# EUROPÄISCHE PATENTSCHRIFT

(11) **EP 2 751 127 B1**
(45) Veröffentlichungstag und Bekanntmachung des Hinweises auf die Patenterteilung: **27.07.2016**
(21) Anmeldenummer: 12751509.6
(22) Anmeldetag: 29.08.2012
(51) Int. Cl.: C07K 7/64, A61K 38/13, A61P 31/12

(54) **CYCLOSPORIN-DERIVATE**
CYCLOSPORIN DERIVATIVES
DÉRIVÉS DE CYCLOSPORINE

(30) Priorität: 30.08.2011 DE 102011111991
(43) Veröffentlichungstag der Anmeldung: 09.07.2014
(73) Patentinhaber: Max-Planck-Gesellschaft zur Förderung der Wissenschaften e.V., 80539 München (DE); Lead Discovery Center GmbH, 44227 Dortmund (DE)
(72) Erfinder: FISCHER, Gunter, 06120 Halle (DE); MALESEVIC, Miroslav, 06108 Halle (DE); ERDMANN, Frank, 06114 Halle (DE); KÜHLING, Jan, 06108 Halle (DE); BUKRINSKY, Michael, Potomac, Maryland 20854 (US); CONSTANT, Stephanie, Herndon, Virginia 20171 (US); RÜHTER, Gerd, 21129 Hamburg (DE); NUSSBAUMER, Peter, 44227 Dortmund (DE); DINKEL, Klaus, 44799 Bochum (DE)
(74) Vertreter: Arth, Hans-Lothar
(86) Internationale Anmeldenummer: PCT/EP2012/066726
(87) Internationale Veröffentlichungsnummer: WO 2013/030208

(56) Entgegenhaltungen:
- GB-A- 2 205 317
- US-A1- 2006 069 016
- US-A1- 2010 209 390

## Beschreibung

Die vorliegende Erfindung betrifft Verbindungen der weiter unten abgebildeten Formel I und Indikator-gekoppelte Verbindungen, die beide Cyclosporinderivate sind. Die erfindungsgemäßen Verbindungen zeigen keine immunosuppressive Wirkung und finden Verwendung in der Medizin, insbesondere in der Behandlung von Erkrankungen wie viralen Infektionen, entzündlichen Erkrankungen, Krebs, degenerativen Muskelerkrankungen, neurodegenerativen Erkrankungen und Schädigungen, welche mit Beeinträchtigung der Calcium-Homöostase einhergehen. Die vorliegende Erfindung betrifft zudem eine pharmazeutische Zusammensetzung enthaltend eine erfindungsgemäße Verbindung und optional verschiedene Zusatzmittel. Desweiteren betrifft die vorliegende Erfindung ein Verfahren zur Anreicherung von Wirkstoffen in einem extrazellulären Raum eines multizellularen Objekts. Ein weiterer Gegenstand der vorliegenden Erfindung ist die Verwendung diverser Cyclosporinderivate zur Herstellung von erfindungsgemäßen Verbindungen.

Biologisch wirksame Moleküle, sogenannte "Wirkstoffe", bei denen es sich in der Regel um pharmazeutische Wirkstoffe handelt, entfalten ihre Wirkung meist sowohl innerhalb wie auch außerhalb von biologischen Zellen. Dabei ist bisher vornehmlich das Problem aufgetreten, dass Wirkstoffe, die ihre Wirkung nur innerhalb der Zelle entfalten sollen, bereits vor Durchtritt durch die Zellmembran unerwünschte Veränderungen im extrazellulären Raum verursachen. Hierbei tritt zusätzlich das Problem auf, dass ein und derselbe Wirkstoff innerhalb und außerhalb der Zelle eine unterschiedliche Wirkung entfalten kann. Die eigentliche Wirkung umfasst somit zwei Komponenten - die erwünschte intrazelluläre und die nicht erwünschte extrazelluläre Wirkung. Um die intrazelluläre Wirkung zu erreichen, musste - da der Transport zur Zelle in der Regel die Durchquerung eines extrazellulären Raumes beinhaltet - notwendigerweise oft auch die extrazelluläre (Neben)Wirkung in Kauf genommen werden.

Ebenso kann auch nur die extrazelluläre Wirkung erwünscht und die intrazelluläre Wirkung unerwünscht sein. Vor allem in der Medizin ist eine Reihe von Wirkstoffen bekannt, die in der Zelle nicht nur nicht die beabsichtigte Wirkung entfalten, sondern sogar toxisch wirken bzw. eine anderweitig schädigende Wirkung verursachen. Hinzu kommt, dass zur Erzielung einer bestimmten, extrazellulären Wirkung eine weit höhere Dosis verabreicht werden muss als eigentlich benötigt, um den "Verlust" der in das Zellinnere abgewanderten Wirkstoffe zu kompensieren.

Wirkstoffe können extrazellulär und/oder intrazellulär auf Moleküle oder Strukturen wirken. Solche biologischen Moleküle können beispielsweise Enzyme, Inhibitoren, Aktivatoren oder Rezeptoren sein. Unter "Strukturen" ist beispielsweise die extrazelluläre Matrix zu verstehen, die aus der Gesamtheit der Makromoleküle, die sich außerhalb der Plasmamembran von Zellen in Geweben und Organen befinden, gebildet ist.

Mehrere Veröffentlichungen zeigen, dass Cyclosporine sowohl intrazelluläre als auch extrazelluläre Wirkungen entfalten können. Cyclosporine können sowohl intrazellulär als auch extrazellulär an Proteine, wie Cyclophiline binden. Diese Proteine sind in der wissenschaftlichen und Patent-Literatur umfassend beschrieben, wie z.B.in WO 2010/012073. Cyclosporine und ihre Derivate bewirken eine Vielzahl von Effekten, die therapeutisch genutzt werden können. So können sie beispielsweise einen Einfluss auf Neuroprotektion/Neurogeneration, Chaperon-Aktivität, HIV-Infektion, Krebs oder Alzheimer haben. Ein Beispiel für die erwünschte Wirkung dieser Verbindungen ist die PPIase-Inhibition. Die PPIase(Peptidyl-Prolyl-Isomerase)-Inhibitoren können zwar zwischen den einzelnen PPIase-Familien (Nature Chemical Biology. 3(10):619-29, 2007; Cellular & Molecular Life Sciences. 63(24):2889-900, 2006; Current Topics in Medicinal Chemistry. 3(12):1315-47, 2003; Advances in Protein Chemistry. 59:243-82, 2001) unterscheiden, haben aber oft ähnliche Hemmkraft gegenüber Sequenz-ähnlichen Familienmitgliedern. Da PPIasen innerhalb einer Familie unterschiedlichste biochemische Reaktionen beeinflussen können, hängt die diagnostische oder pharmakologische Wirkung verabreichter Wirkstoffe unmittelbar von der erreichten Konzentration in unterschiedlichsten Verteilungsräumen ab. So sind z.B. einige dieser PPIase-Inhibitoren (z.B. Biopolymers 84(2006)125-146; Chemical & Pharmaceutical Bulletin. 54(3):372-376, 2006; Chemistry & Biology. 10(1):15-24, 2003; Nucleic Acids Research. 29(3):767-773, 2001), wie z.B. das therapeutisch verwendete Cyclosporin A, in Wasser nur schwer löslich. (DE 19859910).

Das ursprünglich aus dem Pilz *Tolypocladium inflatum* isolierte Cyclosporin A wird beispielsweise in medizinischen Anwendungen zur Unterdrückung von Immunreaktionen verwendet.

Cyclosporine sind im Sinne dieser Erfindung zyklische Peptide aus 11 Aminosäuren (Undecapeptide). Darunter sollen alle Cyclosporine A bis I sowie deren Derivate wie in Helvetica Chimica Acta 65 (1982), 1655-1677 beschrieben und alle Cyclosporine K bis Z sowie deren Derivate wie in Helvetica Chimica Acta 70 (1987), 13-36 beschrieben verstanden werden. Die Cyclosporine können erfindungsgemäß auch - wie in der WO 99/18120 beschrieben - deuteriert sein.

Cyclosporin A (auch Ciclosporin) ist ein zyklisches Oligopeptid, welches intrazellulär eine Calcineurin-hemmende Wirkung entfaltet. Ein so wirkendes Cyclosporin zeichnet sich durch einen selektiven und reversiblen Mechanismus der Immunosuppression aus. Es blockiert selektiv die Aktivierung von T-Lymphozyten über die Produktion von bestimmten Zytokinen, die an der Regulierung dieser T-Zellen beteiligt sind. Dabei wird vor allem die Synthese von Interleukin-2 gehemmt, wodurch gleichzeitig die Proliferation von zytotoxischen T-Lymphozyten, die z. B. für die Abstoßung von fremdem Gewebe verantwortlich sind, supprimiert wird. Das Cyclosporin wirkt intrazellulär durch Bindung an die so genannten Cyclophiline oder Immunophiline, die zur Familie der Cyclosporin-bindenden Proteine gehören. Es ist erfindungsgemäß bevorzugt, dass die erfindungsgemäßen Verbindungen diese intrazelluläre Wirkung nicht aufweisen.

Inhibitoren von Cyclophilinen weisen ein sehr großes therapeutisches Spektrum auf, wie z.B. die Behandlung von Erkrankungen des Atmungstraktes, wie z.B. Asthma, chronisch obstruktive Lungenerkrankung (COPD), Lungenentzündung oder Emphysem (Expert Opinion on Investigational Drugs 12(2003)647-653, Biodrugs 8(1997) 205-215, American Journal of Respiratory Cell & Molecular Biology 20(1999)481-492), Stoffwechselerkrankungen wie Diabetes (Transplantation Proceedings 37(2005)1857-1860, Molecular Pharmacology 60(2001)873-879), entzündliche Erkrankungen des Verdauungstraktes (Bone Marrow Transplantation 26(2000):545-551, Pharmaceutical Research 20(2003)910-917), Störungen des Immunsystems (Immunology Letters 84(2002)137-143, Acta Biochimica Polonica 49(2002)233-247, Entzündungen (Journal of Periodontal Research 42(2007)580-588, Journal of Neurology, Neurosurgery & Psychiatry 76(2005)1115-1120, Transplant Immunology 12(2004):151-157), kardiovaskuläre Erkrankungen (Journal of Hypertension 17(1999)1707-1713, Drug & Chemical Toxicology 21(1998)27-34), neurologische Erkrankungen (Annals of Vascular Surgery. 20(2006) 243-249), Erkrankungen welche mit Störung der Angiogenese einhergehen (Blood Purification. 25(2007)466-472, International Angiology 24(2005)372-379, Nefrologia. 23(2003):44-48), zur Unterdrückung der Immunantwort bei Organtransplantation (Bone Marrow Transplantation. 38(2006)169-174), Biodrugs. 14(2000)185-193, Clinical Immunotherapeutics. 5(1996)351-373) und von Autoimmunerkrankungen (Immunology & Immunopathology. 82(3):197-202, 1997), Erkrankungen des arthritischen Formenkreises (British Journal of Rheumatology. 36(1997)808-811, Biodrugs. 7(1997)376-385), Dermatididen (Veterinary Dermatology 17(2006)3-16), Psoriasis (Journal of Dermatological Treatment 16(2005)258-277, Hautarzt 44(1993)353-360), bei Allergien (Cornea 27(2008)625, Journal of Small Animal Practice 47(2006)434-438, Clinical & Experimental Ophthalmology 34(2006)347-353), bei multipler Sklerose (Immunopharmacology & Immunotoxicology 21(1999)527-549, Journal of Neuroimaging 7(1997)1-7), Erkrankungen die durch Ischemie verursacht wurden, wie z.B. Infarkte des Herzens (Annals of Thoracic Surgery 86(2008)1286-1292, Acta Anaesthesiologica Scandinavica 51(2007)÷909-913), des Pankreas (Pancreas 32(2006)145-151) oder des Hirns (Annals of Vascular Surgery 20(2006)243-249, Neurological Research 27(2005)827-834), Nierenerkrankungen wie z.B. Glomerulonephritis (Nephrology Dialysis Transplantation 19(2004)3207, Nephron 91(2002)509-511), Geschwulste (Journal of Investigative Dermatology 128(2008)2467-2473, Endocrinology 148(2007)4716-4726), bei multiplen Myelomen (Leukemia 12(1998)505-509, Leukemia & Lymphoma 16(1994)167-170), bei akuter oder chronischer Leukemie (Cancer Chemotherapy & Pharmacology 52(2003)449-452, Cancer 97(2003)1481-1487), Muskel-Degeneration (Neuroscience Research Communications 31(2002)85-92, Kachexie (International Journal of Cardiology 85(2002)173-183, Drugs 58(1999)953-963, 1999), Reiter's Syndrome (Rheumatology 40(2001)945-947), Knochenabbauerkrankungen (European Journal of Pharmacology 564(2007)226-231, Biochemical & Biophysical Research Communications 254(1999)248-252), bei der Alzheimer'schen Erkrankung (Biochemical & Biophysical Research Communications 248(1998)168-173, Chinese Medical Journal 115(2002)884-887), Malaria (Molecular & Biochemical Parasitology 99(1999)167-181), dem septischen und toxischen Schock-Syndrom (Journal of Pharmacology & Experimental Therapeutics 311(2004)1256-1263), Myalgie (British Journal of Dermatology 147(2002)606-607), bei der Infektion mit Viren (Expert Opinion on Emerging Drugs 13(2008)393-416) wie z.B. HIV-1, HIV-2, HIV-3 (Journal of Infectious Diseases 194(2006)1677-1685, Molecular Medicine Today 1(1995)287-291, 1995), Cytomegaloviren (Journal of Virology 81(2007)9013-9023) oder Adenoviren (Ophthalmologe 105(2008)592-594, Ophthalmologe 97(2000)764-768) und zur Förderung des Haarwachstums (Archives of Dermatological Research 296(6):265-269, 2004, Annales de Dermatologie et de Venereologie 127(2000)769). Aufgrund der therapeutischen Anwendungsbreite stellen sie in der Medizin eine wichtige Substanzklasse dar.

Sollen Cyclosporine oder deren Derivate für die Behandlung der genannten Krankheiten eingesetzt werden, so ist ein Nachteil vieler bisher bekannter Cyclosporinderivate, dass sie neben der erwünschten therapeutischen Wirkung auch immunsuppressiv wirken. Die immunsuppressive Wirkung ist in diesen Fällen jedoch eine unerwünschte Nebenwirkung, die es zu unterdrücken gilt. Es ist bekannt, dass Cyclosporine und ihre Derivate in der Lage sind Cyclophiline zu inhibieren. Dringen die Cyclosporinverbindungen jedoch in eine Zelle ein, so können die Komplexe aus Cyclosporinverbindung und Cyclophilin die Serin-Threonin-Phosphatase Calcineurin inhibieren. Die Inhibition von Calcineurin führt dann zu der unerwünschten Nebenwirkung der Immunsuppression. Es existieren jedoch auch Cyclophiline im extrazellulären Raum, deren Inhibition mit Cyclosporinverbindungen zu erwünschten therapeutischen Wirkungen führt. Ist es also möglich, Cyclosporinverbindungen bereitzustellen, die sowohl hinsichtlich des erwünschten therapeutischen Zwecks Wirkung zeigen, als auch nicht in der Lage sind in Zellen einzudringen, dann könnten die unerwünschten Nebenwirkungen vermieden werden.

Es war somit die Aufgabe der vorliegenden Erfindung, neue Cyclosporinderivate bereitzustellen, die die erwünschten therapeutischen Wirkungen aufweisen, ohne dass es zu der Nebenwirkung der Immunsuppression kommt. Insbesondere war es die Aufgabe der vorliegenden Erfindung Wirkstoffe bereitzustellen, die therapeutisch wirksam sind, jedoch nicht in das Zellinnere eindringen können.

Insbesondere war es eine Aufgabe der vorliegenden Erfindung Verbindungen bereitzustellen, die gegen folgende Erkrankungen therapeutisch helfen, ohne dabei immunsuppressiv zu wirken:
a) virale Infektionen
b) akute und chronische entzündliche Erkrankungen
c) Krebs
d) degenerative Muskelerkrankungen
e) neurodegenerative Erkrankungen, und
f) Schädigungen, welche mit Beeinträchtigung der Calcium-Homöostase einhergehen,
wobei die virale Infektion durch Viren wie HIV, Hepatitis A, Hepatitis B, Hepatitis C, Hepatitis D und Hepatitis E ausgelöst werden kann,
wobei die entzündliche Erkrankung vorzugsweise Asthma, chronische Entzündungen, chronische Prostatitis, Glomerulonephritis, multiple chemische Empfindlichkeit, entzündliche Darmerkrankungen, Sepsis, Entzündungen der vaskulären glatten Muskelzellen, Aneurysma, Entzündungen im Beckenbereich, Reperfusionsschäden, rheumatoide Arthritis, Vasculitis, Psoriasis und ulzerative Kolitis einschliesst,
wobei die Krebserkrankung vorzugsweise Lungenkrebs, Blasenkrebs, Leberkrebs, Pankreaskrebs und Brustkrebs umfaßt,
wobei die degenerative Muskelerkrankung vorzugsweise Muskeldystrophy, Kollagen IV-Myopathien und den myokardialen Reperfusionsschaden betrifft,
wobei die neurodegenerative Erkrankung vorzugsweise ausgewählt ist aus der Alzheimer Erkrankung, Parkinson, Huntington, multiple systemische Atrophie, multiple Sklerose, zerebrale Kinderlähmung, Schlaganfall, diabetische Neuropathie, amyotrophe Lateral-Sklerose, Rückenmarkschäden und Zerebralsklerose,
wobei die Erkrankung, die mit einem Verlust der zellulären Calcium-Homöostase einhergeht, vorzugsweise Herzinfarkt, Schlaganfall, akute Hepatotoxizität, Cholestase und Reperfusionsschäden von transplantierten Organen betrifft, und
Überraschenderweise wurde gefunden, dass die Derivatisierung der Aminosäure 1 von Cyclosporinen mit geeigneten Imidazol-, Oxazol- oder Thiazolderivaten deren Eigenschaften so verändern kann, dass die so veränderten Cyclosporine eine veränderte Zellpermeabilität aufweisen. Insbesondere die Einführung von chemischen Gruppierungen in die oben beschriebenen erfindungsgemäßen Imidazol-, Oxazol- oder Thiazolderivate, wie z.B. einer Säuregruppierung, kann die Eigenschaften der Cyclosporine so verändern, dass sie sich im extrazellulären Raum anreichern und nicht in die Zellen eindringen.

Eine wesentliche Eigenschaft, welche durch die Derivatisierung von Cyclosporinen mit geeigneten Imidazol-, Oxazol- oder Thiazolderivaten erhalten wird, ist die Fähigkeit der so erhaltenen neuen Cyclosporin-Derivate die Peptidyl-Prolyl-cis/trans-Isomeraseaktivität von humanen Cyclophilinen weiterhin zu hemmen, vorzugsweise mit einem IC₅₀-Wert von < 100 nMol, ohne dabei die Serin-Threonin-Phosphatase Calcineurin zu hemmen und ohne dabei in die Zellen einzudringen.

Die oben genannte Aufgabe wird durch die Bereitstellung von Verbindungen der folgenden Formel I gelöst: 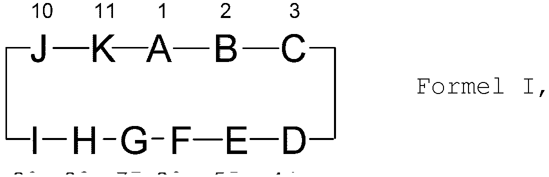 worin
A eine Aminosäure der folgenden Formel II ist, 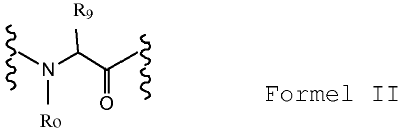 wobei die Bindungen, die an den gewellten Linien enden, Verknüpfungen zu den Einheiten B und K darstellen und R₉ eine Gruppe der folgenden Formel III ist, und 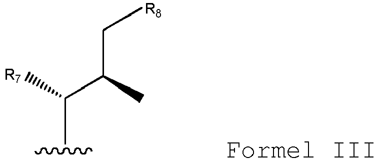 bei der R₈ einer Gruppe der folgenden Formel IV entspricht, oder 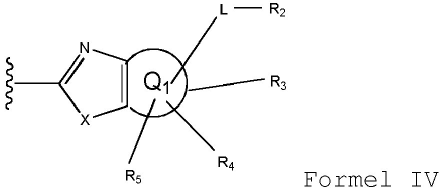 bei der R₈ einer Gruppe der folgenden Formel V entspricht, oder 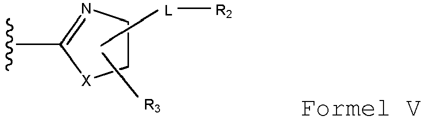 bei der R₈ einer Gruppe der folgenden Formel VI entspricht, oder 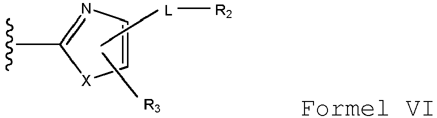 bei der R₈ einer Gruppe der folgenden Formel VII entspricht, oder 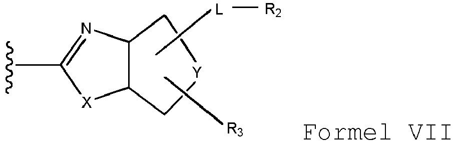 bei der R₈ einer Gruppe der folgenden Formel VIII entspricht, in der die Reste L-R₂ und R₃ an den gleichen oder verschiedenen Phenylringen gebunden sein können, oder 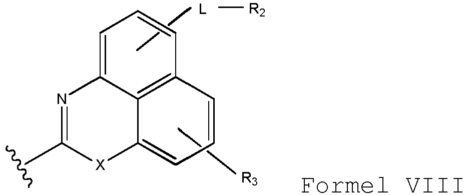 bei der R₈ einer Gruppe der folgenden Formel IX entspricht, und in der die Reste L-R₂ und R₃ an den gleichen oder an verschiedenen Ringen Q₂ oder Q₃ gebunden sein können, oder 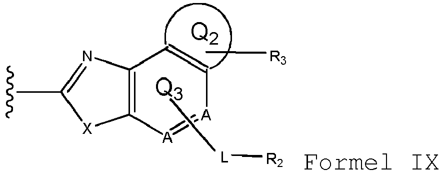 bei der R₈ einer Gruppe der folgenden Formel X entspricht, in der die Reste L-R₂ und R₃ an den gleichen oder an verschiedenen Ringen Q₂ oder Q₃ gebunden sein können, oder 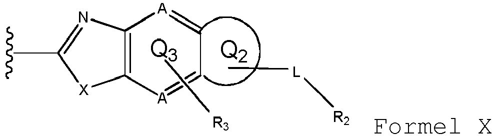 bei der R₈ einer Gruppe der folgenden Formel XI entspricht, in der die Reste L-R₂ und R₃ an den gleichen oder an verschiedenen Ringen Q₂ oder Q₃ gebunden sein können, oder 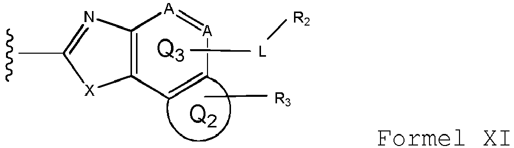 bei der R₈ einer Gruppe der folgenden Formel XII entspricht, in der die Reste L-R₂ und R₃ an den gleichen oder verschiedenen Phenylringen gebunden sein können, oder 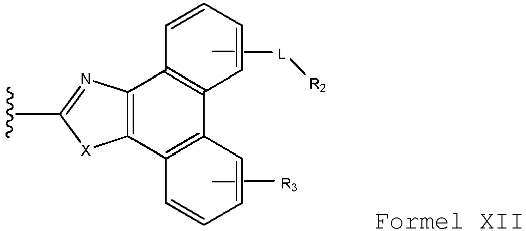 bei der R₈ einer Gruppe der folgenden Formel XIII entspricht, 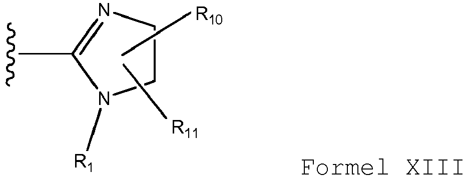 wobei
   die Bindungen in den Formeln IV bis XIII, die an den gewellten Linien enden, an die CH₂-Gruppe der Formel III binden, an der R₈ verknüpft ist;
   wobei
   die Gruppe der Formel II über ihr CO kovalent zu der alpha-Aminosäure B in Formel I unter Ausbildung einer Amidbindung gebunden ist und N-Rₒ in der Formel II kovalent zu einer Carboxylgruppe von K unter Ausbildung einer Amidbindung gebunden ist, wobei
B eine Aminosäure ist, die aus der folgenden Gruppe ausgewählt ist:
   alpha-Aminobuttersäure;
   Alanin;
   Threonin;
   Valin;
   Norvalin; und
   in der Seitenkette mit einer Hydroxylgruppe modifizierte Moleküle von alpha-Aminobuttersäure, Alanin, Threonin, Valin oder Norvalin;
C für Sarkosin steht;
D eine Aminosäure ist, die aus der folgenden Gruppe ausgewählt ist:
   Leucin;
   N-Methylleucin;
   Valin;
   Gamma-Hydroxy-N-Methylleucin; und
   Gamma-Hydroxyleucin;
E eine Aminosäure ist, die aus der folgenden Gruppe ausgewählt ist:
   Valin;
   Norvalin; und
   einem modifizierten Valin oder Norvalin, bei dem ein Kohlenstoffatom der Seitenkette mit einer Hydroxylgruppe substituiert ist;
F eine Aminosäure ist, die aus der folgenden Gruppe ausgewählt ist:
   Leucin;
   N-Methylleucin;
   Gamma-Hydroxy-N-Methylleucin; und
   Gamma-Hydroxyleucin;
G gleich alpha-Aminobuttersäure oder Alanin ist;
H gleich D-Alanin oder D-Serin ist;
I und J Aminosäuren sind, die unabhängig voneinander aus der folgenden Gruppe ausgewählt sind:
   Leucin;
   N-Methylleucin;
   Gamma-Hydroxy-N-Methylleucin; und
   Gamma-Hydroxyleucin;
K gleich N-Methylvalin oder Valin ist;
   wobei
   die Aminosäuren B bis K unter Ausbildung von Amidbindungen miteinander verknüpft sind;
   wobei
   die verwendeten Symbole und Indices die folgenden Bedeutungen aufweisen:
   - X: steht für O, S, oder N-R₁;
   - Y: steht für O, S, N-R₁, -CH₂- oder -CH₂CH₂-;
   - A: steht für CH oder N;
   - L: steht für eine Bindung, -CH₂-, -CH₂CH₂-, -CH₂CH₂CH₂-,-CH₂CH₂CH₂CH₂-, -CH=CH-, -CH=CHCH₂-, -CH₂CH=CH-,-CH₂CH₂CH=CH-, -CH₂CH=CHCH₂-, -CH=CHCH₂CH₂-, -OCH₂-, -OCH₂CH₂-, -OCH₂CH₂CH₂-, -OCH₂CH=CH-, -CONH-, -CONHCH₂-,-CONHCH₂CH₂-, -CONHCH₂CH₂OCH₂CH₂-, -CONHCH₂CH₂OCH₂CH₂OCH₂CH₂-;
   - Q₁: steht zusammen mit den beiden Atomen des ankondensierten Rings für einen aromatischen oder heteroaromatischen Ring aus der Gruppe Benzol, Pyridin, Pyrimidin, Pyridazin, Pyrazin, Triazin, Thiophen, Furan, Pyrrol, Thiazol, Isothiazol, Oxazol, Isoxazol, Imidazol, Pyrazol;
   - Q₂: steht zusammen mit den beiden Atomen des ankondensierten Rings für einen aromatischen oder heteroaromatischen Ring aus der Gruppe Benzol, Pyridin, Pyrimidin, Pyridazin, Pyrazin, Triazin, Thiophen, Furan, Pyrrol, Thiazol, Isothiazol, Oxazol, Isoxazol, Imidazol, Pyrazol, Thiadiazol, Oxadiazol, Triazol;
   - Q₃: bezeichnet jeweils den Sechsring in dessen Mitte es steht;
   - Rₒ: steht für H oder CH₃;
   - R₁: steht für H, (C1-C4)-Alkyl oder Benzyl, die auch durch eine Gruppe -COOH substituiert sein können;
   - R₂: steht für eine polare deprotonierbare Gruppe Pₛ, oder eine Gruppe Pₛ`, die unter physiologischen Bedingungen in Pₛ umgewandelt werden kann, oder eine polare protonierbare Gruppe P_{b}, oder eine Gruppe P_{b}', die unter physiologischen Bedingungen in P_{b} umgewandelt werden kann;
   - R₃: steht für H, (C1-C6)-Alkyl, (C1-C6)-Alkoxy, -OH, (C1-C6)-Alkylthio, (C1-C6)-Alkylsulfonyl, -SH, -CF₃, -COOH, -COO((C₁-C6)Alkyl)), -CONH₂, -CONH((C1-C6)Alkyl) ,-CON((C1-C6)Alkyl)₂, Nitro, Halogen, Cyano, Amino, (C1-C6)Alkyl-amino, (C1-C6)Dialkyl-amino; R₃ kann auch in Form eines freien Elektronenpaares vorliegen, insbesondere dann, wenn es an ein Heteroatom des Ringes Q₁, Q₂ oder Q₃ bindet/binden würde;

   - R₄ und R₅: stehen unabhängig voneinander für H, (C1-C6)-Alkyl, (C1-C6)-Alkoxy, -CF₃, Halogen oder können zusammen eine Gruppe -OCH₂O- oder -OCH₂CH₂O- bilden, wenn R₄ und R₅ in Positionen ortho zueinander am Ring gebunden sind; R₄ und R₅ können auch unabhängig voneinander in Form eines freien Elektronenpaares vorliegen, insbesondere dann, wenn R₄ oder R₅ an ein Heteroatom des Ringes Q₁ bindet/binden würde;
   - R₇: steht für H, -OH, -OCOOR₁₂, -OCOR₁₃, -OCONR₁₄R₁₅, -O-(tetrahydropyran-2-yl), -O-(tetrahydrofuran-2-yl), -O-CHR₁₆-OR₁₇, -SiR₁₈R₁₉R₂₀:

   - R₁₀ und R₁₁: stehen unabhängig voneinander für H, (C1-C6)-Alkyl, -CF₃, Halogen oder können zusammen eine Gruppe -CH₂CH₂-, -CH₂CH₂CH₂-, -CH₂CH₂CH₂CH₂- oder-CH₂CH₂CH₂CH₂CH₂- bilden;

   - R₁₂: steht für (C1-C6)-Alkyl, Benzyl oder Phenyl, wobei die Alkylgruppe gegebenenfalls durch Fluor oder Chlor substituiert und Benzyl und Phenyl gegebenenfalls durch einen oder mehrere Substituenten aus der Gruppe (C1-C6)-Alkyl, (C1-C6)-Alkoxy, -CF₃, Cyano, Nitro oder Halogen substituiert sein kann;
   - R₁₃: steht für H oder R₁₂;

   - R₁₄ und R₁₅: stehen unabhängig voneinander für H, (C1-C6)-Alkyl, Benzyl oder Phenyl, wobei Benzyl und Phenyl gegebenenfalls durch einen oder mehrere Substituenten aus der Gruppe (C1-C6)-Alkyl, (C1-C6)-Alkoxy, -CF₃, Cyano, Nitro oder Halogen substituiert sein können;

   - R₁₆: steht für H oder (C1-C6)-Alkyl;
   - R₁₇: steht für (C1-C6)-Alkyl, Benzyl oder Phenyl; und

   - R₁₈, R₁₉ und R₂₀: stehen unabhängig voneinander für (C1-C6)-Alkyl, Benzyl oder Phenyl.

Die Erfindung beinhaltet auch alle pharmazeutisch akzeptablen Salze, sowie tautomere, enantiomere und andere stereoisomere Formen von Verbindungen der Formel I.

Wenn Namen von Aminosäuren ohne die Nomenklatur-Zusätze D- und L- nach Fischer verwendet werden, so sind erfindungsgemäß beide Varianten denkbar, vorzugsweise ist es jedoch das in der Natur natürlich vorkommende Isomer (meist L-Isomer). Wird ein entsprechender Nomenklatur-Zusatz verwendet, so ist das entsprechende Isomer erfindungsgemäß bevorzugt.

Die Gruppen Pₛ und P_{b} sind entweder ionische Gruppen, d.h. anionische Gruppen Pₛ oder kationische Gruppen P_{b}, oder Gruppen, die in Lösung leicht in ionische Gruppen Pₛ oder P_{b} übergehen. Vorzugsweise liegen diese Gruppen im Bereich von pH6 bis pH8 ganz oder teilweise in ionischer Form vor. Gruppen Pₛ können aber auch polare Gruppen sein, die ein Wasserstoffatom tragen, welches mit stärkeren Basen abgespalten werden kann, wobei dieses Wasserstoffatom vorzugsweise an ein Heteroatom gebunden ist.

Beispiele von sauren Gruppen Pₛ sind die folgenden:
a) -COOH, -SO₃H, -CONH₂, -CONHNH₂, -SO₂NH₂, -PO₃H₂, - PO(OH)(NH₂), -CO(NHOH), -CO(NH(O-C1-C4-Alkyl)), -CSNH₂, - NHCONH₂, -N(OH)CONH₂, -NHCO(NHOH)-, -NHCSNH₂, -CSNHNH₂;
b) 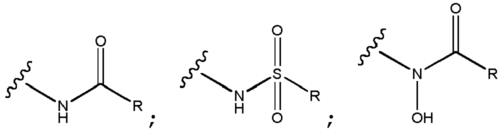 und R = -(C1-C4-Alkyl), -O(C1-C4-Alkyl), -NH(C1-C4-Alkyl), -NH(C1-C4-Alkenyl), (substituiertes) Aryl, (substituiertes) O-Aryl, (substituiertes) NH-Aryl, -CF₃ und andere fluorierte (C1-C4-Alkyl)gruppen;
c) 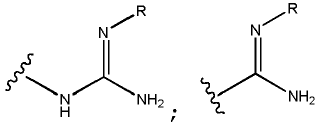 und R = -OH, -CN, -NO₂;
d) 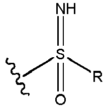 und R = (C1-C4-Alkyl), (substituiertes) Aryl, -CF₃ und andere fluorierte (C1-C4-Alkyl)gruppen;
e) 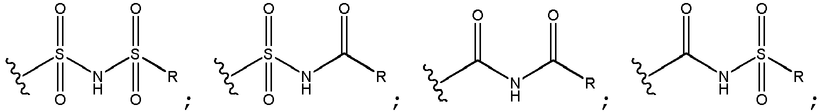 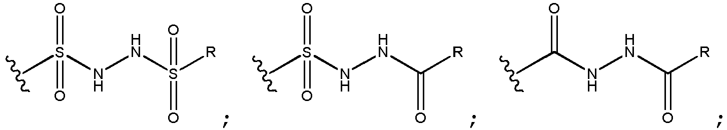 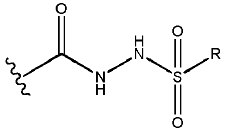 und R = - (C1-C4-Alkyl) , -O(C1-C4-Alkyl) , - NH(C1-C4-Alkyl), -NH(C1-C4-Alkenyl), (substituiertes) Aryl, (substituiertes) O-Aryl, (substituiertes) NH-Aryl, -CF₃ und andere fluorierte (C1-C4-Alkyl)gruppen;
f) 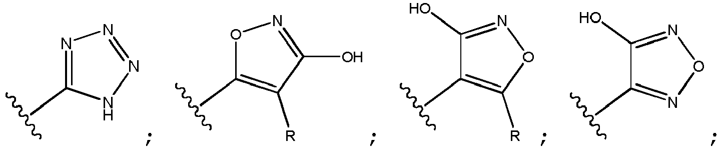 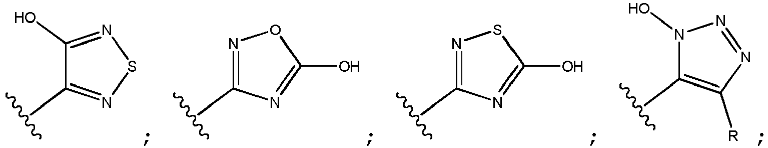 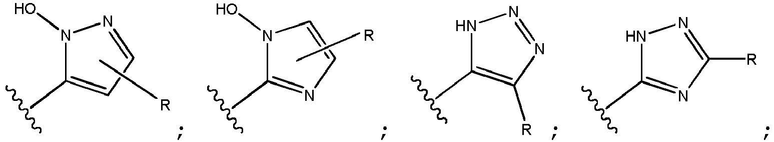 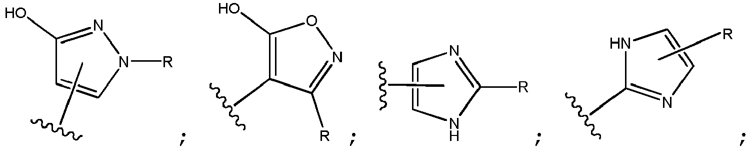 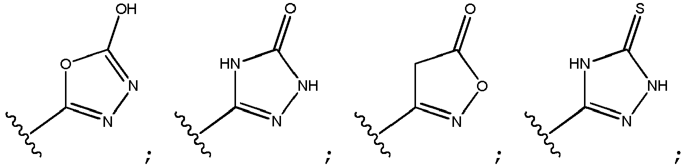 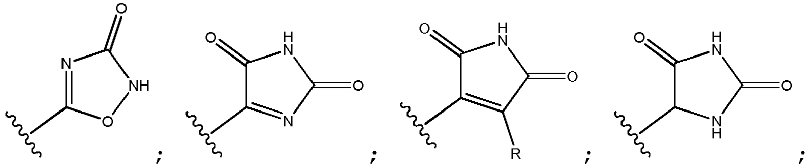 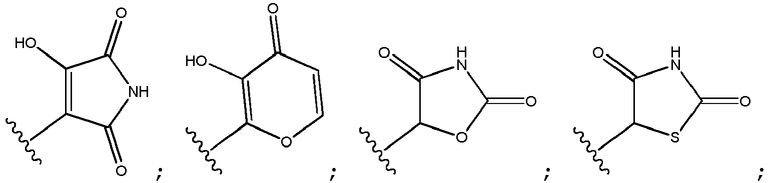 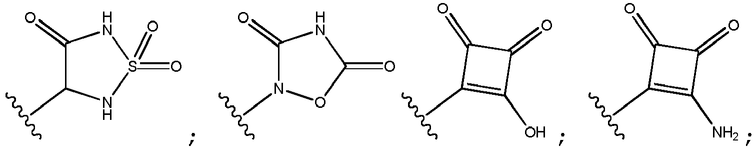 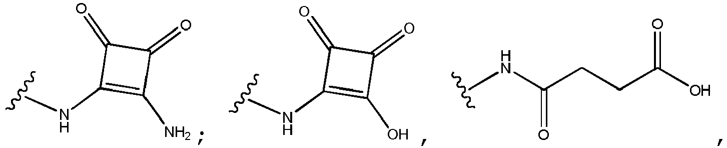 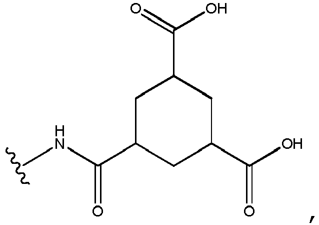 in denen und R = H, -(C1-C4-Alkyl), -CF₃ und andere fluorierte (C1-C4-Alkyl)gruppen.
g) -OH und -SH, mit den Maßgaben, dass L eine kovalente Einfachbindung darstellt, und dass-OH oder -SH nur als Substituent an einem Ring Q₁, Q₂ oder Q₃ auftreten kann und dabei an ein Kohlenstoffatom gebunden ist, das in Nachbarschaft zu einem Stickstoffatom steht.

Es ist erfindungsgemäß besonders bevorzugt, dass die Gruppe Pₛ eine der folgenden Gruppen ist, welche vorzugsweise direkt über eine kovalente Bindung an den Heterocyclus der Formeln IV - XII gebunden ist:
-COOH, -OH, -SH, -CONH₂, -CONHNH₂, -SO₃H, -SO₂NH₂, und die Gruppe 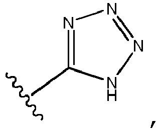 mit den Maßgaben, dass, wenn Pₛ gleich -OH oder -SH ist, L eine kovalente Einfachbindung darstellt, und - OH oder -SH an einem Ring Q₁, Q₂ oder Q₃ an ein Kohlenstoffatom gebunden ist, das in Nachbarschaft zu einem Stickstoffatom steht.

Beispiele von basischen Gruppen P_{b} sind die folgenden:
a) 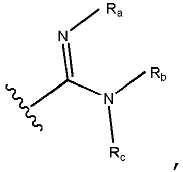 in der Rₐ, R_{b} und R_{c} für H und - (C1-C4-Alkyl) stehen, Rₐ und R_{b} zusammen - (CH₂CH₂) - oder - (CH₂CH₂CH₂) - und R_{b} und R_{c} zusammen mit dem Stickstoffatom Pyrrolidin, Piperidin oder Morpholin sein können;
b) 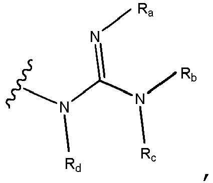 in der Rₐ, R_{b}, R_{c} und R_{d} für H und - (C1-C4-Alkyl) stehen, Rₐ und R_{b}, Rₐ und R_{d} sowie R_{b}, und R_{d} zusammen - (CH₂CH₂) - oder - (CH₂CH₂CH₂) - und R_{b} und R_{c} zusammen mit dem Stickstoffatom Pyrrolidin, Piperidin oder Morpholin sein können;
c) eine Aminogruppe ausgewählt aus -NH₂, (C1-C6)Alkyl-amino, (C1-C6)Dialkyl-amino, Pyrrolidin, Piperidin, Morpholin oder Piperazin, welches in Position 4 durch (C1-C6)Alkyl, -(C1-C6)Alkanoyl, Benzyl, Benzoyl oder Phenyl substituiert sein kann, wobei diese Substituenten optional eine Gruppe -COOH tragen können.

Es ist erfindungsgemäß besonders bevorzugt, dass die Gruppe P_{b} eine der folgenden Gruppen ist, welche direkt über eine kovalente Bindung an den Heterocyclus der Formeln IV - XII gebunden ist:

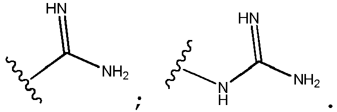

Die Gruppen Pₛ oder P_{b} können auch eine Gruppe 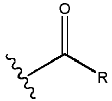 sein, in der R eine Aminosäure oder ein Aminosäureamid ist, die über ihre Aminogruppe gebunden sind, oder ein Peptid, welches aus 2 - 10 Aminosäuren besteht und in dem die endständige Aminosäure auch ein Aminosäureamid sein kann. Bevorzugte Peptidgruppen enthalten mindestens eine Aminosäure aus der Gruppe D-Glutaminsäure, L-Glutaminsäure, D-Asparaginsäure, L-Asparaginsäure, D-Glutamin, L-Glutamin, D-Asparagin, L-Asparagin, D-Cysteinsulfonsäure, L-Cysteinsulfonsäure, D-Arginin oder L-Arginin. Bevorzugt sind Peptidgruppen der Formel

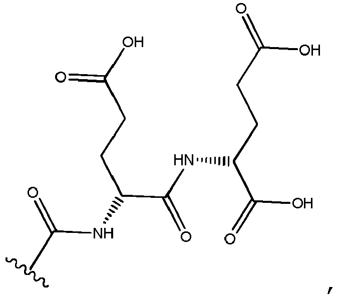

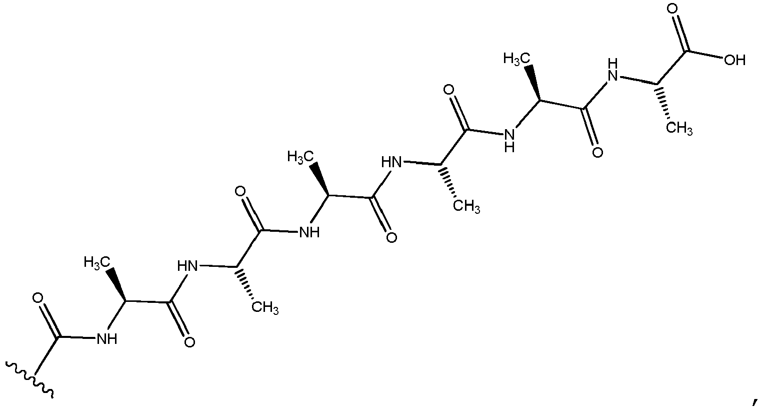

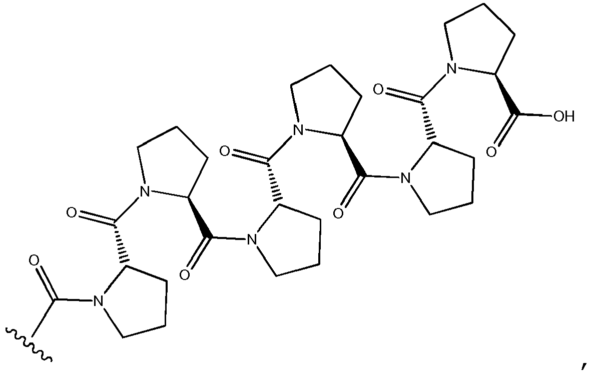

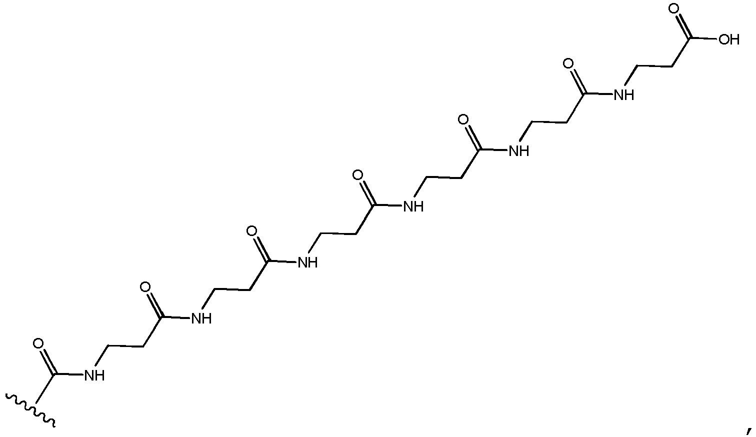

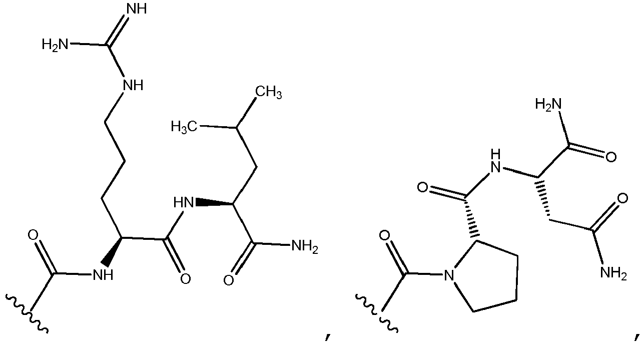

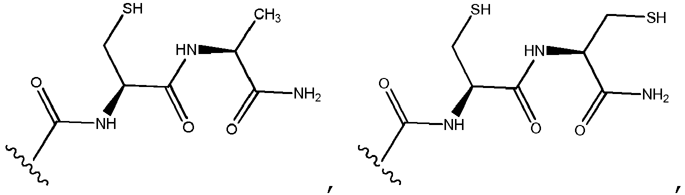

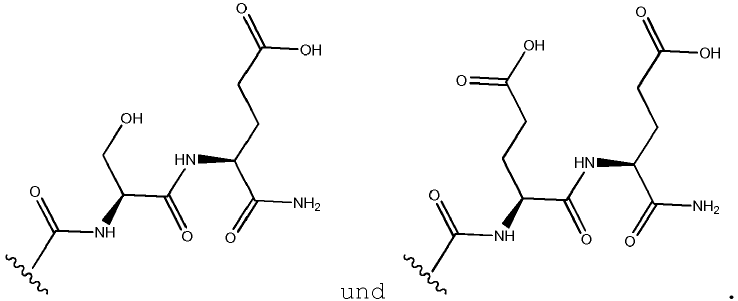

Unter einer Aminosäure wird in der vorliegenden Anmeldung jegliche organische Verbindung verstanden, die eine Aminogruppe und eine Carbonsäuregruppe oder Sulfonsäuregruppe enthält. Bevorzugt sind in der vorliegenden Anmeldung jedoch die natürlich vorkommenden Aminosäuren.

Die Gruppen Pₛ' und P_{b}' sind Gruppen, die unter physiologischen Bedingungen in Gruppen Pₛ bzw. P_{b} umgewandelt werden können. Verbindungen, die Gruppen P_{s'} oder P_{b}' enthalten, werden als "Prodrugs" bezeichnet. Solche Verbindungen müssen nicht zwangsläufig nicht-zellgängig sein, aber sie wandeln sich idealerweise nach Applikation in nicht-zellgängige Derivate um, vorzugsweise nach oraler Applikation, so dass sie dann am Wirkort nicht in die Zelle penetrieren können.

Beispielsweise können Carbonsäueester als Prodrugs für Carbonsäuren (Pₛ = -COOH) verwendet werden (K. Beaumont, et al., Current Drug Metabolism 4 (2003), 461-485). Beispiele von Carbonsäureestern P_{s'} = -COOR enthalten die Reste R
a) (C1-C6-Alkyl), (C2-C6-Alkenyl), Phenyl oder Benzyl, in denen der aromatische Ring weitere Substituenten tragen kann, vorzugsweise -CH₃, -CH₂CH₃, -CH₂CH₂CH₃, -CH(CH₃)₂, - CH₂CH₂CH₂CH₃, -CH(CH₃)CH₂CH₃, -CH₂CH=CH₂, Benzyl;
b) (C1-C6-Alkyl), in welchem Wasserstoffatome durch Halogen ersetzt sind, vorzugsweise -CF₃, -CH₂CCl₃;
c) (C1-C6-Alkyl), in welchem ein Wasserstoffatom durch -OH, (C1-C6-Alkoxy) oder eine substituierte Aminogruppe ersetzt ist, vorzugsweise -CH₂OCH₃, -CH₂CH₂OCH₃, -CH₂CH₂OH, -CH₂CH₂N (CH₃)₂, -CH₂CH₂ (morpholin-4-yl) ;
d) 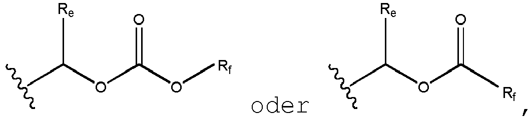 in denen Rₑ für H oder (C1-C4-Alkyl) und R_{f} für (C1-C4-Alkyl) oder (C3-C6-Cycloalkyl) steht, vorzugsweise Rₑ = H, -CH₃, -CH₂CH₃,-CH (CH₃)₂ und R_{f} = -CH₃, -CH₂CH₃, -CH(CH₃)₂, -C(CH₃)₃, Cyclohexyl;
e)

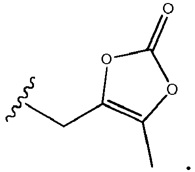

Es ist besonders bevorzugt, dass die Gruppe Pₛ' eine der folgenden Gruppen ist: -COOCH₃, -COOCH₂CH₃, -COOCH₂CH₂CH₃, -COOCH₂CH₂CH₂CH₃, -COOCH₂CH(CH₂CH₃)₂, -COOCH(CH₃)₂, -COOC(CH₃)₃, -COOCH₂CH₂N (CH₃)₂ oder -COOCH₂CH₂ (morpholin-4-yl) .

Als Prodrugs von basischen Gruppen P_{b} wie Amidine und Guanidine können ihre acylierten Derivate P_{b}' = 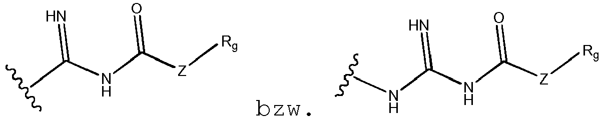 verwendet werden, in denen Z = O, S, NH oder NCH₃ und R_{g} = (C1-C4-Alkyl), (C3-C6-Cycloalkyl) oder Benzyl ist, vorzugweise Z = O und R_{g} = -CH₃, - CH₂CH₃, -CH₂CH₂CH₃, -CH(CH₃)₂, Benzyl.

Es ist insbesondere bevorzugt, dass R₂ aus der Gruppe von Resten ausgewählt ist, die aus folgendem besteht:
-COOH, -OH, -SH, -CONH₂, -CONHNH₂, -SO₃H, -SO₂NH₂, -COOCH₃, - COOCH₂CH₃, -COOCH₂CH₂CH₃, -COOCH₂CH₂CH₂CH₃, -COOCH₂CH(CH₂CH₃)₂, -COOCH(CH₃)₂, -COOC(CH₃)₃, -COOCH₂CH₂N(CH₃)₂ oder - COOCH₂CH₂(morpholin-4-yl), die Gruppen

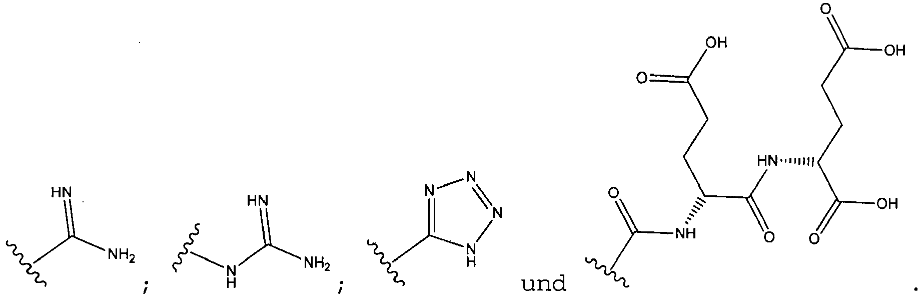

Weiterhin bevorzugt ist in der erfindungsgemäßen Verbindung nach Formel I der Rest R₀ gleich -CH₃.

Die Gruppe R₇ ist bevorzugt aus der Gruppe ausgewählt, die aus -OH, -OCOCH₃ , -OCOOCH₃ , -OCH₂OCH₃, -Si(CH₃)₃ und -Si(CH₃)₂C(CH₃)₃ besteht. R₇ steht besonders bevorzugt für -OH.

Die bivalente Gruppe L ist vorzugsweise aus der Gruppe ausgewählt, die aus einer Bindung, vorzugsweise einer kovalenten Einfachbindung, -CH₂-, -CH₂CH₂-, -CH=CH-, -CONH- und -OCH₂- besteht. Besonders bevorzugt ist L eine kovalente Einfachbindung.

Weiterhin besonders bevorzugt ist es, dass R₂ für -COOH, -SO₃H, -SO₂NH₂, Tetrazol-5-yl, -C=NH(NH₂) oder -NH-C=NH (NH₂) steht und die bivalente Gruppe L eine kovalente Bindung darstellt.

Die Gruppe R₁ steht besonders bevorzugt für H oder CH₃, noch stärker bevorzugt für H.

Desweiteren ist es bevorzugt, dass X für N-R₁, stärker bevorzugt für NH, steht.

Der Ring Q₁ ist besonders bevorzugt Benzol, Pyridin oder Pyrimidin, am stärksten bevorzugt Benzol.

Die Ringe Q₂ und Q₃ sind jeweils unabhängig voneinander bevorzugt Benzol. Am meisten bevorzugt ist es, wenn in einer Verbindung, die beide Ringe trägt, beide Ringe Q₂ und Q₃ Benzol sind.

Die bivalente Einheit Y ist besonders bevorzugt O, S, -CH₂-oder -CH₂CH₂-.

Bevorzugt ist es zudem, wenn R₃ aus der Gruppe ausgewählt ist, die aus H, -COOH, CH₃, OCH₃, F, Cl, Br und CN besteht. Besonders bevorzugt steht R₃ für H und F, ganz besonders bevorzugt für H.

R₄ und R₅ sind besonders bevorzugt jeweils unabhängig voneinander gleich H oder F, noch stärker bevorzugt gleich H.

Die Gruppe A steht besonders bevorzugt für CH.

Die Gruppen R₁₀ und R₁₁ sind besonders bevorzugt jeweils unabhängig voneinander gleich H oder CH₃, noch stärker bevorzugt gleich H.

Desweiteren ist es bevorzugt, dass die Gruppe R₈ eine Gruppe der Formel IV, V, VII, IX oder XI ist. Besonders bevorzugt ist die Gruppe R₈ eine Gruppe der Formel IV.

Ist die Gruppe R₈ eine Gruppe der Formel IV, so ist in einer bevorzugten Ausführungsform Q₁ gleich Benzol und X gleich NH, L gleich eine kovalente Einfachbindung oder -CH=CH-, R₂ gleich - COOH, -CONH₂, -CONHNH₂, -SO₃H, -SO₂NH₂, -COOCH₃, -COOCH₂CH₃, - COOCH₂CH₂CH₃, -COOCH₂CH₂CH₂CH₃, -COOCH₂CH(CH₂CH₃)₂, -COOCH(CH₃)₂, - COOC(CH₃)₃, -COOCH₂CH₂N(CH₃)₂ oder -COOCH₂CH₂ (morpholin-4-yl) oder eine der Gruppen

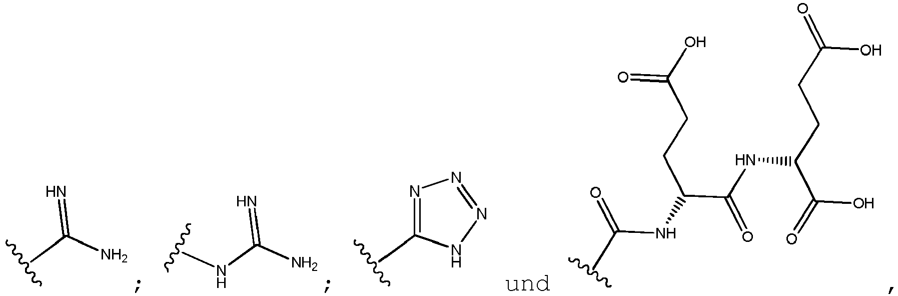

R₃ gleich H, -COOH, -COOMe oder F, vorzugsweise H, R₄ gleich H oder F, vorzugsweise H, und R₅ gleich H.

Ist die Gruppe R₈ eine Gruppe der Formel IV, so ist in einer weiteren bevorzugten Ausführungsform Q₁ gleich Pyridin oder Pyrimidin und X gleich NH. Dabei ist es besonders bevorzugt, dass Q₁ gleich Pyrimidin, L gleich eine kovalente Einfachbindung, R₂ gleich SH, OH oder NH₂, vorzugsweise SH oder OH, R₃ gleich H, SH, OH oder NH₂, vorzugsweise H, SH oder OH, und R₄ und R₅ vorzugsweise nicht vorhanden sind (da an dieser Stelle die Heteroatome im Pyrimidinring sitzen).

Ist die Gruppe R₈ eine Gruppe der Formel IX, X oder XI, so ist in einer weiteren bevorzugten Ausführungsform X gleich NH, A gleich CH, Q₂ gleich Benzol, L gleich eine kovalente Einfachbindung, R₂ gleich -SO₃H oder -COOH und R₃ gleich H.

Ist die Gruppe R₈ eine Gruppe der Formel XIII, so sind in einer weiteren bevorzugten Ausführungsform R₁, R₁₀ und R₁₁ gleich H.

Desweiteren ist es bevorzugt, dass das Grundgerüst der Formel I eine substituierte Cyclosporin A Verbindung ist, die der folgenden Formel entspricht: 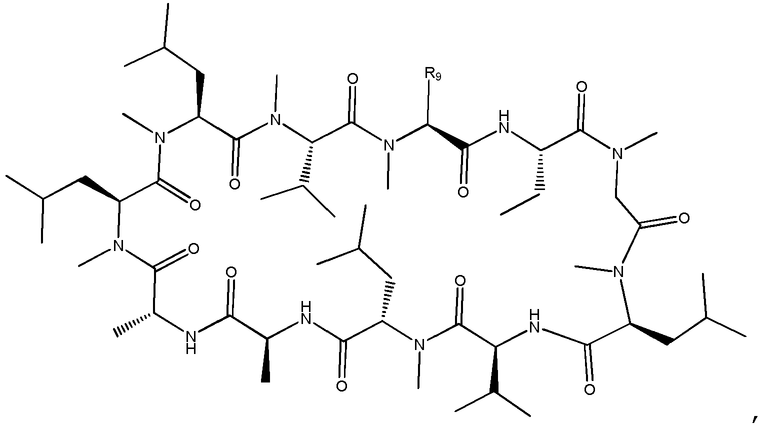 wobei R₉ die gleichen Bedeutungen wie weiter oben oder weiter unten definiert aufweist.

Zudem ist es besonders bevorzugt, dass die Verbindung der Formel I ein substituiertes Cyclosporin A-Derivat der folgenden Formel ist:

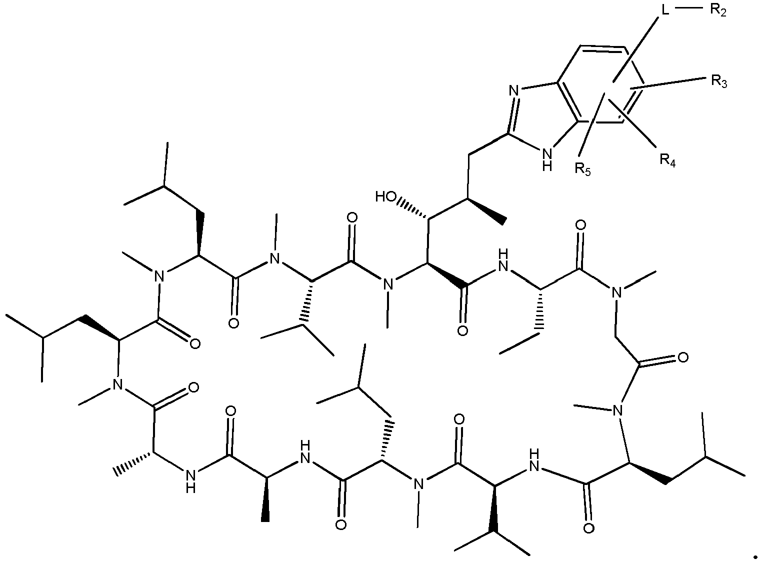

Alle in den verschiedenen Ausführungsformen angeführten Merkmale der Verbindung der voranstehenden Formel I können - sofern sie sich nicht gegenseitig ausschließen - miteinander erfindungsgemäß kombiniert werden. Sofern in den genannten Ausführungsformen Reste der Verbindung der voranstehenden Formel I nicht explizit in der genannten Ausführungsform definiert sind, so können sie erfindungsgemäß jedwede in dieser Anmeldung angegebene allgemeine oder spezielle Definition innehaben.

Beispielhaft für Verbindungen der voranstehenden Formel I werden die folgenden Verbindungen Ac-CsA-Aldehyd, TBDMS-CsA-Aldehyd und Ac-CsA-Säure angeführt, welche als Ausgangsverbindungen zur Synthese von Verbindungen der Formel I dienen können, die sich vom Cyclosporin A ableiten:
Verbindungen 1, 2 und 3: Ac-CsA-Aldehyd, TBDMS-CsA-Aldehyd und Ac-CsA-Säure (CsA heißt Cyclosporin A) (nachstehend): 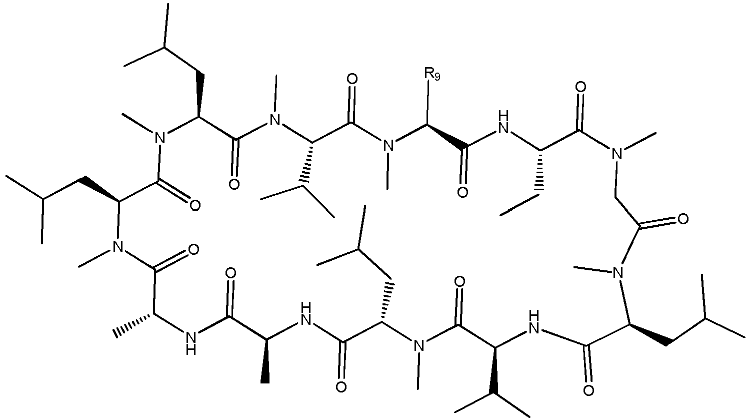
wobei R₉ folgender Struktur entspricht: 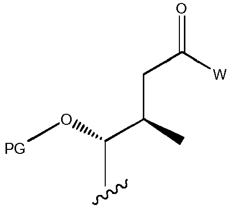
und wobei im Falle der Verbindung 1 PG gleich eine Acetylgruppe (Ac) und W = H, im Falle von der Verbindung 2 PG gleich eine tert-Butyldimethylsilylgruppe (TBDMS) und W = H, und im Falle von der Verbindung 3 PG gleich eine Acetylgruppe (Ac) und W = OH ist.

Besonders bevorzugt sind in der vorliegenden Erfindung die folgenden Verbindungen 4 bis 68 als Verbindungen der Formel I:
Verbindung 4: 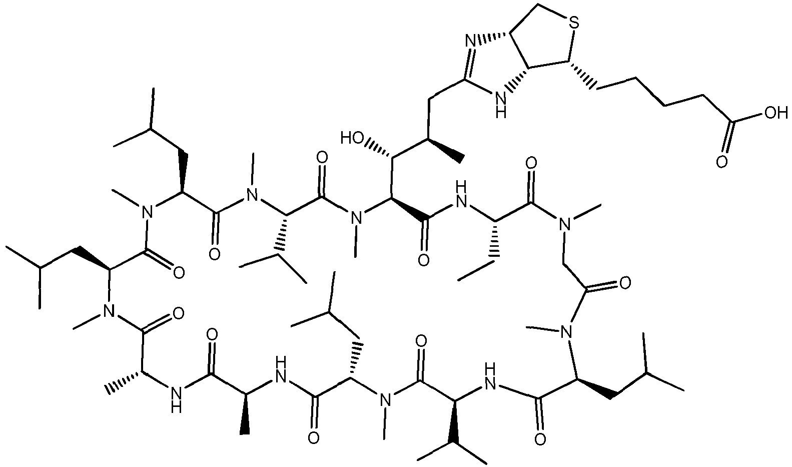
Verbindung 5: 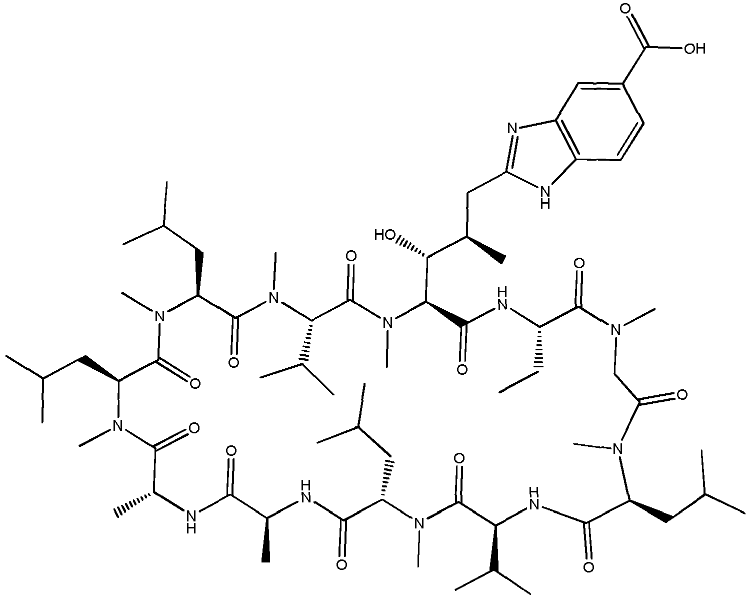
Verbindung 6: 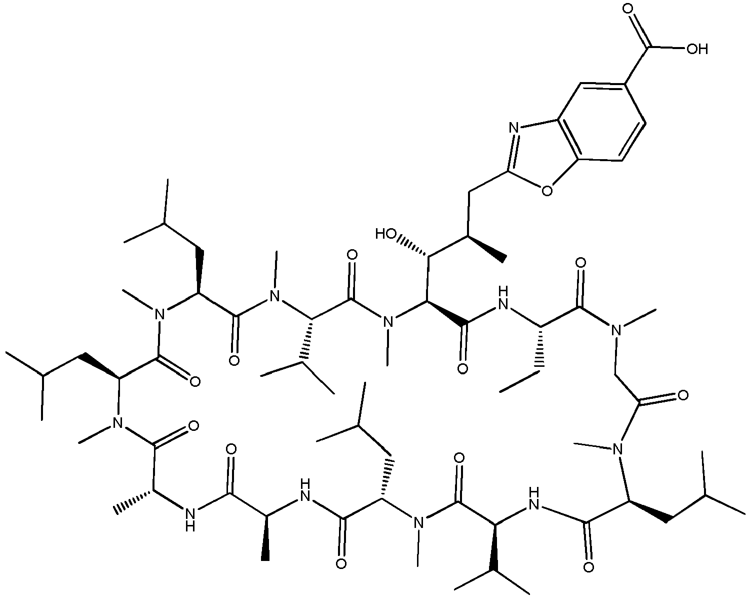
Verbindung 7: 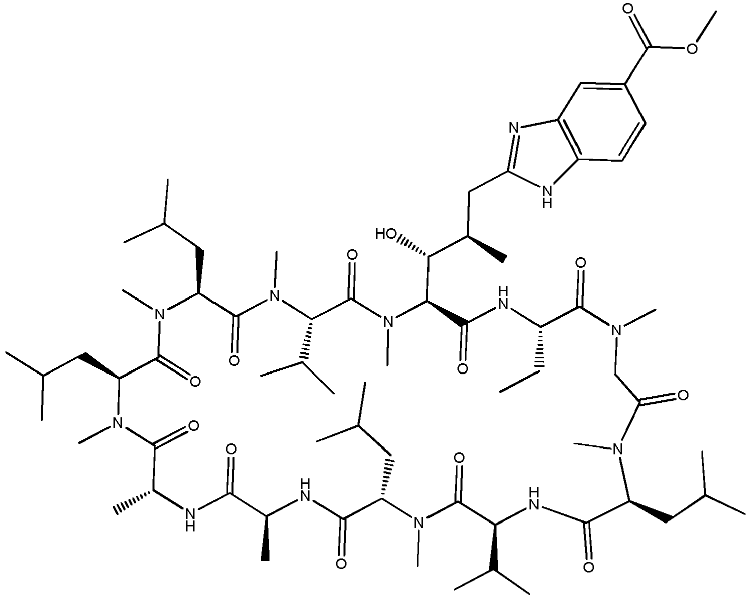
Verbindung 8: 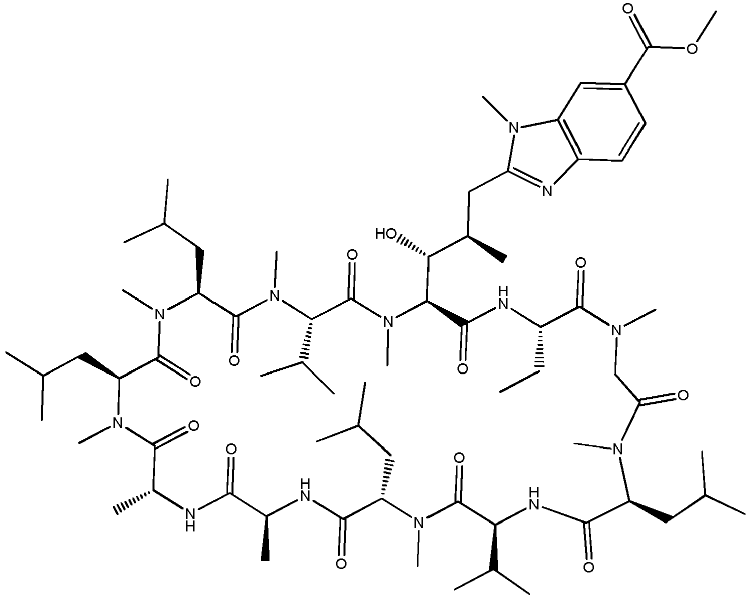
Verbindung 9: 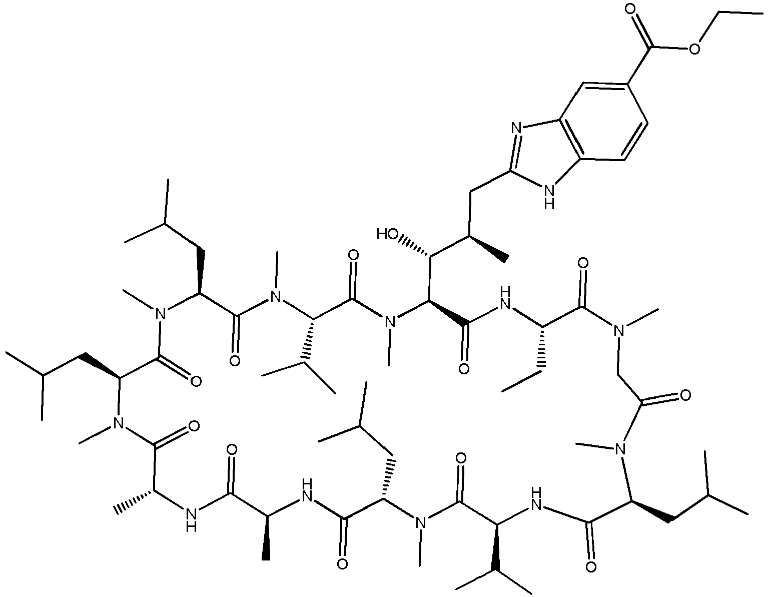
Verbindung 10: 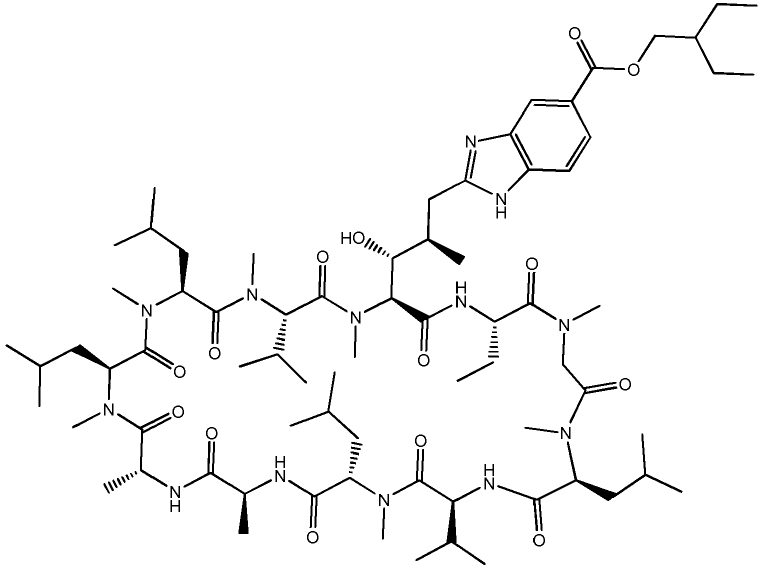
Verbindung 11: 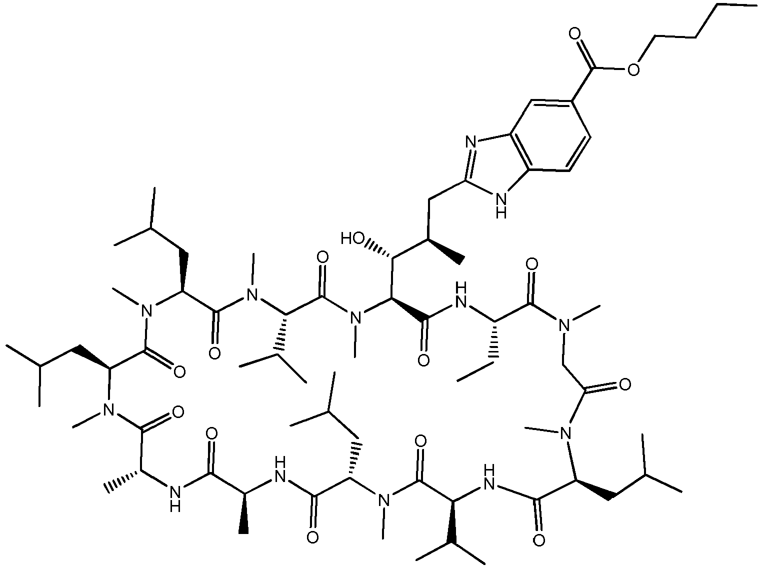
Verbindung 12: 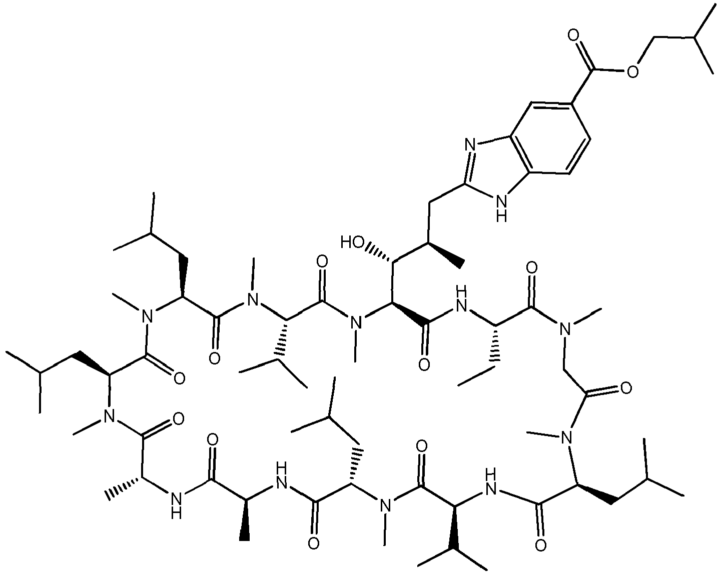
Verbindung 13: 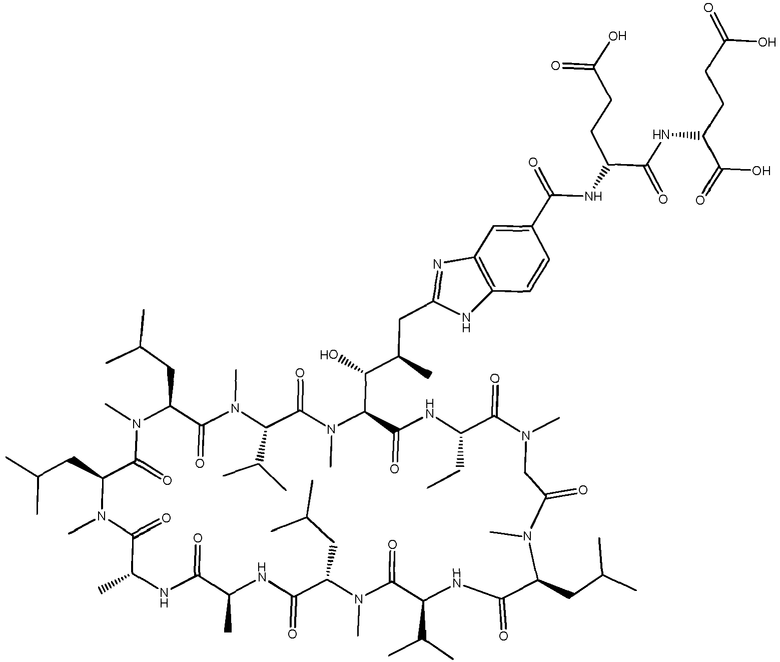
Verbindung 14: 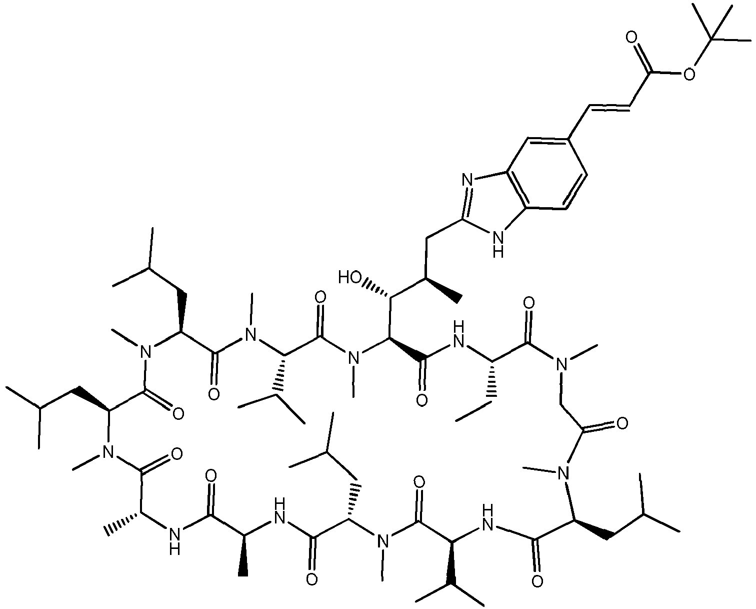
Verbindung 15: 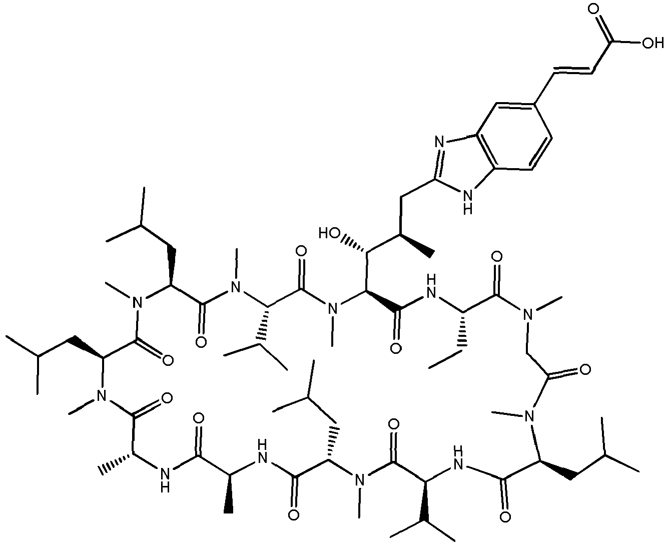
Verbindung 16: 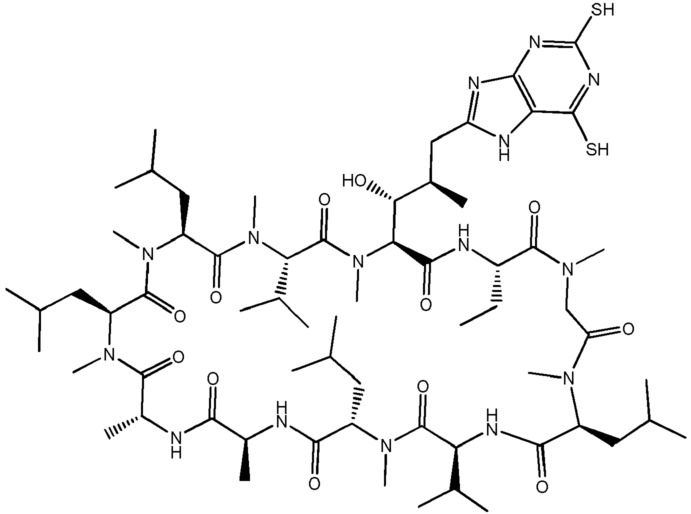
Verbindung 17: 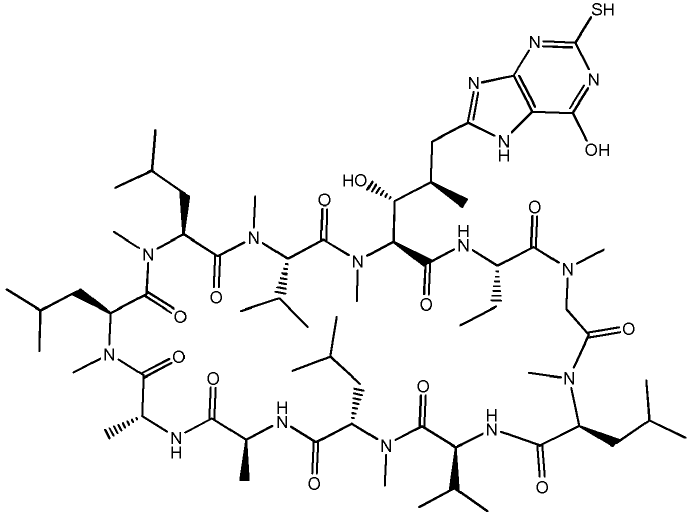
Verbindung 18: 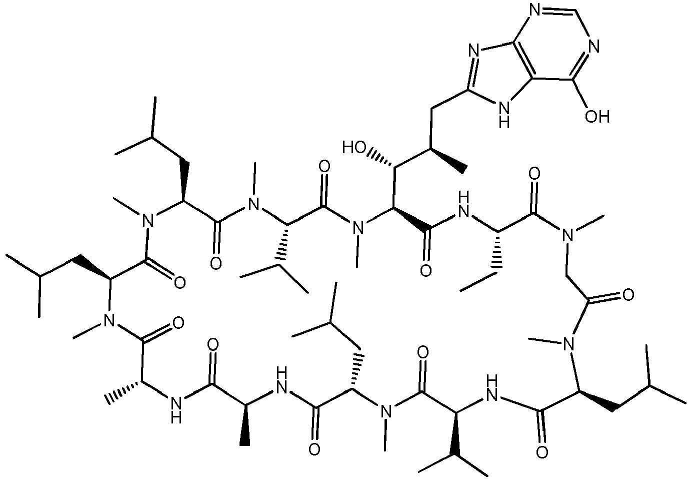
Verbindung 19: 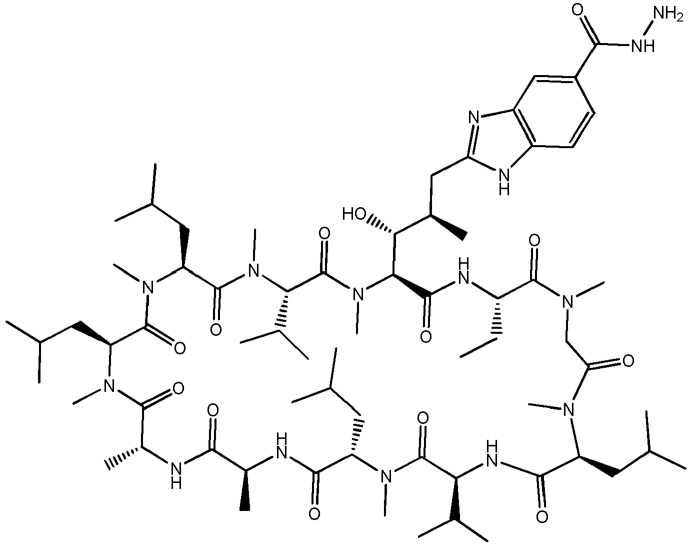
Verbindung 20: 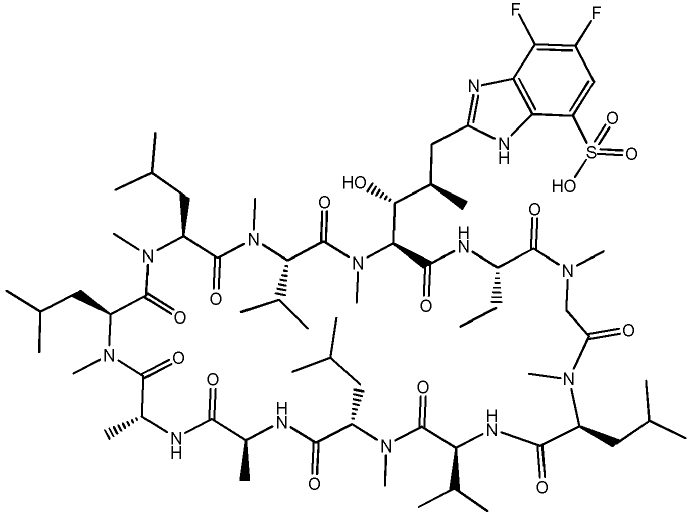
Verbindung 21: 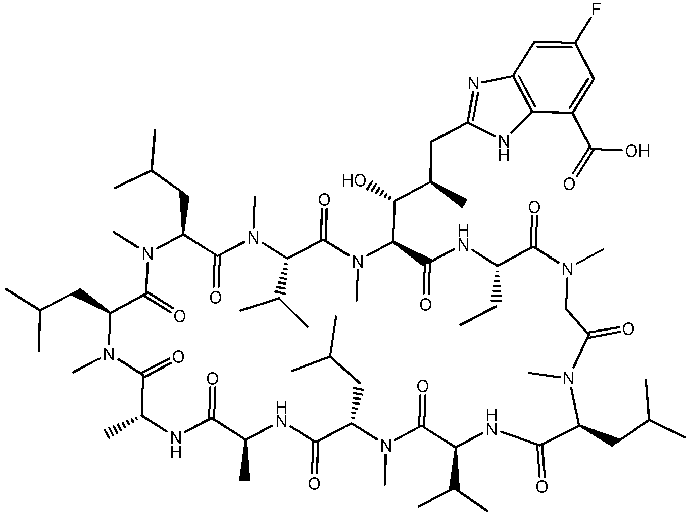
Verbindung 22: 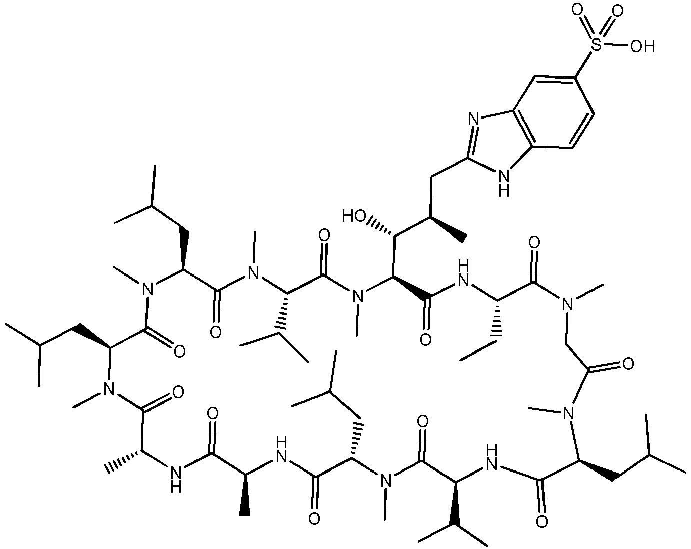
Verbindung 23: 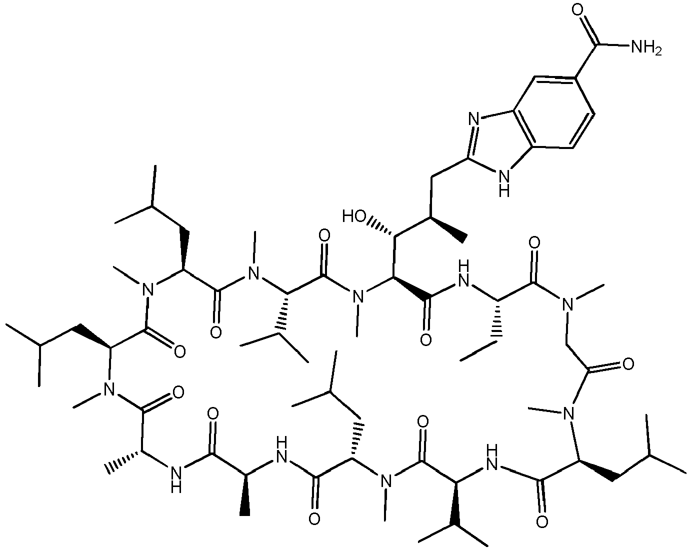
Verbindung 24: 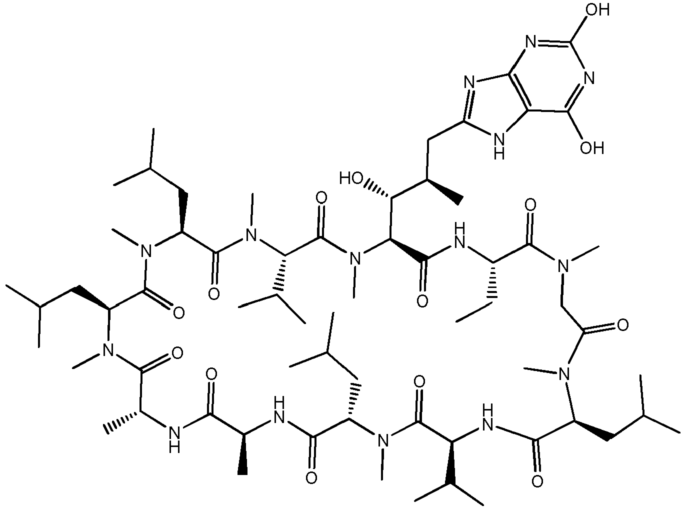
Verbindung 25: 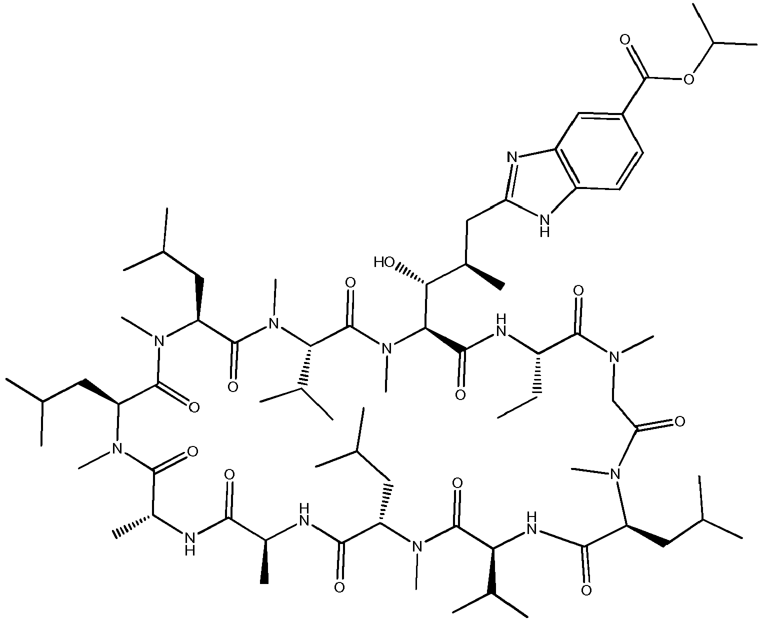
Verbindung 26: 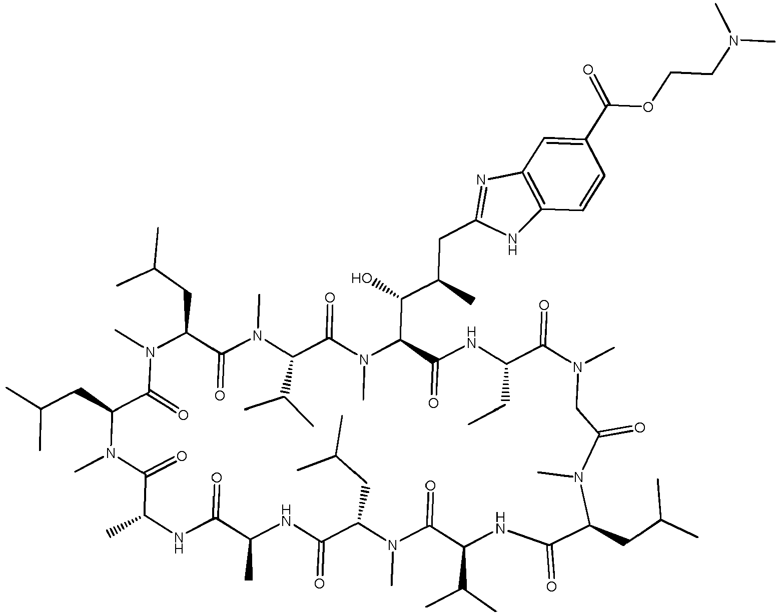
Verbindung 27: 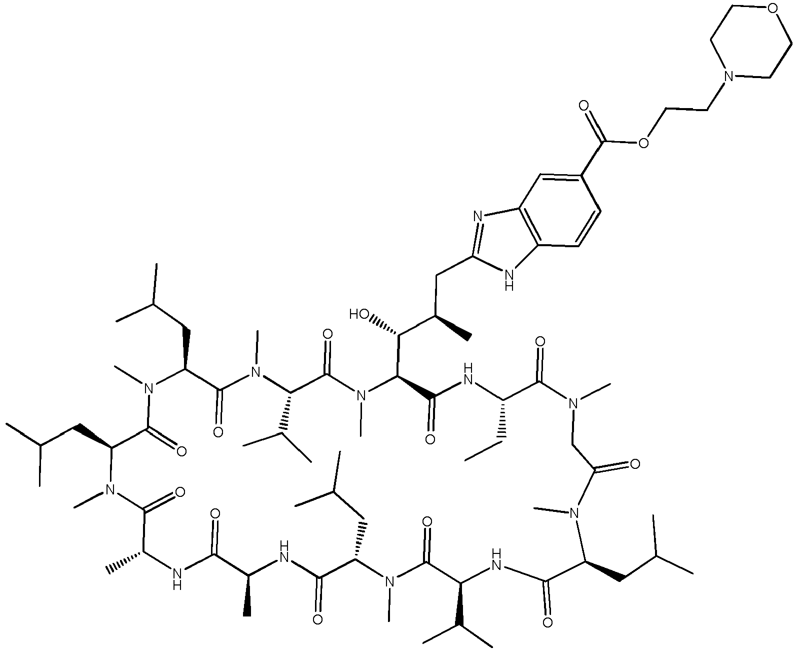
Verbindung 28: 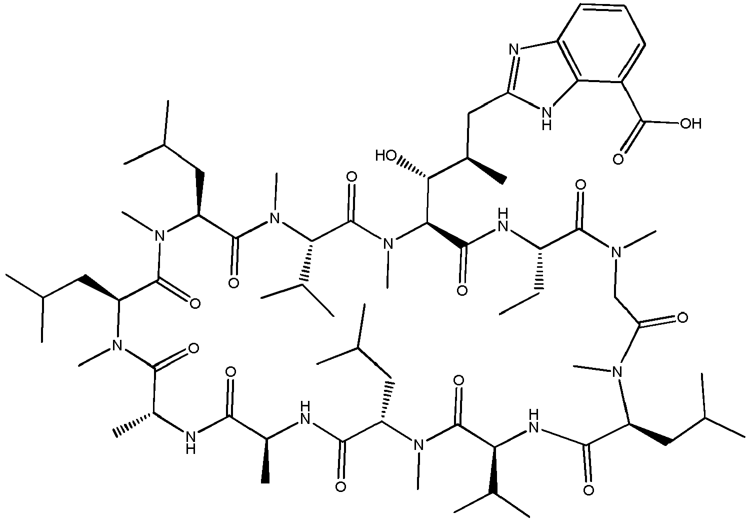
Verbindung 29: 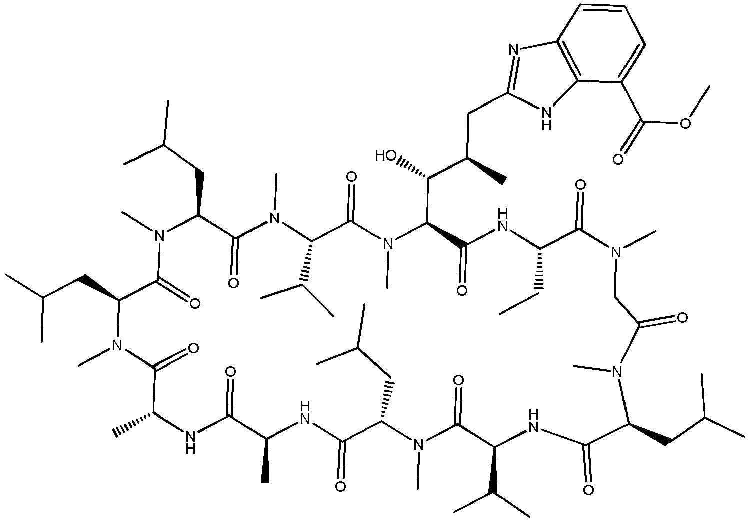
Verbindung 30: 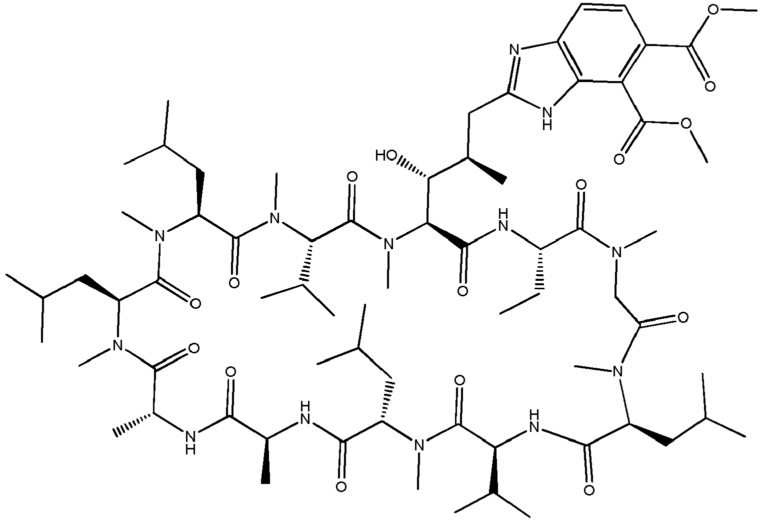
Verbindung 31: 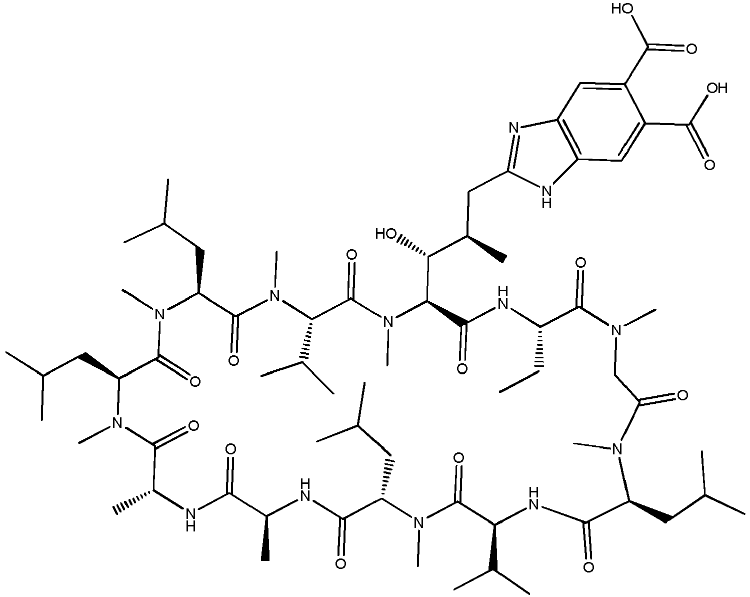
Verbindung 32: 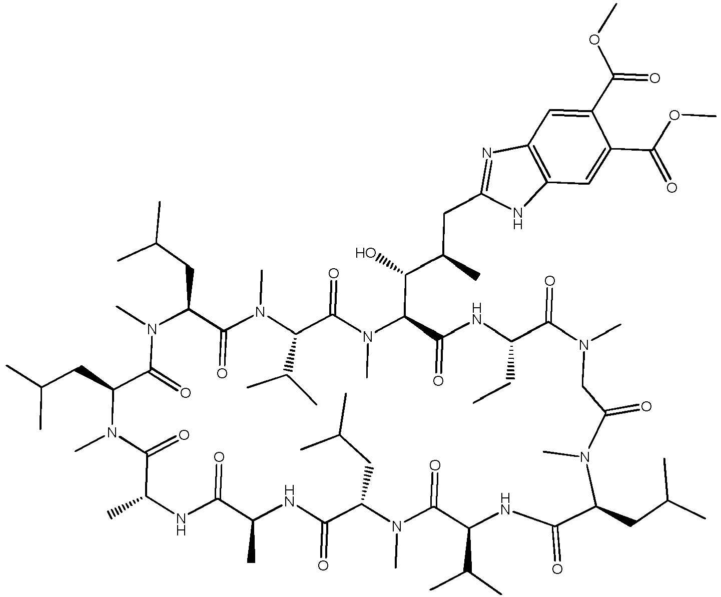
Verbindung 33: 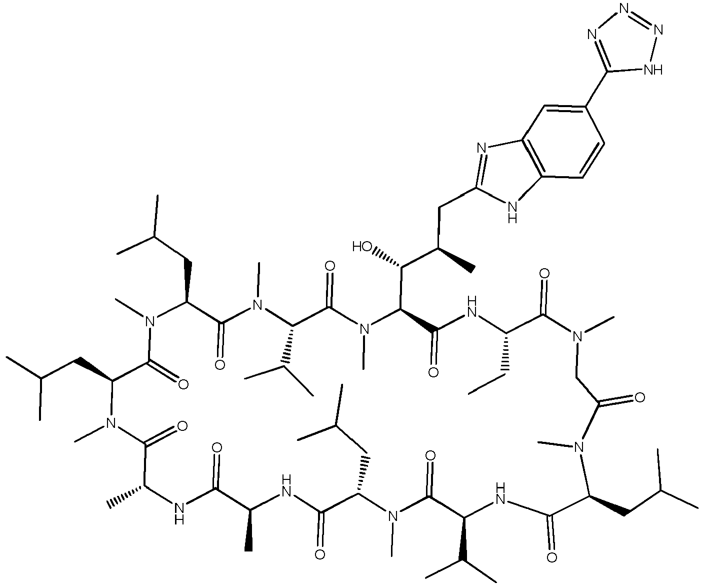
Verbindung 34: 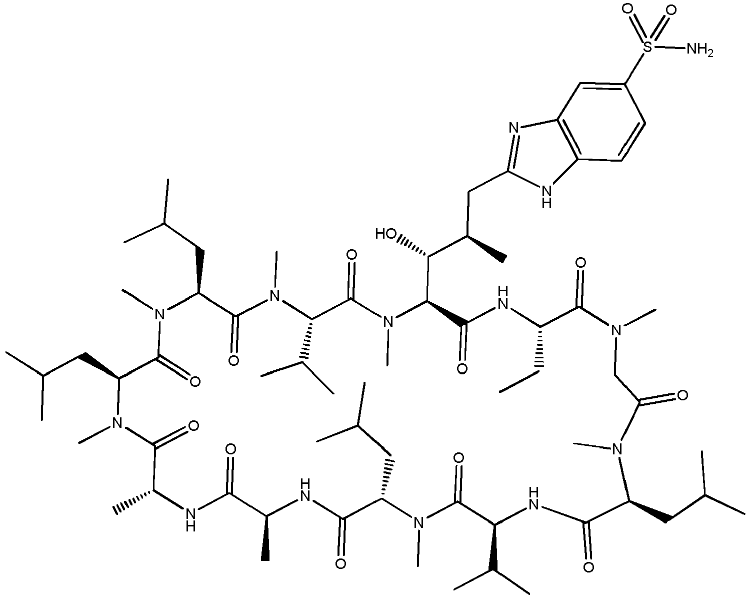
Verbindung 35: 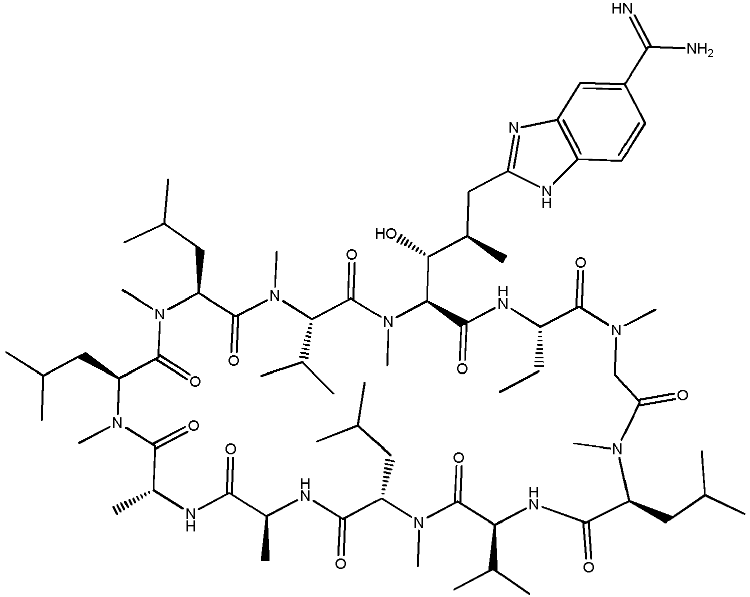
Verbindung 36: 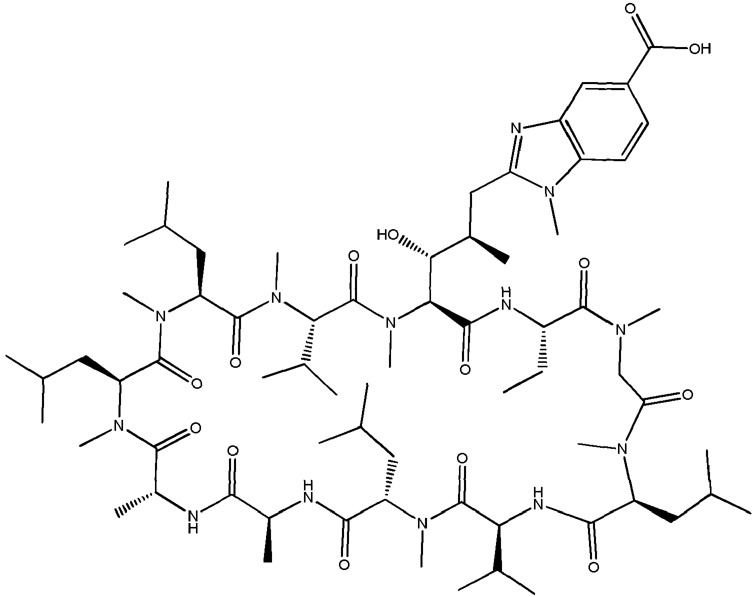
Verbindung 37: 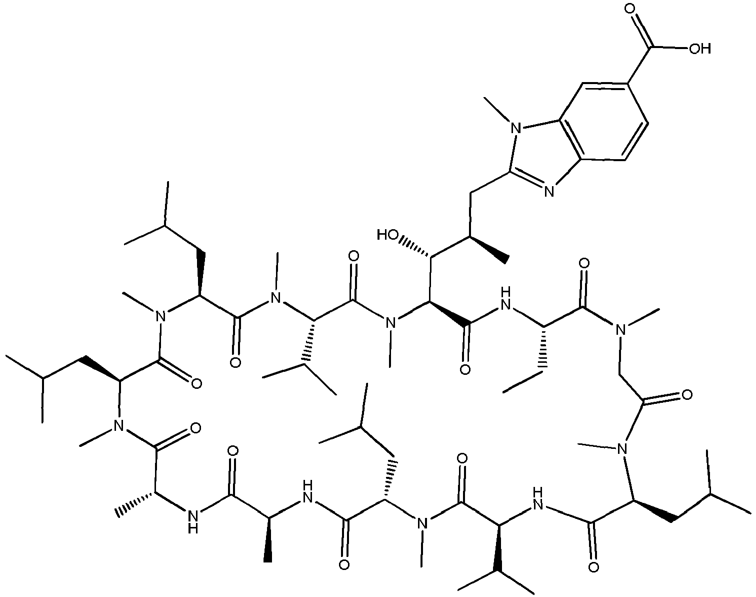
Verbindung 38: 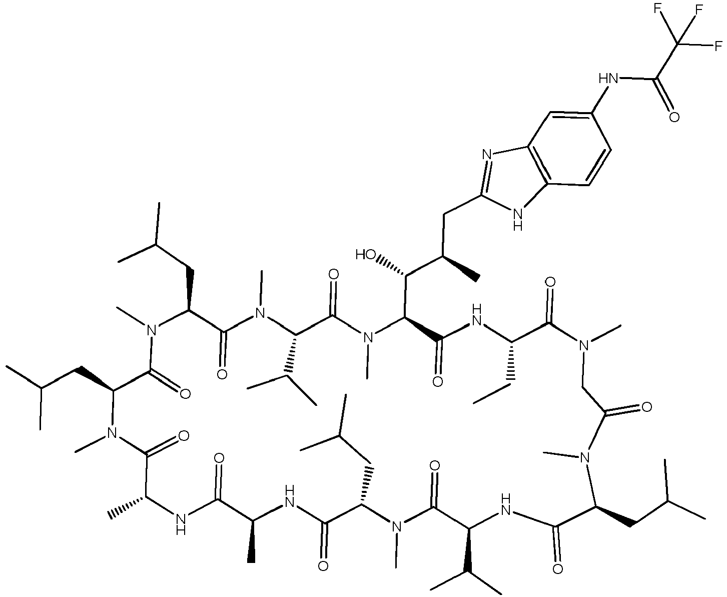
Verbindung 39: 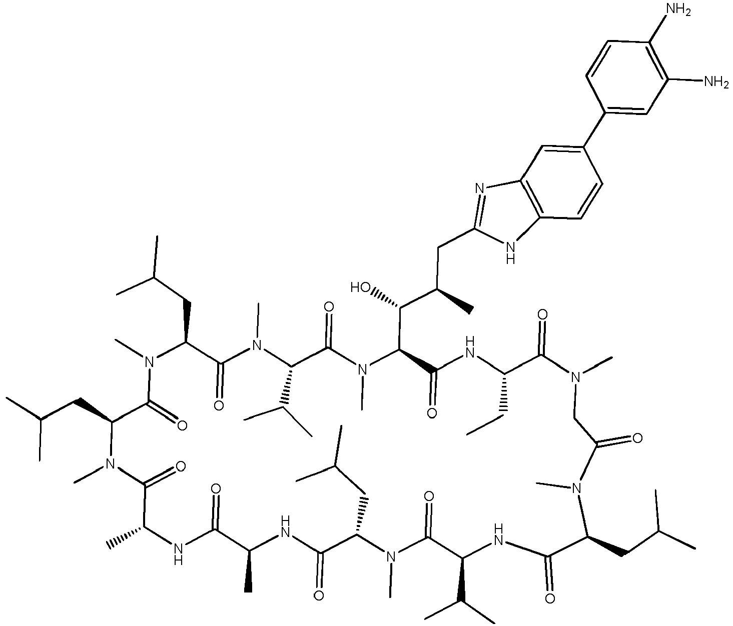
Verbindung 40: 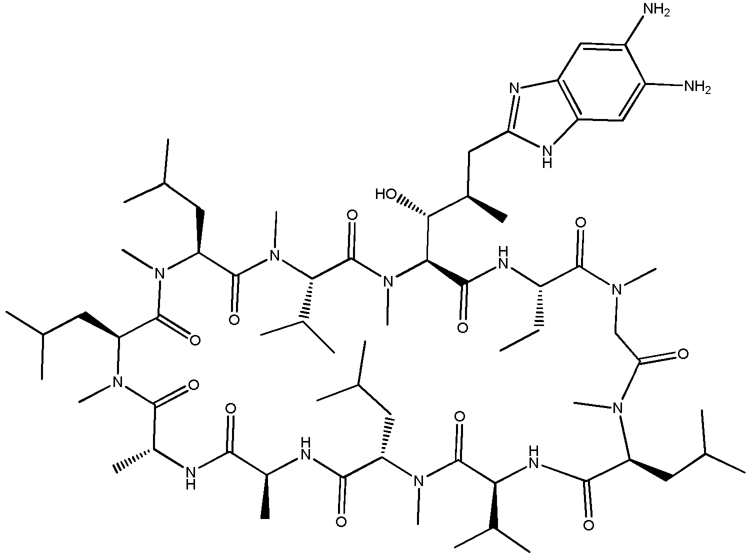
Verbindung 41: 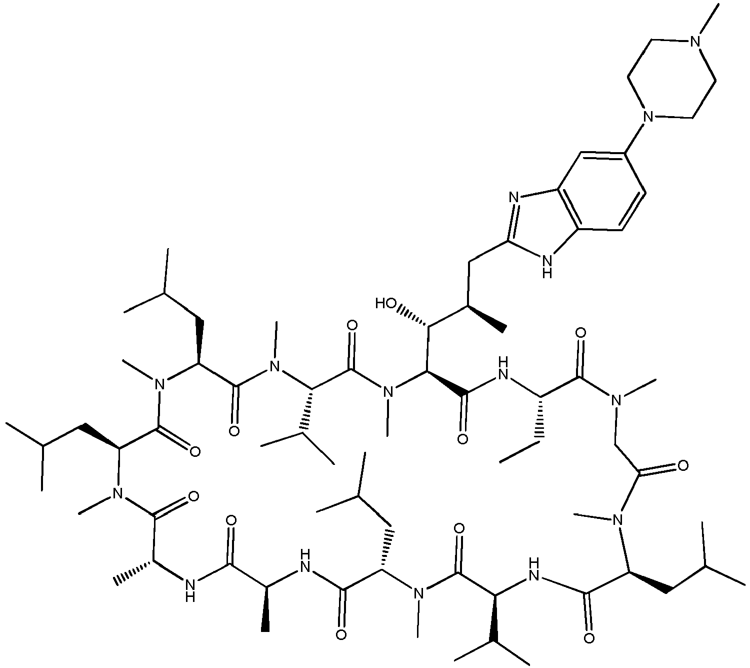
Verbindung 42: 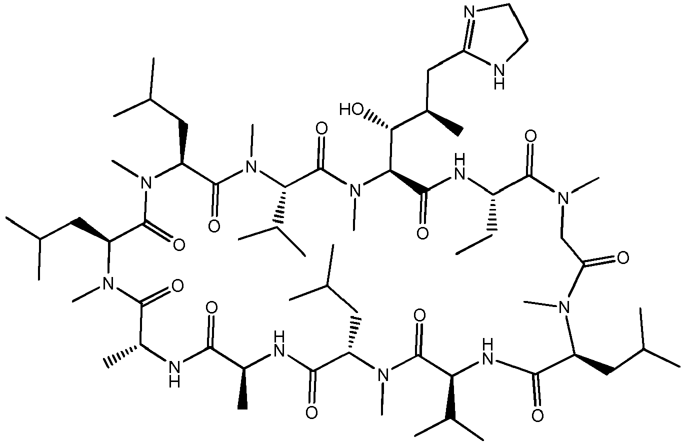
Verbindung 43: 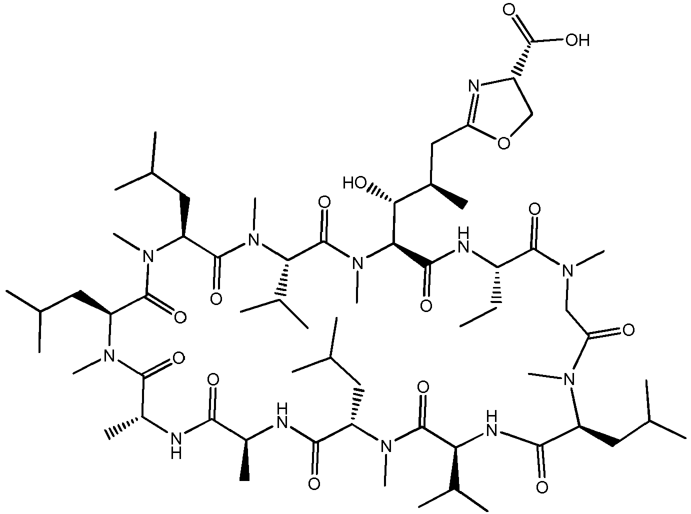
Verbindung 44: 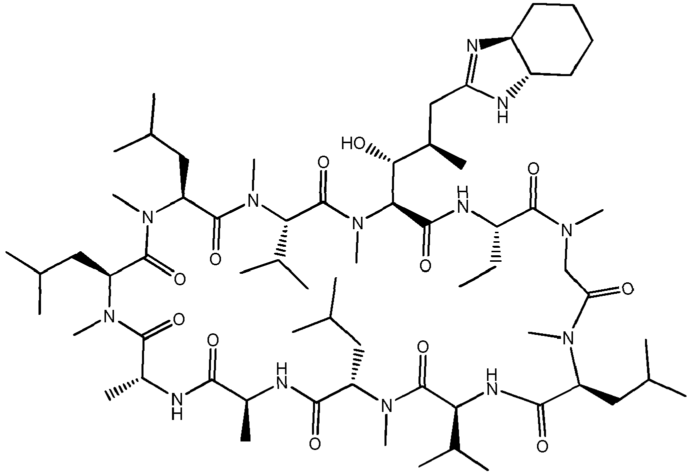
Verbindung 45: 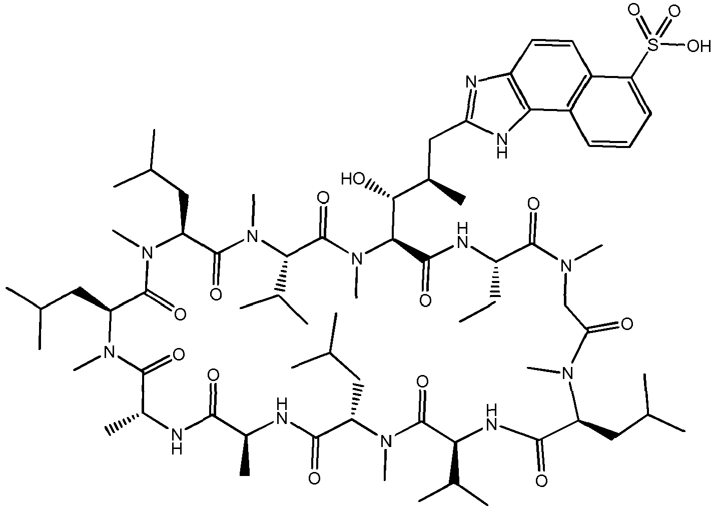
Verbindung 46: 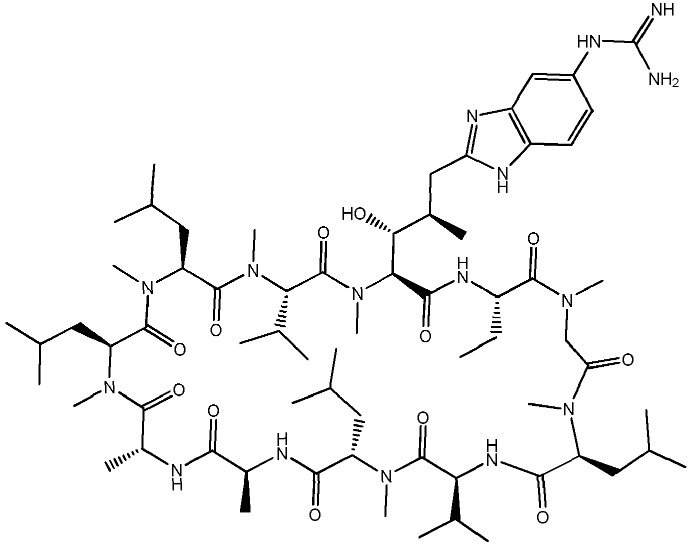
Verbindung 47: 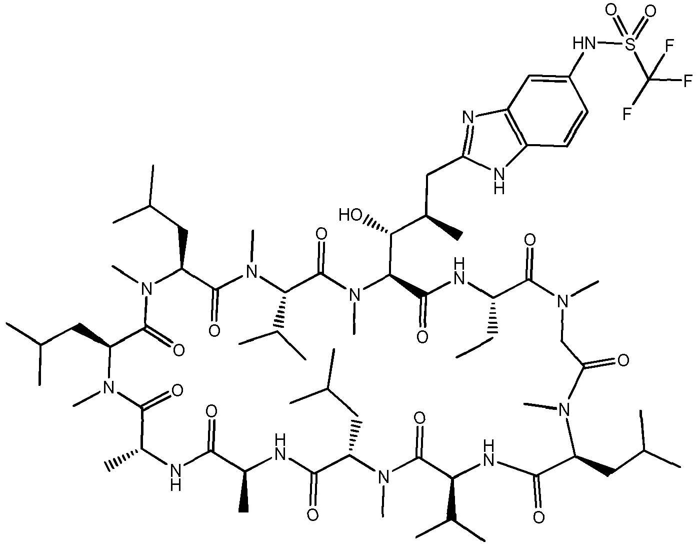
Verbindung 48: 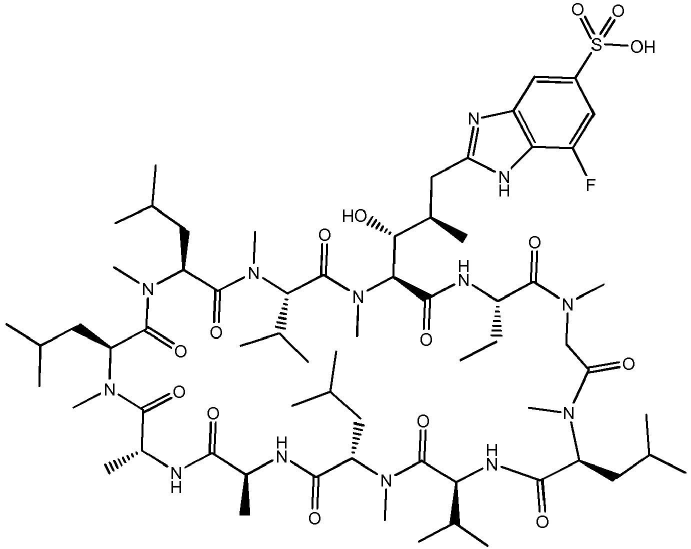
Verbindung 49: 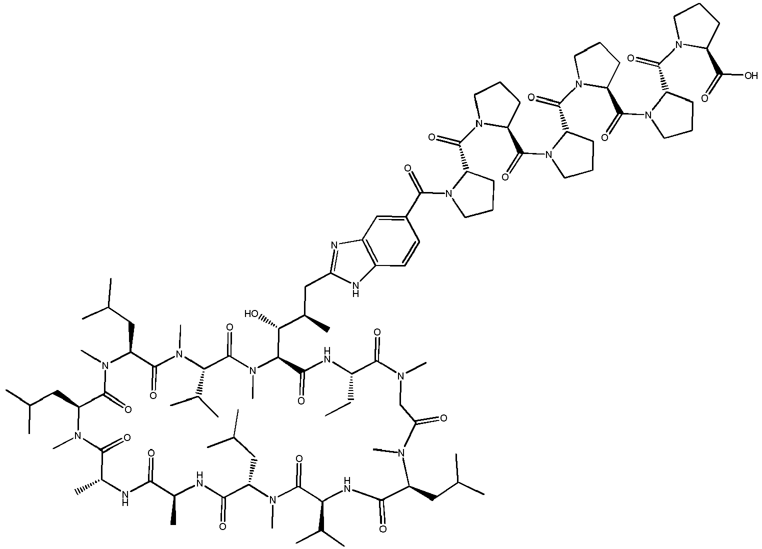
Verbindung 50: 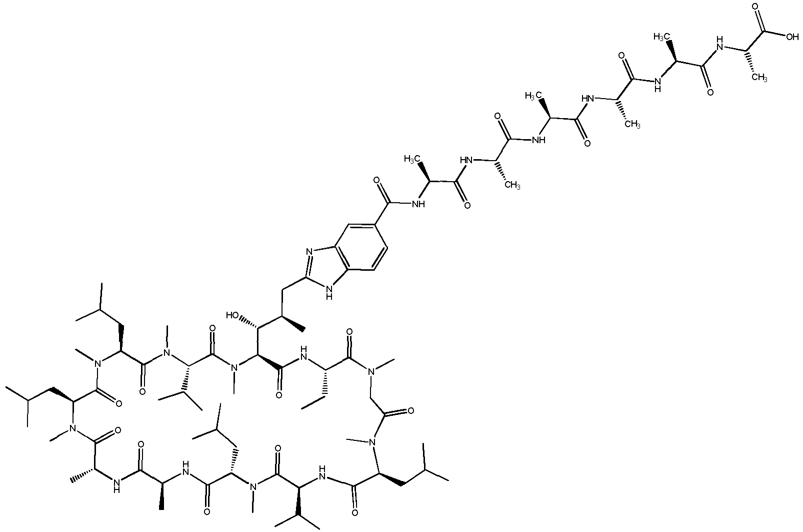
Verbindung 51: 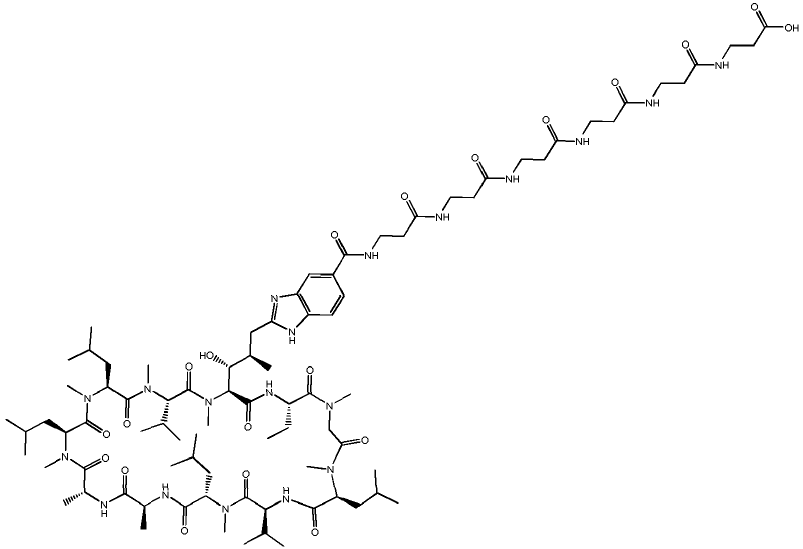
Verbindung 52: 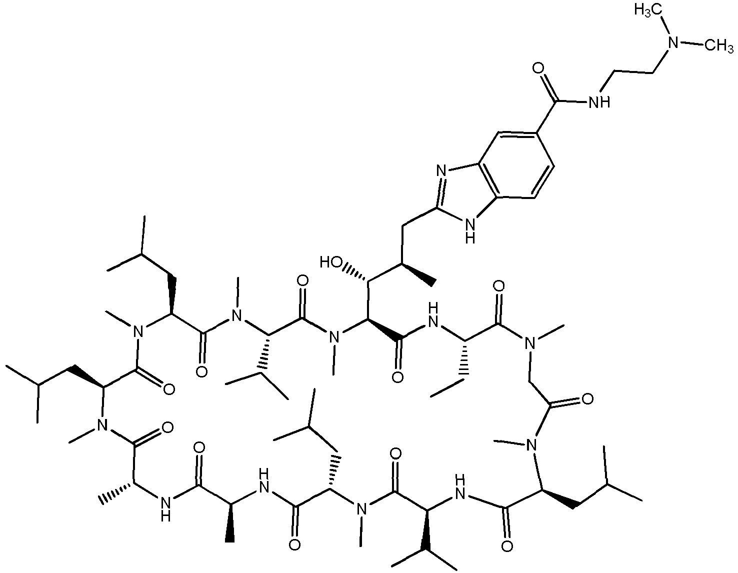
Verbindung 53: 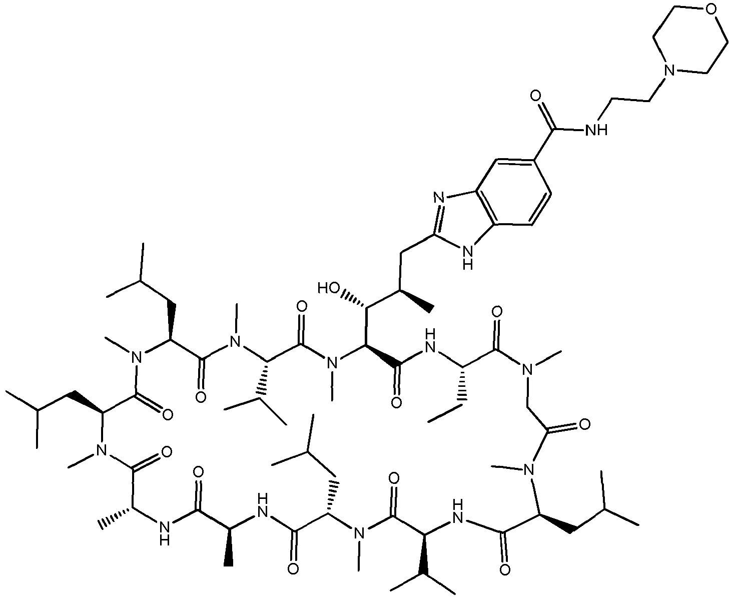
Verbindung 54: 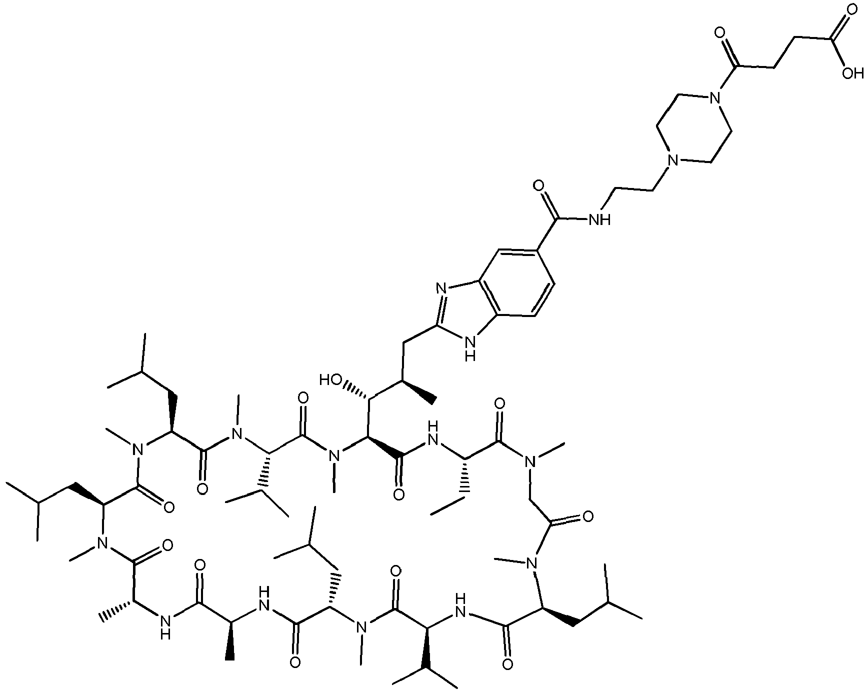
Verbindung 55: 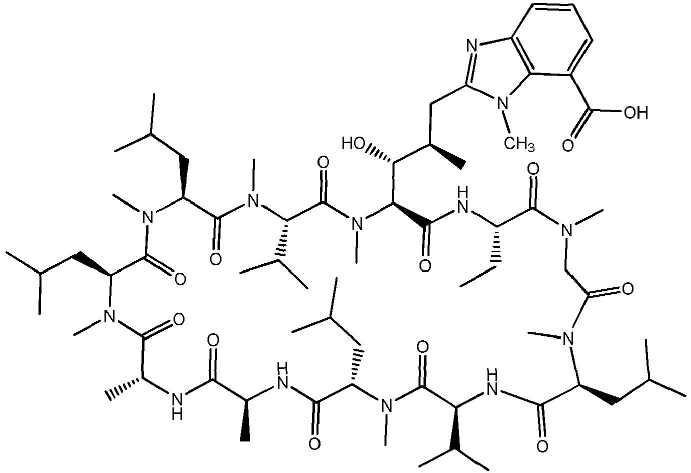
Verbindung 56: 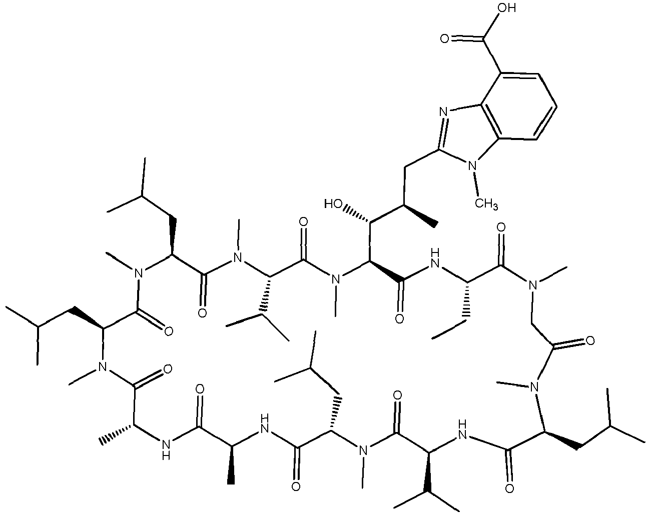
Verbindung 57: 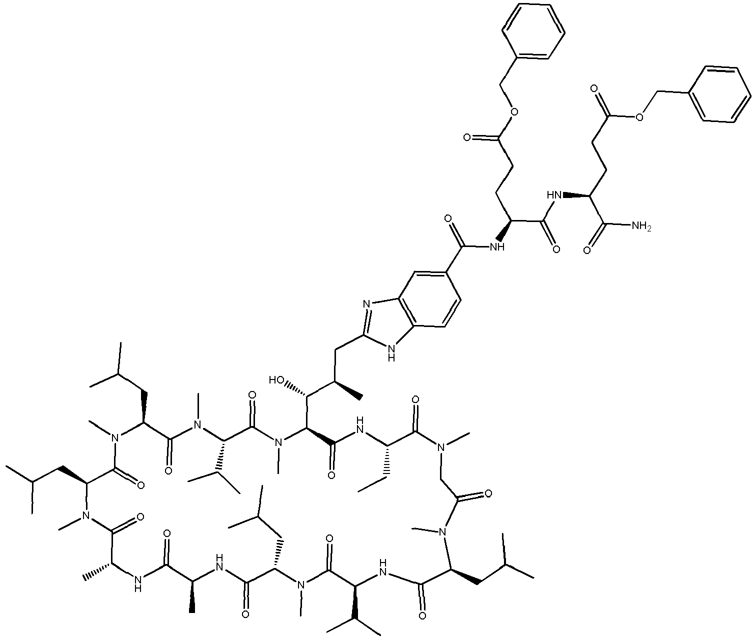
Verbindung 58: 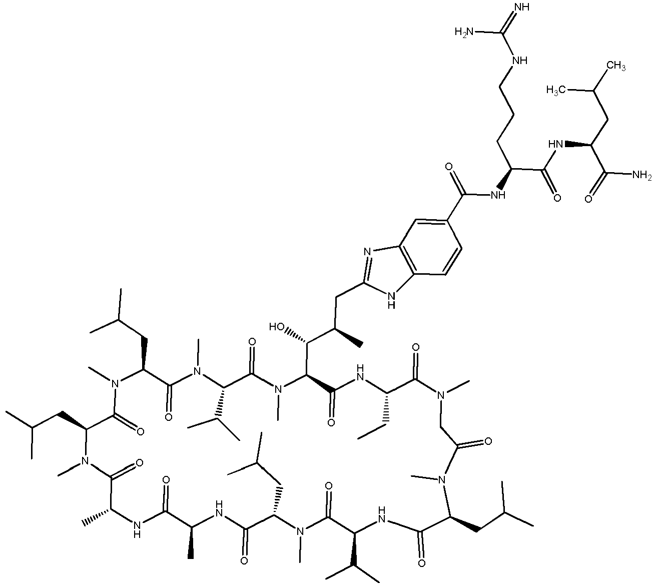
Verbindung 59: 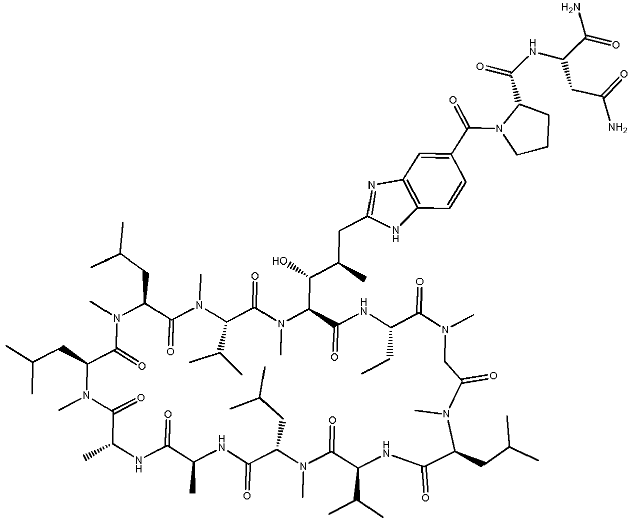
Verbindung 60: 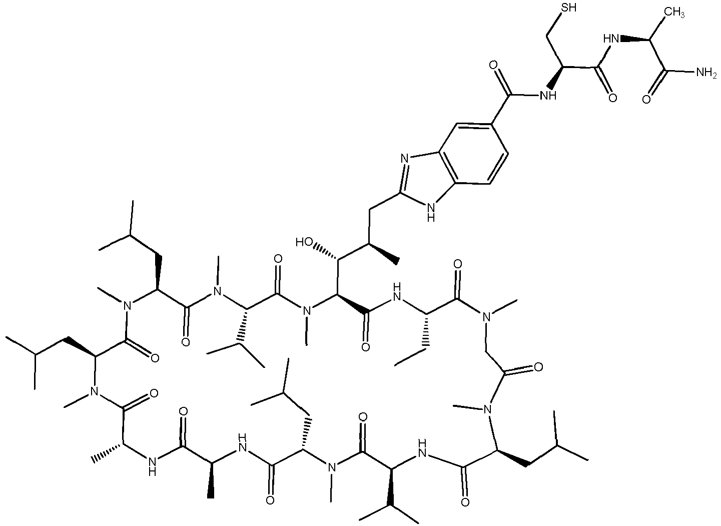
Verbindung 61: 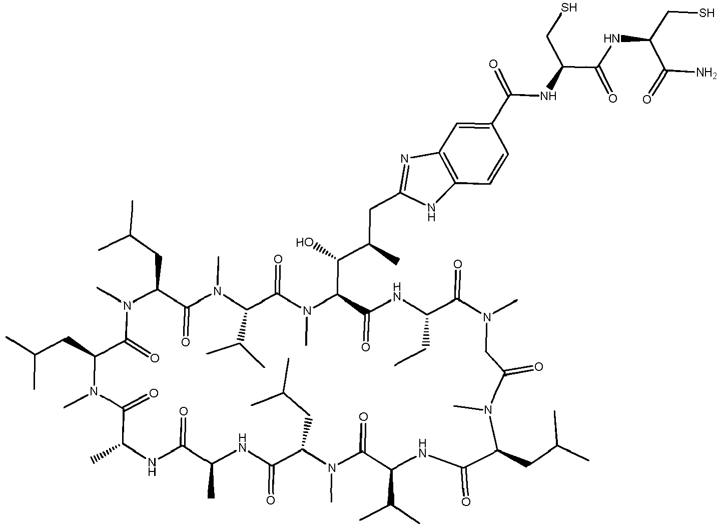
Verbindung 62: 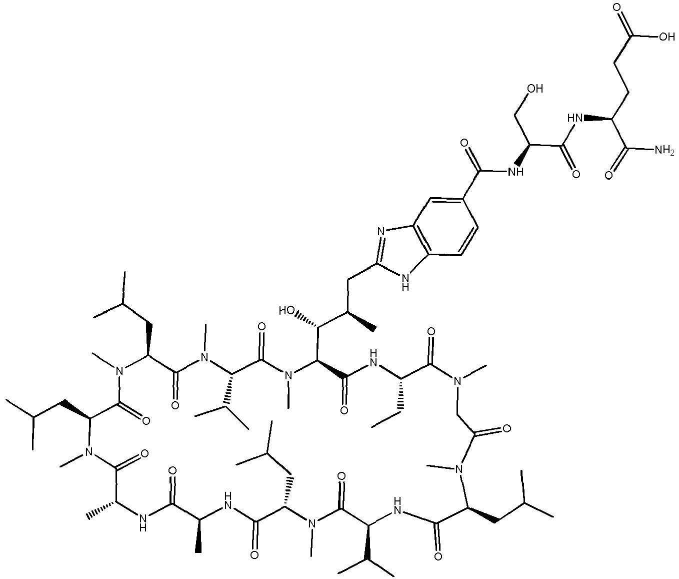
Verbindung 63: 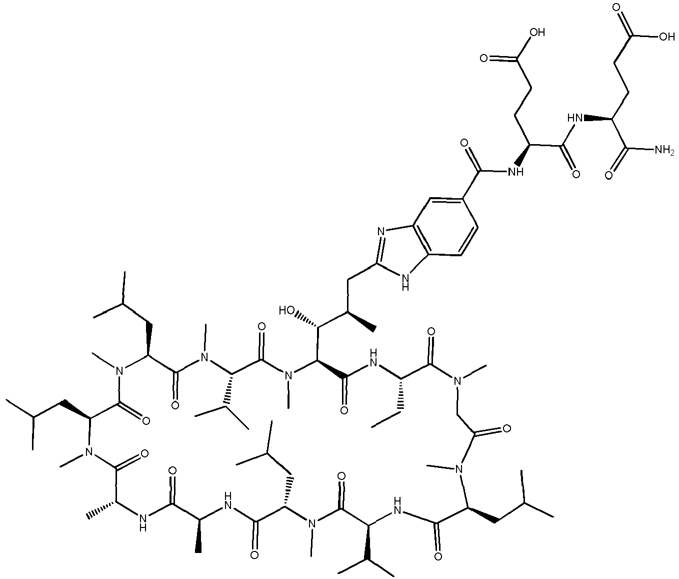
Verbindung 64: 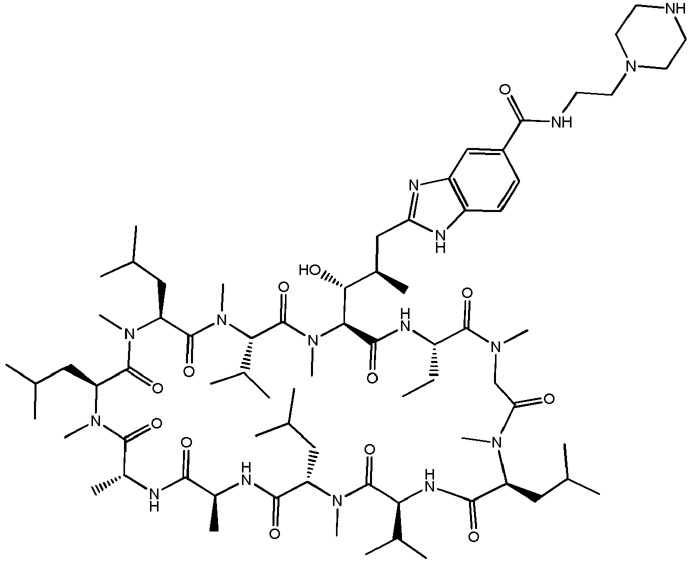
Verbindung 65: 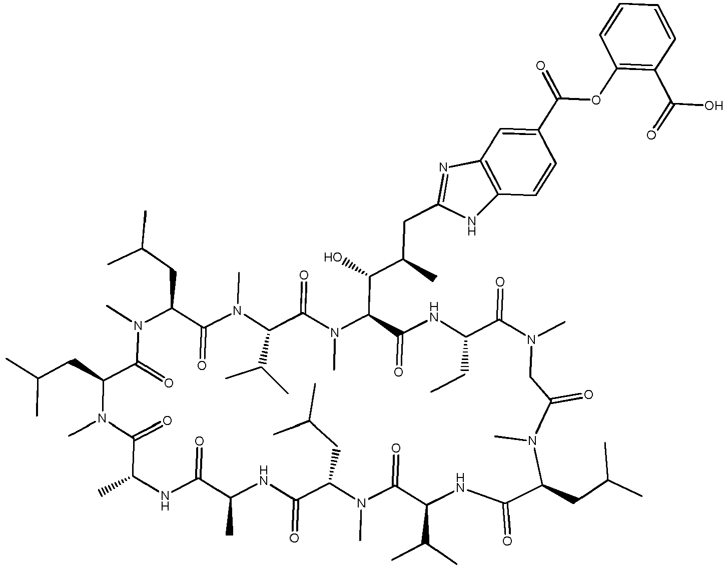
Verbindung 66: 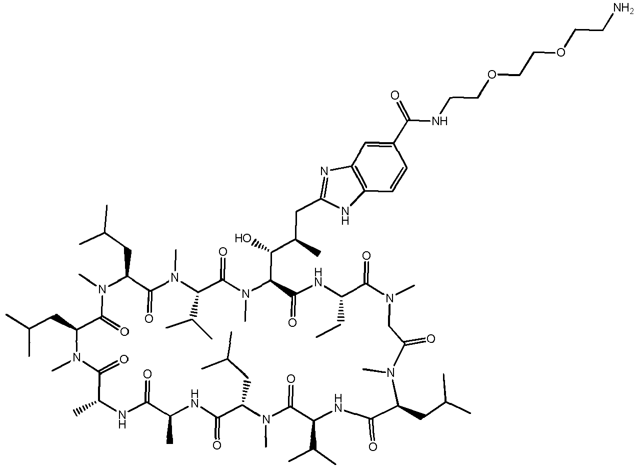
Verbindung 67: 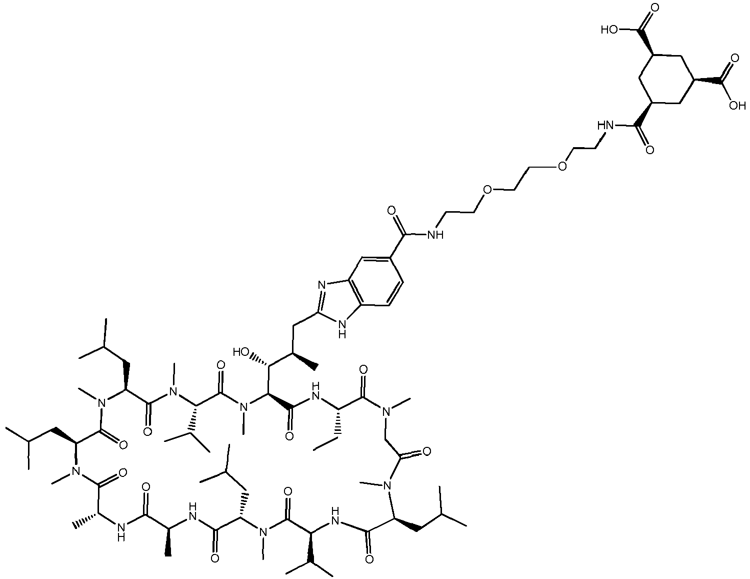
Verbindung 68:

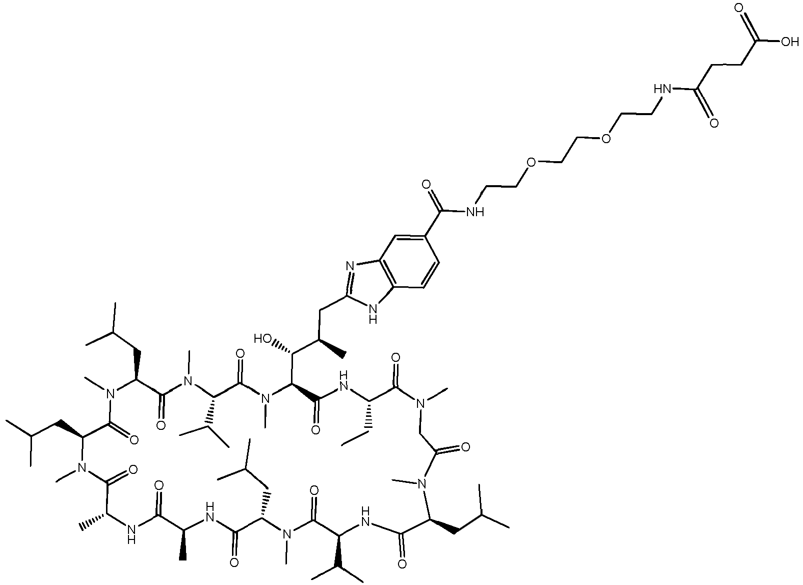

Die vorstehend und nachstehend aufgelisteten Gruppen in dieser Anmeldung binden an der Stelle, an der die Bindung mit der gewellten Linie endet.

Wenn bei den Verbindungen 4 bis 68 tautomere Formen denkbar sind, sollen diese ebenso mit dem angegebenen Tautomer abgedeckt und Gegenstand der vorliegenden Anmeldung sein.

Als erfindungsgemäß besonders bevorzugt haben sich die Verbindungen 5, 13, 20, 22, 28, 31, 33, 34, 35, 37, 42, 43, 45, 46 und 48, insbesondere die Verbindungen 5 und 33, erwiesen.

Unter einem (C1-C4)-Alkyl bzw.(C1-C6)-Alkyl versteht man in der vorliegenden Anmeldung eine geradkettige oder verzweigte Alkylgruppe mit 1 bis 4 bzw. 1 bis 6 C-Atomen, stärker bevorzugt 1 bis 4C-Atomen und noch stärker bevorzugt 1 bis 3 C-Atomen. Einzelne -CH- oder -CH₂-Gruppen können durch N, NH, O oder S ersetzt sein. Ebenso können 1 oder mehrere H-Atome der Alkylgruppe durch Fluoratome ersetzt sein. Beispiele für diese Gruppen sind die folgenden: Methyl, Ethyl, n-Propyl, isoPropyl, n-Butyl, iso-Butyl, s-Butyl, t-Butyl, 2-Methylbutyl, n-Pentyl, s-Pentyl, t-Pentyl, n-Hexyl, 2,2-(Di-ethyl)-ethyl, Trifluormethyl, Pentafluorethyl und 2,2,2-Trifluorethyl, wobei Methyl und Ethyl bevorzugt sind.

Unter einem (C2-C6)-Alkenyl versteht man in der vorliegenden Anmeldung eine geradkettige oder verzweigte Gruppe aus Kohlenwasserstoffeinheiten mit 2 bis 6C-Atomen, vorzugsweise 2 bis 4 C-Atomen und besonders bevorzugt 2 bis 3C-Atomen, bei denen eine oder mehrere Doppelbindungen, vorzugsweise eine Doppelbindung, zwischen zwei C-Atomen vorliegt. Einzelne -CH- oder -CH₂-Gruppen können durch N, NH, O oder S ersetzt sein. Ebenso können 1 oder mehrere H-Atome der Alkenylgruppe durch Fluoratome ersetzt sein. Beispiele für diese Gruppen sind die folgenden: Ethenyl, Propenyl, Butenyl, Pentenyl, Hexenyl, Insbesondere bevorzugt sind die Reste Ethenyl und Propenyl.

Unter einem -NH(C1-C4-Alkenyl) versteht man in der vorliegenden Erfindung ein wie vorstehend definiertes (C1-C4)-Alkenyl das über eine Einheit -NH- gebunden ist. Es gelten die gleichen Bevorzugungen wie sie für (C1-C4)-Alkenyl genannt sind.

Unter einem (C3-C6)-Cycloalkyl versteht man in der vorliegenden Erfindung eine cyclische Kohlenwasserstoffgruppe mit 3 bis 6 Ring-C-Atomen. Einzelne -CH₂-Gruppen können durch N, NH, O oder S ersetzt sein. Ebenso können 1 oder mehrere H-Atome der Alkylgruppe durch Fluoratome ersetzt sein. Beispiele für diese Gruppen sind die folgenden: Cyclopropyl, Cyclobutyl, Cyclopentyl und Cyclohexyl. Besonders bevorzugt ist Cyclohexyl.

Unter einem (C1-C6)-Alkoxy bzw. einem -O(C1-C4-Alkyl) versteht man in der vorliegenden Erfindung ein (C1-C6)-Alkyl bzw. ein (C1-C4)-Alkyl wie vorstehend definiert, das über ein Sauerstoffatom gebunden ist. Besonders bevorzugt sind hier Methoxy und Ethoxy.

Unter einem (C1-C6)-Alkylthio versteht man in der vorliegenden Erfindung ein (C1-C6)-Alkyl wie vorstehend definiert, das über ein Schwefelatom gebunden ist. Besonders bevorzugt sind hier Methylthio und Ethylthio.

Unter einem (C1-C6)-Alkylsulfonyl versteht man in der vorliegenden Erfindung ein (C1-C6)-Alkyl wie vorstehend definiert, das über eine Sulfonylgruppe gebunden ist.

Besonders bevorzugt sind hier Methylsulfonyl und Ethylsulfonyl.

Unter einem -COO((C1-C4)Alkyl)) versteht man in der vorliegenden Erfindung ein (C1-C4)-Alkyl wie vorstehend definiert, das über eine -COO-Gruppe gebunden ist, wobei das (C1-C4)-Alkyl an dem Sauerstoffatom bindet. Besonders bevorzugt sind hier -COO-Methyl und -COO-Ethyl.

Unter einem (C1-C6)Alkyl-amino bzw. einem -NH(C1-C4-Alkyl) versteht man in der vorliegenden Erfindung ein (C1-C6)-Alkyl bzw. (C1-C4)-Alkyl wie vorstehende definiert, das über eine Einheit -NH- bindet. Beispiele hierfür sind Methylamino oder Ethylamino.

Unter einem (C1-C6)Dialkyl-amino versteht man eine sekundäre Aminogruppe, die zwei (C1-C6)-Alkyle wie vorstehend definiert trägt. Beispiele hierfür sind Dimethylamino und Diethylamino.

Unter einem (substituierten) Aryl versteht man in der vorliegenden Erfindung einen mono- oder polycyclischen (vorzugsweise mono-, bi- oder tricyclischen) aromatischen oder heteroaromatischen Kohlenwasserstoffrest mit vorzugsweise 5 bzw. 6 bis 10, stärker bevorzugt 5 oder 6 aromatischen Ringatomen. Der aromatische oder heteroaromatische Kohlenwasserstoffrest kann weiterhin Substituenten tragen.

Dabei wird unter einer aromatischen bzw. heteroaromatischen Einheit entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Chinolin, Isochinolin, Benzothiophen, Benzofuran und Indol etc., verstanden. Erfindungsgemäße Beispiele für die aromatische oder heteroaromatische Einheit sind demgemäß: Benzol, Naphthalin, Furan, Benzofuran, Isobenzofuran, Thiophen, Benzothiophen, Isobenzothiophen, Pyrrol, Indol, Isoindol, Pyridin, Chinolin, Isochinolin, Pyrazol, Indazol, Imidazol, Benzimidazol, Oxazol, 1,2-Thiazol, 1,3-Thiazol, Benzothiazol, Pyridazin, Benzopyridazin, Pyrimidin, Benzpyrimidin, Chinoxalin, Pyrazin, Phenazin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin und Indolizin. Bevorzugt sind dabei Benzol, Naphthalin, Furan, Thiophen, Pyrrol, Pyrimidin, Pyrazin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, besonders bevorzugt Benzol.

Unter (substituiertes) O-Aryl bzw. (substituiertes) NH-Aryl versteht man in der vorliegenden Erfindung ein wie vorstehend definiertes (substituiertes) Aryl, das über ein Sauerstoffatom oder eine Einheit -NH- bindet.

Der Substituent am substituierten Aryl kann beispielsweise Methyl, Ethyl, Methoxy, Ethoxy, Fluor, Chlor, Brom, Trifluormethyl, Pentafluorethyl, -OH, -SH, -NH₂, -CN oder -NO₂ sein.

Wie bereits weiter oben erwähnt, betrifft eine weitere Ausführungsform der vorliegenden Erfindung eine Indikator-gekoppelte Verbindung, die eine Verbindung der voranstehenden Formel I und eine Indikatorverbindung (Indikator) umfasst, wobei diese über eine kovalente Bindung miteinander verknüpft sind. Die Verknüpfung der Verbindung mit der Formel I liegt so vor, dass anstelle eines endständigen Atoms oder einer endständigen Gruppe der Verbindung der Formel I eine Bindung zu dem Indikator, gegebenenfalls über einen Linker, vorliegt.

Der Indikator ist vorzugsweise an die Verbindung der Formel I kovalent gebunden. Der Indikator kann jedoch grundsätzlich mit dem Wirkstoff auf jede Art verbunden werden, die dem Fachmann als für den erfindungsgemäßen Zweck geeignet bekannt ist. Besonders bevorzugt ist der Indikator kovalent über einen Linker an die Verbindung der Formel I gebunden.

Bei dem Linker kann es sich im Rahmen der vorliegenden Erfindung um jede Gruppe handeln, die dem Fachmann als für den erfindungsgemäßen Zweck geeignet bekannt ist. Bevorzugt handelt es sich dabei jedoch um eine Gruppe, die frei von einer Protease-Schnittstelle ist. Besonders bevorzugt wird der Linker ausgewählt aus Gruppen, welche einen Atomabstand von vier bis 40 Atomen, vorzugsweise 5 bis 30 und am bevorzugtesten 6 bis 20 Atomen ausbilden.

Unter dem Begriff "Indikator" sind im Sinne der Erfindung Stoffe wie z.B. Farbstoffe, Spannungs-sensitive Indikatoren, pH-Wert Indikatoren, Calcium-sensitive Indikatoren, radioaktive Elemente, NMR-Label oder aber Elektronenspinlabel zusammengefasst und in der wissenschaftlichen Literatur mehrfach beschrieben (WO/2005/022158, EP 0649022*,* US 6,596,499, US 7,090,995, US 4,672,044). Der Begriff Indikator umfasst im Sinne der Erfindung einzelne Atome oder Moleküle, welche kovalent mit dem Wirkstoffmolekül verbunden sind. Dabei kann ein Indikator oder auch mehrere Indikatoren direkt an das Wirkstoffmolekül kovalent gebunden sein. Der Indikator oder auch mehrere Indikatoren können aber auch an einen mehrfunktionellen Linker gebunden sein oder der Indikator oder auch mehrere Indikatoren können auch innerhalb eines sauren Peptids oder endständig an ein saures Peptid kovalent gebunden sein.

"Farbstoffe" sind im Sinne der Erfindung Stoffe, die optisch durch Detektion der von ihnen ausgesandten oder der durch sie nicht absorbierten elektromagnetischen Strahlung nachgewiesen werden können. Dazu gehören z.B. Farbstoffe wie Fluoresceinisocyanat (FIC), Flouresceinisothiocyanat (FITC), Dimethylaminonaphthalen-S-sulphonylchlorid (DANSC), Tetramethylrhodaminisothiocyanat (TRITC), Lissaminrhodamin B200-Sulphonylchlorid (RB 200 SC) usw. Eine Beschreibung zahlreicher geeigneter Moleküle ist z.B. zu finden bei DeLuca, "Immunofluorescence Analysis", in "Antibody As A Tool", Marchalonis et al., Eds., John Wiley & Sons, Ltd., pp. 189-231, (1985).

"Spannungs-sensitive Indikatoren" sind im Sinne der Erfindung Stoffe, die in Abhängigkeit einer anliegenden elektrischen Potentialdifferenz oder des vorliegenden elektrischen Potentials derart ihre physikalischen, optischen oder katalytischen Eigenschaften ändern, dass diese ein detektierbares Signal hervorrufen. Dem Fachmann bekannt sind spannungs-sensitive Indikatoren wie z. B. DIBAC [Japanese Journal of Pharmacology 86(2001)342-350, American Journal of Physiology - Heart & Circulatory Physiology 287(2004)H985-H993).

"pH-Wert-sensitive Indikatoren" sind im Sinne der Erfindung Stoffe, die in Abhängigkeit des pH-Wertes derart ihre physikalischen, optischen oder katalytischen Eigenschaften ändern, dass diese ein detektierbares Signal hervorrufen.

Solche Indikatorfarbstoffe, wie z.B. Phenolrot, Bromthymolblau, Bromphenolblau u. v. a. sind dem Fachmann zahlreich bekannt.

"Calcium-sensitive Indikatoren" sind im Sinne der Erfindung Stoffe, die in Anwesenheit von Calcium derart ihre physikalischen, optischen oder katalytischen Eigenschaften ändern, dass diese ein detektierbares Signal hervorrufen. Dem Fachmann bekannte Calcium-sensitive Indikatoren sind z.B. das Aequorin und andere Calcium-sensitive Farbstoffe wie z.B. FURA-2.

"Radioaktive Elemente" im Sinne der Erfindung erzeugen z.B. Gammastrahlung, wie z.B. folgende Isotope ¹²⁴J, ¹²⁵J, ¹²⁸J, ¹³¹J, ¹³²J oder ⁵¹Cr, wobei besonders das ¹²⁵J bevorzugt sein soll. Andere, wie z.B. ¹¹C, ¹⁸F, ¹⁵O oder ¹³N, können mittels ihrer Positronenstrahlung und entsprechender Detektoren (Positronen-Emissions-Tomographie) nachgewiesen werden und andere, wie z.B. ¹¹¹In, lassen sich mittels Elektroneneinfang ("electron capture") nachweisen.

"NMR-Label" im Sinne der Erfindung sind Substanzen, in denen Atome mit ungerader Nukleonenanzahl (Summe der Protonen und Neutronen) enthalten sind. Solche Atomkerne, z.B. ¹³C, ¹⁵N oder ¹⁹F, besitzen einen Kernspin und damit ein kernmagnetisches Moment.

"Elektronenspinlabel" dienen im Sinne der Erfindung der Messung der "Electron Paramagnetic Resonance" mittels Elektronenspinresonanz. Dabei wird die resonante Mikrowellenabsorption einer Probe in einem äußeren Magnetfeld gemessen. Damit lassen sich Moleküle nachweisen, die über ein permanentes magnetisches Moment (ungepaarte Elektronen) verfügen (Physics in Medicine & Biology. 43(1998)U 3-U 4, Clinical Chemistry & Laboratory Medicine. 46(2008)1203-1210).

Die Verwendung von Indikatoren ist besonders vorteilhaft, wenn die Indikator-gekoppelte Verbindung der voranstehenden Formel I in einem Diagnoseverfahren eingesetzt werden soll (z.B. Anamneseerhebung, körperliche Untersuchung, Anwendung bildgebender Verfahren wie Röntgen/MRT oder Analytik mit Laborwerten des Bluts und anderen Körperflüssigkeiten). Enthalten die erfindungsgemäßen Verbindungen der Formel I noch einen oder mehrere Indikatoren, kann der Verteilungsraum des Wirkstoffs anhand dieser Indikatoren erkannt werden. Indikatoren können überdies genutzt werden, um den Wirkstoff zu quantifizieren.

Eine solche - beispielhaft erwähnte - erfindungsgemäße Indikator-gekoppelte Verbindung ist beispielsweise die folgende Verbindung 69: 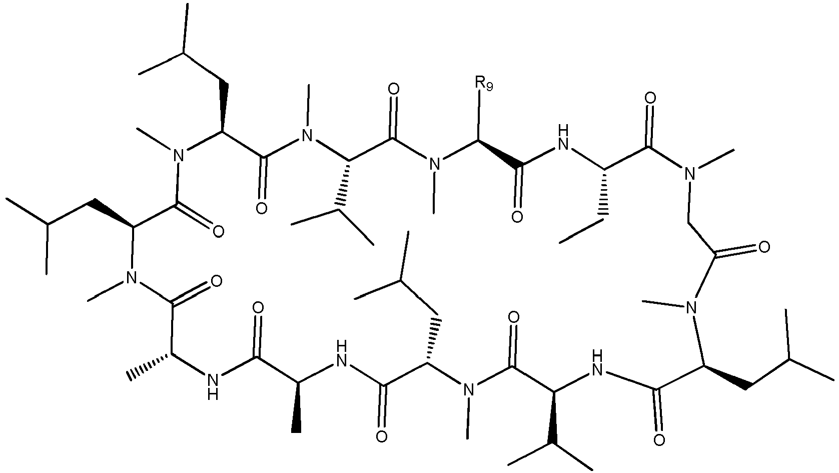 wobei R₉ folgender Struktur entspricht:

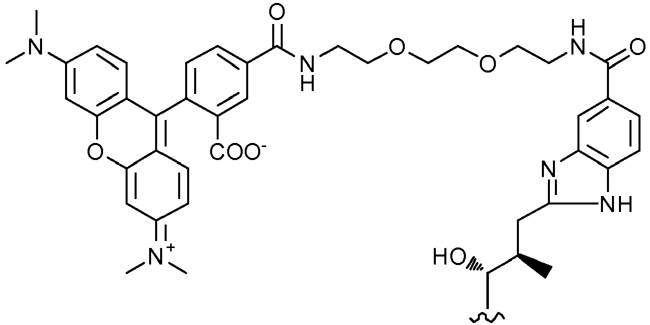

Eine weitere Ausführungsform der vorliegenden Erfindung betrifft eine Verbindung nach der voranstehenden Formel I oder die erfindungsgemäße Indikator-gekoppelte Verbindung zur Verwendung in der Medizin. In anderen Worten wird eine Verbindung der Formel I als pharmazeutisch-aktive Substanz bzw. als Arzneimittel, die erfindungsgemäße Indikator-gekoppelte Verbindung als Diagnosemittel in der Medizin eingesetzt. Die erfindungsgemäße Verbindung kann dabei zur Herstellung jedes Arzneimittels verwendet werden, das dem Fachmann als für die erfindungsgemäße Verbindung geeignet erscheint. Bevorzugt wird die erfindungsgemäße Verbindung zur Herstellung eines nicht-immunosuppressiv wirkenden Arzneimittels verwendet. Arzneimittel werden als nicht-immunosuppressiv verstanden, wenn sie die Funktionen des Immunsystems nicht vermindern.

Das Arzneimittel bzw. die erfindungsgemäße Verbindung (im folgenden nur Arzneimittel genannt) kann dabei in jeder Form verabreicht werden, die dem Fachmann als für den beabsichtigten Zweck geeignet bekannt ist. Beispielsweise kann das Arzneimittel in einer Form, ausgewählt aus der Gruppe bestehend aus Injektionen, Infusionen, Tabletten, Cremes, Gels, Salben, Sprays, Kapseln, Sirupen, Emulsionen, Pudern, Pulvern, Zäpfchen oder Ähnlichen eingesetzt werden. Besonders bevorzugt ist dabei, dass das Arzneimittel in Form von Sprays, Salben, Injektionen oder Tabletten eingesetzt wird. Hierbei werden oft Zusatzmittel benötigt, die das Arzneimittel in eine verabreichbare Darreichungsform bringen.

Deshalb betrifft eine weitere Ausführungsform der vorliegenden Erfindung eine pharmazeutische Zusammensetzung, die eine Verbindung nach der voranstehenden Formel I und vorzugsweise ein oder mehrere Zusatzmittel enthält.

Die Wahl des Zusatzmittels hängt hierbei besonders von der Art der Verabreichungsform ab. Die Zusatzmittel sind vorzugsweise solche, die physiologisch verträglich sind und selbst nicht pharmazeutisch aktiv sind.

Bei der pharmazeutischen Zusammensetzung kann es sich um jede pharmazeutische Zusammensetzung handeln, die dem Fachmann als geeignet bekannt ist. In einer bevorzugten Ausführungsform handelt es sich bei der pharmazeutischen Zusammensetzung um Sprays, Salben, Injektionen oder Tabletten.

Anwendungsbereich der Verbindungen der voranstehenden Formel I, der erfindungsgemäßen pharmazeutischen Zusammensetzung und der erfindungsgemäßen Indikator-gekoppelten Verbindung können Therapie und Diagnose von Krankheiten aber auch kosmetischer Art sein. Unter Therapie wird in erster Linie die Therapie von Krankheiten von Menschen und Tieren verstanden.

Besondere Vorteile der Verbindung nach der voranstehenden Formel I, der erfindungsgemäßen pharmazeutischen Zusammensetzung und der erfindungsgemäßen Indikator-gekoppelten Verbindung liegen dabei in der Tier- und Humanmedizin, bei der Applikation von Stoffen auf oder in Zellsuspensionen, Gewebekulturen, Transplantaten oder dem gesamten Säugetier.

Die vorliegende Erfindung betrifft des Weiteren die Verwendung von Verbindungen der voranstehenden Formel I bzw. der erfindungsgemäßen pharmazeutischen Zusammensetzung, insbesondere der erfindungsgemäßen Indikator-gekoppelten Verbindungen als Mittel zur Diagnosefindung.

In einer weiteren Ausführungsform betrifft die vorliegende Erfindung eine Verbindung nach der voranstehenden Formel I oder eine erfindungsgemäße pharmazeutische Zusammensetzung zur Behandlung von folgenden Erkrankungen:
a) virale Infektionen
b) akute und chronische entzündliche Erkrankungen
c) Krebs
d) degenerative Muskelerkrankungen
e) neurodegenerative Erkrankungen, und
f) Schädigungen, welche mit Beeinträchtigung der Calcium-Homöostase einhergehen.

In anderen Worten betrifft die vorliegende Erfindung auch die Verwendung einer Verbindung der voranstehenden Formel I oder der erfindungsgemäßen pharmazeutischen Zusammensetzung zur Herstellung eines Medikaments zur Behandlung der oben unter a) bis f) genannten Erkrankungen. In noch anderen Worten betrifft die vorliegende Erfindung ein Verfahren zur Behandlung einer der unter a) bis f) genannten Erkrankung in einem behandlungsbedürftigen Individuum umfassend die Verabreichung einer therapeutisch wirksamen Menge eines Arzneimittels umfassend/bestehend aus eine(r) Verbindung nach der voranstehenden Formel I oder die/der erfindungsgemäße(n) pharmazeutische(n) Zusammensetzung. Das zu behandelnde Individuum ist vorzugsweise ein Säugetier. Säugetiere können Menschen und Tiere sein.

In einer weiteren Ausführungsform betrifft die vorliegende Erfindung eine erfindungsgemäße Indikator-gekoppelte Verbindung zur Diagnose von folgenden Erkrankungen:
a) eine virale Infektionen
b) akute und chronische entzündliche Erkrankungen
c) Krebs
d) degenerative Muskelerkrankungen
e) neurodegenerative Erkrankungen, und
f) Schädigungen, welche mit Beeinträchtigung der Calcium-Homöostase einhergehen.

In anderen Worten betrifft die vorliegende Erfindung auch die Verwendung einer erfindungsgemäßen Indikator-gekoppelten Verbindung zur Herstellung eines Medikaments zur Diagnose der oben unter a) bis f) genannten Erkrankungen. In noch anderen Worten betrifft die vorliegende Erfindung ein Verfahren zur Diagnose einer der unter a) bis f) genannten Erkrankung in einem behandlungsbedürftigen Individuum umfassend die Verabreichung einer diagnostisch wirksamen Menge eines Arzneimittels umfassend/bestehend aus eine(r) erfindungsgemäßen Indikator-gekoppelten Verbindung. Das zu behandelnde Individuum ist vorzugsweise ein Säugetier. Säugetiere können Menschen und Tiere sein.

Die zuvor genannte virale Infektion wird erfindungsgemäß vorzugsweise durch Viren wie HIV, Hepatitis A, Hepatitis B, Hepatitis C, Hepatitis D und Hepatitis E ausgelöst.

Die zuvor genannten entzündlichen Erkrankungen schließt erfindungsgemäß vorzugsweise Asthma, chronische Entzündungen, chronische Prostatitis, Glomerulonephritis, multiple chemische Emfindlichkeit, entzündliche Darmerkrankungen, Sepsis, Entzündungen der vaskulären glatten Muskelzellen, Aneurysma, Entzündungen im Beckenbereich, Reperfusionsschäden, rheumatoide Arthritis und Vasculitis ein.

Unter der zuvor genannten Krebserkrankung versteht man erfindungsgemäß vorzugsweise - aber nicht abschließend - Lungenkrebs, Blasenkrebs, Leberkrebs, Pankreaskrebs und/oder Brustkrebs.

Die zuvor genannte degenerative Muskelerkrankung betrifft erfindungsgemäß vorzugsweise Muskeldystrophy, Kollagen IV-Myopathien und den myokardialen Reperfusionsschaden.

Die zuvor genannten neurodegenerativen Erkrankungen werden erfindungsgemäß vorzugsweise ausgewählt aus: der Alzheimer Erkrankung, Parkinson, Huntington, multiple systemische Atrophie, multiple Sklerose, zerebrale Kinderlähmung, Schlaganfall, diabetische Neuropathie, amyotrophe laterale Sklerose, Rückenmarkschäden und Zerebralsklerose.

Die zuvor genannte Erkrankung, die mit einem Verlust der zellulären Calcium-Homöostase einhergeht, betrifft erfindungsgemäß vorzugsweise Herzinfarkt, Schlaganfall, akute Hepatotoxizität, Cholestase und Reperfusionsschäden von transplantierten Organen.

Verbindungen der vorstehenden Formel I aus der vorliegenden Erfindung sind nicht-immunsuppressive, extrazellulär wirksame Inhibitoren der enzymatischen Aktivität von extrazellulären Cyclophilinen. Als solche sind die Verbindungen geeignet zur Behandlung und/oder Prävention von Cyclophilin-vermittelten akuten und chronischen Erkrankungen. Die Verbindungen sind bevorzugt aber nicht ausschließlich geeignet zur Behandlung oder Prävention von persistent oder chronisch entzündlichen Erkrankungen, neurogener Entzündung sowie entzündungsassoziierter Fibrose und Ödembildung. Dies beinhaltet, ist aber nicht beschränkt auf akut überschießende Entzündungsreaktionen (Verbrennungen, post-operative Entzündung), gastrointestinale Entzündung (Colitis, Morbus Crohn), Sepsis, Erkrankungen des respiratorischen Systems (Asthma, chronisch obstruktive Lungenerkrankung), entzündliche Vaskulopathien (Atherosklerose, Reperfusionsschaden), rheumatoide Arthritis, entzündliche Hauterkrankungen (Psoriasis, atopische Dermatitis), Augenerkrankungen (Keratokonjunktivitis) und entzündliche Erkrankungen des peripheren und zentralen Nervensystems (Parkinson, Schlaganfall, Multiple Sklerose).

In einem weiteren Aspekt betrifft die Erfindung ein Verfahren zur Anreicherung von Wirkstoffen in einem extrazellulären Raum eines multizellulären Objekts, umfassend die Schritte:
- Bereitstellen einer Verbindung nach der voranstehenden Formel I oder der erfindungsgemäßen Indikator-gekoppelten Verbindung;
- Inkontaktbringen einer der genannten Verbindungen mit einem multizellulären Objekt.

Insbesondere handelt es sich bei der Anreicherung in dem erfindungsgemäßen Verfahren um eine in-vitro-Anreicherung. Darunter versteht man, dass das multizelluläre Objekt ein vom lebenden Organismus abgetrenntes Objekt ist.

Unter einem "extrazellulären Raum" sollen all die Bereiche verstanden werden, welche sich außerhalb des Zytosols und der das Zytosol umschließenden Membran befinden. Dazu gehört auch die beispielsweise in Zellsuspensionen vorhandene Kulturlösung.

Bei dem multizellulären Objekt kann es sich um jedes Objekt handeln, das aus mindestens zwei gleichen oder unterschiedlichen biologischen Zellen besteht.

Der Begriff "biologische Zelle" umfasst dabei sowohl menschliche, tierische als auch pflanzliche und bakterielle Zellen sowie einzellige Lebewesen. Handelt es sich bei den biologischen Zellen um bakterielle Zellen oder einzellige Lebewesen, so wird unter dem Begriff "multizelluläres Objekt" eine Ansammlung mehrerer Zellen, wie beispielsweise eine Zellkolonie einer Bakterienkultur verstanden. Handelt es sich bei den biologischen Zellen um menschliche oder tierische Zellen, so wird unter dem Begriff "multizelluläres Objekt" ein separiertes Körperteil, wie beispielsweise ein Transplantat, insbesondere ein Organ-, ein Zell-, ein Gliedmaßen- oder ein Gewebetransplantat, Blut oder eine Blutfraktion, wie beispielsweise Blutplasma oder eine in-vitro Kultur menschlicher und/oder tierischer Zellen, wie beispielsweise eine zweidimensionale Gewebekultur oder eine Spheroidkultur der Zellen verstanden. Handelt es sich bei den biologischen Zellen um Pflanzenzellen, so wird unter dem Begriff "multizelluläres Objekt" ein Teil einer Pflanze, wie Beispielsweise Blätter, Wurzel oder Stängel oder auch eine ganze Pflanze verstanden.

Erfindungsgemäß handelt es sich bei dem multizellulären Objekt um ein separiertes Organ oder Körperteil, Blut oder eine Blutfraktion, eine Zellkultur oder eine Pflanze.

Desweiteren betrifft die vorliegende Erfindung auch die Verwendung einer Verbindung der unten abgebildeten Formel XIV - unter die die Verbindungen 1 bis 3 fallen - zur Herstellung einer Verbindung nach der voranstehenden Formel I oder der erfindungsgemäßen Indikator-gekoppelten Verbindung, die sich von Cyclosporin A ableiten. In anderen Worten betrifft die vorliegende Erfindung also auch ein Verfahren zur Herstellung einer solchen Verbindung nach der voranstehenden Formel I oder erfindungsgemäßen Indikator-gekoppelten Verbindung unter Verwendung einer Verbindung der Formel XIV.

Verbindung der Formel XIV: 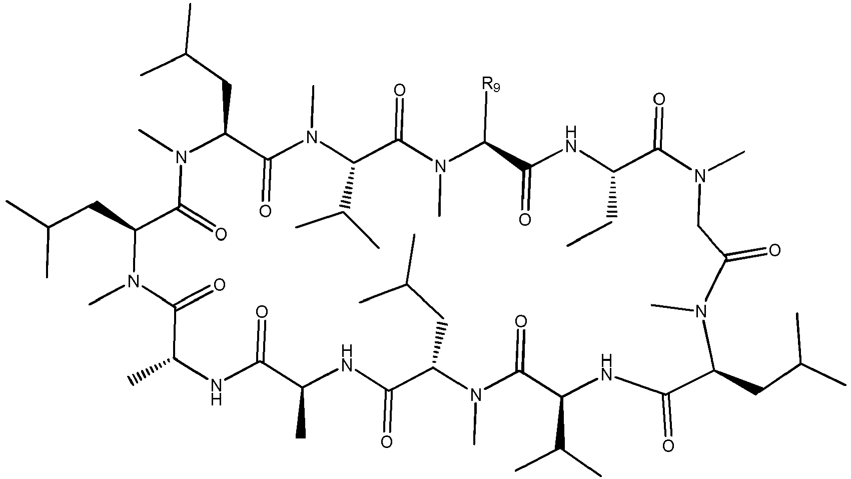 worin Rg einen Rest der folgenden Formel darstellt: 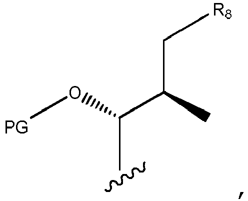 wobei R₈ für -CHO oder -COOH steht; und wobei PG eine Alkohol-Schutzgruppe darstellt. Die Schutzgruppe PG ist in der Lage, eine Hydroxylgruppe zu schützen, so dass diese bei Umwandlungen an anderen funktionellen Gruppen im Molekül unverändert bleibt oder die ungeschützte Hydroxylgruppe diese Umwandlungen nicht stört. Beispiele derartiger Schutzgruppen sowie Methoden zu ihrer Einführung und ihrer Abspaltung sind beschrieben in P.G.M.Wuts und T.W.Greene: "Protective Groups in Organic Synthesis", 4.Auflage, 2006; Kapitel "Schutz der Hydroxylgruppe". Bevorzugte Schutzgruppen sind
Ether, insbesondere substituierte Ether wie z.B. Methoxymethyl-, Methylthiomethyl-, Benzyloxymethyl-, tert-Butoxymethyl-, 2-Methoxyethoxymethyl-, 2-(Trimethylsilyl)ethoxymethyl-, 2,2,2-Trichlorethoxymethyl-, Tetrahydropyranyl-, Tetrahydrofuranyl-, 1-Ethoxyethyl-, 1-Methyl-1-methoxyethyl-, 1-Methyl-1-benzyloxyethyl-, 2,2,2-Trichlorethyl-, 2-(Trimethylsilyl)ethyl-, Allyl-, Benzyl-, 4-Methoxybenzyl-, 3,4-Dimethoxybenzyl-, Diphenylmethyl- oder Triphenylmethylether;
Silylether wie z.B. Trimethylsilyl-, Triethylsilyl-, Triisopropylsilyl-, tert-Butyldimethylsilyl-, tert-Butyldiphenylsilyl-, Triphenylsilyl- oder Diphenylmethylsilylether;
Ester wie z.B. Ester der Ameisen-, Essig-, Chloressig-, Dichloressig-, Trichloressig-, Trifluoressig-, Pivalin- oder Benzoesäure;
Carbonate wie z.B. Methyl-, 9-Fluorenylmethyl-, Ethyl-, 2,2,2-Trichlorethyl-, 2-(Trimethylsilyl)ethyl-, Isobutyl-, Allyl-, 2-Propenyl-, 4-Nitrophenyl-, Benzyl-, 4-Methoxybenzyl- oder 3,4-Dimethoxybenzylcarbonat.
Besonders bevorzugt sind Ether wie Methoxymethylether oder Tetrahydropyranylether, Silylether wie Trimethylsilyl- (TMS), tert-Butyldiphenylsilyl- (TBDPS) oder tert-Butyldimethylsilylether (TBDMS) oder eine Esterschutzgruppe wie Acetyl. Ganz besonders bevorzugt sind TBDMS oder Acetyl.

Derivate des Cyclosporin A der allgeneinen Formel I, welche Substituenten R₈ der Formel IV bis Formel XIII tragen, können aus einer Verbindung der Formel XIV mit geeigneten aromatischen, heteroaromatischen, aliphatischen oder heteroaliphatischen primären Aminen hergestellt werden, welche an einem benachbarten Kohlenstoffatom eine unsubstituierte oder substituierte Aminogruppe (X = NR₁), eine Hydroxylgruppe (X = O) oder eine Thiolgruppe (X = S) tragen. Vorzugsweise können die Aldehyde der Formel XIV (R₈ = -CHO) verwendet werden. Im Falle dieser Umsetzung von Aldehyden der Formel XIV mit den Aminen wird die Reaktion in einem inerten Lösungsmittel wie z.B. Ethanol, Methanol, Isopropanol, Tetrahydrofuran (THF), Dichlormethan, Chloroform, Dimethylformamid (DMF), N,N-Dimethylacetamid (DMA), Acetonitril oder Ethylylacetat, oder auch in Gemischen dieser Lösungsmittel, gegebenenfalls auch in Gegenwart von Wasser durchgeführt. Bevorzugte Lösungsmittel sind Methanol, Acetonitril oder DMF. Die Reaktion kann bei Raumtemperatur oder erhöhter Temperatur bis zum Sieden des Reaktionsgemisches erfolgen. Gegebenenfalls wird zur Vervollständigung der Reaktion ein Oxidationsmittel hinzugegeben. Beispiele für Oxidationsmittel sind Chinone wie 2,3-Dichlor-5,6-dicyano-1,4-benzochinon (DDQ); Diacetoxyiodbenzol; Benzofuroxan; Nitrobenzol; Dimethylsulfoxid (DMSO); Metallverbindungen mit Metallen erhöhter Oxidationsstufe wie z.B. Bariummanganat, Mangandioxid, Nickeloxid, Bleitetraacetat, Pyridiniumchlorochromat, Scandium(III)triflat, Ytterbium(III)triflat, Kupfer(II)triflat, Mangantriacetat; Halogene wie z.B. Iod; tert-Butylhypochlorid; Schwefelverbindungen höherer Oxidationsstufe wie Oxone, Natriumdisulfit, Natriumhydrogensulfit, Kaliumhydrogensulfat, Kaliummonopersulfat; N-Bromsuccinimid; N-Chlorsuccinimid; N-Iodsuccinimid oder Sauerstoff der Luft. Bevorzugte Oxidationsmittel sind N-Bromsuccinimid oder Luftsauerstoff. Die Umsetzung der Amine mit Carbonsäuren der Formel XIV (R₈ = - COOH) kann in Gegenwart starker Säuren erfolgen wie z.B. Polyphosphorsäure, vorzugsweise bei erhöhter Temperatur. In einer bevorzugten Variante wird die Umsetzung in zwei Stufen durchgeführt, wobei zunächst aus einer Carbonsäure der Formel XIV und dem Amin unter Abspaltung von Wasser ein Amid erhalten wird, aus dem in einem zweiten Schritt unter erneuter Abspaltung von Wasser die heterocyclische Verbindung der Formel I gebildet wird. Für die Bildung des Amids können Reagenzien verwendet werden, die dem Fachmann zur Knüpfung einer Amid-Bindung unter Bedingungen der Dehydratisierung bekannt sind, z.B. unter Verwendung von (Benzotriazol-1-yl)oxytripyrrolidinophosphonium Hexafluorphosphat (PyBOP) in Gegenwart einer Base wie z.B. Diisopropylethylamin (DIPEA). Der zweite Schritt kann beispielsweise erfolgen durch Erhitzen mit einem anorganischen Säurechlorid wie Thionylchlorid oder POCl₃, durch Erhitzen in Gegenwart einer Säure wie p-Toluolsulfonsäure in einem Lösungsmittel wie Toluol oder Xylol, durch Erhitzen in einer organischen Carbonsäure wie Essigsäure oder Propionsäure oder in Gegenwart von Wasser entziehenden Reagenzien wie z.B. Dicyclohexylcarbodiimid (DCC). Verbindungen der Formel I können auch in andere Verbindungen der Formel I überführt werden, indem Substituenten R₂, R₃, R₄, R₅, R₁₀ oder R₁₁, die an Gruppen R₈ der Formel IV bis Formel XIII gebunden sind, unter Verwendung von Standardreaktionen der organischen Synthese umgewandelt werden, die dem Fachmann bekannt sind. Beispielsweise können Nitrogruppen zu Aminogruppen reduziert werden. Aminogruppen können mit Sulfonylchloriden zu Sulfonamiden oder mit Acylchloriden oder anderen aktivierten Carbonsäure-Derivaten zu Amiden umgesetzt werden. Carbonsäuren R₂ = -COOH können mit Alkylhalogeniden in ihre Ester überführt werden. Ebenso kann unter ähnlichen Reaktionsbedingungen eine Verbindung der Formel I, in der X = NH, in eine Verbindung X = N(C1-C4-Alkyl) oder N-Benzyl überführt werden. Ferner können Carbonsäuren R₂ = -COOH mit primären oder mit sekundären Aminen unter Standardbedingungen der Amidkupplung in entsprechende Carbonsäureamide überführt werden.

Eine Ausführungsform (i) der vorliegenden Erfindung betrifft eine pharmazeutische Zusammensetzung enthaltend eine erfindungsgemäße Verbindung nach Formel I oder eine erfindungsgemäße Indikator-gekoppelte Verbindung.

Eine weitere Ausführungsform (ii) der vorliegenden Erfindung betrifft ein Verfahren zur Behandlung/Diagnose von/der folgenden Erkrankungen a) bis f) in einem behandlungsbedürftigen Individuum umfassend die Verabreichung einer therapeutisch wirksamen Menge eines Arzneimittels umfassend eine Verbindung nach Formel I oder einer erfindungsgemäßen pharmazeutischen Zusammensetzung nach Ausführungsform (i):
a) virale Infektionen
b) akute und chronische entzündliche Erkrankungen
c) Krebs
d) degenerative Muskelerkrankungen
e) neurodegenerative Erkrankungen,
f) Schädigungen, welche mit Beeinträchtigung der Calcium-Homöostase einhergehen, und

Eine weitere Ausführungsform (iii) der vorliegenden Erfindung betrifft ein Verfahren nach Ausführungsform (ii) zur Behandlung von viralen Infektionen, die durch Viren wie HIV, Hepatitis A, Hepatitis B, Hepatitis C, Hepatitis D und Hepatitis E ausgelöst werden, Asthma, chronischen Entzündungen, chronischer Prostatitis, Glomerulonephritis, multipler chemischer Emfindlichkeit, entzündlichen Darmerkrankungen, Sepsis, Entzündungen der vaskulären glatten Muskelzellen, Aneurysma, Entzündungen im Beckenbereich, Peritonitis, Reperfusionsschäden, rheumatoider Arthritis, Vasculitis, Lungenkrebs, Blasenkrebs, Leberkrebs, Pankreaskrebs, Bruskrebs, Muskeldystrophy, Kollagen IV-Myopathien, myokardialem Reperfusionsschaden, Alzheimer Erkrankung, Parkinson, Huntington, multipler systemischer Atrophie, multipler Sklerose, zerebraler Kinderlähmung, Schlaganfall, diabetischer Neuropathie, amyotrophe laterale Sklerose, Rückenmarksschäden, Zerebralsklerose, Herzinfarkt, Schlaganfall, akuter Hepatotoxizität, Cholestase, Reperfusionsschäden von transplantierten Organen, Asthma, Psoriasis, atopischer Dermatitis und ulzerativer Colitis.

Eine weitere Ausführungsform (iv) der vorliegenden Erfindung betrifft ein Verfahren zur Anreicherung von Wirkstoffen/Diagnosestoffen in einem extrazellulären Raum eines multizellulären Objekts, umfassend die Schritte:
- Bereitstellen einer erfindungsgemäßen Verbindung der Formel I oder einer erfindungsgemäßen Indikator-gekoppelten Verbindung;
- Inkontaktbringen einer der Verbindungen mit dem multizellulären Objekt.

Eine weitere Ausführungsform (v) der vorliegenden Erfindung betrifft ein Verfahren zur Herstellung eines erfindungsgemäßen Derivats von Cyclosporin A durch Umsetzung einer Verbindung der folgenden Formel 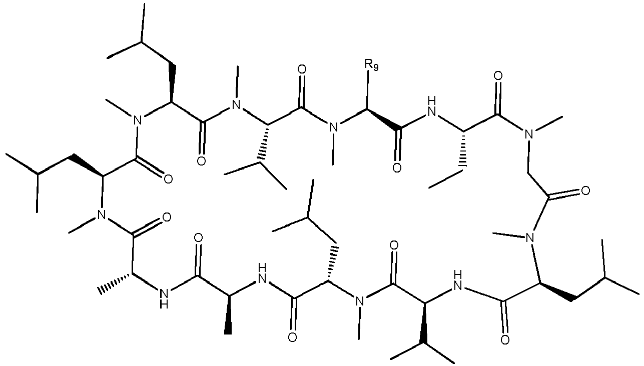 worin Rg einen Rest der folgenden Formel darstellt: 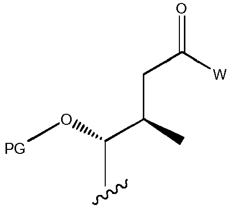 wobei PG eine Alkohol-Schutzgruppe und W gleich H oder OH darstellt und der jeweilige Rest über die Bindung, an deren Ende eine gewellte Linie eingezeichnet ist, verknüpft ist.

Eine weitere Ausführungsform (vi) der vorliegenden Erfindung betrifft ein Verfahren zur Herstellung einer Indikator-gekoppelten Verbindung durch Umsetzung einer Verbindung nach Formel I mit einer einen Indikator enthaltenden Verbindung.

### Figuren und Beispiele

Die vorliegende Erfindung soll nun anhand der folgenden Figuren und Beispiele näher beschrieben werden. Die Figuren und Beispiele haben dabei rein veranschaulichenden Charakter und sollen den Rahmen der vorliegenden Erfindung keinesfalls einschränken.

### Es zeigen

- **Fig. 1:**: Figur 1 zeigt zwei Diagramme, in denen die jeweilige Konzentration der Verbindungen 5 und 33 gegen die Prozent der Kontrolle aufgetragen ist. In anderen Worten zeigen die Diagramme in Figur 1 die Zellpermeabilität von Verbindungen 5 und 33 sowie von Cyclosporin A in Jurkat-Zellen.
- **Fig. 2:**: Überprüfung der immunsuppressiven Wirkung von Verbindung 5 im Vergleich zu Cyclosporin A im Proliferationsassay.
- **Fig. 3:**: Einfluß von 200 µg der Verbindung 5 auf die Anzahl der eosinophilen Granulozyten (Eosinophile) in der Bronchiallavage.
- **Fig. 4:**: Einfluß von 200 µg der Verbindung 5 auf die Anzahl der eosinophilen Granulozyten (Eosinophile) im Lungengewebe.
- **Fig. 5:**: Einfluß von der Verbindung 5 auf die Anzahl der CD4 positiven T-Zellen in der Bronchiallavage.
- **Fig. 6:**: Einfluß von der Verbindung 5 auf die Anzahl der CD4 positiven T-Zellen im Lungengewebe.

### Beispiele:

Die folgenden Abkürzungen werden in den Beispielen verwendet:
- CsA: Cyclosporin A
- DBU: 1,8-Diazabicyclo[5.4.0]undec-7-en
- DCM: Dichlormethan
- DIC: N,N'-Diisopropylcarbodiimid
- DIPEA: Diisopropylethylamin
- DMAP: 4-Dimethylamino)pyridin
- DMEM: Dulbecco's Modified Eagle Medium
- DMF: N,N-Dimethylformamid
- DMSO: Dimethylsulfoxid
- DTT: Dithiothreitol
- EDO: Ethylendioxid
- ES: Elektronenspray
- FACS: Fluorescence Activated Cell Sorter
- FCS: Fetal Calve Serum
- FITC: Fluoresceinisothiocyanat
- Fmoc: Fluorenylmethoxycarbonyl
- FSC: Forward Scatter
- HATU: 2-(7-Aza-1H-benzotriazol-1-yl)-1,1,3,3-tetramethyluronium Hexafluorophosphat
- HBSS: Hank's Buffered Salt Solution
- HOAc: Essigsäure
- HPLC: High-performance Liquid Chromatography
- MeOH: Methanol
- MS: Massenspektrum
- MTT: (Methylthiazol-2-yl)-diphenyltetrazolium Bromid
- NMP: N-Methylpyrrolidon
- OVA: Ovalbumin
- Pbf: 2,2,4,6,7-Pentamethyldihydrobenzofuran-5-sulfonyl
- PBS: Phosphate Buffered Saline
- PMA: Phorbol Myristate Acetate
- PyBOP: (Benzotriazol-1-yl)oxytripyrrolidinophosphonium Hexafluorphosphat
- SSC: Side Scatter
- TAMRA: Tetramethyl-6-Carboxyrhodamine
- TBDMS: tert-Butyldimethylsilyl
- TFA: Trifluoressigsäure
- THF: Tetrahydrofuran
- Trt: Trityl

### Beispiel 1: Synthese von Verbindung 1 (Acetyl-CsA-Aldehyd):

Zu einer Lösung von Acetyl-Cyclosporin A (3.0 g; 2.4 mmol) und Ruthenium(III)chloridhydrat (25 mg; 0.125 mmol) in einem Gemisch aus Acetonitril (30 ml) und Wasser (4 ml) wurde eine Lösung von Natriumperiodat (1.03 mg; 4.81 mmol) in Wasser (7 ml) vorsichtig tropfenweise zugegeben. Anschließend wurde die Mischung über Nacht bei Raumtemperatur gerührt. Danach wurde Ethylacetat (225 ml) zugegeben und mittels gesättigter Kochsalzlösung (3x110 ml) extrahiert. Die organische Phase wurde über MgSO₄ und anschließend im Vakuum getrocknet. Die Verbindung 1 konnten mittels Flash-Chromatographie (300 g Silicagel, 0.043-0.063 mm; Laufmittel: 0.1 % Essigsäure in Ethylacetat) von der als Nebenprodukt entstandenen Acetyl-CsA-Carbonsäure (Verbindung 3) getrennt werden. Es wurde eine Ausbeute von 1.76 g (60 %) für Verbindung 1 erhalten.

### Beispiel 2: Synthese von Verbindung 2 (TBDMS-CsA-Aldehyd):

### Stufe 1:

Eine Lösung von Cyclosporin A (4,0 g; 3,33 mmol) in trockenem Dichlormethan (20 ml) wurde unter einer Schutzgasatmosphäre von Stickstoff auf -20°C gekühlt. Anschließend wurden bei dieser Temperatur langsam nacheinander 2,6-Lutidin (1,43 g; 13,3 mmol) und tert.-Butyldimethylsilyl Trifluormethansulfonat (1,76 g; 6,66 mmol) hinzugegeben. Nach Rühren über Nacht bei Raumtemperatur wurde noch einmal die gleiche Menge von der Silylverbindung zugesetzt und weitere 3 Stunden gerührt. Das Lösungsmittel wurde im Vakuum entfernt, der Rückstand chromatographiert (Kieselgel; Dichlormethan / Methanol 100:0 - 90:10) und 4,2 g TBDMS-CsA erhalten.

### Stufe 2:

Zu einer Lösung von TBDMS-CsA (4,2 g; 3,19 mmol) in einem Gemisch aus Acetonitril (80 ml) und Wasser (10 ml) wurde Ruthenium(III)chloridhydrat (33 mg; 0,16 mmol) gegeben und anschließend langsam eine Lösung von Natriumperiodat (1,36 g; 6,36 mmol) in Wasser (30 ml) zugetropft. Nach Rühren über Nacht wurde filtriert, das Filtrat im Vakuum weitgehend eingeengt und mit Essigester versetzt. Die organische Phase wurde mit gesättigter NaCl-Lösung gewaschen, über Na₂SO₄ getrocknet und eingeengt. Der Rückstand wurde an Kieselgel chromatographiert (Cyclohexan/Essigester-Gradient) und dabei TBDMS-CsA-Aldehyd erhalten, der ohne weitere Reinigung für die folgenden Umsetzungen verwendet wurde. Ausbeute: 2,78 g (64 % über beide Stufen); MS (ES) C₆₆H₁₂₁N₁₁O₁₃Si berechnet 1304, gefunden 1305 (M+H)⁺.

### Beispiel 3: Synthese von Verbindung 3 (Acetyl-CsA-Carbonsäure):

Die Verbindung wurde nach einer Literaturvorschrift (Bioconjugate Chem 3 (1992), 32-36) durch Oxidation von Acetyl-Cyclosporin A mit Natriumperiodat / Kaliumpermanganat dargestellt.

### Beispiel 4: Synthese von Verbindung 4:

50 mg (0.041 mmol) Verbindung 1 und 11.25 mg cis-Diaminobiotin in 20 ml MeOH wurden für 1h unter Rückfluss erwärmt und anschließend über Nacht bei Raumtemperatur gerührt. Nach Evaporation des Methanols wurde der Rückstand in 10 ml DCM aufgenommen, mit 9 mg N-Bromsuccinimid versetzt und danach für eine Stunde gerührt. Das Acetyl-geschützte Produkt wurde anschließend mittels präparativer HPLC abgetrennt und lyophilisiert. Danach wurde die Acetyl-Schutzgruppe mit 3 ml 0.1M LiOH in 50% Tetrahydrofuran entfernt und das Endprodukt mittels präparativer HPLC isoliert. Ausbeute: 10 mg(18 %).

### Beispiel 5: Synthese von Verbindung 5:

### Methode A:

Eine Lösung von 100 mg (0.081mmol) Verbindung 1 und 190 mg (1.215 mmol, 15 eq) 3,4-Diaminobenzoesäure in MeOH (10 ml) wurde bis zum Reaktionsende (Kontrolle mittels analytischer HPLC) für 12-48 Stunden verrührt. Anschließend wurde filtiert und der Feststoff mit 10 ml MeOH gewaschen. 1.5 ml der methanolischen Lösung wurde für die nächste Stufe abgetrennt und der Rest des Filtrats im Vakuum eingeengt. Die Acetyl-Verbindung wurde anschließend mittels präparativer HPLC gereinigt (Säule RP C18 250x25mm; Gradient Wasser+0.05% TFA / Acetonitril+0.05% TFA) und nach Lyophilisieren als weißer Feststoff erhalten. Ausbeute: 70 mg (69 %).

Zu 1.5 ml der methanolischen Lösung der vorstehend beschriebenen Acetyl-Verbindung (∼6.1µmol) wurden 1.5 ml 0.2 M LiOH zugegeben und bis zum Ende der Hydrolyse verrührt (ca. 3 Stunden; Kontrolle mittels analytischer HPLC). Nach Ansäuern mit verdünnter Salzsäure wurde Verbindung 5 mittels präparativer HPLC gereinigt (Säule RP C18 250x25mm; Gradient Wasser+0.05% TFA / Acetonitril+0.05% TFA), lyophilisiert und als weißer Feststoff isoliert. Ausbeute: 7 mg (87 %).

### Methode B:

### Stufe 1:

Zu einer Lösung von TBDMS-CsA-Aldehyd (2,78 g; 2,13 mmol) in Methanol (10 ml) wurde ein Überschuss an 3,4-Diaminobenzoesäure (4,86 g; 31,9 mmol) gegeben und das Gemisch über Nacht unter Durchleiten eines schwachen Luftstroms gerührt. Es wurde filtriert, das Filtrat eingeengt und der Rückstand ohne weitere Reinigung in der nächsten Stufe eingesetzt. MS (ES) C₇₃H₁₂₅N₁₃O₁₄Si berechnet 1436, gefunden 1437 (M+H)⁺.

### Stufe 2:

Der Rückstand aus der vorherigen Stufe wurde mit einer 1M-Lösung von Tetrabutylammonium Fluorid in THF (15 ml; 15 mmol) versetzt und das Gemisch 2,5 Stunden gerührt bis die TBDMS-Verbindung nicht mehr nachweisbar war. Die Lösung wurde direkt ohne Entfernen des Lösungsmittels zur Auftrennung über RP-HPLC (Säule C18; Gradient H₂O (0,1% TFA) / MeOH (0,1% TFA)) verwendet und die Titelverbindung nach Gefriertrocknung als farbloser Feststoff erhalten. Ausbeute: 909 mg (32 % über beide Stufen); MS (ES) C₆₇H₁₁₁N₁₃O₁₄ berechnet 1322, gefunden 1323 (M+H)⁺.

### Beispiel 6: Synthese von Verbindung 6:

Ein Gemisch aus Acetyl-CsA-Carbonsäure (50 mg; 0,04 mmol), 3-Amino-4-hydroxybenzoesäure Methylester (10 mg; 0,06 mmol), (Benzotriazol-1-yl)oxytripyrrolidinophosphonium Hexafluorphosphat (PyBOP) (23 mg; 0,044 mmol) und DIPEA (21 µl) in DMF (3 ml) wurde über Nacht gerührt und anschließend die Zwischenstufe des Carbonsäureamids durch präparative HPLC gereinigt. Das Amid wurde in Toluol (10 ml) gelöst, mit Thionylchlorid (3 µl) versetzt und die Lösung für einige Stunden unter Rückfluss erhitzt. Es wurde nochmals Thionylchlorid (10 µl) hinzugegeben und nach Erhitzen für weitere 24 Stunden konnte ein Umsatz zum Benzoxazol von ca. 80% durch analytische HPLC nachgewiesen werden. Das Lösungsmittel wurde im Vakuum entfernt und der Rückstand mit MeOH (1 ml) und 0,2M NaOH (1 ml) versetzt. Das Gemisch wurde auf 5°C gekühlt und über Nacht gerührt. Es wurde mit 18%iger Salzsäure (100 µl) angesäuert und Verbindung 6 nach Reinigung über präparative HPLC isoliert.

### Beispiel 7: Synthese von Verbindung 7 und Verbindung 8:

### Methode A:

Eine Lösung von 10 mg (0.00732 mmol) der Verbindung 5 in trockenem THF wurde mit 17 mg CH₃I und 5-6 mg Benzyltriethylammoniumchlorid versetzt und über Nacht bei Raumtemperatur geschüttelt. Nach Zugabe von Wasser wurde die Lösung auf ca. pH 2-3 eingestellt und anschließend im Vakuum lyophilisiert. Es wurden 0.1M LiOH (4 ml) und MeOH (4 ml) hinzugegeben, zwei Stunden gerührt. Neben der Verbindung 7 wurde mittels präparativer HPLC auch die Verbindung 8 isoliert. Ausbeute: Verbindung 7: 3.5 mg (36 %) und Verbindung 8: 1 mg (9 %).

### Methode B:

Verbindung 7 wurde wie Verbindung 5 (Methode B)hergestellt, mit dem Unterschied, dass die folgenden Substanzen in den folgenden Mengen verwendet wurden: TBDMS-CsA-Aldehyd (300 mg; 0,23 mmol) und 3,4-Diaminobenzoesäure Methylester (38 mg; 0,23 mmol); Ausbeute: 52,3 mg (17 %), farbloser Feststoff; MS (ES) C₆₈H₁₁₃N₁₃O₁₄ berechnet 1336, gefunden 1337 (M+H)⁺.

### Beispiel 8: Synthese von Verbindung 9:

### Methode A:

Verbindung 9 wurde wie Verbindung 5 (Methode B) hergestellt, mit dem Unterschied, dass die folgenden Substanzen in den folgenden Mengen verwendet wurden: TBDMS-CsA-Aldehyd (300 mg; 0,23 mmol) und 3,4-Diaminobenzoesäure Ethylester (42 mg; 0,23 mmol); Ausbeute: 46 mg (15 %), farbloser Feststoff; MS (ES) C₆₉H₁₁₅N₁₃O₁₄ berechnet 1350, gefunden 1351 (M+H)⁺.

### Methode B:

25 mg der Verbindung 5 wurden in trockenem THF gelöst, mit 20 mg Ethylbromid, 8 mg K₂CO₃ und 5-6 mg Benzyltriethylammoniumchlorid versetzt und für 12-48 Stunden bis zum Reaktionsende geschüttelt (Kontrolle mittels analytischer HPLC). Danach wurde das Gemisch im Vakuum eingeengt und mittels präparativer HPLC getrennt. Die Acetylgruppe wurde innerhalb von 2h bei Raumtemperatur mittels 4 ml 0.1 M NaOH in 50% THF abgespalten und das Endprodukt mittels präparativer HPLC isoliert. Ausbeute: 10 mg (40 %).

### Beispiel 9: Synthese von Verbindung 10:

Verbindung 10 wurde wie Verbindung 9 in Beispiel 8 (Methode B) hergestellt, mit dem Unterschied, dass nicht Ethylbromid sondern 1-Brom-2-Ethylbutan als Alkylierungsreagenz eingesetzt wurde.

### Beispiel 10: Synthese von Verbindung 11:

Aus 25 mg Verbindung 5 und zugesetztem 1-Brombutan konnten mittels der im Beispiel 7 angegebenen Prozedur (Methode A) 7 mg der Verbindung 11 hergestellt werden. Ausbeute: 7 mg (28 %).

### Beispiel 11: Synthese von Verbindung 12:

Aus 25 mg Verbindung 5 und zugesetztem Isobutylbromid konnte mittels der im Beispiel 7 angegeben Prozedur (Methode A) 5 mg der Verbindung 12 hergestellt werden. Ausbeute: 5 mg (20 %).

### Beispiel 12: Synthese von Verbindung 13:

Das Dipeptid H-D-Glu(Ot-Bu)-D-Glu(Ot-Bu)-OH wurde über Standard Fluorenylmethoxycarbonyl(Fmoc)-Peptid-Synthese an ein 2-Chlor-tritylchlorid-Harz gebunden. Zu einer Lösung aus 13 mg der Verbindung 5 und 4 mg HATU in einem Gemisch aus DMF (1 ml) und DCM (1 ml) wurden nacheinander 152 mg des Dipeptid-Harzes und DIPEA (10 µl) hinzugegeben. Es wurde eine Stunde bei Raumtemperatur gerührt, danach der Feststoff abfiltriert und nacheinander mit DMF (3x) und DCM (3x) gewaschen. Verbindung 13 wurde durch Rühren mit 2 ml 100 % Trifluoressigsäure (TFA) über 2h bei 5 °C vom Harz abgespalten. Nach Entfernen der TFA im Vakuum wurde das Endprodukt mittels präparativer HPLC gereinigt. Ausbeute: 4 mg (27 %).

### Beispiel 13: Synthese von Verbindung 14:

Eine Lösung von Acetyl-CsA-Aldehyd (Verbindung 1; 20 mg) und 3-(3,4-Diamino-phenyl)-acrylsäure-tert-butylester (50 mg; 0,21 mmol) in MeOH (1 ml) wurde bis zum Reaktionsende (Kontrolle mittels analytischer HPLC) bei Raumtemperatur geschüttelt. Das Reaktionsgemisch wurde auf 5°C gekühlt, 0.2M NaOH (1 ml) zugegeben und das Reaktionsgemisch bei 5°C bis zum Ende der Hydrolyse geschüttelt (Kontrolle mittels analytischer HPLC). Danach wurde mit 18%iger Salzsäure (100 µl) angesäuert und das Produkt mittels präparativer HPLC gereinigt. Ausbeute: 5 mg (22.2 %).

### Beispiel 14: Synthese von Verbindung 15:

Zu ∼2.5 mg Verbindung 14 wurde Trifluoressigsäure(1 ml) gegeben. Das Gemisch wurde bei Raumtemperatur 30 min geschüttelt. Nach Abdampfen der TFA im Vakuum wurde das Produkt mittels präparativer HPLC gereinigt. Ausbeute: 0,3 mg (11.1 %).

### Beispiel 15: Synthese von Verbindung 16:

Eine Lösung von Acetyl-CsA-Aldehyd (Verbindung 1; 10 mg; 8 µmol) und 4,5-Diamino-2,6-dimercaptopyrimidin (6,9 mg; 40 µmol) in MeOH (1 ml) wurde bis zum Reaktionsende (Kontrolle mittels analytischer HPLC) bei Raumtemperatur geschüttelt. Hydrolyse des Acetats, Aufarbeitung des Reaktionsansatzes und Reinigung von Verbindung 16 wurden in der gleichen Weise durchgeführt wie für Verbindung 14 beschrieben. Ausbeute: 3.1 mg (28.8 %).

### Beispiel 16: Synthese von Verbindung 17:

Eine Lösung von Acetyl-CsA-Aldehyd (Verbindung 1; 10 mg; 8 µmol) und 4,5-Diamino-6-hydroxy-2-mercaptopyrimidin (3,2 mg; 18 µmol) in DMF (1 ml) wurde über Nacht bei einer Temperatur von 100 °C gerührt. Nach Entfernen des Lösungsmittels im Vakuum wurde die Hydrolyse des Acetats, die Aufarbeitung des Reaktionsansatzes und die Reinigung der Verbindung 17 in der gleichen Weise durchgeführt wie für Verbindung 14 beschrieben. Ausbeute 1.5 mg (14 %).

### Beispiel 17: Synthese von Verbindung 18:

Eine Lösung von Acetyl-CsA-Aldehyd (Verbindung 1; 10 mg; 8 µmol) und 5,6-Diamino-1H-pyrimidin-4-on Hemisulfat (7,3 mg; 21 µmol) in MeOH (1 ml) wurde bis zum Reaktionsende (Kontrolle mittels analytischer HPLC) bei Raumtemperatur geschüttelt. Hydrolyse des Acetats, Aufarbeitung des Reaktionsansatzes und Reinigung von Verbindung 18 wurden in der gleichen Weise durchgeführt wie für Verbindung 14 beschrieben. Ausbeute: 0.83 mg (8 %).

### Beispiel 18: Synthese von Verbindung 19:

Verbindung 19 wurde in der gleichen Weise hergestellt wie in Beispiel 17 beschrieben, mit dem Unterschied, dass als Diamin 3,4-Diaminobenzoesäurehydrazid (4.4 mg; 26 µmol) eingesetzt wurde. Ausbeute: 2.5 mg (23.4 %).

### Beispiel 19: Synthese von Verbindung 20:

Verbindung 20 wurde in der gleichen Weise hergestellt wie in Beispiel 17 beschrieben, mit dem Unterschied, dass als Diamin 2,3-Diamino-4,5-difluorbenzolsulfonsäure (3.4 mg; 15 µmol) eingesetzt wurde. Ausbeute: 2.5 mg (22.4 %).

### Beispiel 20: Synthese von Verbindung 21:

Verbindung 21 wurde in der gleichen Weise hergestellt wie in Beispiel 17 beschrieben, mit dem Unterschied, dass als Diamin 2,3-Diamino-5-fluorbenzoesäure (3.8 mg; 22 µmol) eingesetzt wurde. Ausbeute: 7.6 mg (70.9 %).

### Beispiel 21: Synthese von Verbindung 22:

### Methode A:

Verbindung 22 wurde wie Verbindung 5 (Methode B) hergestellt, mit dem Unterschied, dass die folgenden Substanzen in den folgenden Mengen verwendet wurden: TBDMS-CsA-Aldehyd (300 mg; 0,23 mmol) und 3,4-Diaminobenzolsulfonsäure (51,8 mg; 0,275 mmol); Ausbeute: 116,1 mg (37 %), farbloser Feststoff; MS (ES) C₆₆H₁₁₁N₁₃O₁₅S berechnet 1358, gefunden 1359 (M+H)⁺.

### Methode B:

Eine Lösung von 10 mg (8 µmol) von Verbindung 1 und 4.9 mg (26 µmol) 3,4-Diaminobenzolsulfonsäure wurde in 1 ml MeOH bis zum Reaktionsende (Kontrolle mittels analytischer HPLC) bei Raumtemperatur geschüttelt. Das Reaktionsgemisch wurde auf 5°C abgekühlt und zur Abspaltung der Acetyl-Schutzgruppe 1.0 ml 0.2M NaOH zugegeben. Das Reaktionsgemisch wurde bei 5°C bis zum Ende der Hydrolyse geschüttelt (Kontrolle mittels analytischer HPLC). Danach wurden 100 ml 18% HCl zugegeben und das Produkt mittels präparativer HPLC gereinigt. Ausbeute: 2.3 mg (21.1 %).

### Beispiel 22: Synthese von Verbindung 23:

### Methode A:

3,4-Diaminobenzamid wurde nach Literaturvorschrift erhalten (J. Med. Chem. 48 (2005), 1873-1885) und das Diamin (35 mg; 0,23 mmol) mit TBDMS-CsA-Aldehyd (300 mg; 0,30 mmol) umgesetzt in der gleichen Weise wie für Verbindung 5 (Methode B) beschrieben. Ausbeute: 43,7 mg (14 %), bräunlicher Feststoff; MS (ES) C₆₇H₁₁₂N₁₄O₁₃ berechnet 1321, gefunden 1322 (M+H)⁺.

### Methode B:

Verbindung 23 wurde in der gleichen Weise hergestellt wie in Beispiel 17 beschrieben, mit dem Unterschied, dass als Diamin 3,4-Diaminobenzonitril (2.3 mg; 17 µmol) eingesetzt wurde. Ausbeute: 0.4 mg (3.8 %).

### Beispiel 23: Synthese von Verbindung 24:

Eine Lösung von Acetyl-CsA-Aldehyd (Verbindung 1; 10 mg; 8 µmol) und 5,6-Diaminouracil Sulfat (3.2 mg; 14 µmol) in DMF (1 ml) wurde über Nacht bei einer Temperatur von 100 °C gerührt. Nach Entfernen des Lösungsmittels im Vakuum wurde die Hydrolyse des Acetats, die Aufarbeitung des Reaktionsansatzes und die Reinigung der Verbindung 24 in der gleichen Weise durchgeführt wie für Verbindung 14 beschrieben. Ausbeute: 1.7 mg (16.2 %).

### Beispiel 24: Synthese von Verbindung 25:

Verbindung 25 wurde wie Verbindung 5 (Methode B) hergestellt, mit dem Unterschied, dass die folgenden Substanzen in den folgenden Mengen verwendet wurden: 3,4-Diaminobenzoesäure Isopropylester wurde nach Literaturvorschrift (US2007/0032525) hergestellt und der Ester (45 mg; 0,23 mmol) mit TBDMS-CsA-Aldehyd (300 mg; 0,23 mmol) umgesetzt; Ausbeute: 99,1 mg (32 %), farbloser Feststoff; MS (ES) C₇₀H₁₁₇N₁₃O₁₄ berechnet 1364, gefunden 1365 (M+H)⁺.

### Beispiel 25: Synthese von Verbindung 26:

### Stufe 1:

Zu 3,4-Diaminobenzoesäure (300 mg; 1,97 mmol) wurden nacheinander konz. Schwefelsäure (2 ml) und 2-(Dimethylamino)ethanol (176 mg; 1,97 mmol) gegeben. Das Gemisch wurde 1 Stunde bei Raumtemperatur verrührt, anschließend auf Eis gegeben und mit 20%iger Natronlauge auf pH14 gebracht. Es wurde mit Essigester extrahiert, die organische Phase über MgSO₄ getrocknet, zur Trockene eingeengt und als Rückstand 3,4-Diaminobenzoesäure 2-(Dimethylamino)ethylester erhalten (154 mg; 35 %), der ohne weitere Reinigung in der nächsten Stufe eingesetzt wurde.

### Stufe 2:

Die Verbindung 26 wurde in der gleichen Weise wie Verbindung 5 (Methode B) aus TBDMS-CsA-Aldehyd (300 mg; 0,23 mmol) und 3,4-Diaminobenzoesäure 2-(Dimethylamino)ethylester (52 mg; 0,23 mmol) hergestellt. Ausbeute: 4,3 mg (1,3 %), farbloser Feststoff; MS (ES) C₇₁H₁₂₀N₁₄O₁₄ berechnet 1393, gefunden 1394 (M+H)⁺.

### Beispiel 26: Synthese von Verbindung 27:

### Stufe 1:

3,4-Diaminobenzoesäure 2-(Morpholin-4-yl)ethylester wurde aus 3,4-Diaminobenzoesäure (300 mg; 1,97 mmol) und 2-(Morpholin-4-yl)ethanol (258 mg; 1,97 mmol) hergestellt wie unter Stufe 1 des Beispiels 25 beschrieben. Ausbeute: 61 mg (12 %).

### Stufe 2:

Die Verbindung 27 wurde in der gleichen Weise wie Verbindung 5 (Methode B) aus TBDMS-CsA-Aldehyd (300 mg; 0,23 mmol) und 3,4-Diaminobenzoesäure 2-(Morpholin-4-yl)ethylester (61 mg; 0,23 mmol) hergestellt. Ausbeute: 8,5 mg (2,6 %), farbloser Feststoff; MS (ES) C₇₃H₁₂₂N₁₄O₁₅ berechnet 1435, gefunden 1436 (M+H)⁺.

### Beispiel 27: Synthese von Verbindung 28:

Verbindung 28 wurde wie Verbindung 5 (Methode B) hergestellt, mit dem Unterschied, dass die folgenden Substanzen in den folgenden Mengen verwendet wurden: TBDMS-CsA-Aldehyd (300 mg; 0,23 mmol) und 2,3-Diaminobenzoesäure (35 mg; 0,23 mmol); Ausbeute: 65 mg (21 %), farbloser Feststoff; MS (ES) C₆₇H₁₁₁N₁₃O₁₄ berechnet 1322, gefunden 1323 (M+H)⁺.

### Beispiel 28: Synthese von Verbindung 29:

Verbindung 29 wurde wie Verbindung 5 (Methode B) hergestellt, mit dem Unterschied, dass die folgenden Substanzen in den folgenden Mengen verwendet wurden: TBDMS-CsA-Aldehyd (300 mg; 0,23 mmol) und 2,3-Diaminobenzoesäure Methylester (38 mg; 0,23 mmol); Ausbeute: 50,8 mg (17 %), farbloser Feststoff; MS (ES) C₆₈H₁₁₃N₁₃O₁₄ berechnet 1336, gefunden 1337 (M+H)⁺.

### Beispiel 29: Synthese von Verbindung 30:

3,4-Diaminophthalsäure Dimethylester wurde nach Literaturvorschrift hergestellt (J. Heterocyclic Chem. 10 (1973), 891-898) und der Ester (30 mg; 0,13 mmol) mit TBDMS-CsA-Aldehyd (175 mg; 0,13 mmol) in der gleichen Weise umgesetzt wie für Verbindung 5 beschrieben (Methode B). Ausbeute: 38,6 mg (21 %), farbloser Feststoff; MS (ES) C₇₀H₁₁₅N₁₃O₁₆ berechnet 1394, gefunden 1395 (M+H)⁺.

### Beispiel 30: Synthese von Verbindung 31:

### Stufe 1:

Eine ethanolische Lösung von Na-Ethanolat, hergestellt aus Natrium (181 mg, 7,87 mmol) in Ethanol (5 ml), wurde zu einer Suspension von 4,5-Diaminophthalsäure Dimethylester (820 mg; 3,66 mmol) in Ethanol (3,5 ml) und Wasser (0,15 ml) gegeben und das Gemisch 2 Stunden unter Rückfluss erhitzt. Nach Abkühlen wurde der Feststoff abfiltriert, in Wasser (7 ml) gelöst und die Lösung mit 1M HCl neutralisiert. Es wurde lyophilisiert und der Rückstand mit Methanol (50 ml) verrührt. Nach erneuter Filtration wurde das Filtrat im Vakuum eingeengt und 4,5-Diaminophthalsäure aus dem Rückstand nach RP-HPLC (Säule C18; Gradient H₂O / MeOH 95:5) und Gefriertrocknung erhalten. Ausbeute: 553 mg (77 %).

### Stufe 2:

Die Verbindung 31 wurde in der gleichen Weise wie Verbindung 5 (Methode B) aus TBDMS-CsA-Aldehyd (300 mg; 0,23 mmol) und 4,5-Diaminophthalsäure (45 mg; 0,23 mmol) hergestellt. Ausbeute: 16,5 mg (5,3 %), farbloser Feststoff; MS (ES) C₆₈H₁₁₁N₁₃O₁₆ berechnet 1366, gefunden 1367 (M+H)⁺.

### Beispiel 31: Synthese von Verbindung 32:

4,5-Diaminophthalsäure Dimethylester wurde nach Literaturvorschrift hergestellt (J. Heterocyclic Chem. 10 (1973), 891-898) und der Ester (51 mg; 0,23 mmol) mit TBDMS-CsA-Aldehyd (300 mg; 0,23 mmol) in der gleichen Weise umgesetzt wie für Verbindung 5 (Methode B) beschrieben. Ausbeute: 63,9 mg (20 %), farbloser Feststoff; MS (ES) C₇₀H₁₁₅N₁₃O₁₆ berechnet 1394, gefunden 1395 (M+H)⁺.

### Beispiel 32: Synthese von Verbindung 33:

5-(3,4-Diaminophenyl)tetrazol Dihydrochlorid wurde nach Literaturvorschrift hergestellt (EP1944311) und das Tetrazol (57 mg; 0,23 mmol) mit TBDMS-CsA-Aldehyd (300 mg; 0,23 mmol) in der gleichen Weise umgesetzt wie für Verbindung 5 (Methode B) beschrieben. Ausbeute: 32,9 mg (11 %), farbloser Feststoff; MS (ES) C₆₇H₁₁₁N₁₇O₁₂ berechnet 1346, gefunden 1346 (M⁺).

### Beispiel 33: Synthese von Verbindung 34:

Verbindung 34 wurde wie Verbindung 5 (Methode B) hergestellt, mit dem Unterschied, dass die folgenden Substanzen in den folgenden Mengen verwendet wurden: TBDMS-CsA-Aldehyd (174 mg; 0,13 mmol) und 3,4-Diaminobenzolsulfonsäureamid (25 mg; 0,13 mmol); Ausbeute: 14,5 mg (8 %), farbloser Feststoff; MS (ES) C₆₆H₁₁₂N₁₄O₁₄S berechnet 1357, gefunden 1358 (M+H)⁺.

### Beispiel 34: Synthese von Verbindung 35:

3,4-Diaminobenzamidin wurde nach Literaturvorschrift hergestellt (WO1999/24395) und das Amidin (35 mg; 0,23 mmol) mit TBDMS-CsA-Aldehyd (300 mg; 0,23 mmol) in der gleichen Weise um gesetzt wie für Verbindung 5 (Methode B) beschrieben. Ausbeute: 86,9 mg (29 %), farbloser Feststoff; MS (ES) C₆₇H₁₁₃N₁₅O₁₂ berechnet 1320, gefunden 1321 (M+H)⁺.

### Beispiel 35: Synthese von Verbindung 36:

3-Amino-4-(methylamino)benzoesäure wurde nach Literaturvorschrift hergestellt (WO2005/030704) und das Diamin (39 mg; 0,23 mmol) mit TBDMS-CsA-Aldehyd (300 mg; 0,23 mmol) in der gleichen Weise umgesetzt wie für Verbindung 5 (Methode B) beschrieben. Ausbeute: 87,7 mg (29 %), farbloser Feststoff; MS (ES) C₆₈H₁₁₃N₁₃O₁₄ berechnet 1336, gefunden 1337 (M+H)⁺.

### Beispiel 36: Synthese von Verbindung 37:

4-Amino-3-(methylamino)benzoesäure wurde nach Literaturvorschrift hergestellt (WO2000/020400) und das Diamin (39 mg; 0,23 mmol) mit TBDMS-CsA-Aldehyd (300 mg; 0,23 mmol) umgesetzt in der gleichen Weise wie für Verbindung 5 (Methode B) beschrieben. Ausbeute: 80,2 mg (26 %), farbloser Feststoff; MS (ES) C₆₈H₁₁₃N₁₃O₁₄ berechnet 1336, gefunden 1337 (M+H)⁺.

### Beispiel 37: Synthese von Verbindung 38:

N-(3,4-Diaminophenyl)trifluoracetamid wurde nach Literaturvorschrift hergestellt (WO2004/039318) und das Diamin (51 mg; 0,23 mmol) mit TBDMS-CsA-Aldehyd (300 mg; 0,23 mmol) umgesetzt in der gleichen Weise wie für Verbindung 5 (Methode B) beschrieben. Ausbeute: 23,7 mg (7,4 %), farbloser Feststoff; MS (ES) C₆₈H₁₁₁F₃N₁₄O₁₃ berechnet 1389, gefunden 1390 (M+H)⁺.

### Beispiel 38: Synthese von Verbindung 39:

Verbindung 39 wurde in der gleichen Weise hergestellt wie in Beispiel 17 beschrieben, mit dem Unterschied, dass als Diamin 3,3'-Diaminobenzidin Tetrahydrochlorid Dihydrat (18.3 mg; 46 µmol) eingesetzt wurde. Ausbeute: 4.5 mg (40.6 %).

### Beispiel 39: Synthese von Verbindung 40:

Verbindung 40 wurde in der gleichen Weise hergestellt wie in Beispiel 17 beschrieben, mit dem Unterschied, dass als Diamin 1,2,4,5-Tetraaminobenzol Tetrahydrochlorid (5.4 mg; 19 µmol) eingesetzt wurde. Ausbeute: 4.3 mg (41.1 %).

### Beispiel 40: Synthese von Verbindung 41:

Verbindung 41 wurde in der gleichen Weise hergestellt wie in Beispiel 17 beschrieben, mit dem Unterschied, dass als Diamin 4-(4-Methylpiperazin-1-yl)-1,2-diaminobenzol (4.1 mg; 20 µmol) eingesetzt wurde. Ausbeute: 2.3 mg (20.9 %).

### Beispiel 41: Synthese von Verbindung 42:

Eine Lösung von Acetyl-CsA-Aldehyd (25 mg; 20 µmol) und Ethylendiamin (1.5 µl) in Dichlormethan (5 ml) wurde 1 h bei Raumtemperatur und danach für 20 min bei 5°C gerührt. Anschließend wurde N-Bromsuccinimid (4 mg) hinzugegeben und über Nacht gerührt. Das Lösungsmittel wurde im Vakuum entfernt und der Rückstand mit Ethylacetat (30 ml) aufgenommen. Es wurde nacheinander mit gesättigter NaHCO₃- (2 x 10ml), 5%iger KHSO₄-(2 x 10ml) und gesättigter Kochsalz-Lösung (2 x 10ml) gewaschen. Nach Trocknen über MgSO₄ wurde das Lösungsmittel im Vakuum entfernt und anschließend der Rückstand mit einer Lösung von 0.1M NaOH (4 ml) in dem gleichen Volumen Tetrahydrofuran aufgenommen. Es wurde bis zur Komplettierung der Reaktion (Kontrolle mittels analytischer HPLC) für 2-5 Stunden gerührt. Das Produkt konnte mittels präparativer HPLC abgetrennt werden. Ausbeute: 7 mg(28 %).

### Beispiel 42: Synthese von Verbindung 43:

Acetyl-CsA-Carbonsäure (50 mg; 0.04 mmol), L-Serin Methylester Hydrochlorid (7 mg) und (Benzotriazol-1-yl)oxytripyrrolidinophosphonium Hexafluorphosphat (PyBOP) (23 mg; 0,044 mmol) wurden in DMF (3 ml) gelöst und anschließend mit DIPEA (21 µl) versetzt. Nach 50 min Rühren bei Raumtemperatur konnte das Zwischenprodukt mittels präparativer HPLC abgetrennt werden. Ausbeute: 27 mg (50 %).

27 mg des Amid-Zwischenprodukts wurden in Toluol (20 ml) gelöst und danach Triethylamin (20 µl) und Methansulfonylchlorid (10 µl) hinzugegeben. Nach Entfernen des Lösungsmittels im Vakuum wurde der Rückstand mit 0.2M NaOH (2 ml) und THF (2 ml) versetzt und das Gemisch bis zur Komplettierung der Reaktion gerührt (Kontrolle mittels analytischer HPLC). Das Endprodukt wurde durch präparative HPLC isoliert. Ausbeute: 7 mg (27 %).

### Beispiel 43: Synthese von Verbindung 44:

Verbindung 44 wurde in der gleichen Weise hergestellt wie in Beispiel 17 beschrieben, mit dem Unterschied, dass als Diamin (R,R)-(-)-trans-1,2-Diaminocyclohexan Hydrochlorid (3.3 mg; 22 µmol) eingesetzt wurde. Ausbeute: 5.8 mg (56.4 %).

### Beispiel 44: Synthese von Verbindung 45:

Eine Lösung von Acetyl-CsA-Aldehyd (11 mg; 8.8 µmol) und 5,6-Diamino-naphthalin-1-sulfonsäure (2.8 mg; 12 µmol) in DMF (1 ml) wurde bei Raumtemperatur über Nacht geschüttelt. Das Lösungsmittel wurde im Vakuum entfernt und zu dem Rest wurde MeOH (1 ml) und 0.2M NaOH (1 ml) gegeben. Das Reaktionsgemisch wurde bei 5°C über Nacht geschüttelt und das Produkt mittels präparativer HPLC gereinigt. Ausbeute: 8.0 mg (71.0 %).

### Beispiel 45: Synthese von Verbindung 46:

N-(3,4-Diaminophenyl)guanidin Hydrochlorid wurde nach Literaturvorschrift hergestellt (WO1998/045275) und das Diamin (62 mg; 0,307 mmol) mit TBDMS-CsA-Aldehyd (400 mg; 0,307 mmol) umgesetzt in der gleichen Weise wie für Verbindung 5 (Methode B) beschrieben. Ausbeute: 121,7 mg (30 %), farbloser Feststoff; MS (ES) C₆₇H₁₁₄N₁₆O₁₂ berechnet 1335, gefunden 1336 (M+H)⁺.

### Beispiel 46: Synthese von Verbindung 47:

### Stufe 1:

Eine Lösung von 2,4-Diaminonitrobenzol (8 g; 52,2 mmol) und DIPEA (9,9 g; 76,6 mmol) in DCM (300 ml) wurde auf 0°C gekühlt und bei dieser Temperatur Trifluormethansulfonsäureanhydrid (23 g; 81,5 mmol) innerhalb von 90 min zugetropft. Es wurde über Nacht gerührt, mit DCM verdünnt, mit gesättigter NaHCO₃-Lsg. gewaschen, über MgSO₄ getrocknet und im Vakuum eingeengt. Der Rückstand wurde mit Ether verrührt (3 x 400 ml). Die vereinigten überstehenden Lösungen wurden eingeengt und N-(4-Amino-3-nitrophenyl)trifluormethansulfonamid nach chromatographischer Reinigung an Kieselgel mit DCM erhalten; Ausbeute: 12,2 g (82 %).

### Stufe 2:

Zu einer Lösung der vorstehenden Nitroverbindung (12,2 g; 42,8 mmol) in MeOH (150 ml) wurde 10% Pd-C (1,2 g) gegeben. Das Gemisch wurde über Nacht unter H₂-Atmosphäre bei Raumtemperatur gerührt, die Feststoffe abfiltriert und mit MeOH gewaschen. Es wurde im Vakuum eingeengt, an Kieselgel chromatographiert (DCM/MeOH-Gradient) und N-(3,4-Diaminophenyl)-trifluormethansulfonamid erhalten; Ausbeute: 3,87 g (35 %).

### Stufe 3:

Verbindung 47 wurde aus N-(3,4-Diaminophenyl)-trifluormethansulfonamid (40 mg; 0,157 mmol) und TBDMS-CsA-Aldehyd (205 mg; 0,157 mmol) in der gleichen Weise erhalten wie für Verbindung 5 (Methode B) beschrieben. Ausbeute: 14 mg (6,3 %), rötlicher Feststoff; MS (ES) C₆₇H₁₁₁F₃N₁₄O₁₄S berechnet 1425, gefunden 1426 (M+H)⁺.

### Beispiel 47: Synthese von Verbindung 48:

### Stufe 1:

Eine Lösung von 1,2-Diamino-3-fluorbenzol (100 mg; 0,8 mmol) in 100%iger Schwefelsäure (5 ml) wird in einer Mikrowelle für 30 min auf 150°C erhitzt und der Ablauf der Reaktion über HPLC kontrolliert. Danach wird die Lösung in 200 g Eis eingerührt und mit 40%iger NaOH neutralisiert. Der gefriergetrocknete Rückstand wird in Acetonitril (100 ml) suspendiert. Nach Filtrieren und anschließendem Abziehen des Lösungsmittels wird die 3,4-Diamino-5-fluor-benzolsulfonsäure in Wasser (5 ml) gelöst und über eine Dowex Cl⁻ Säule gereinigt. Ausbeute: 148 mg (90 %).

### Stufe 2:

Eine Lösung von Ac-CsA-Aldehyd (Verbindung 1; 12 mg; ∼10 µmol) in DMF (3 ml) wird zu 3,4-Diamino-5-fluor-benzolsulfonsäure (3.6 mg; 17.5 µmol) in DMF (1 ml) gegeben. Nach Zugabe einer Lösung von Kaliummonopersulfat (6.1 mg; ∼10 µmol) in Wasser (0.1 ml) wird das Gemisch gerührt. Die Reaktion ist nach 3h beendet und das Produkt wird über eine präparative HPLC gereinigt. Ausbeute: 7 mg (49 %).
Zur gereinigten Fraktion wird MeOH (1 ml) und eiskalte 0.2M NaOH (1 ml) gegeben und die Mischung über Nacht bei 5°C geschüttelt. Danach wird angesäuert und der Rückstand über präparative HPLC gereinigt. Ausbeute: 4.9 mg (72 %).

### Beispiel 48: Synthese von Verbindung 49:

### Stufe 1: Synthese des Hexapeptids H-(L-Pro)₆-OH

Das Hexapeptid H-(L-Pro)₆-OH wurde über ein Standard-Protokoll zur Fmoc-Festphasen-Peptidsynthese an einem 2-Chlortritylchlorid-Harz hergestellt. In jedem Synthesezyklus wurde Fmoc-geschütztes L-Prolin mit PyBOP und DIPEA in DMF aktiviert und für 2 h gekuppelt. Die Fmoc-Schutzgruppe wurde mit einer 20%igen Lösung von Piperidin in DMF abgespalten und dabei für je einmal 5 min und einmal 15 min mit dem Amin gerührt. Nach dem letzten Kupplungsschritt wurde das Peptid vom Harz mit 100%iger TFA abgespalten und über präparative HPLC gereinigt.

### Stufe 2:

Zu einer Lösung der Verbindung 5 (10 mg; 7.6 µmol) und HATU (3.3 mg; 8.7 µmol) in NMP (1 ml) wurde DIPEA (5 µl; 29 µmol) gegeben. Das Gemisch wurde für 5 min geschüttelt und anschließend mit H-(L-Pro)₆-OH (10 mg; 16 µmol) versetzt. Nach Schütteln des Gemischs für 2h wurde das Produkt mittels präparativer HPLC gereinigt. Ausbeute: 8.7 mg (60%).

### Beispiel 49: Synthese von Verbindung 50:

### Stufe 1: Synthese des Hexapeptids H-(L-Ala)₆-OH

Das Hexapeptid wurde aus Fmoc-geschütztem L-Alanin analog dem Protokoll zur Herstellung von H-(L-Pro)₆-OH synthetisiert wie unter der Stufe 1 des Beispiels 48 beschrieben.

### Stufe 2:

Verbindung 50 wurde aus der Verbindung 5 (10 mg; 7.6 µmol) und H-(L-Ala)₆-OH (10 mg; 23 µmol) in der gleichen Weise erhalten wie unter Beispiel 48 (Stufe 2) beschrieben. Ausbeute: 8.1 mg (61%).

### Beispiel 50: Synthese von Verbindung 51:

### Stufe 1: Synthese des Hexapeptids H-(β-Ala)₆-OH

Das Hexapeptid wurde aus Fmoc-geschütztem β-Alanin analog dem Protokoll zur Herstellung von H-(L-Pro)₆-OH synthetisiert wie unter der Stufe 1 des Beispiels 48 beschrieben.

### Stufe 2:

Verbindung 51 wurde aus der Verbindung 5 (10 mg; 7.6 µmol) und H-(β-Ala)₆-OH (10 mg; 23 µmol) in der gleichen Weise erhalten wie unter Beispiel 48 (Stufe 2) beschrieben. Ausbeute: 4.2 mg (32 %).

### Beispiel 51: Synthese von Verbindung 52:

Zu einer Lösung von Verbindung 5 (10 mg; 7.6 µmol) und HATU (3.3 mg; 8.7 µmol) in NMP (1 ml) und wurde DIPEA (5 µl; 29 µmol) gegeben und das Gemisch 5 min geschüttelt. Nach Zugabe von 2-(N,N-Dimethylamino)ethylamin (2.3 µl; 21 µmol) wurde für weitere 2h geschüttelt und das Produkt anschließend mittels präparativer HPLC gereinigt. Ausbeute: 7.9 mg (75 %).

### Beispiel 52: Synthese von Verbindung 53:

Verbindung 53 wurde aus Verbindung 5 (10 mg; 7.6 µmol) und 4-(2-Aminoethyl)morpholin (2.1 µl; 16 µmol) nach der unter Beispiel 51 beschriebenen Methode erhalten. Ausbeute: 9.0 mg (83 %).

### Beispiel 53: Synthese von Verbindung 54:

### Stufe 1: N-(2-(Piperazin-1-yl)ethyl)-tritylamin

Eine Lösung von 1-(2-Aminoethyl)-piperazin (100 µl; 0.76 mmol) und Triethylamin (106 µl; 0.76 mmol) in DCM (20 ml) wurden auf 5°C gekühlt und mit Tritylchlorid (212 mg; 0.76 mmol) versetzt. Das Gemisch wurde bei 20°C über Nacht gerührt und nach Entfernen des Lösungsmittels das Produkt über eine präparative HPLC gereinigt. Ausbeute: 84 mg (30 %).

### Stufe 2: 4-[4-(2-Aminoethyl)piperazin-1-yl]-4-oxo-buttersäure

Zu einer Lösung von N-(2-(Piperazin-1-yl)ethyl)-tritylamin (55 mg; 0.15 mmol) in DMF (2 ml) wurden Bernsteinsäureanhydrid (30 mg; 0.30 mmol) und DIPEA (51 µl; 0.3 mmol) gegeben. Das Gemisch wurde für 2h gerührt und das Produkt 4-Oxo-4-{4-[2-(trityl-amino)ethyl]piperazin-1-yl}-buttersäure über präparative HPLC gereinigt. Nach Gefriertrocknung wurde zu diesem Produkt TFA (2 ml) gegeben und das Gemisch für 30 min gerührt. Die TFA wurde im Vakuum abdestilliert und der Rückstand mittels präparativer HPLC gereinigt. Ausbeute: 25 mg (36 %).

### Stufe 3:

Eine Lösung von Verbindung 5 (10 mg; 7.6 µmol), HATU (8 mg; 8.7 µmol) und DIPEA (10 µl; 29 µmol) in NMP (1 ml) wurde 5 min geschüttelt. Nach Zusatz von 4-[4-(2-Aminoethyl)piperazin-1-yl]-4-oxo-buttersäure (24 mg; 106 µmol) wurde für weitere 2h geschüttelt. Das Produkt wurde mittels präparativer HPLC gereinigt. Ausbeute: 18 mg (67 %).

### Beispiel 54: Synthese von Verbindung 55:

3-Amino-2-(methylamino)benzoesäure wurde nach Literaturvorschrift hergestellt (WO2008/008431) und die Säure (38 mg; 0,23 mmol) mit TBDMS-CsA-Aldehyd (300 mg; 0,23 mmol) umgesetzt in der gleichen Weise wie für Verbindung 5 (Methode B) beschrieben. Ausbeute: 60,3 mg (20 %), farbloser Feststoff; MS (ES) C₆₈H₁₁₃N₁₃O₁₄ berechnet 1336, gefunden 1337 (M+H)⁺.

### Beispiel 55: Synthese von Verbindung 56:

### Stufe 1:

3-(N-Acetyl-N-methyl-amino)-2-nitrobenzoesäure wurde nach Literaturvorschrift hergestellt (WO1998/24771) und die Säure (5,35 g; 22,46 mmol) zusammen mit 6N HCl (46 ml) 90 min in einem Bombenrohr auf 120°C erhitzt. Nach Abdestillieren des Lösungsmittels wurde in Methanol aufgenommen, auf Diatomeenerde aufgezogen, an Kieselgel chromatographiert (CHCl₃/MeOH 19:1 + 0,5% HOAc) und 3,4 g (77 %) 3-(Methylamino)-2-nitrobenzoesäure erhalten.

### Stufe 2:

Eine Lösung der vorstehend beschriebenen Nitroverbindung (3,35 g; 17,1 mmol) und Hydrazin Hydrat (2,5 ml; 51,2 mmol) in trockenem MeOH (100 ml) wurde vorsichtig portionsweise mit einer Suspension von Raney-Ni (3,4 g) in dem gleichen Lösungsmittel versetzt. Das Gemisch wurde 30 min bei 50°C gerührt, eingeengt, danach der Rückstand mit MeOH über Kieselgel filtriert und 1,24 g (44 %) 2-Amino-3-(methylamino)benzoesäure als dunkler Feststoff erhalten.

### Stufe 3:

Die Benzoesäure aus der vorstehenden Stufe (32 mg; 0,19 mmol) wurde mit TBDMS-CsA-Aldehyd (250 mg; 0,19 mmol) umgesetzt in der gleichen Weise wie für Verbindung 5 (Methode B) beschrieben. Ausbeute: 89 mg (35 %), brauner Feststoff; MS (ES) C₆₈H₁₁₃N₁₃O₁₄ berechnet 1336, gefunden 1337 (M+H)⁺.

### Beispiel 56: Synthese von Verbindung 57:

Das Dipeptid H-L-Glu(OCH₂Ph)-L-Glu(OCH₂Ph)-NH₂ wurde über Standard Fluorenylmethoxycarbonyl(Fmoc)-Peptid-Synthese an ein Rink-Amid-Harz gebunden (Aktivierung von Fmoc-L-Glu(OCH₂Ph)-OH mit PyBOP / DIPEA und Abspaltung der Fmoc-Gruppe mit 2% DBU in DMF und 2% Piperidin in DMF). Zu dem Harz wurde eine Lösung der Verbindung 5 (16 mg; 0,012 mmol), HATU (7 mg; 0,18 mmol) und DIPEA (6 µl) in DMF (2 ml) gegeben. Nach Schütteln über Nacht wurde das Reaktionsprodukt mit TFA/DCM 1:1 (2 ml) innerhalb von 1 h bei Raumtemperatur vom Harz abgespalten. Es wurde filtriert, Toluol (2 x 2 ml) hinzugegeben und das Lösungsmittel jeweils im Vakuum entfernt. Das Endprodukt wurde aus dem Rückstand mittels präparativer HPLC abgetrennt. Ausbeute: 6 mg (28 %).

### Beispiel 57: Synthese von Verbindung 58:

Verbindung 58 wurde nach der unter Beispiel 56 beschriebenen Methode aus Fmoc-L-Leu-OH, Fmoc-L-Arg(Pbf)-OH, Verbindung 5 (35 mg; 0,0265 mmol), HATU (11 mg; 0,029 mmol) und DIPEA (9 µl) erhalten. Nach Waschen des Harzes mit DMF und DCM wurde die Titelverbindung mit TFA abgespalten und aus dem Rückstand mittels präparativer HPLC isoliert. Ausbeute: 2 mg (5 %).

### Beispiel 58: Synthese von Verbindung 59:

Verbindung 59 wurde in der gleichen Weise wie unter Beispiel 57 beschrieben aus Fmoc-L-Pro-OH, Fmoc-L-Asn(Trt)-OH und Verbindung 5 (35 mg; 0,0265 mmol) erhalten. Ausbeute: 28 mg (69 %).

### Beispiel 59: Synthese von Verbindung 60:

Verbindung 60 wurde in der gleichen Weise wie unter Beispiel 57 beschrieben aus Fmoc-L-Cys(Trt)-OH, Fmoc-L-Ala-OH und Verbindung 5 (35 mg; 0,0265 mmol) hergestellt. Die Abspaltung der Endverbindung vom Harz erfolgte durch TFA (3 ml), welches DTT (100 mg) enthielt. Ausbeute: 12 mg (30 %).

### Beispiel 60: Synthese von Verbindung 61:

Verbindung 61 wurde in der gleichen Weise wie unter Beispiel 59 beschrieben aus Fmoc-L-Cys(Trt)-OH und Verbindung 5 (35 mg; 0,0265 mmol) hergestellt. Ausbeute: 4,4 mg (11 %).

### Beispiel 61: Synthese von Verbindung 62:

Verbindung 62 wurde in der gleichen Weise wie unter Beispiel 57 beschrieben aus Fmoc-L-Ser(t-Bu)-OH, Fmoc-L-Glu(Ot-Bu)-OH und Verbindung 5 (35 mg; 0,0265 mmol) hergestellt. Ausbeute: 11,6 mg (28 %).

### Beispiel 62: Synthese von Verbindung 63:

Verbindung 62 wurde in der gleichen Weise wie unter Beispiel 57 beschrieben aus Fmoc-L-Glu(Ot-Bu)-OH und Verbindung 5 (35 mg; 0,0265 mmol) hergestellt. Ausbeute: 13 mg (31 %).

### Beispiel 63: Synthese von Verbindung 64:

Zu einer Lösung von Verbindung 5 (10 mg; 7,6 µmol) in NMP (1 ml) wurden HATU (3,3 mg; 8,7 µmol) und DIPEA (5 µl) gegeben. Nachdem das Gemisch 2 Minuten verrührt worden war, wurde 1-(2-Aminoethyl)-piperazin (1,2 mg; 9,4 µmol) hinzugegeben und 3 Stunden geschüttelt. Verbindung 64 wurde durch präparative HPLC abgetrennt. Ausbeute: 3,7 mg (34 %).

### Beispiel 64: Synthese von Verbindung 65:

Zu einer Lösung von Verbindung 5 (10 mg; 7,6 µmol) in DCM (1 ml) wurden DIC (2 µl) und DMAP (1,4 mg; 11,4 µmol) gegeben. Es wurde 30 Minuten gerührt und das Lösungsmittel im Vakuum entfernt. Der Rückstand wurde mit einer Lösung von Salicyclsäure (2 mg; 14,5 µmol) in NMP (1 ml) versetzt, das Gemisch über Nacht geschüttelt und anschließend Verbindung 65 durch präparative HPLC isoliert. Ausbeute: 1,5 mg (13 %).

### Beispiel 65: Synthese von Verbindung 66:

### Stufe 1:

N-(Trityl)-2-(2-(2-aminoethoxy)ethoxy)ethylamin wurde nach einer Literaturvorschrift (Eur. J. Org. Chem. 2009, 3953-3963) dargestellt.

### Stufe 2:

Verbindung 5 (50 mg; 0,038 mmol) und PyBOP (19,7 mg; 0,038 mmol) wurden in NMP (3 ml) gelöst und nacheinander DIPEA (19,3 µl) und das Amin der Stufe 1 (17,7 mg; 0,045 mmol) hinzugegeben. Es wurde 1 Stunde gerührt, mit Essigester (50 ml) verdünnt und nacheinander jeweils zweimal mit 5%iger KHSO₄-, 5%iger NaHCO₃- und gesättigter Kochsalz-Lösung gewaschen. Die organische Phase wurde über MgSO₄ getrocknet, im Vakuum eingeengt und mit TFA (2 ml) versetzt. Nach 15 Minuten bei Raumtemperatur wurde eingeengt und anschließend Verbindung 66 durch präparative HPLC isoliert. Ausbeute: 29 mg (52 %).

### Beispiel 66: Synthese von Verbindung 67:

Zu einer Lösung von cis,cis-1,3,5-Cyclohexantricarbonsäure (22 mg; 0,1 mmol), Verbindung 66 (7,3 mg; 0,005 mmol) und PyBOP (3 mg; 0,0058 mmol) in NMP (1 ml) wurde DIPEA (10 µl) gegeben. Das Gemisch wurde 2 Stunden bei Raumtemperatur gerührt und Verbindung 67 anschließend mittels präparativer HPLC isoliert. Ausbeute: 5 mg (61 %).

### Beispiel 67: Synthese von Verbindung 68:

Zu einer Lösung von Bernsteinsäureanhydrid (20 mg; 0,2 mmol) und Verbindung 66 (7,3 mg; 0,005 mmol) in NMP (1 ml) wurde DIPEA (10 µl) gegeben und das Gemisch über Nacht bei Raumtemperatur gerührt. Verbindung 68 wurde anschließend mittels präparativer HPLC isoliert. Ausbeute: 2,9 mg (37 %).

### Beispiel 68: Herstellung von Verbindung 69 durch

Derivatisierung von Verbindung 5 mit einem Farbstoffmolekül:
a) Synthese von TAMRA-EDO (Indikator):
   Eine Lösung von 5(6)-Carboxytetramethylrhodamin (20 mg; 46,5 µmol) in DMF (5 ml) wurde mit 2-(7-Aza-1H-benzotriazole-1-yl)-1,1,3,3-tetramethyluronium Hexafluorophosphate (HATU) (19 mg; 8,3 µmol) versetzt und für 5 min zur Präaktivierung gerührt. Zu dieser Lösung wurde anschließend) 2,2-(Ethylenedioxy)ethyldiamin (68µl; 465 µmol) zugegeben. Es wurde für 2h gerührt und das Produkt mittels präparativer HPLC isoliert. Ausbeute: 15 mg (57.6%).
b) Synthese von Verbindung 69:
   Verbindung 5 (10 mg; 7.56 µmol) wurde in DMF (1 ml) gelöst und nach Zugabe von HATU (3,2 mg; 8.3 µmol) und DIPEA (3.9 µl; 22.68 µmol) zur Präaktivierung für 5 min gerührt. Danach wurde die Lösung zu TAMRA-EDO (8.5 mg; 15.12 µmol), gelöst in DMF (1 ml), gegeben. Es wurde für 2 h gerührt und das Produkt (Verbindung 69) mittels präparativer HPLC isoliert. Ausbeute: 7 mg (50%).

### Beispiel 69: Hemmung der Peptidyl-Prolyl-cis/trans-Isomerase (PPIase) Aktivität des Cyclophilins Cyp18wt

Die Bestimmung wurde an einem UV/Vis Spektrometer bei 10°C Küvettentemperatur durchgeführt, wobei das Gerät bei 390 nm im Kinetik-Modus betrieben wurde (Meßpuffer: 35 mM HEPES/NaOH pH 7.8 (AppliChem A1069)). In der Küvette befanden 0.58 nM rekombinantes humanes Wildtyp Cyp18 (Cyp18wt), 2.33 nM Rinderserumalbumin (BSA) und 1mg/ml Chymotrypsin (Merck), gelöst in 35 mM HEPES/NaOH pH 7.8, sowie gegebenenfalls unterschiedliche Konzentrationen der jeweiligen Testsubstanz. Die Reaktion wurde gestartet mit einer Stammlösung von Suc-Ala-Ala-Pro-Phe-4-nitroanilid (Bachem, 10 mg/ml DMSO) als Substrat, so dass eine finale Substratkonzentration von 32 µM resultierte. Die Extinktions-Zeit-Kurven wurden nach Ablauf der schnellen Phase der Reaktion nach ca. 60 sec nach einem Geschwindigkeitsgesetz 1. Ordnung ausgewertet. IC₅₀-Werte der Inhibitoren resultieren aus der Geschwindigkeitskonstanten 1. Ordnung der nicht-inhibierten Reaktion im Verhältnis zur inhibierten Reaktion (% Inhibition), gemessen in Abhängigkeit von der Inhibitorkonzentration in der Küvette. Die resultierenden IC₅₀-Werte der Testverbindungen sind in der Tabelle 1 aufgeführt.

### Beispiel 70: Hemmung der Phosphataseaktivität von Calcineurin

Die Bestimmung der dephosphorylierenden Calcineurinaktivität in Gegenwart und in Abwesenheit von Inhibitoren wurde in einem Scintillations-Proximity-Assay durchgeführt (siehe auch R. Baumgrass, et al.; J. Biol. Chem. 276 (2001), 47914-47921). Die Messung wurde durch Inkubation von 10 pMol des ³³P markierten, biotinylierten Phosphopeptids Asp-Leu-Asp-Val-Pro-Ile-Pro-Gly-Arg-Phe-Asp-Arg-Arg-Val-pSer-Val-Ala-Ala-Glu (MW = 2192.0) (RII-Peptid) in einem Probepuffer (40 mM Tris-HCl, pH 7.5, 100 mM NaCl, 6 mM MgCl₂, 0.5 mM Dithiothreitol, 1 mM CaCl₂, 0.1 mg/ml Rinderserumalbumin) in Gegenwart von 50 nM Calmodulin und 1.32 nM Calcineurin bei 30 °C für 20 min in einer 96-well Mikrotiterplatte (Costar, Bodenheim, Germany) durchgeführt. Das Gesamtvolumen des Assays/well betrug 100 µl. Nach dieser Reaktionszeit wurde ein 90 µl Aliquot der Reaktionsmischung in eine mit Streptavidin beschichtete Scintillationskavität überführt und für 20 min bei 22°C inkubiert. Nach einem Waschschritt wurde die RII Phosphopeptid-assoziierte ³³P Radioaktivität an einem MicroBeta Top Counter (Wallac) vermessen und die Enzymaktivität als Mittelwert von drei Messungen ± S.D. angegeben. Die uninhibierte Dephosphorylierungsaktivität wurde auf 100 % gesetzt. Die Calcineurin-Inhibition potentieller Cyclophilin-Inhibitor-Komplexe wurde in Gegenwart von 10nM bis 50 µM Cyp18 Konzentrationen bei konstanter Inhibitorkonzentration von 10-50 µM (typische 1 mM Stammlösungen in DMSO) gemessen. Die Ergebnisse für die Testverbindungen sind in der Tabelle 1 aufgeführt.

### Beispiel 71: Überprüfung der immunsuppressiven Wirkung im zellulären NFAT-Reporter-Gen Assay

Eine antigenspezifische Stimulation des T-Zell-Rezeptors bewirkt die Aktivierung von Transkriptionsfaktoren wie z.B. NFAT (nuclear factor of activated T cells). NFAT-Aktivierung ist essentiell für die Expression von Interleukin-2 und damit für die Proliferation von T-Zellen im Rahmen der Immunantwort. Eine stabile NFAT-Reporter-Gen Zelllinie ermöglicht die Analyse von Modulatoren des NFAT-Pathways und damit die Untersuchung der immunsuppressiven Wirkung von Verbindungen.

Die stabil transfizierte Reporter-Gen Zelllinie GloResponse NFAT-RE-luc2P HEK293 (Promega Corp. USA) wurde in DMEM (10% FCS; 2mM Glutamin; 200µg/ml Hygromycin) kultiviert und auf 96well-Platten (20.000 Zellen/well) ausgelegt. Die zu testenden Verbindungen wurden entweder in einer 2-Punkt-Bestimmung (5µM und 10µM) oder einer IC₅₀-Bestimmung (Konzentrationsreihe 4.1 nM - 1 µM) zu den Zellen gegeben. Als immunsuppressive Referenzverbindung wurde unmodifiziertes Cyclosporin A verwendet. Unmittelbar danach wurden die Zellen mit 5 nM PMA und 2 µM Ionomycin stimuliert und für 16h inkubiert (37°C; Zellinkubator). Diese Doppelstimulation simuliert eine T-Zellrezeptor-Stimulation und bewirkt eine NFAT-Aktivierung mit anschließender Expression des Luziferase-Reporter-Gens. Die Menge an Luziferase wurde nach 16h quantitativ durch Messung der Biolumineszenz mittels Bright-Glo Luziferase-Assay-System (Promega Corp., USA) bestimmt. Die gemittelten Luziferase-Werte der mit Vehikel (1% DMSO) behandelten und mit PMA/Ionomycin stimulierten Zellen wurden als Positivkontrolle und Referenzwert (100% Aktivität; 0% Inhibition) verwendet. Die Ergebnisse für die Testverbindungen sind in der Tabelle 1 aufgeführt.

In der folgenden Tabelle 1 sind die erfindungsgemäßen Verbindungen 4-68 im Vergleich zu Cyclosporin A bezügl. ihrer Aktivität zur Inhibierung von Cyp18 und Calcineurin sowie ihrer Aktivität im NFAT-Reporter-Gen Assay aufgelistet:

**Tabelle 1:**

| Verbindung | Inhibierung von Cyp18wt IC₅₀ [nM] | Indirekte Inhibierung von Calcineurin IC₅₀ [nM] | NFAT-Reporter-Gen Assay IC₅₀ [nM] |
|---|---|---|---|
| Cyclosporin A | ~8 | 85 | 31,4 |
| 4 | 264 | >10000 | |
| 5 | 9, 6 | >10000 | >10000 |
| 6 | 700 | | |
| 7 | 11,9 | 6900 | >10000 |
| 8 | 90,4 | | |
| 9 | 13,9 | 7000 | >10000 |
| 10 | 11,5 | 6900 | >10000 |
| 11 | 13,0 | 4700 | 10000 |
| 12 | 31,4 | | 2000 |
| 13 | 11,1 | >10000 | >10000 |
| 14 | 36,4 | 3200 | |
| 15 | 4,8 | >10000 | >10000 |
| 16 | 15,1 | 7500 | |
| 17 | >50 | >10000 | |
| 18 | 1,4 | 10000 | >10000 |
| 19 | 5,4 | 7000 | >10000 |
| 20 | 6,7 | >10000 | >10000 |
| 21 | 22,2 | >10000 | 5300 |
| 22 | 8,7 | >10000 | >10000 |
| 23 | 3,8 | >10000 | >10000 |
| 24 | 6,2 | >10000 | >10000 |
| 25 | 22,0 | 7900 | 10000 |
| 26 | 10,8 | 6200 | >10000 |
| 27 | 5, 9 | 6400 | 7400 |
| 28 | 60,0 | >10000 | >10000 |
| 29 | 29, 5 | >10000 | 10000 |
| 30 | 29,1 | 10000 | >10000 |
| 31 | 39,9 | 10000 | >10000 |
| 32 | 24,9 | >10000 | >10000 |
| 33 | 9,8 | >10000 | >10000 |
| 34 | 16,4 | 6400 | >10000 |
| 35 | 12,0 | 4800 | >10000 |
| 36 | 1000 | >10000 | >10000 |
| 37 | 61,5 | >10000 | >10000 |
| 38 | 14,3 | 3340 | 3800 |
| 39 | 6,1 | >10000 | 10000 |
| 40 | 5,5 | 8000 | >10000 |
| 41 | 3,0 | >10000 | >10000 |
| 42 | 57,7 | 9500 | 10000 |
| 43 | 45,3 | 10000 | 5300 |
| 44 | 9,2 | >10000 | >10000 |
| 45 | 3,2 | >10000 | >10000 |
| 46 | 13,1 | 6630 | >10000 |
| 47 | 13,2 | >10000 | >10000 |
| 48 | 8,7 | >10000 | |
| 49 | 30,9 | >10000 | |
| 50 | 7,3 | 10000 | |
| 51 | 7,3 | >10000 | |
| 52 | 8,0 | >10000 | |
| 53 | 12,7 | 4200 | |
| 54 | 7,1 | 7200 | |
| 55 | 209,3 | >10000 | |
| 56 | >1000 | >10000 | |
| 57 | 16,9 | | |
| 60 | ∼10 | | |
| 61 | ∼10 | | |
| 62 | 4,4 | | |
| 63 | 5, 1 | | |
| 64 | 17,2 | 10000 | |
| 65 | 20,2 | >10000 | |
| 66 | 15,0 | >10000 | |
| 67 | 8,4 | >10000 | |
| 68 | 8,5 | >10000 | |

### Beispiel 72: Bestimmung der Zellpermeabilität in Jurkat-Zellen

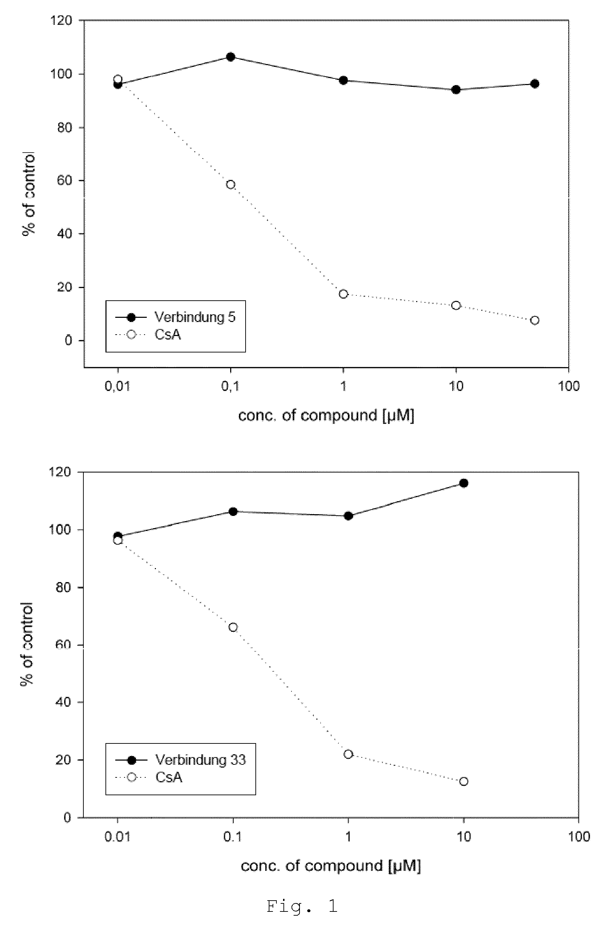

Jurkat-Zellen der Zellzucht wurden geerntet und zentrifugiert. Das Medium wurde verworfen und die Zellen einmal mit RPMI (ohne Phenolrot, 10%FCS, Hepes, Gentamycin) gewaschen. Die Zellen wurden in einem Medium re-suspendiert, auf eine Zellkonzentration von 1.0x105⁻³.0x10⁵ Zellen/ml eingestellt und anschließend mit einem fluoreszierenden CsA-Derivat (0.5µM-2.0µM) bei 37°C, 5-10% CO₂ und 100% Luftfeuchtigkeit inkubiert. Die Zellen wurden danach zentrifugiert, das Medium verworfen, das Zellpellet einmal mit Medium gewaschen und die Zellen in einem neuen Medium re-suspendiert. Anschließend wurden die Zellen mit den entsprechenden Konzentration (0.01 µM bis 100 µM) der zu untersuchenden Testverbindung für eine halbe bis vier Stunden inkubiert und danach erneut zentrifugiert. Das Medium wurde verworfen, einmal mit 1 ml PBS gewaschen und in 500 µl PBS suspendiert. Die Analyse erfolgte mittels FACS-Messung durch Quantifizierung des verdrängten fluoreszierenden CsA-Derivats. Die Referenz (ohne Inhibitor) wurde dabei als 100% gesetzt. Die Ergebnisse für Verbindung 5 und für Cyclosporin A aus diesem Testsystem zeigen (Figur 1), dass die Verbindung 5 im Vergleich zu CsA nicht-zellgängig ist.

### Beispiel 73: Bestimmung der Zellpermeabilität in Caco2-Zellmembranen

Die Testsubstanzen wurden von einer 10 mM DMSO-Lösung auf eine finale Konzentration von 5 µM in HBSS Puffer pH 7.4 verdünnt. Anschließend wurde für 2 Stunden bei 37°C und 5% CO₂ auf einer differenzierten Monoschicht aus Caco2-Zellen inkubiert, die für 10 Tage auf einer Transwell-Membran gewachsen war. Die Konzentration der Testsubstanz wurde im Ausgangs- als auch im Empfänger-Well bestimmt. Die apparente Permeabilität (Pₐₚₚ) in Richtung apikal nach basolateral (A-B) und umgekehrt (B-A) wurde nach folgender Formel berechnet: Pₐₚₚ = 1/AC₀ (dQ/dt), wobei A die Fläche der Transwell-Membran, C₀ die Substanzkonzentration zum Zeitpunkt t = 0 und dQ/dt die pro Zeiteinheit migrierende Substanzmenge darstellt. Die Ergebnisse für die Testverbindungen sind in der Tabelle 2 aufgeführt.

### Beispiel 74: Bestimmung der Zellpermeabilität in PAMPA-Zellmembranen (Parallel-artifizieller Membranpermeabilitätsassay)

Die Testsubstanzen wurden von einer 10 mM DMSO-Lösung auf eine finale Konzentration von 500 µM in 50 mM Hepes Puffer pH 7.4 verdünnt und auf eine Transwell-Membran überführt, die mit einer Membran-bildenden Lösung aus 10% 1,2-Dioleyl-sn-glycer-3-phosphocholin und 0.5% (w/v) Cholesterol in Dodecan bedeckt war. Nach einer Inkubation von 16 Stunden bei Raumtemperatur in einer Feuchtkammer wurde nach Filtration die optische Dichte der Lösung in einem Bereich von 250 bis 500 nm in Schritten von je 10 nm gemessen. Der Prozentsatz "Flux" wurde aus dem Integral des Graphen zwischen 250 und 500 nm berechnet und auf die im Parallelansatz ohne künstliche Membran ermittelte optische Dichte standardisiert. Die Ergebnisse für die Testverbindungen sind in der Tabelle 2 aufgeführt.

Tabelle 2: Zellpermeabilität ausgewählter erfindungsgemäßer Verbindungen im Vergleich zu Cyclosporin A in Caco2- und in PAMPA-Zellmembranen:

| Verbindung | Zellpermeabilitä t in Caco2-Zellmembranen AB [10⁻⁶ cm/s] | Zellpermeabilitä t in Caco2-Zellmembranen B-A [10⁻⁶ cm/s] | Zellpermeabilitä t in PAMPA-Zellmembranen [% flux] |
|---|---|---|---|
| Cyclosporin A | 3,0 | 7, 9 | |
| 5 | 0,17 | 0,13 | 1,7 |
| 22 | 0, 9 | 0,2 | 4,1 |
| 23 | 0,3 | 0, 6 | |
| 28 | <0,4 | 0,3 | 3,3 |
| 31 | <0,15 | <0,18 | 0,2 |
| 33 | <0,15 | <0,15 | 0,25 |
| 34 | <0,6 | 0,87 | 19, 9 |
| 35 | <0, 35 | <0,46 | 4 |
| 36 | 0,3 | 0,2 | 0,7 |
| 37 | 0,1 | 0,2 | 2 |
| 46 | 0,4 | 0,2 | 13,5 |

### Beispiel 75: Überprüfung der immunsuppressiven Wirkung im Proliferationsassay

Für die Isolierung der Immunzellen aus der Milz von Mäusen wurde der Stamm C57BL/6 verwendet und über CHARLES RIVER Laboratories bezogen. Vor der Sektion wurden die 14 Wochen alten Tiere für 15 min in einen mit CO₂ gefluteten Tank gestellt. Nachdem keine Lebenszeichen mehr an den Mäusen zu erkennen waren, erfolgte ein Genickbruch und die Sektion mit der Entnahme der Milz. Die Isolierung der Milzzellen erfolgte bei Raumtemperatur in 5 mL HBSS (PAA) durch Zerdrücken des Organs und Ausstreichen der Zellen mittels der Rückseite des Kolbens einer 1 mL Spritze. Anschließend wurden die Zellen durch ein 40 µm Zellsieb gegeben und vereinzelt. Das Sieb wurde erneut mit 5 mL HBSS gewaschen. Für die Separation der Immunzellen wurde ein Dichtegradient verwendet, wobei 5 mL Lymphocyte Separation Medium (Mediatech, Inc.) mit 5 mL der Zellsuspension überschichtet wurden. Die weiteren Schritte erfolgten nach Angaben des Herstellers.

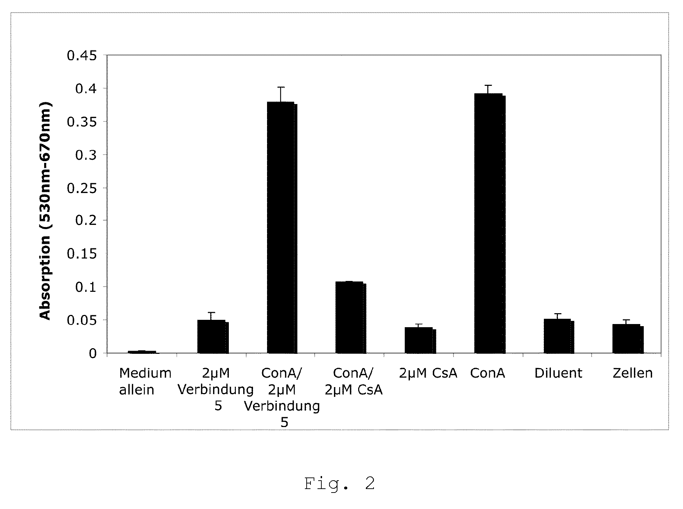

Die Untersuchung auf Inhibierung der Proliferation von Milzzellen durch die Testsubstanzen wurde in Mikrotiterplatten (96well plate) in dreifacher Anordnung durchgeführt. Dafür wurden 2 µM der zu testenden Verbindungen oder adäquate Kontrollen (Medium, DMSO, CsA) in RPMI-1640 Medium (PAA) mit 3,04 x 10⁵ Zellen in 90 µL inkubiert (Gesamtvolumen 100 µL). Die Aktivierung der Proliferation erfolgte durch Zugabe von 0,01 mg/mL Concanavalin A (ConA) in PBS (Sigma). Die Zellen wurden anschließend bei 37°C für 72 Stunden inkubiert. Die Proliferation wurde im Anschluss kolorimetrisch ermittelt. Dazu wurden 11 µL MTT Reagenz (5mg/ mL, Methylthiazolyldiphenyl-tetrazolium-bromid; Sigma) zu den Zellen gegeben und für weitere 5 Stunden bei 37°C inkubiert. Im Anschluss wurde das Medium abgenommen, die Zellen in 100 µL DMSO resuspendiert und die Absorption bei 550 nm und 630 nm mit einem Multiplate Reader (VERSmax, Molecular Devices) ermittelt. Das Testergebnis für Verbindung 5 im Vergleich zu Cyclosporin A ist beispielhaft in Figur 2 gezeigt.

### Beispiel 76: Asthma-Untersuchungen:

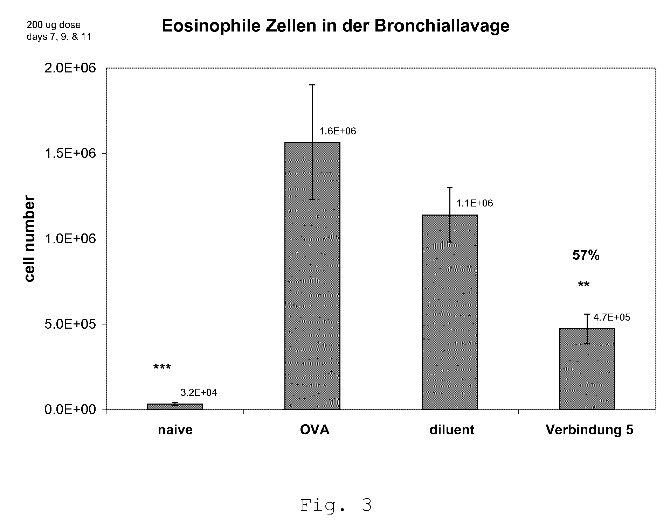

Durch Gabe von Ovalbumin zusammen mit Aluminiumhydroxid wird eine Immunantwort gegen Ovalbumin provoziert. Die Immunantwort kann anhand der in die Bronchialschleimhaut eingewanderten T-Helferzellen-Population (CD4+) und der eingewanderten eosinophilen Granulozyten verfolgt werden. Weibliche Mäuse (BALB/c-Linie) wurden durch intraperitonale (i.p.) Gabe von 50 µg Ovalbumin gelöst in Phosphatpuffer (PBS) gelöst ((OVA) plus 100 µL Aluminiumhydroxid) bei einem Gesamtvolumen von 200 µL per Maus an Tag 0 sensibilisiert. Den Ovalbum-sensibilisierten Mäusen wurde dann unter milder Anästhesie (Isoflurane) an den Tagen 7-10 intranasal jeweils 100 µg OVA in PBS (50 µL Gesamtvolumen) verabreicht. Aus diesen Tieren wurden Gruppen gebildet, welche zusätzlich an den Tagen 7, 9 und 11 entweder 200 µg der Testverbindung in PBS (i.p.), nur PBS (Diluent) oder keinen weiteren Zusatz (-) erhielten. Am Tag 12 wurden alle Tiere durch CO₂-Exposition getötet und Zellen des Bronchialtraktes durch Bronchiallavage (BAL) mittels einer in die Trachea eingeführten Kanüle bei dreimaligem Waschen mit je 1 ml von kaltem PBS erhalten. Die erhaltenen Zellen der BAL wurden dann doppelt angefärbt (a) mit Cy-Chrome-konjugierten anti-Maus-CD4 -Antikörpern und (b) mit FITC-konjugierten anti-Maus-CD62L-Antikörpern. Anschließend wurden die Zellen mittels FACS analysiert. Effector/Memory-CD4⁺-T-Zellen wurden als CD4⁺/CD62L⁻-Lymphocyten und eosinophile Zellen anhand ihrer Streulichteigenschaften (FSC/SSC) unterschieden. Die mit der Verbindung 5 erhaltenen Ergebnisse sind in den Abbildungen Fig. 3 bis 6 zusammengefasst.

Fig. 3 zeigt den Einfluss von Verbindung 5 auf die Anzahl der eosinophilen Granulozyten (Eosinophile), welche durch die Ovalbuminsensibiliserung in die Bronchialschleimhäute einwanderten und sich in der Lungenspülflüssigkeit (Lavage) nachweisen lassen. Die Gabe von Verbindung 5 reduzierte hoch-signifikant die Anzahl der eosinophilen Granulozyten.

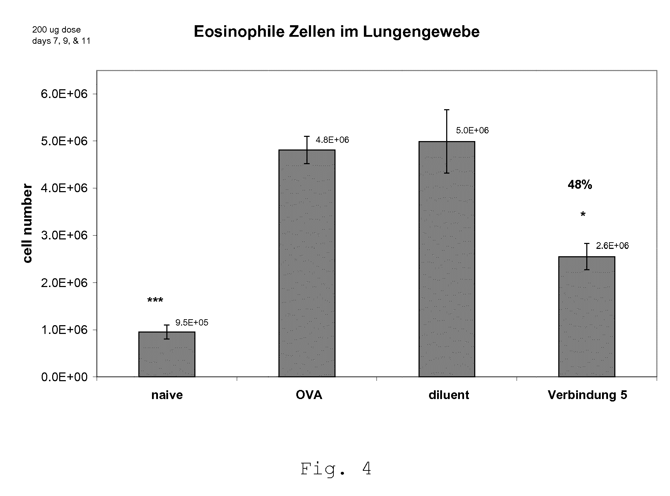

Fig. 4 zeigt den Einfluss von Verbindung 5 auf die Anzahl der eosinophilen Granulozyten (Eosinophile) im Lungengewebe, welche durch die Ovalbuminsensibiliserung in die Bronchialschleimhäute einwanderten. Die Gabe von 200 µg der Verbindung 5 reduzierte hoch-signifikant die Anzahl der eosinophilen Granulozyten.

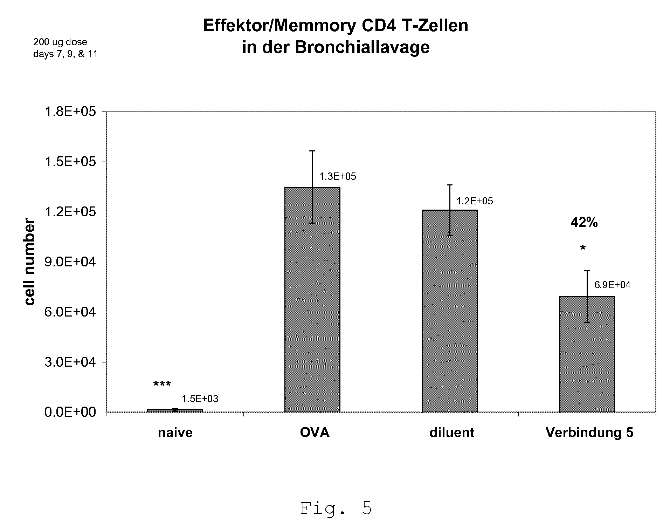

Fig. 5 zeigt den Einfluss von Verbindung 5 auf die Anzahl der CD4 positiven T-Zellen, die durch die Ovalbuminsensibiliserung in die Bronchialschleimhäute einwanderten und sich mittels Lungenspülung in die Lungenspülflüssigkeit (BAL) auswaschen lassen. Auch hier reduzierte die Gabe von 200 µg der Verbindung 5 die Anzahl der CD4 positiven T-Zellen hoch-signifikant.

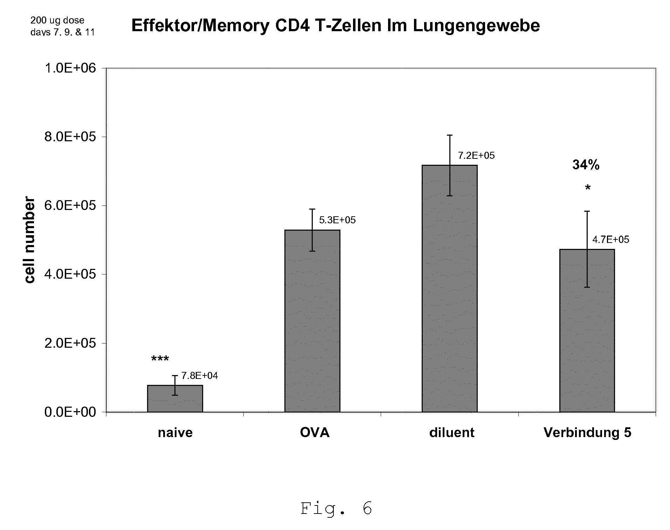

Fig. 6 zeigt den Einfluss der Verbindung 5 auf die Anzahl der CD4 positiven T-Zellen im Lungengewebe, die durch die Ovalbuminsensibilisierung in die Bronchialschleimhäute einwanderten und sich dort nachweisen lassen. Die Gabe von 200 µM der Verbindung 5 reduzierte hoch-signifikant die Anzahl der CD4 positiven T-Zellen.

## Patentansprüche

1. Verbindung der folgenden Formel **I** 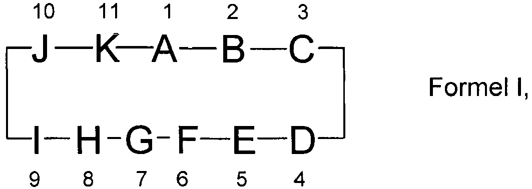 worin
A eine Aminosäure der folgenden Formel **II** ist, 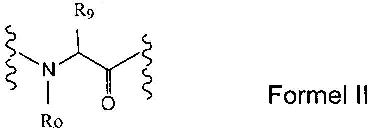 wobei die Bindungen, die an den gewellten Linien enden, Verknüpfungen zu den Einheiten B und K darstellen und
R₉ eine Gruppe der folgenden Formel III ist, und 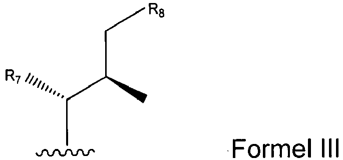 bei der R₈ einer Gruppe der folgenden Formel IV entspricht, oder 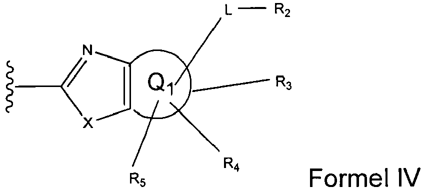 bei der R₈ einer Gruppe der folgenden Formel V entspricht, oder 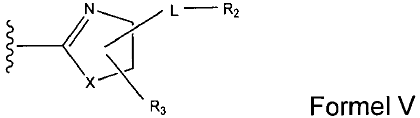 bei der R₈ einer Gruppe der folgenden Formel VI entspricht, oder 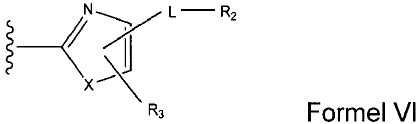 bei der R₈ einer Gruppe der folgenden Formel VII entspricht, oder 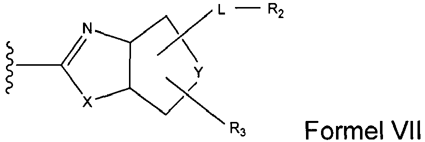 bei der R₈ einer Gruppe der folgenden Formel VIII entspricht, in der die Reste L-R₂ und R₃ an den gleichen oder verschiedenen Phenylringen gebunden sein können, oder 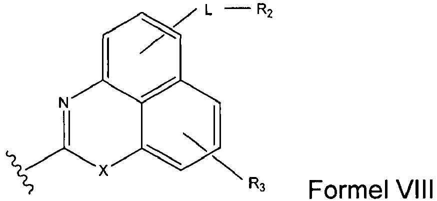 bei der R₈ einer Gruppe der **folgenden** Formel IX entspricht, und in der die Reste L-R₂ und R₃ an den gleichen oder an verschiedenen Ringen Q₂ oder Q₃ gebunden sein können, oder 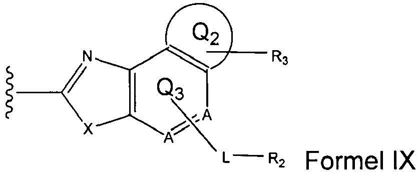 bei der R₈ einer Gruppe der folgenden Formel X entspricht, in der die Reste L-R₂ und R₃ an den gleichen oder an verschiedenen Ringen Q₂ oder Q₃ gebunden sein können, oder 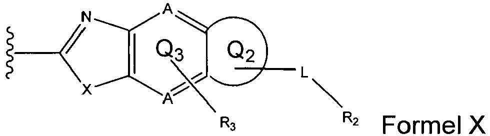 bei der R₈ einer Gruppe der folgenden Formel XI entspricht, in der die Reste L-R₂ und R₃ an den gleichen oder an verschiedenen Ringen Q₂ oder Q₃ gebunden sein können, oder 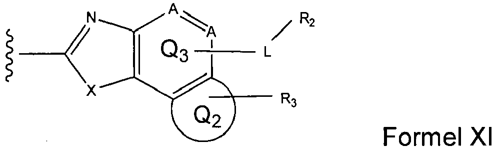 bei der R₈ einer Gruppe der folgenden Formel XII entspricht, in der die Reste L-R₂ und R₃ an den gleichen oder verschiedenen Phenylringen gebunden sein können, oder 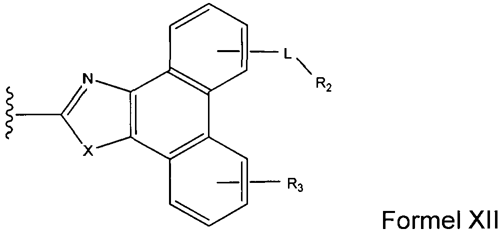 bei der R₈ einer Gruppe der folgenden Formel XIII entspricht, 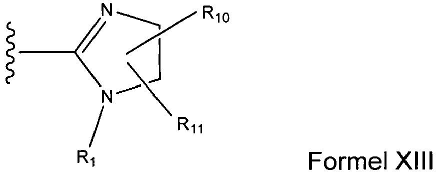 wobei
die Gruppe der Formel II über ihr CO kovalent zu der alpha-Aminosäure B in Formel I unter Ausbildung einer Amidbindung gebunden ist und N-R₀ in der Formel II kovalent zu einer Carboxylgruppe von K unter Ausbildung einer Amidbindung gebunden ist, wobei
B eine Aminosäure ist, die aus der folgenden Gruppe ausgewählt ist:
alpha-Aminobuttersäure;
Alanin;
Threonin;
Valin;
Norvalin; und
in der Seitenkette mit einer Hydroxylgruppe
modifizierte Moleküle von alpha-Aminobuttersäure, Alanin, Threonin,
Valin oder Norvalin;
C für Sarkosin steht;
D eine Aminosäure ist, die aus der folgenden Gruppe ausgewählt ist:
Leucin;
N-Methylleucin;
Valin;
Gamma-Hydroxy-N-Methylleucin; und
Gamma-Hydroxyleucin;
E eine Aminosäure ist, die aus der folgenden Gruppe ausgewählt ist:
Valin;
Norvalin; und
einem modifizierten Valin oder Norvalin, bei dem ein Kohlenstoffatom der Seitenkette mit einer Hydroxylgruppe substituiert ist;
F eine Aminosäure ist, die aus der folgenden Gruppe ausgewählt ist:
Leucin;
N-Methylleucin;
Gamma-Hydroxy-N-Methylleucin; und
Gamma-Hydroxyleucin;
G gleich alpha-Aminobuttersäure oder Alanin ist;
H gleich D-Alanin oder D-Serin ist;
I und J Aminosäuren sind, die unabhängig voneinander aus der folgenden Gruppe ausgewählt sind:
Leucin;
N-Methylleucin;
Gamma-Hydroxy-N-Methylleucin; und
Gamma-Hydroxyleucin;
K gleich N-Methylvalin oder Valin ist;
wobei
die Aminosäuren B bis K unter Ausbildung von Amidbindungen miteinander verknüpft sind;
wobei
die verwendeten Symbole und Indices die folgenden Bedeutungen aufweisen:
X steht für O, S, oder N-R₁;
Y steht für O, S, N-R₁, -CH₂- oder -CH₂CH₂-; A wie verwendet in Formeln IX, X, und XI steht für CH oder N;
L steht für ein Bindung, -CH₂-, -CH₂CH₂-, -CH₂CH₂CH₂-, -CH₂CH₂CH₂CH₂-, -CH=CH-, -CH=CHCH₂-, -CH₂CH=CH-, -CH₂CH₂CH=CH-, -CH₂CH=CHCH₂-, -CH=CHCH₂CH₂-, -OCH₂-, -OCH₂CH₂-, -OCH₂CH₂CH₂-, -OCH₂CH=CH-, -CONH-, -CONHCH₂ oder -CONHCH₂CH₂-, -CONHCH₂CH₂OCH₂CH₂- oder -CONHCH₂CH₂OCH₂CH₂OCH₂CH₂-;
Q₁ steht zusammen mit den beiden Atomen des ankondensierten Rings für einen aromatischen oder heteroaromatischen Ring aus der Gruppe Benzol, Pyridin, Pyrimidin, Pyridazin, Pyrazin, Triazin, Thiophen, Furan, Pyrrol, Thiazol, Isothiazol, Oxazol, Isoxazol, Imidazol oder Pyrazol;
Q₂ steht zusammen mit den beiden Atomen des ankondensierten Rings für einen aromatischen oder heteroaromatischen Ring aus der Gruppe Benzol, Pyridin, Pyrimidin, Pyridazin, Pyrazin, Triazin, Thiophen, Furan, Pyrrol, Thiazol, Isothiazol, Oxazol, Isoxazol, Imidazol, Pyrazol, Thiadiazol, Oxadiazol oder Triazol;
Q₃ bezeichnet jeweils den Sechsring in dessen Mitte es steht;
R₀ steht für H oder CH₃;
R₁ steht für H, (C1-C4)-Alkyl oder Benzyl, die auch durch eine Gruppe -COOH substituiert sein können;
R₂ steht für eine polare deprotonierbare Gruppe Pₛ, ausgewählt aus der Gruppe bestehend aus
a) -COOH, -SO₃H, -CONH₂, -CONHNH₂, -SO₂NH₂, -PO₃H₂,-PO(OH)(NH₂), -CO(NHOH), -CO(NH(O-C₁-C₄-Alkyl)), -CSNH₂,-NHCONH₂, -N(OH)CONH₂, -NHCO(NHOH)-, -NHCSNH₂,-CSNHNH₂;
b) 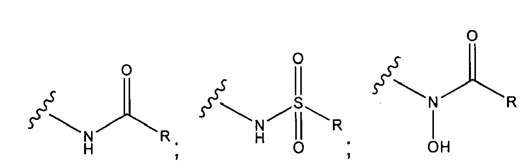 und R = -(C1-C4-Alkyl), -O(C1-C4-Alkyl), -NH(C1-C4-Alkyl), -NH(C1-C4-Alkenyl), (substituiertes) Aryl, (substituiertes) O-Aryl, (substituiertes) NH-Aryl, - CF3 und andere fluorierte (C1-C4-Alkyl)gruppen;
c) 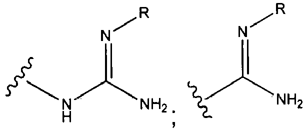 und R = -OH, -CN, -NO2;
d) 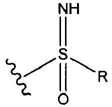 und R = (C1-C4-Alkyl), (substituiertes) Aryl, -CF3 und andere fluorierte (C1-C4-Alkyl)gruppen;
e) 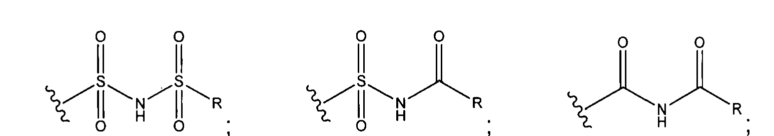 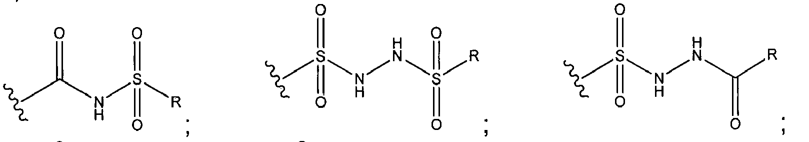 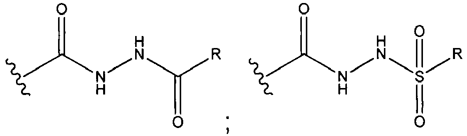 und R = -(C1-C4-Alkyl), - O(C1-C4-Alkyl), -NH(C1-C4-Alkyl), -NH(C1-C4-Alkenyl), (substituiertes) Aryl, (substituiertes) O-Aryl, (substituiertes) NH-Aryl, - CF3 und andere fluorierte (C1-C4-Alkyl)gruppen; 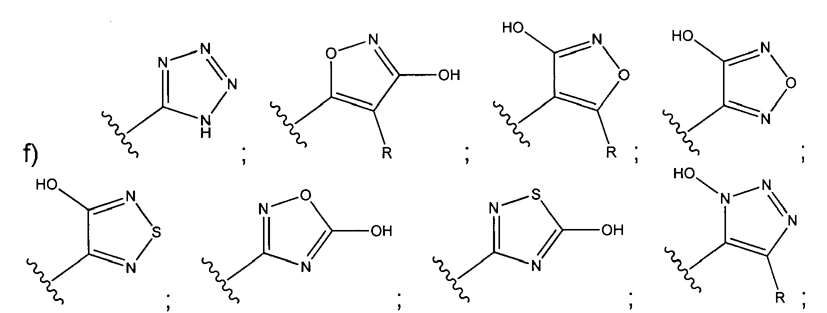 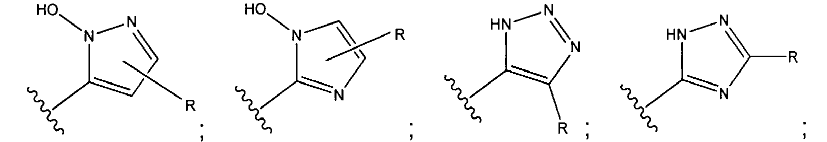 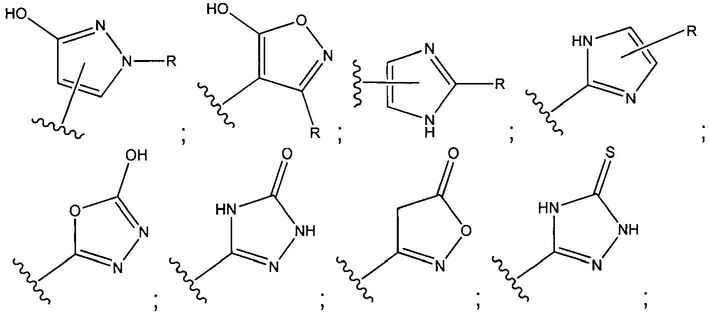 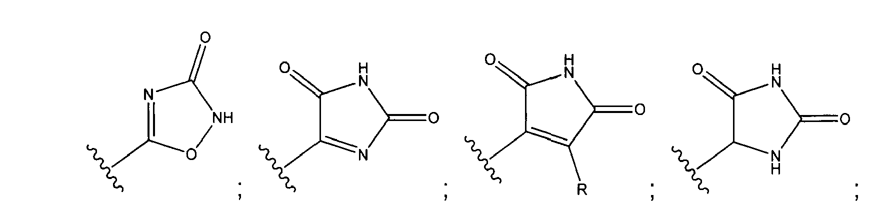 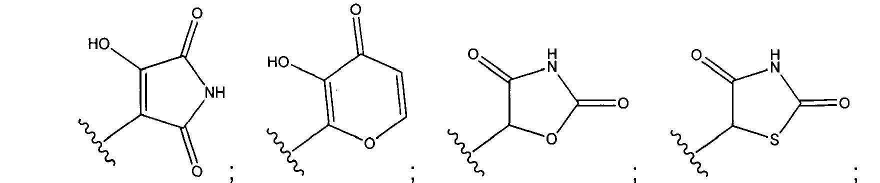 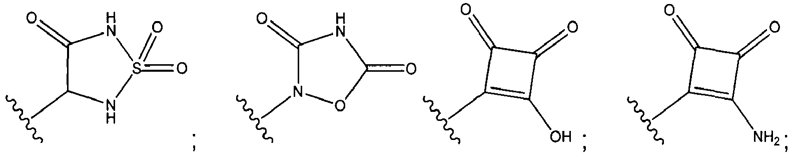 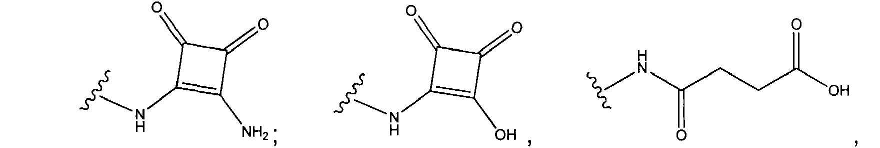 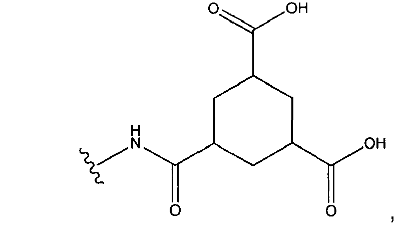 in denen und R = H, -(C1-C4-Alkyl), -CF3 und andere fluorierte (C1-C4-Alkyl)gruppen.
g) -OH und -SH, mit den Maßgaben, dass L eine kovalente Einfachbindung darstellt, und dass-OH oder -SH nur als Substituent an einem Ring Q1, Q2 oder Q3 auftreten kann und dabei an ein Kohlenstoffatom gebunden ist, das in Nachbarschaft zu einem Stickstoffatom steht
oder
eine Gruppe Pₛ', die unter physiologischen Bedingungen in Pₛ umgewandelt werden kann, oder
eine polare protonierbare Gruppe P_{b}, ausgewählt aus der Gruppe bestehend aus
a) 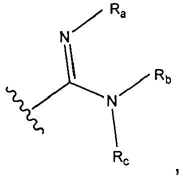 in der Rₐ, R_{b} und R_{c} für H und -(C1-C4-Alkyl) stehen, Rₐ und R_{b} zusammen -(CH₂CH₂)- oder -(CH₂CH₂CH₂)- und R_{b} und R_{c} zusammen mit dem Stickstoffatom Pyrrolidin, Piperidin oder Morpholin sein können;
b) 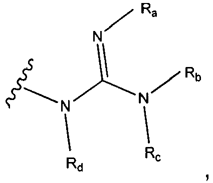 in der Rₐ, R_{b}, R_{c} und R_{d} für H und -(C1-C4-Alkyl) stehen, Rₐ und R_{b}, Rₐ und R_{d} sowie R_{b} und R_{d} zusammen - (CH₂CH₂)- oder -(CH₂CH₂CH₂)- und R_{b} und R_{c} zusammen mit dem Stickstoffatom Pyrrolidin, Piperidin oder Morpholin sein können;
c) eine Aminogruppe ausgewählt aus -NH₂, (C1-C6)Alkyl-amino, (C1-C6)Dialkyl-amino, Pyrrolidin, Piperidin, Morpholin oder Piperazin, welches in Position 4 durch (C1-C6)Alkyl, -(C1-C6)Alkanoyl, Benzyl, Benzoyl oder Phenyl substituiert sein kann, wobei diese Substituenten optional eine Gruppe -COOH tragen können
oder
eine Gruppe P_{b'}, die unter physiologischen Bedingungen in P_{b} umgewandelt werden kann;
R₃ steht für H, (C1-C6)-Alkyl, (C1-C6)-Alkoxy, -OH, (C1-C6)-Alkylthio, (C1-C6)-Alkylsulfonyl, -SH, -CF₃, -COOH, -COO((C1-C6)Alkyl)),-CONH₂, -CONH((C1-C6)Alkyl), -CON((C1-C6)Alkyl)₂, Nitro, Halogen, Cyano, Amino, (C1-C6)Alkyl-amino oder (C1-C6)Dialkyl-amino; oder R₃ liegt in Form eines freien Elektronenpaares vor, wenn es an ein Heteroatom des Ringes Q₁, Q₂ oder Q₃ bindet/binden würde;
R₄ und R₅ stehen unabhängig voneinander für H, (C1-C6)-Alkyl, (C1-C6)-Alkoxy, -CF₃, Halogen oder können zusammen eine Gruppe -OCH₂O- oder -OCH₂CH₂O bilden, wenn R₄ und R₅ in Positionen ortho zueinander am Ring gebunden sind;
R₇ steht für H, -OH, -OCOOR₁₂, -OCOR₁₃, -OCONR₁₄R₁₅, -O-(tetrahydropyran-2-yl), -O-(tetrahydrofuran-2-yl), -O-CHR₁₆-OR₁₇ oder -SiR₁₈R₁₉R₂₀;
R₁₀ und R₁₁ stehen unabhängig voneinander für H, (C1-C6)-Alkyl, -CF₃ oder Halogen; oder R₁₀ und R₁₁ bilden zusammen eine Gruppe -CH₂CH₂-, -CH₂CH₂CH₂-, -CH₂CH₂CH₂CH₂- oder -CH₂CH₂CH₂CH₂CH₂-;
R₁₂ steht für (C1-C6)-Alkyl, Benzyl oder Phenyl, wobei die Alkylgruppe gegebenenfalls durch Fluor oder Chlor substituiert und Benzyl und Phenyl gegebenenfalls durch einen oder mehrere Substituenten aus der Gruppe (C1-C6)-Alkyl, (C1-C6)-Alkoxy, -CF₃, Cyano, Nitro oder Halogen substituiert sein kann;
R₁₃ steht für H oder R₁₂;
R₁₄ und R₁₅ stehen unabhängig voneinander für H, (C1-C6)-Alkyl, Benzyl oder Phenyl, wobei Benzyl und Phenyl gegebenenfalls durch einen oder mehrere Substituenten aus der Gruppe (C1-C6)-Alkyl, (C1-C6)-Alkoxy, -CF₃, Cyano, Nitro oder Halogen substituiert sein können;
R₁₆ steht für H oder (C1-C6)-Alkyl;
R₁₇ steht für (C1-C6)-Alkyl, Benzyl oder Phenyl
R₁₈, R₁₉ und R₂₀ stehen unabhängig voneinander für (C1-C6)-Alkyl, Benzyl oder Phenyl;
oder ein pharmazeutisch akzeptables Salz sowie eine tautomere, enantiomere oder andere stereoisomere Form davon.

2. Verbindung nach Anspruch 1, worin R₀ gleich -CH₃ ist.

3. Verbindung nach Anspruch 1 oder 2, worin R₇ aus der Gruppe ausgewählt ist, die aus -OH, -OCOCH₃, -OCOOCH₃, -OCH₂OCH₃, -Si(CH₃)₃ und -Si(CH₃)₂C(CH₃)₃ besteht.

4. Verbindung nach einem der Ansprüche 1 bis 3, worin R₂ aus der Gruppe ausgewählt ist, die aus -COOH, -OH, -SH, -CONH₂, -CONHNH₂, -SO₃H, -SO₂NH₂, -COOCH₃, -COOCH₂CH₃, -COOCH₂CH₂CH₃, -COOCH₂CH₂CH₂CH₃, -COOCH₂CH(CH₂CH₃)₂, -COOCH(CH₃)₂, -COOC(CH₃)₃, -COOCH₂CH₂N(CH₃)₂, -COOCH₂CH₂(morpholin-4-yl) und den Gruppen 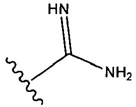 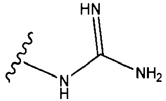 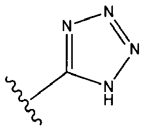 und 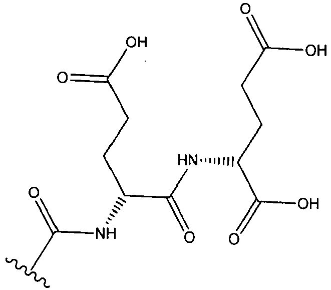 besteht, mit den Maßgaben, dass, wenn R₂ gleich -OH oder -SH ist, L eine kovalente Einfachbindung darstellt, und -OH oder -SH an einem Ring Q₁, Q₂ oder Q₃ an ein Kohlenstoffatom gebunden ist, das in Nachbarschaft zu einem Stickstoffatom steht.

5. Verbindung nach einem der Ansprüche 1 bis 4, worin L aus der Gruppe ausgewählt ist, die aus einer Bindung, -CH₂-, -CH₂CH₂-, -CH=CH-, -CONH- und -OCH₂- besteht.

6. Verbindung nach einem der Ansprüche 1 bis 5, worin X für N-R₁ steht und/oder worin der Ring Q₁ Benzol, Pyridin oder Pyrimidin ist, oder worin die Ringe Q₂ und Q₃ Benzol sind.

7. Verbindung nach einem der Ansprüche 1 bis 6, worin Y gleich O, S, -CH₂- oder -CH₂CH₂- ist und/oder worin R₃ aus der Gruppe ausgewählt ist, die aus H, -COOH, -CH₃, -OCH₃, F, Cl, Br und CN besteht.

8. Verbindung nach einem der Ansprüche 1 bis 7, worin R₄ und R₅ jeweils unabhängig voneinander gleich H oder F sind und/oder worin R₁₀ und R₁₁ jeweils unabhängig voneinander gleich H oder CH₃ sind und/oder worin die Gruppe R₈ eine Gruppe der Formel IV ist.

9. Verbindung nach Anspruch 1, worin die Verbindung der Formel I die folgende ist:

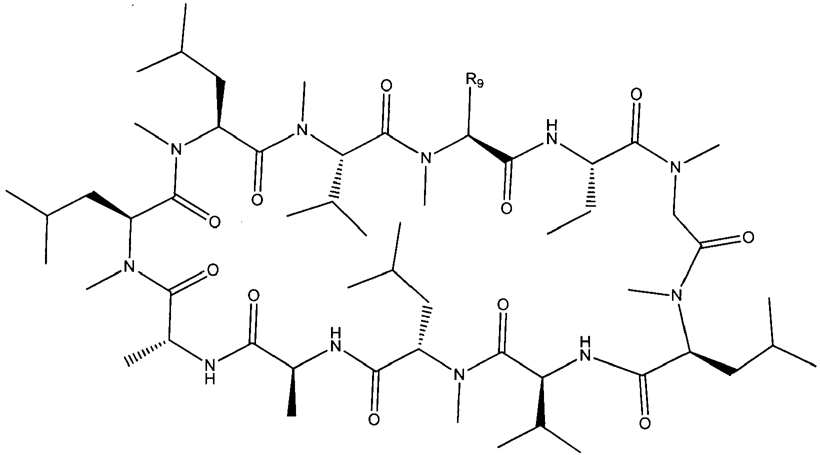

10. Verbindung nach Anspruch 1, worin die Verbindung der Formel I die folgende ist:

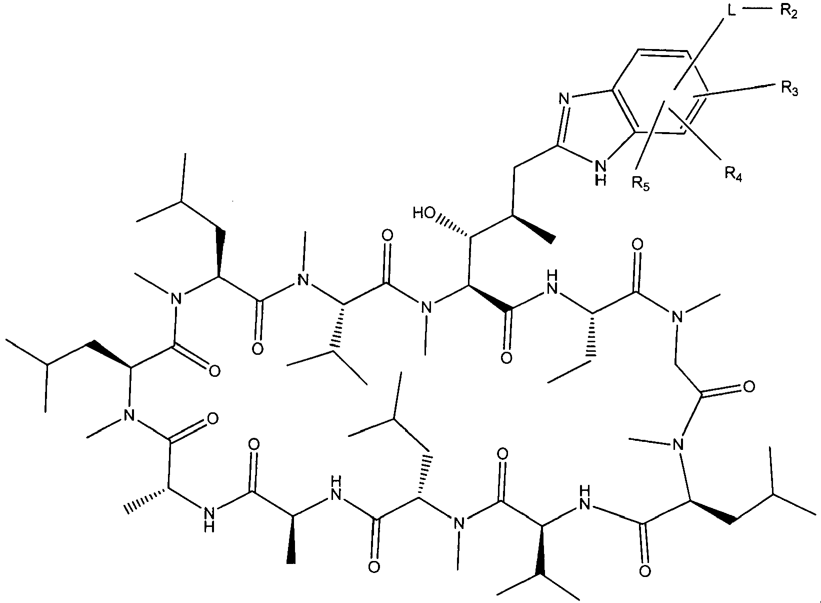

11. Verbindung nach Anspruch 1, worin die Verbindung der Formel I eine der folgenden ist:

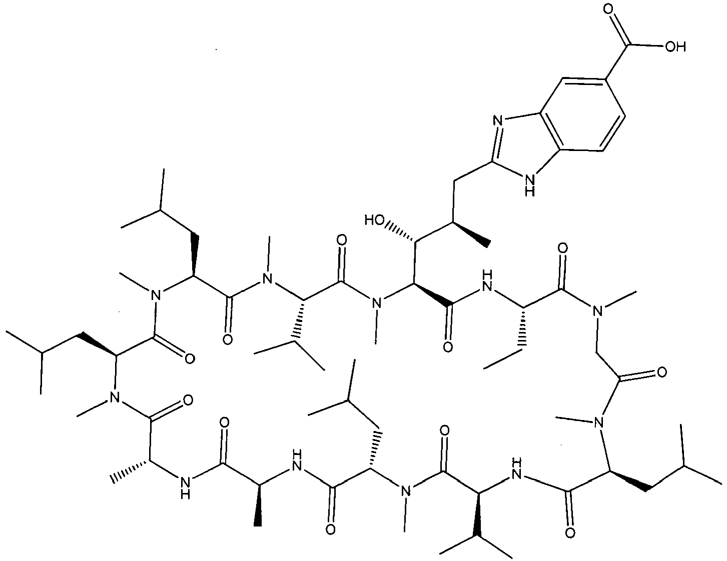

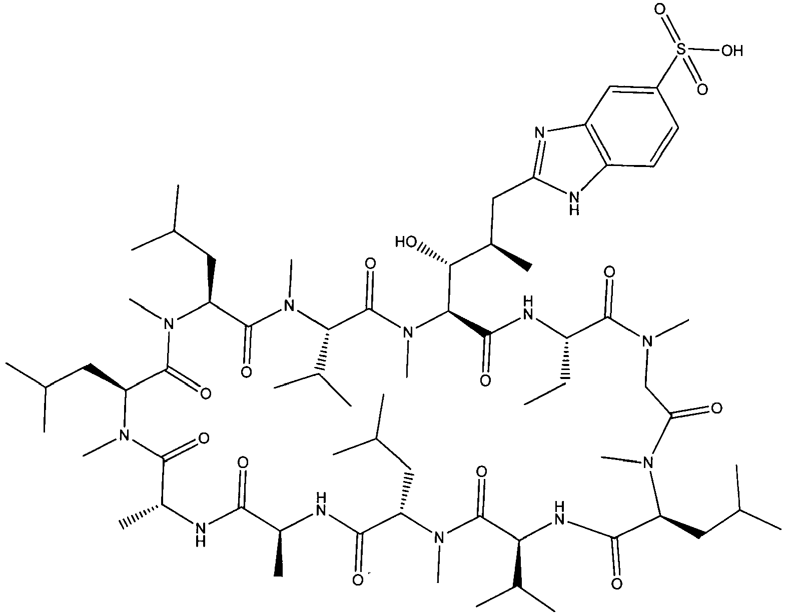

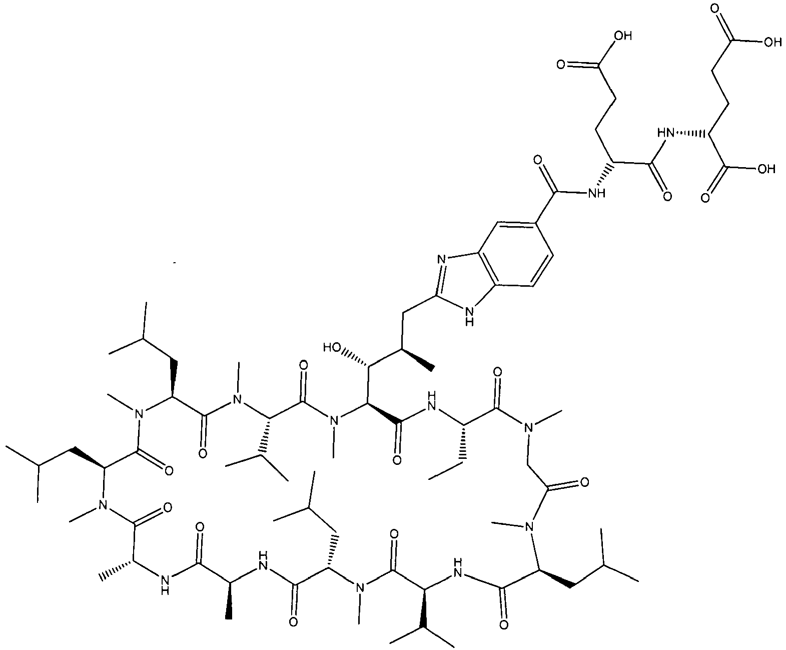

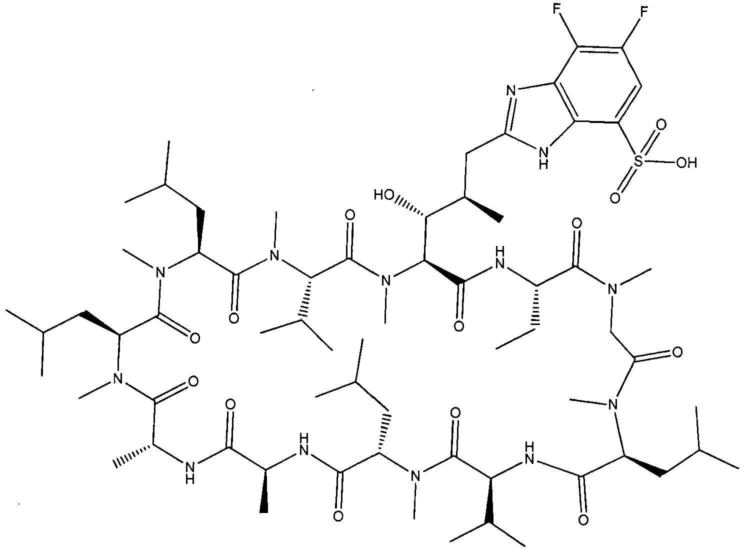

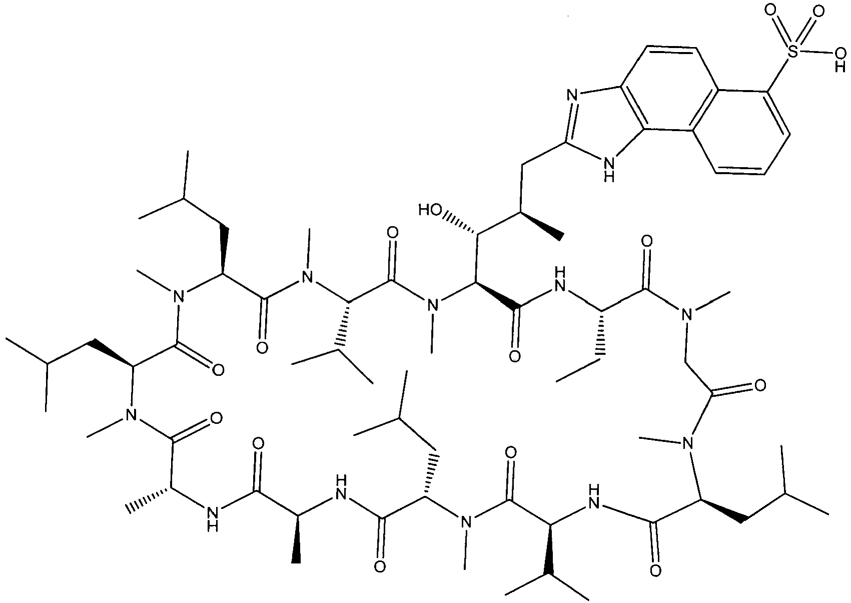

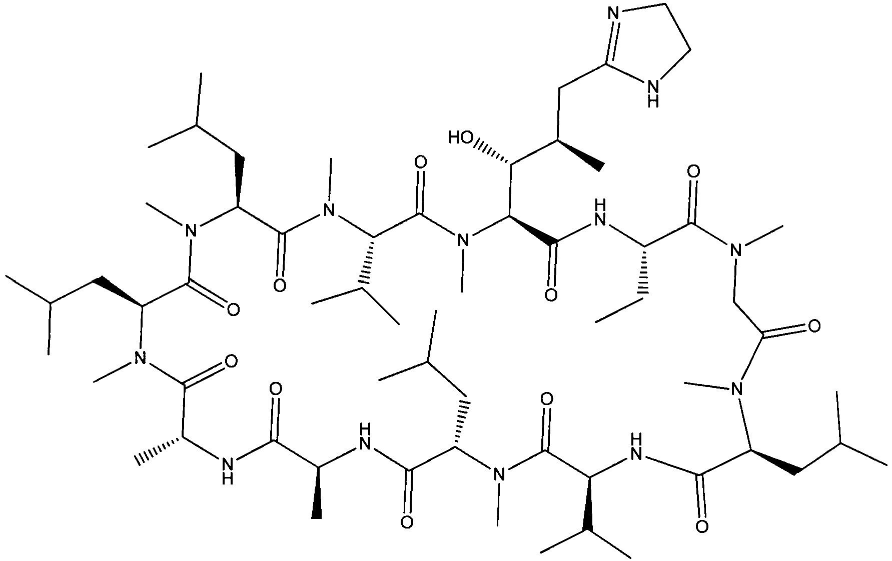

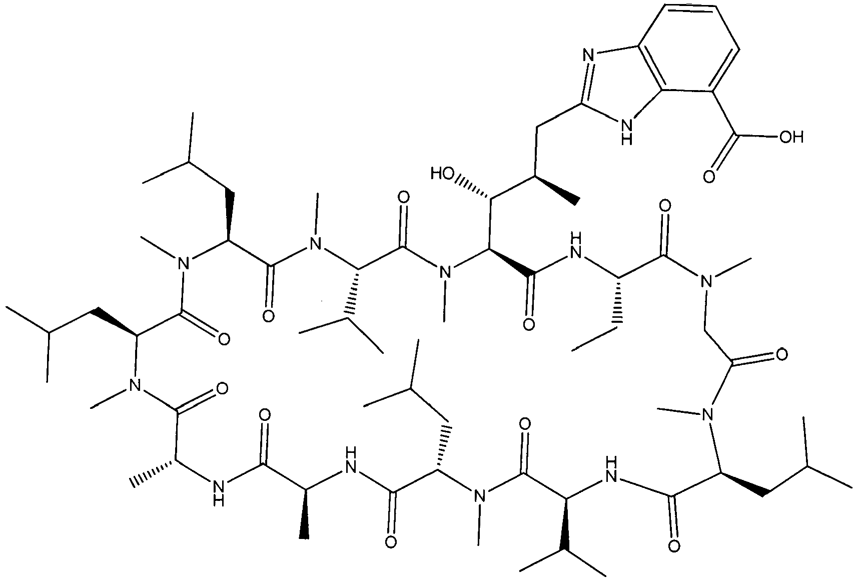

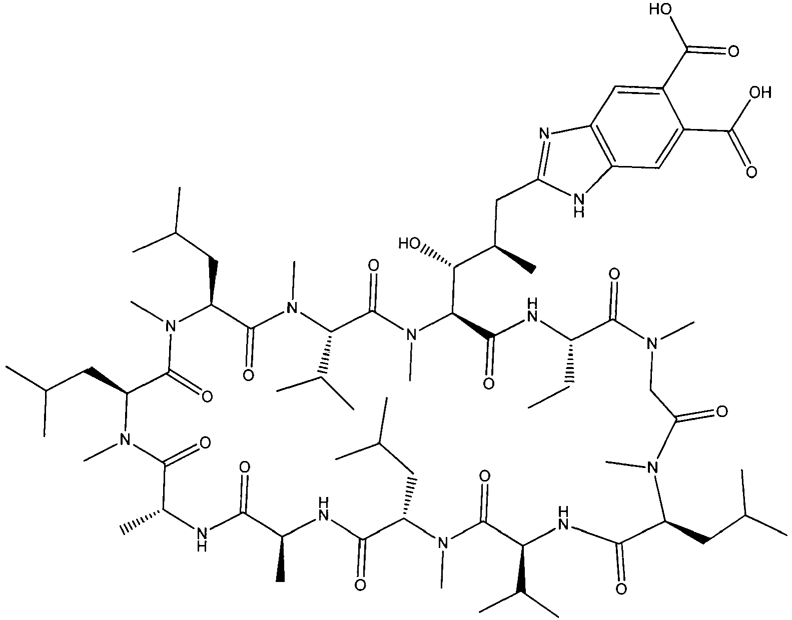

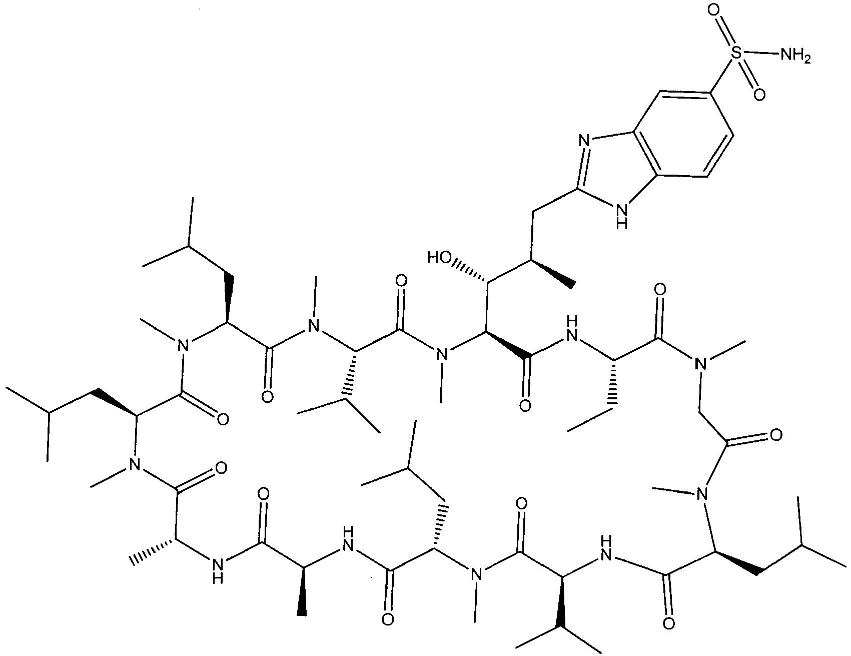

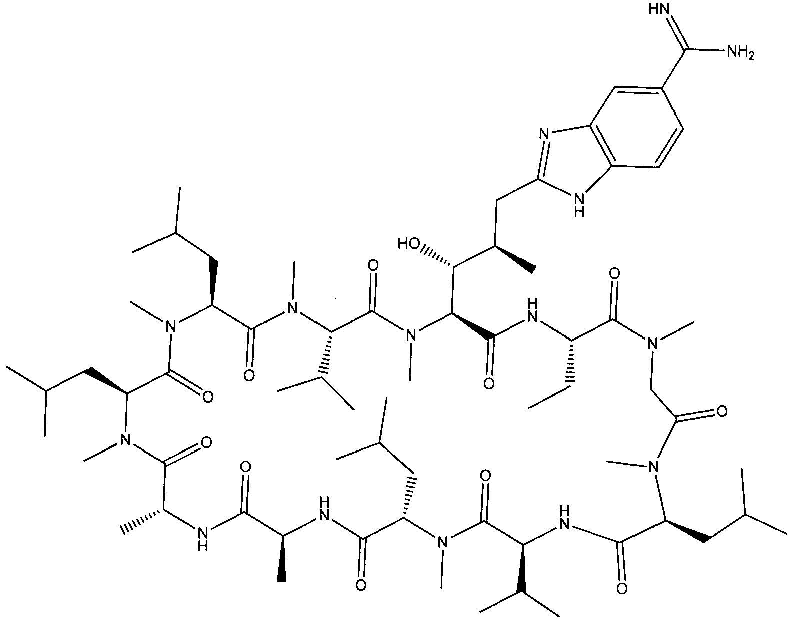

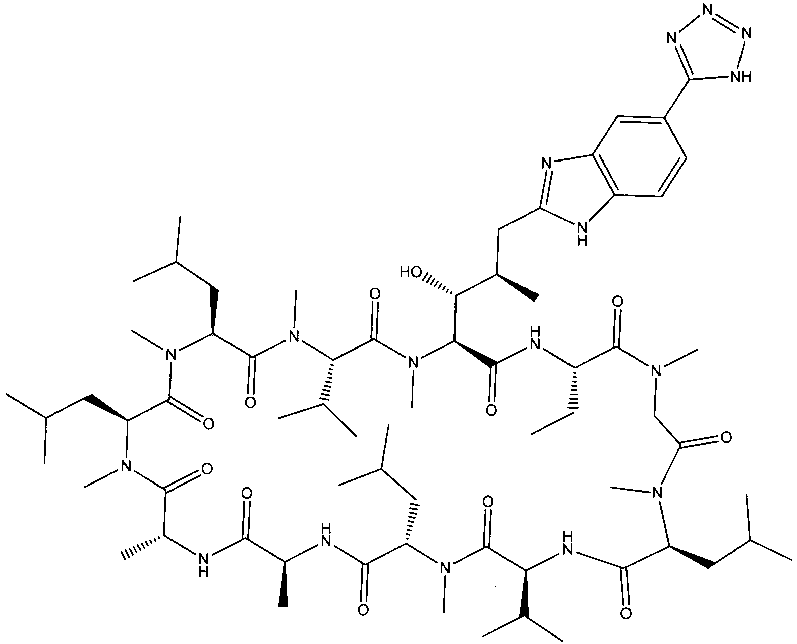

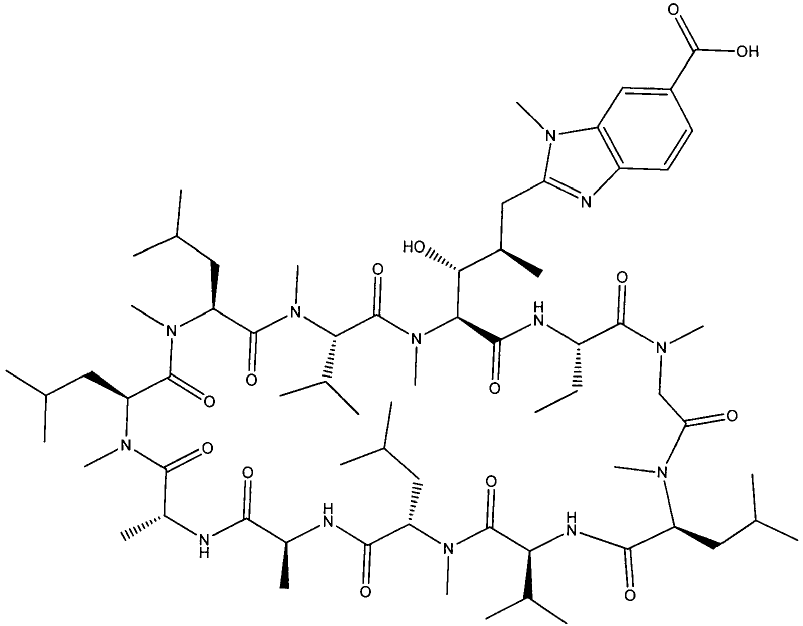

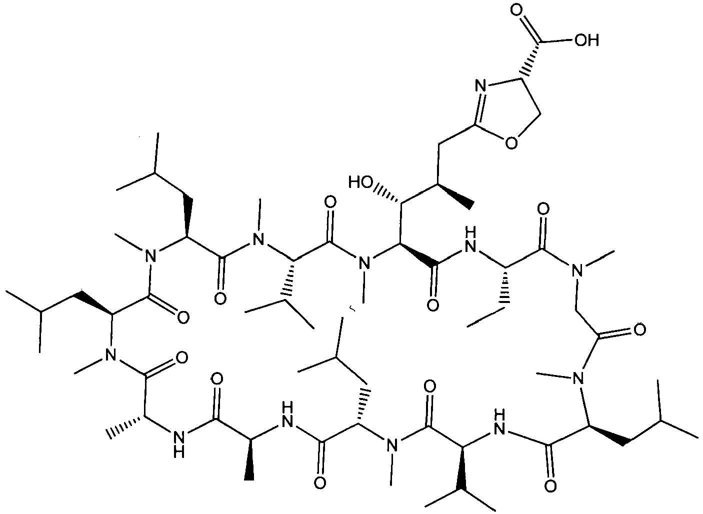

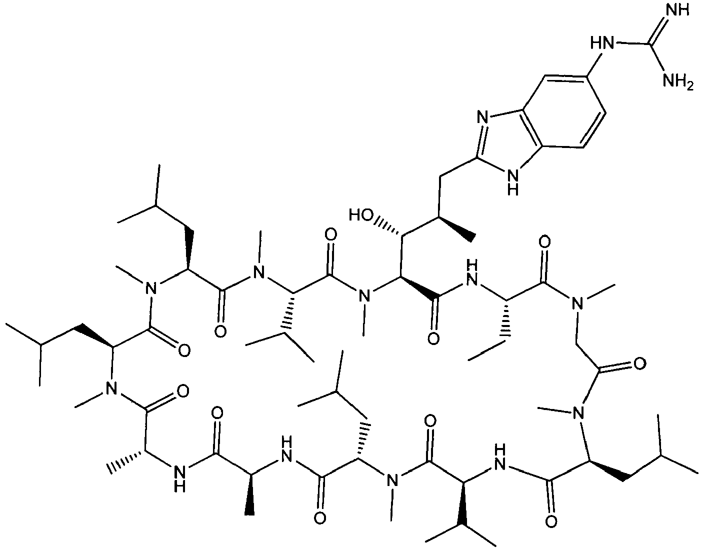

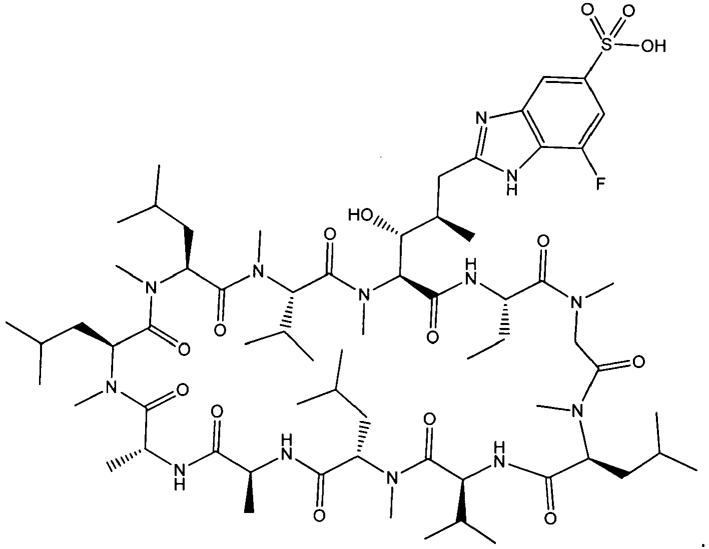

12. Indikator-gekoppelte Verbindung, die eine Verbindung gemäß Formel I nach einem der Ansprüche 1 bis 11 umfasst, bei der ein endständiges Atom nicht vorhanden und an dieser Stelle ein Indikator gebunden ist.

13. Verbindung gemäß einem der Ansprüche 1 bis 12 zur Verwendung in der Medizin.

14. Verbindung nach einem der Ansprüche 1 bis 12 zur Verwendung in der Behandlung und/oder Diagnose von
a) viralen Infektionen
b) akuten und chronischen entzündlichen Erkrankungen
c) Krebs
d) degenerativen Muskelerkrankungen
e) neurodegenerativen Erkrankungen, und
f) Schädigungen, die mit Beeinträchtigung der Calcium-Homöostase einhergehen.

15. Verwendung eines Cyclosporinderivates der **folgenden** Formel 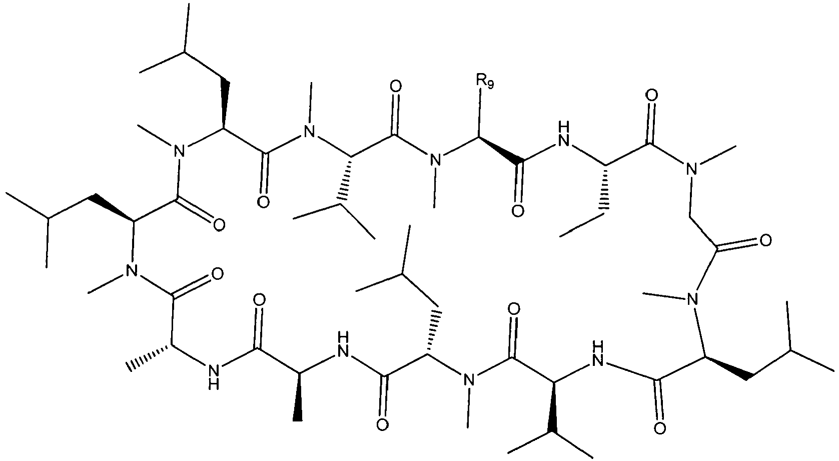 zur Herstellung einer Verbindung nach einem der Ansprüche 1 bis 12 worin R₉ einen Rest der folgenden Formel darstellt: 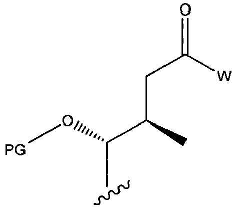 wobei PG eine Alkohol-Schutzgruppe und W gleich H oder OH darstellt und der jeweilige Rest über die Bindung, an deren Ende eine gewellte Linie eingezeichnet ist, verknüpft ist.

## Claims

1. Compound of formula 1 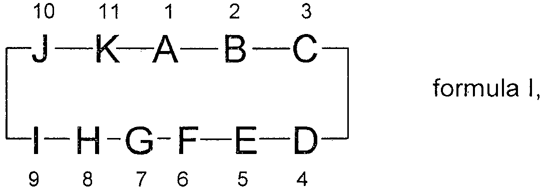 wherein
A is an amino acid of the following formula II, 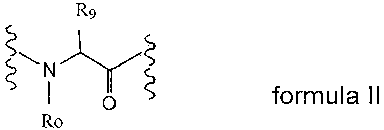 wherein the bonds, which end at the wavy lines represent linkages to units B and K and
R₉ is a group of the following formula III, and 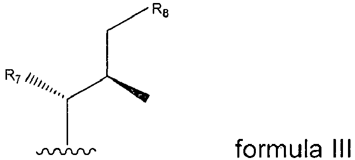 wherein R₈ is a group of the following formula IV, or 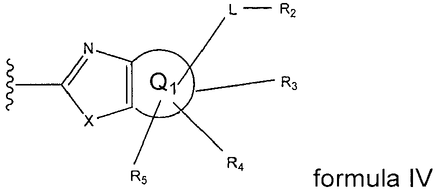 wherein R₈ is a group of the following formula V, or 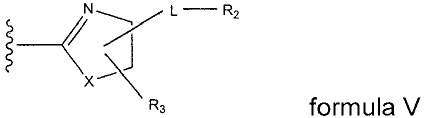 wherein R₈ is a group of the following formula VI, or 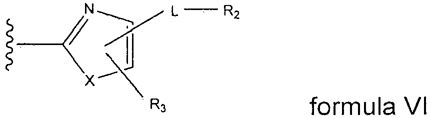 wherein R₈ is a group of the following formula VII, or 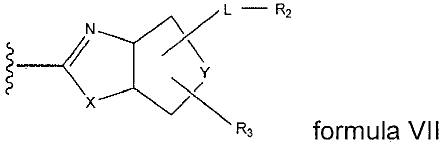 wherein R₈ is a group of the following formula VIII, wherein the residues L-R₂ and R₃ can be bonded to the same or different phenyl rings, or 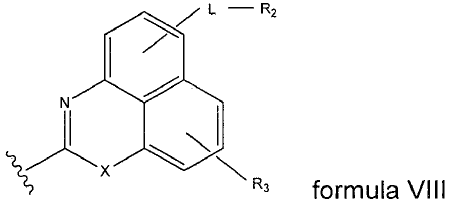 wherein R₈ is a group of the following formula IX, and wherein the residues L-R₂ and R₃ can be bonded to the same or different rings Q₂ or Q₃, or 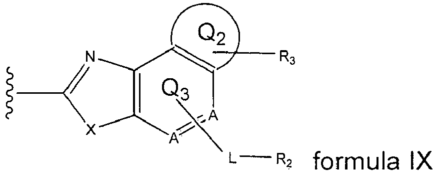 wherein R₈ is a group of the following formula X, and wherein the residues L-R₂ and R₃ can be bonded to the same or different rings Q₂ or Q₃, or 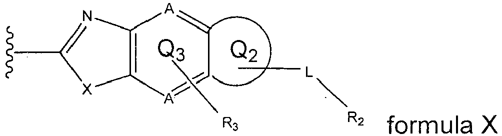 wherein R₈ is a group of the following formula XI, and wherein the residues L-R₂ and R₃ can be bonded to the same or different rings Q₂ or Q₃, or 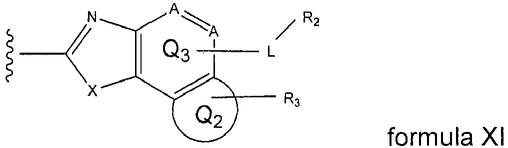 wherein R₈ is a group of the following formula XII, and wherein the residues L-R₂ and R₃ can be bonded to the same or different phenyl rings, or 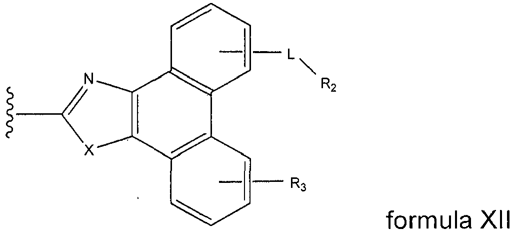 wherein R₈ is a group of the following formula XIII, 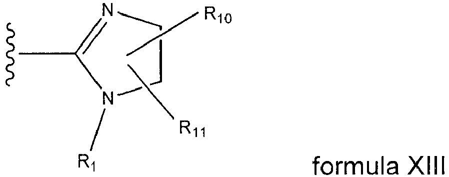 wherein
the group of formula II is covalently linked to the alpha-amino acid B in formula I through its CO by forming an amide bond and N-Rₒ in the formula II is covalently linked to a carboxy group of K by forming an amide bond, wherein
B is an amino acid, selected from the following group:
alpha-aminobutyric acid;
alanine;
threonine;
valine;
norvaline; and
molecules of alpha-aminobutyric acid, alanine, threonine, valine or
norvaline modified with a hydroxy group in the sidechain;
C represents sarcosine;
D is an amino acid, selected from the following group:
leucine;
N-methylleucine;
valine;
gamma-hydroxy-N-methylleucine; and
gamma-hydroxyleucine;
E is an amino acid, selected from the following group:
valine;
norvaline; and
a modified valine or norvaline in which a carbon atom of the side chain is substituted by a hydroxy group;
F is an amino acid, selected from the following group:
leucine;
N-methylleucine;
gamma-hydroxy-N-methylleucine; and
gamma-hydroxyleucine;
G equals alpha-aminobutyric acid or alanine;
H equals D-alanine or D-serine;
I and J are amino acids, selected independently of each other from the following group:
leucine;
N-methylleucine;
gamma-hydroxy-N-methylleucine; and
gamma-hydroxyleucine;
K equals N-methylvaline or valine;
wherein
the amino acids B to K are linked to each other by forming of amide bonds;
wherein
the used symbols and indices have the following meanings:
X represents O, S, or N-R₁;
Y represents O, S, N-R₁, -CH₂- or-CH₂CH₂-;
A as used in formulas IX, X, and XI represents CH or N;
L represents a bond, -CH₂-, -CH₂CH₂-, -CH₂CH₂CH₂-, -CH₂CH₂CH₂CH₂-, -CH=CH-, -CH=CHCH₂-, -CH₂CH=CH-, -CH₂CH₂CH=CH-, -CH₂CH=CHCH₂-, -CH=CHCH₂CH₂-, -OCH₂-, -OCH₂CH₂-, -OCH₂CH₂CH₂-, -OCH₂CH=CH-, -CONH-, -CONHCH₂ or -CONHCH₂CH₂-, -CONHCH₂CH₂OCH₂CH₂- or -CONHCH₂CH₂OCH₂CH₂OCH₂CH₂-;
Q₁ represents together with both atoms of the condensed ring an aromatic or heteroaromatic ring from the group of benzene, pyridine, pyrimidine, pyridazine, pyrazine, triazine, thiophene, furane, pyrrole, thiazole, isothiazole, oxazole, isoxazole, imidazole or pyrazole;
Q₂ represents together with both atoms of the condensed ring an aromatic or heteroaromatic ring from the group of benzene, pyridine, pyrimidine, pyridazine, pyrazine, triazine, thiophene, furane, pyrrole, thiazole, isothiazole, oxazole, isoxazole, imidazole, pyrazole, thiadiazole, oxadiazole or triazole;
Q₃ defines the six-membered ring in which it is located;
Rₒ represents H or CH₃;
R₁ represents H, (C1-C4)-alkyl or benzyl, that may also be substituted by a -COOH group;
R₂ represents a polar deprotonatable group Pₛ selected from the group consisting of:
a) -COOH, -SO₃H, -CONH₂, -CONHNH₂, -SO₂NH₂, -PO₃H₂, -PO(OH)(NH₂), -CO(NHOH), -CO(NH(O-C₁-C₄-Alkyl)), -CSNH₂, -NHCONH₂, -N(OH)CONH₂, -NHCO(NHOH)-, -NHCSNH₂, -CSNHNH₂;
b) 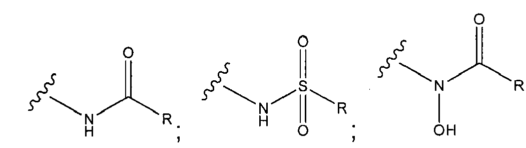 and R = -(C1-C4-alkyl), -O(C1-C4-alkyl), -NH(C1-C4-alkyl), -NH(C1-C4-alkenyl), (substituted) aryl, (substituted) O-aryl, (substituted) NH-aryl, -CF₃ and other fluorinated (C1-C4-alkyl)groups;
c) 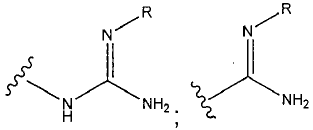 and R = -OH, -CN, -NO₂;
d) 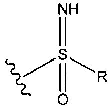 and R = (C1-C4-alkyl), (subsituted) aryl, -CF₃ and other fluorinated (C1-C4-alkyl)groups;
e) 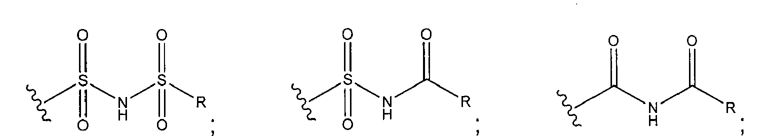 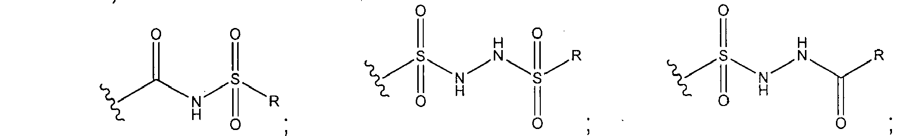 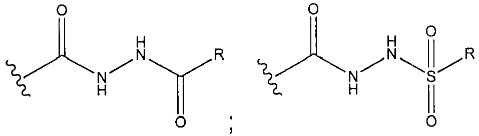 and R = -(C1-C4-alkyl), -O(C1-C4-alkyl), -NH(C1-C4-alkyl), -NH(C1-C4-alkenyl), (substituted) aryl, (substituted) O-aryl, (substituted) NH-aryl, -CF₃ and other fluorinated (C1-C4-alkyl)groups;
f) 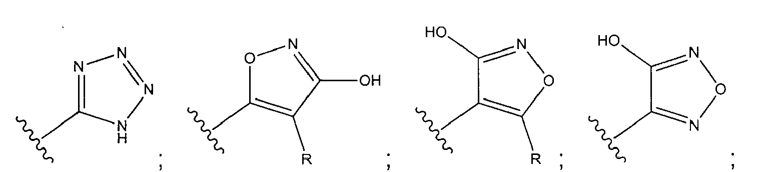 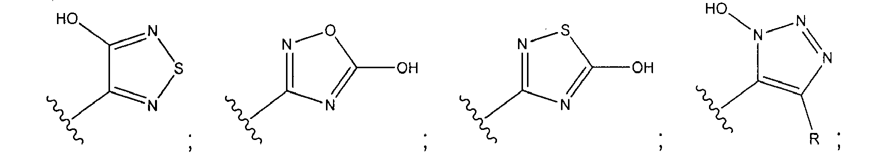 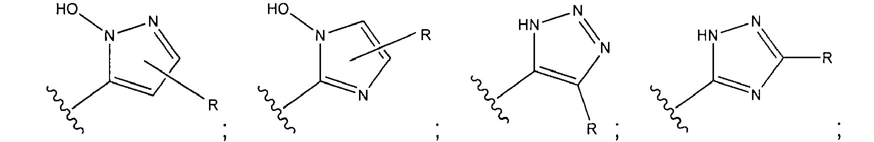 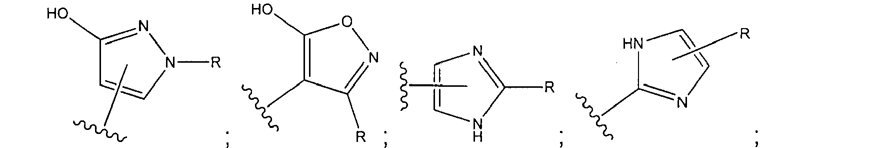 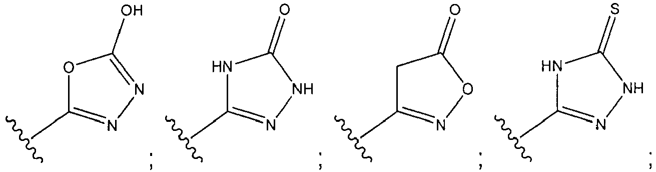 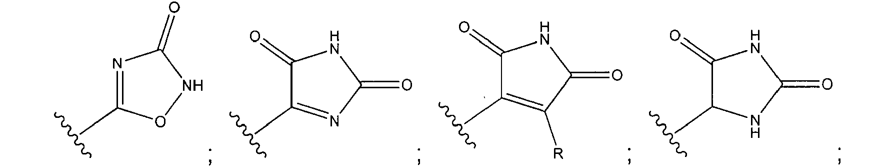 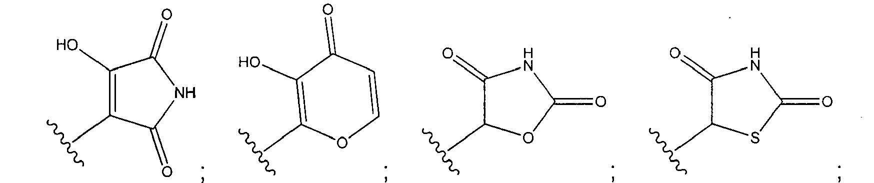 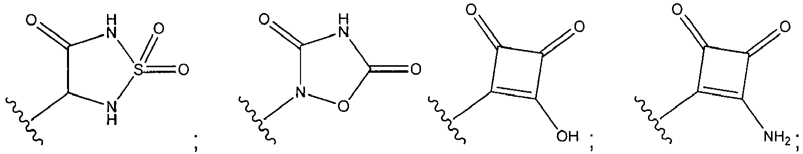 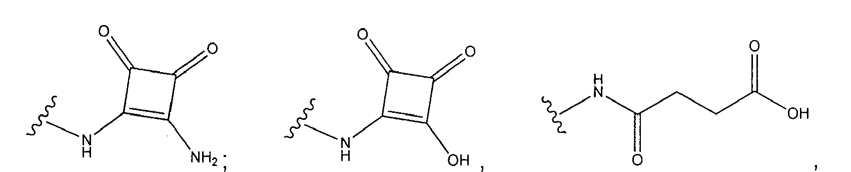 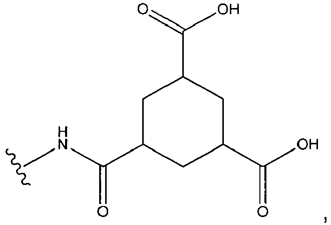 in which R = H, -(C1-C4-alkyl), -CF₃ and other fluorinated (C1-C4-alkyl)groups.
g) -OH and -SH, providing that L represents a covalent single bond, and that-OH or-SH can only be a substituent on one ring Q1, Q2 or Q3 and is at the same linked to a carbon atom that is adjacent to a nitrogen atom
or
a group Pₛ', that can be converted under physiological conditions to Pₛ, or
a polar protonatable group P_{b} selected from the group consisting of
a) 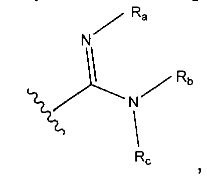 in which Rₐ, R_{b} and R_{c} represent H and -(C1-C4-alkyl), Rₐ and R_{b} may be together -(CH₂CH₂)- or -(CH₂CH₂CH₂)-and R_{b} and R_{c} may be together with the nitrogen atom pyrrolidine, piperidine or morpholine;
b) 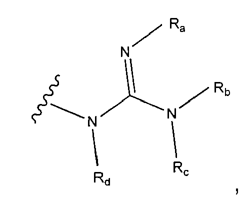 in which Rₐ, R_{b}, R_{c} and R_{d} represent H and -(C1-C4-alkyl), Rₐ and R_{b}, Rₐ and R_{d} as well as R_{b} and R_{d} may be together -(CH₂CH₂)- or -(CH₂CH₂CH₂)- and R_{b} and R_{c} may be togetherwith the nitrogen atom pyrrolidine, piperidine or morpholine;
c) an amino group selected from -NH₂, (C1-C6)alkyl-amino, (C1-C6)dialkyl-amino, pyrrolidine, piperidine, morpholine or piperazine, which can be substituted in position 4 by (C1-C6)alkyl, -(C1-C6)alkanoyl, benzyl, benzoyl or phenyl, wherein these substituents optionally bear a -COOH group
or
a group P_{b}', that can be converted under physiological conditions to P_{b};
R₃ represents H, (C1-C6)-alkyl, (C1-C6)-alkoxy, -OH, (C1-C6)-alkylthio, (C1-C6)-alkylsulfonyl, -SH, -CF₃, -COOH, -COO((C1-C6)alkyl)), -CONH₂, -CONH((C1-C6)alkyl), -CON((C1-C6)alkyl)₂, nitro, halogen, cyano, amino, (C1-C6)alkyl-amino or (C1-C6)dialkyl-amino; or R₃ exists in form of a free electron pair, if it would bind to a heteroatom of rings Q₁, Q₂ or Q₃;
R₄ and R₅ represent indepedently from each other H, (C1-C6)-alkyl, (C1-C6)-alkoxy, -CF₃, halogen or they can form together -OCH₂O-or -OCH₂CH₂O-, if R₄ and R₅ are bonded to the ring in ortho position to each other;
R₇ represents H, -OH, -OCOOR₁₂, -OCOR₁₃, -OCONR₁₄R₁₅, -O-tetrahydropyran-2-yl), -O-(tetrahydrofuran-2-yl), -O-CHR₁₆-OR₁₇ or -SiR₁₈R₁₉R₂₀;
R₁₀ and R₁₁ represent independently of each other H, (C1-C6)-alkyl, -CF₃ or halogen; or R₁₀ and R₁₁ form together -CH₂CH₂-, -CH₂CH₂CH₂-, -CH₂CH₂CH₂CH₂- or -CH₂CH₂CH₂CH₂CH₂-;
R₁₂ represents (C1-C6)-alkyl, benzyl or phenyl, wherein optionally the alkyl group is substituted by fluorine or chlorine and benzyl and phenyl are optionally substituted by one or more substituents selected from the group of (C1-C6)-alkyl, (C1-C6)-alkoxy, -CF₃, cyano, nitro or halogen;
R₁₃ represents H or R₁₂;
R₁₄ and R₁₅ represent independently of each other H, (C1-C6)-alkyl, benzyl or phenyl, wherein, if necessary, benzyl and phenyl may be substituted by one or more substituents selected from the group of (C1-C6)-alkyl, (C1-C6)-alkoxy, -CF₃, cyano, nitro or halogen;
R₁₆ represents H or (C1-C6)-alkyl;
R₁₇ represents (C1-C6)-alkyl, benzyl or phenyl
R₁₈, R₁₉ and R₂₀ represent independently of each other (C1-C6)-alkyl, benzyl or phenyl;
or a pharmaceutically acceptable salt as well as a tautomeric, enatiomeric or other stereoisomeric form thereof.

2. Compound according to claim 1, wherein R₀ is -CH₃.

3. Compound according to claim 1 or 2, wherein R₇ is selected from the group consisting of: -OH, -OCOCH₃, -OCOOCH₃, -OCH₂OCH₃, -Si(CH₃)₃ and -Si(CH₃)₂C(CH₃)₃.

4. Compound according to any one of claims 1 to 3, wherein R₁₂ is selected from the group consisting of: -COOH, -OH, -SH, -CONH₂, -CONHNH₂, -SO₃H, -SO₂NH₂, -COOCH₃, -COOCH₂CH₃, -COOCH₂CH₂CH₃, -COOCH₂CH₂CH₂CH₃, -COOCH₂CH(CH₂CH₃)₂, -COOCH(CH₃)₂, -COOC(CH₃)₃, -COOCH₂CH₂N(CH₃)₂, -COOCH₂CH₂(morpholin-4-yl) and the groups 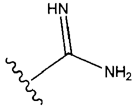 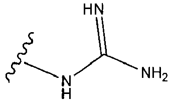 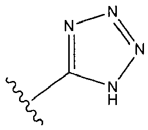 and 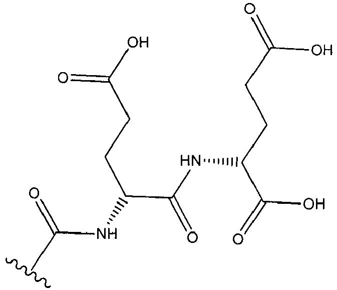 providing that, if R₁₂ is -OH or -SH, L represents a covalent single bond, and -OH or -SH are linked to a ring Q₁, Q₂ or Q₃ to a carbon atom that is adjacent to a nitrogen atom.

5. Compound according to any one of claims 1 to 4, wherein L is selected from the group consisting of: a bond, -CH₂-, -CH₂CH₂-, -CH=CH-, -CONH- and -OCH₂-.

6. Compound according to any one of claims 1 to 5, wherein X represents N-R₁ and/or wherein the ring Q₁ is benzene, pyridine or pyrimidine, or wherein the rings Q₂ and Q₃ are benzene.

7. Compound according to any one of claims 1 to 6, wherein Y is O, S, -CH₂-or -CH₂CH₂- and/or wherein R₃ is selected from the group consisting of H, -COOH, -CH₃, -OCH₃, F, Cl, Br and CN.

8. Compound according to any one of claims 1 to 7, wherein R₄ and R₅ are independently of each other H or F and/or wherein R₁₀ and R₁₁ are independently of each other H or CH₃ and/or wherein the group R₈ is a group of formula IV.

9. Compound according to claim 1, wherein the compound of formula I is the following:

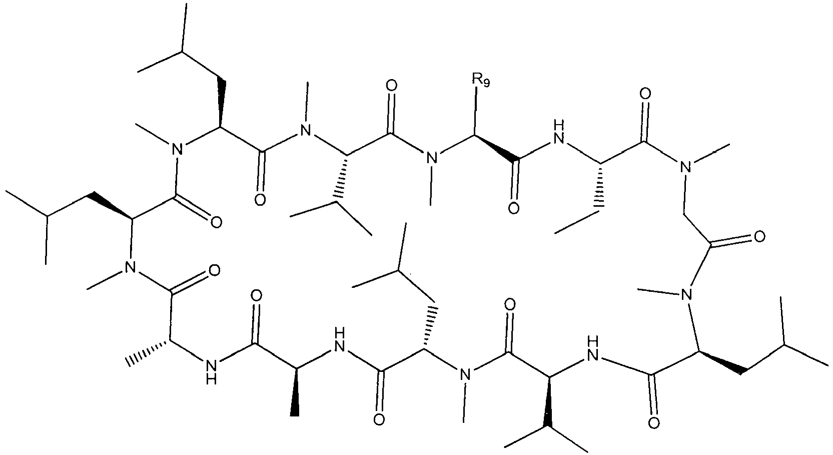

10. Compound according to claim 1, wherein the compound of formula I is the following:

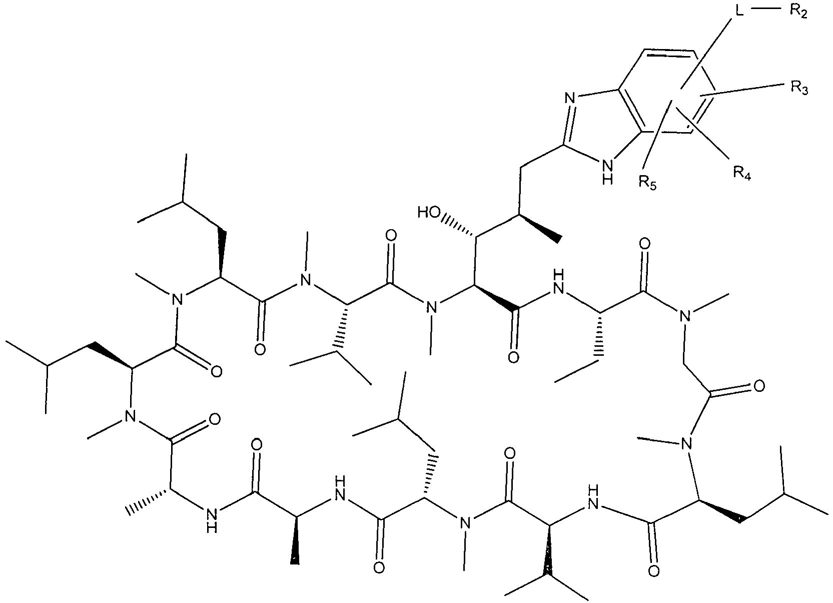

11. Compound according to claim 1, wherein the compound of formula I is one of the following:

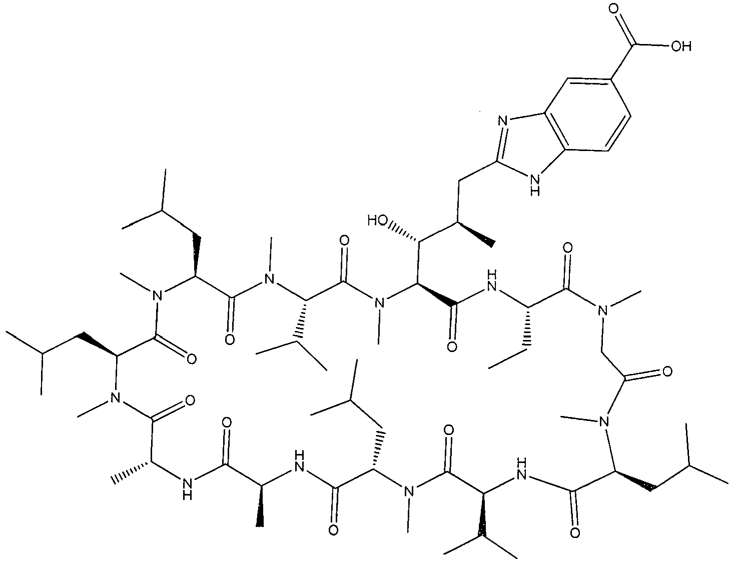

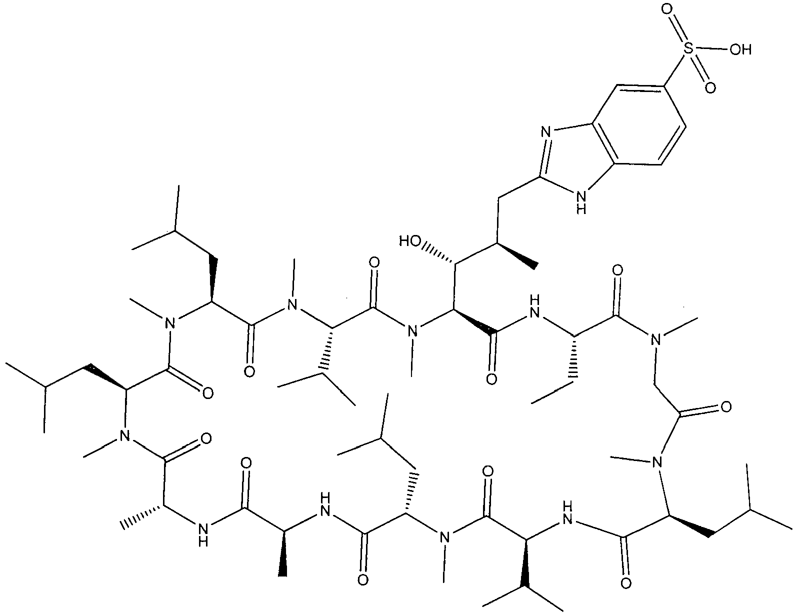

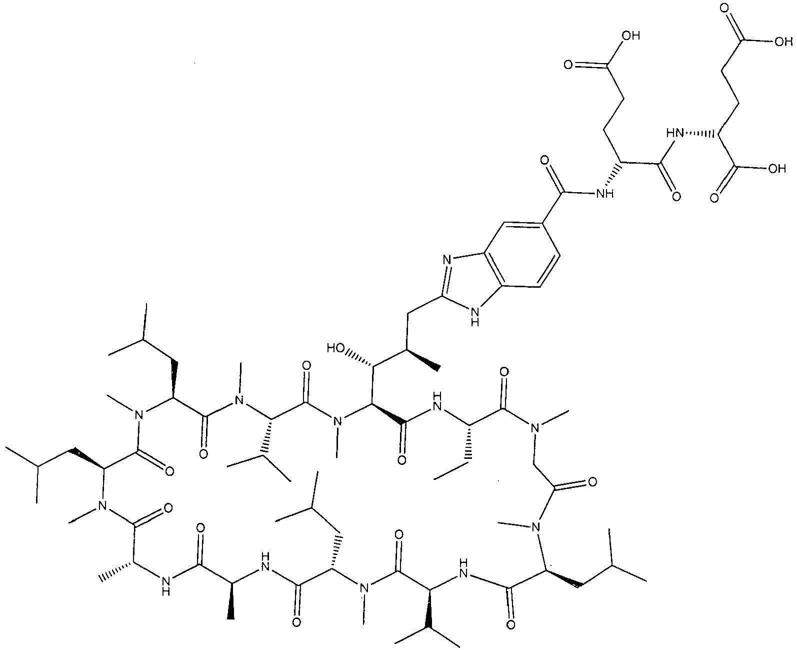

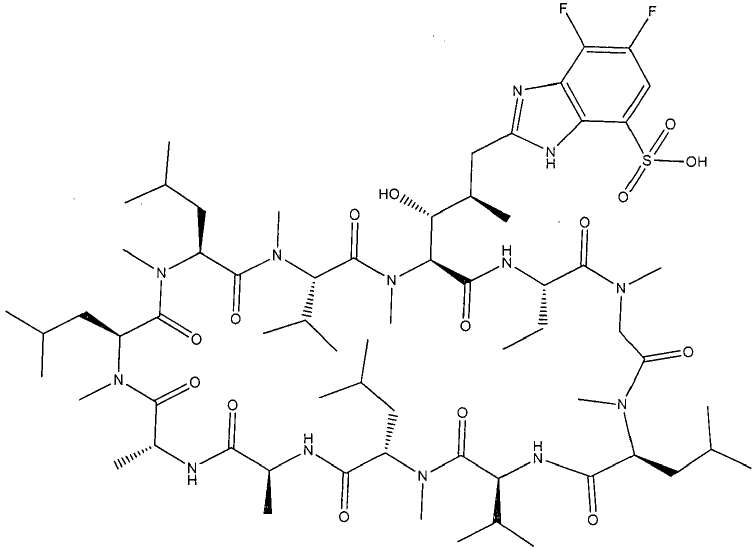

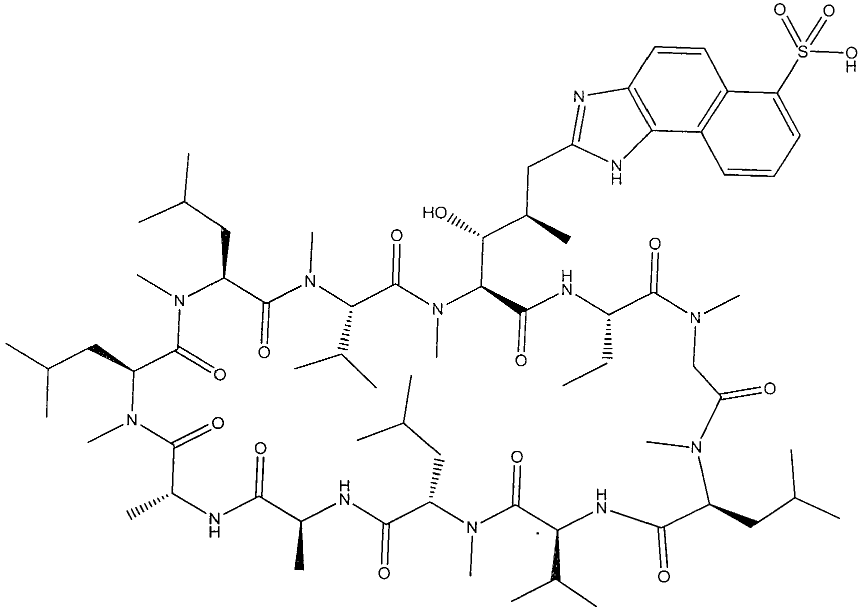

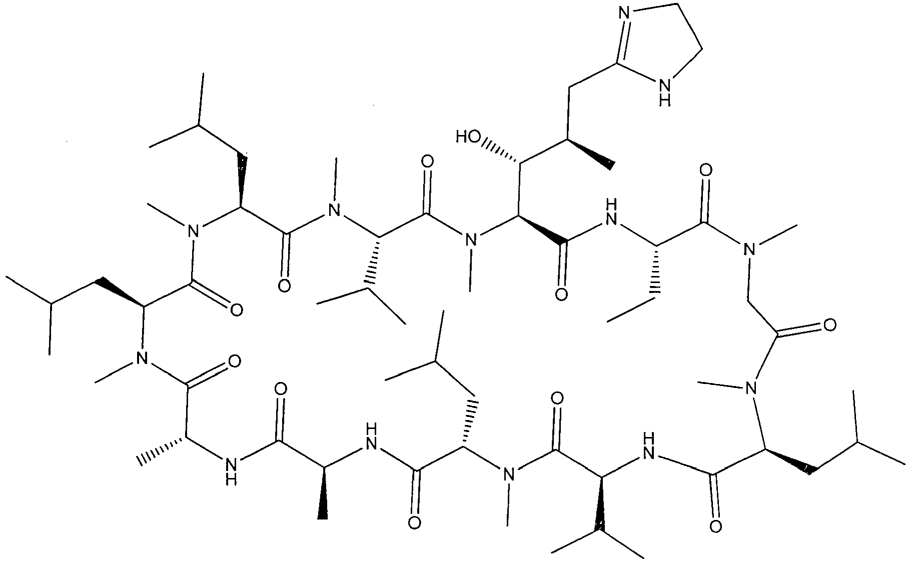

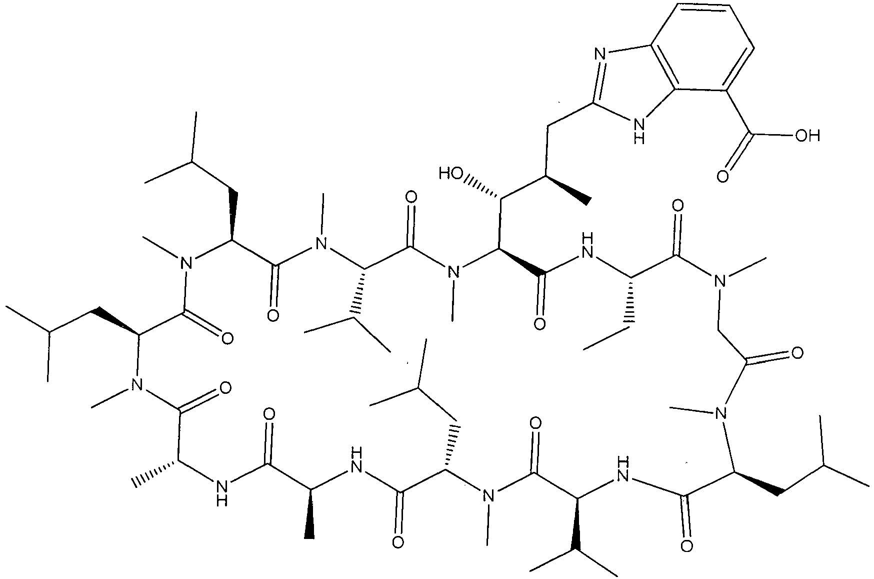

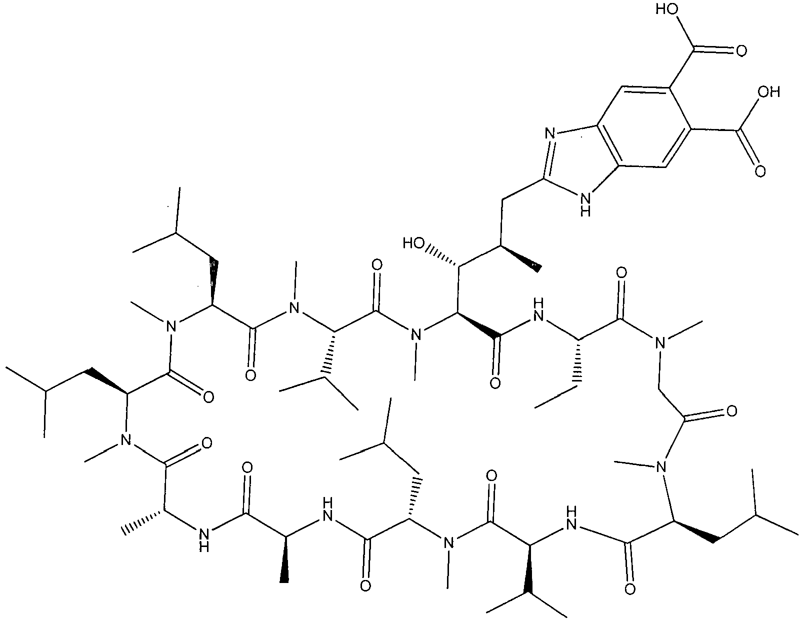

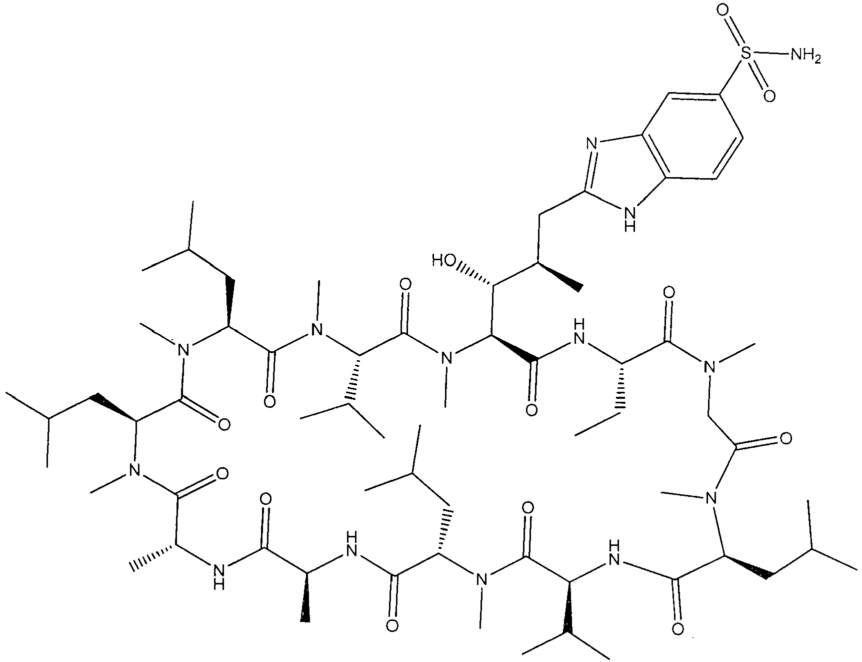

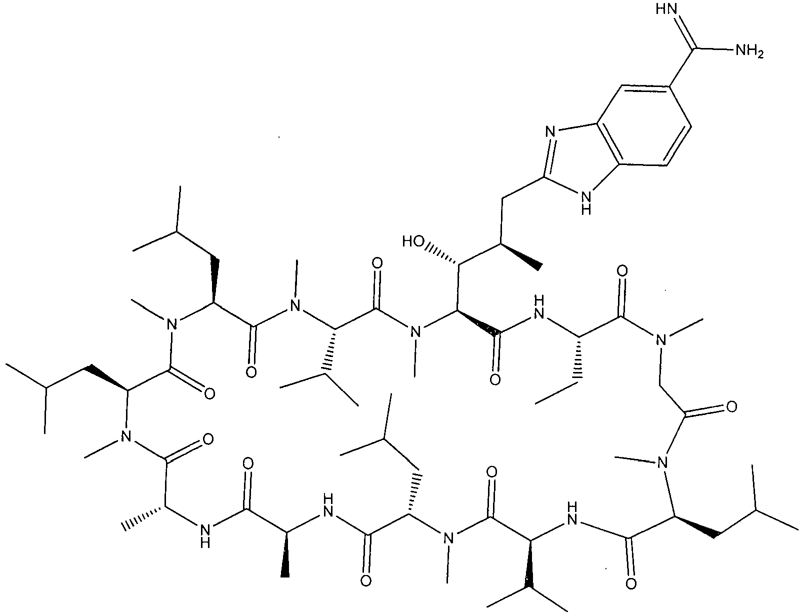

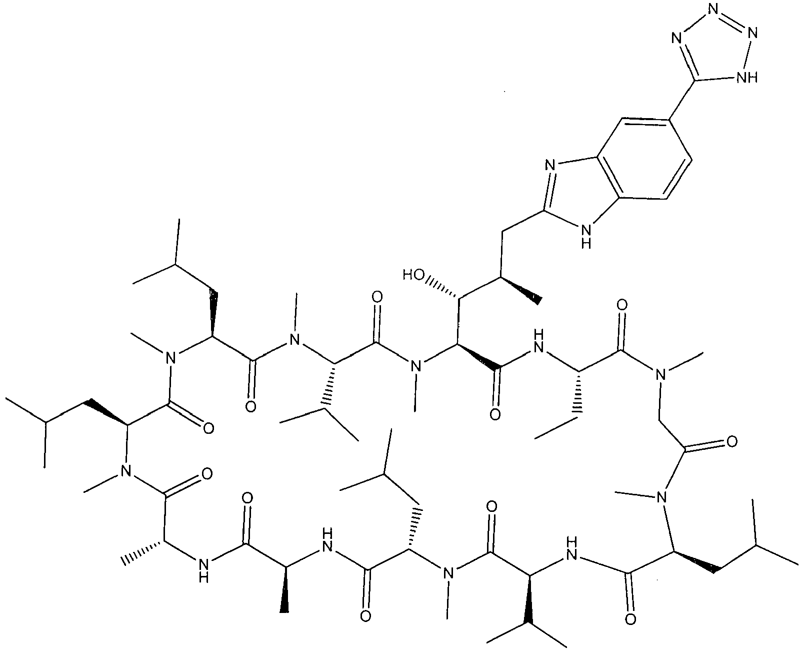

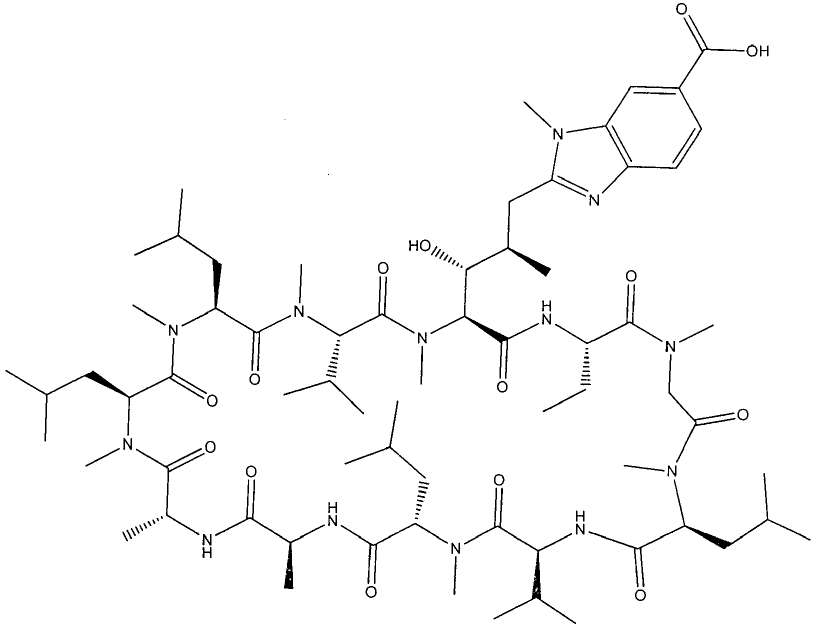

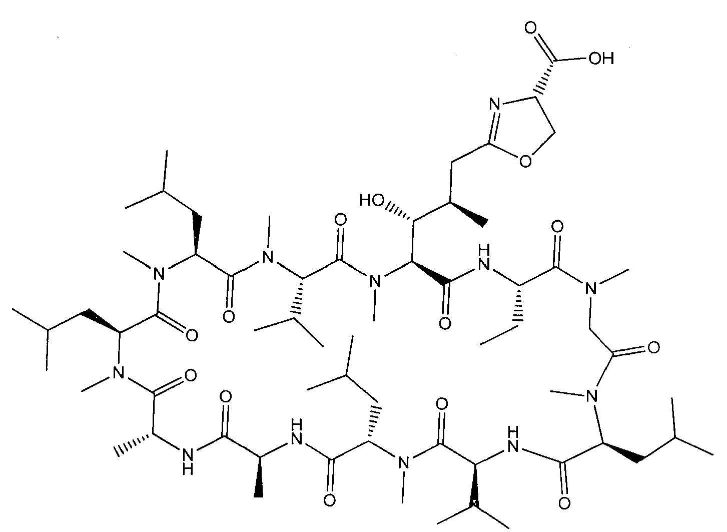

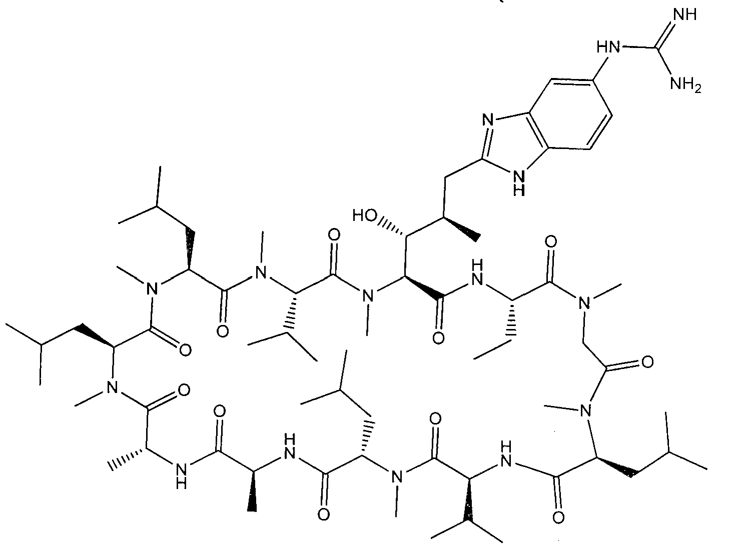

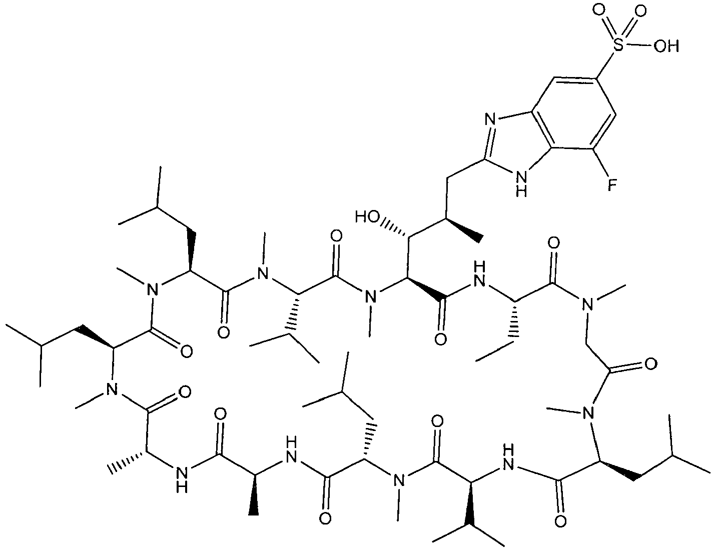

12. Indicator-coupled compound comprising a compound of formula I according to any one of claims 1 to 11, wherein one terminal atom is missing and an indicator is linked to this position.

13. Compound according to any one of claims 1 to 12 for use in medicine.

14. Compound according to any one of claims 1 to 12 for use in treatment and/or diagnosis of
a) viral Infections
b) acute and chronic inflammatory diseases
c) cancer
d) degenerative muscular diseases
e) neurodegenerative diseases, and
f) damages accompanied by impairment of the calcium homeostasis.

15. Use of a cyclosporine derivative of the following formula 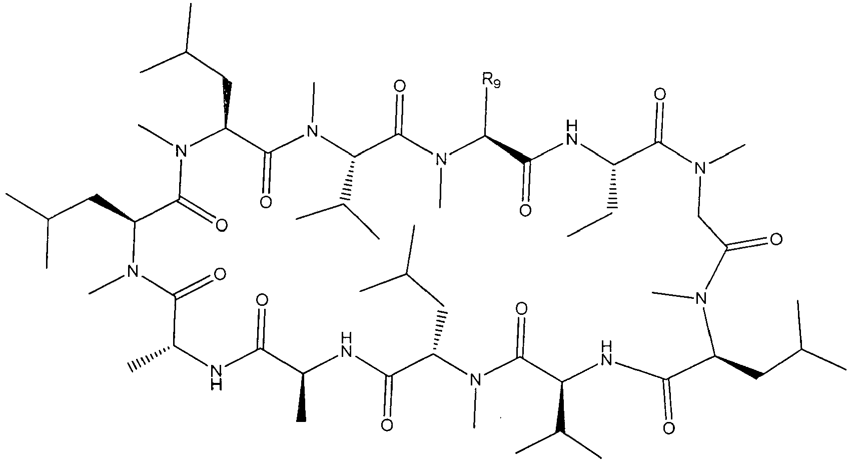 for preparation of a compound according to any one of claims 1 to 12, wherein R₉ represents a residue of the following formula: 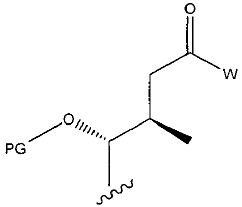 wherein PG represents an alcohol protective group and W is H or OH and the corresponding residue is linked through the bond at which end a wavy line is drawn.

## Revendications

1. Un composé de la formule (I) suivante 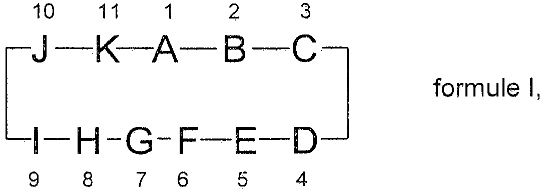 dans lequel
A est un acide aminé ayant la formule II suivante 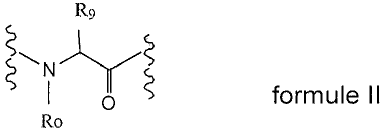 où les liaisons qui aboutissent aux lignes ondulées représentent les liens aux unités B et K, et
R⁹ est un groupement de la formule III suivante et 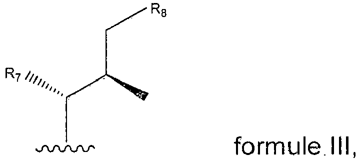
R₈ est un groupement de la formule IV suivante formule IV, ou
R₈ est un groupement de la formule V suivante 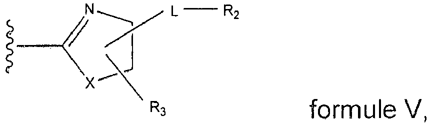 ou
R₈ est un groupement de la formule VI suivante 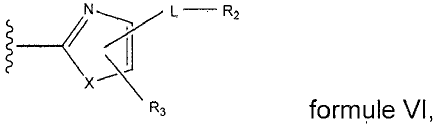 ou
R₈ est un groupement de la formule VII suivante 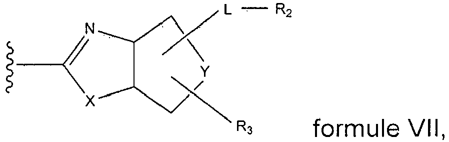 ou
R₈ est un groupement de la formule VIII suivante, dans laquelle les résidus L-R₂ et R₃ peuvent être connectés au même noyau phényle ou à des noyaux phényle différents 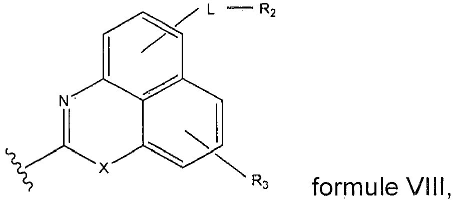 ou
R₈ est un groupement de la formule IX suivante, dans laquelle les résidus L-R₂ et R₃ peuvent être connectés au même cycle Q₂ ou Q₃, ou à des cycles différents 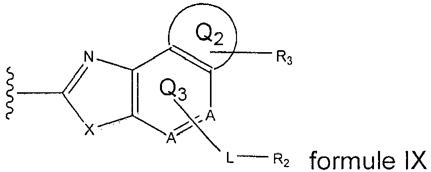
R₈ est un groupement de la formule X suivante, dans laquelle les résidus L-R₂ et R₃ peuvent être connectés au même cycle Q₂ ou Q₃, ou à des cycles différents 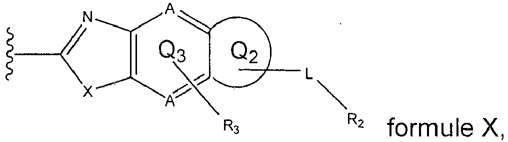 ou
R₈ est un groupement de la formule XI suivante, dans laquelle les résidus L-R₂ et R₃ peuvent être connectés au même cycle Q₂ ou Q₃, ou à des cycles différents 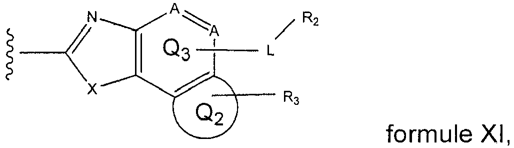 ou
R₈ est un groupement de la formule XII suivante, dans laquelle les résidus peuvent être connectés au même noyau phényle ou à des noyaux phényle différents 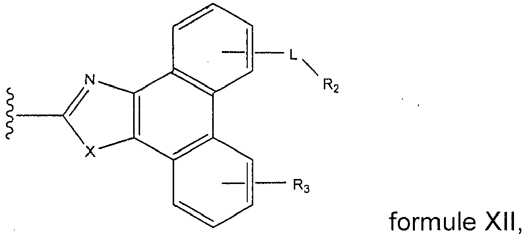 ou
R₈ est un groupement de la formule XIII suivante, 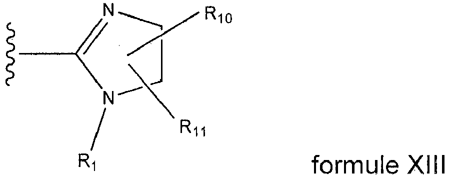 où
le groupement de formule Il est connecté de manière covalente à travers CO à l'acide alpha-aminé B dans la formule (I) par formation d'une liaison amide et N-Rₒ dans la formule Il est connecté de manière covalente à un groupement carboxylique de K par formation d'une liaison amide, où
B est un acide aminé sélectionné dans le groupe suivant:
acide alpha-aminobutyrique;
alanine;
thréonine;
valine;
norvaline; et
molécules modifiées de l'acide alpha-aminobutyrique, l'alanine, la thréonine, la valine ou la norvaline ayant dans la chaine latérale un groupement hydroxyle;
C représente sarcosine;
D est un acide aminé sélectionné dans le groupe suivant:
leucine;
N-méthyl-leucine;
valine;
gamma-hydroxy-N-méthyl-leucine; et
gamma-hydroxy-leucine;
E est un acide aminé sélectionné dans le groupe suivant:
valine;
norvaline; et
valine ou norvaline modifié, dans lesquelles un atome de carbone de la chaîne latérale est substituée par un groupement hydroxyle;
F est un acide aminé sélectionné dans le groupe suivant:
leucine;
N-méthyl-leucine;
gamma-hydroxy-N-méthyl-leucine; et
gamma-hydroxy-leucine.
G représente l'acide alpha-aminobutyrique ou l'alanine;
H représente la D-alanine ou la D-sérine;
I et J sont des acides aminés qui sont indépendamment l'un de l'autre sélectionnés dans le groupe suivant:
leucine;
N-méthyl-leucine;
gamma-hydroxy-N-méthyl-leucine; et
gamma-hydroxy-leucine;
K représente la N-méthyl-valine ou la valine;
où les acides aminés B jusqu'à K sont couplés les uns aux autres via des liaisons amide;
où les symboles et indices utilisés ont les significations suivantes:
X représente O, S ou N-R₁;
Y représente O, S, N-R₁, -CH₂- ou -CH₂CH₂-;
A comme utilisé dans les formules IX, X et XI représente CH ou N;
L représente une liaison, -CH₂-, -CH₂CH₂-, -CH₂CH₂CH₂-, -CH₂CH₂CH₂CH₂-, -CH=CH-, -CH=CHCH₂-, -CH₂CH=CH-, -CH₂CH₂CH=CH-, -CH₂CH=CHCH₂-, -CH=CHCH₂CH₂-, -OCH₂-, -OCH₂CH₂-, -OCH₂CH₂CH₂-, -OCH₂CH=CH-, -CONH-, -CONHCH₂ ou -CONHCH₂CH₂-, -CONHCH₂CH₂OCH₂CH₂- ou -CONHCH₂CH₂OCH₂CH₂OCH₂CH₂-;
Q₁ représente conjointement avec les deux atomes du cycle condensé un cycle aromatique ou hétéroaromatique choisi parmi le groupe constitué par benzène, pyridine, pyrimidine, pyridazine, pyrazine, triazine, thiophène, furane, pyrrole, thiazole, isothiazole, oxazole, isoxazole, imidazole ou pyrazole;
Q₂ représente conjointement avec les deux atomes du cycle condensé un cycle aromatique ou hétéroaromatique choisi parmi le groupe constitué par benzène, pyridine, pyrimidine, pyridazine, pyrazine, triazine, thiophène, furane, pyrrole, thiazole, l'isothiazole, l'oxazole, isoxazole, imidazole, pyrazole, thiadiazole, oxadiazole ou triazole;
Q₃ désigne le cycle à six chainons au milieu duquel il est situé;
Ro représente H ou CH₃;
R₁ représente H, (C1-C4)-alkyle ou benzyle, qui peuvent également être substitués par un groupement -COOH;
R₂ représente un groupement polaire déprotoné Ps, sélectionné à partir du groupe constitué par:
a) -COOH, -SO₃H, -CONH₂, -CONHNH₂, -SO₂NH₂, -PO₃H₂, -PO(OH)(NH₂), -CO(NHOH), -CO(NH(O-C₁-C₄-alkyle)), -CSNH₂, -NHCONH₂, -N(OH)CONH₂, -NHCO(NHOH)-, -NHCSNH₂, -CSNHNH₂;
b) 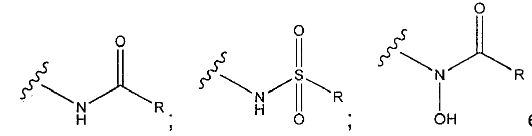 et R = -(C1-C4-alkyle), -O(C1-C4-alkyle), -NH(C1-C4-alkyle), -NH(C1-C4-alcényle), aryle substitué, O-aryle substitué, NH-aryle substitué, -CF₃ et d'autres groupements (C1-C4-alkyle) fluorés;
c) 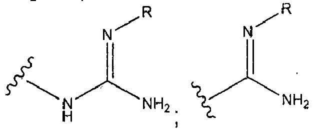 et R = -OH, -CN, -NO₂;
d) 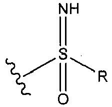 et R = (C1-C4-alkyle), aryle substitué, -CF₃ et d'autres groupements (C1-C4-alkyle) fluorés;
e) 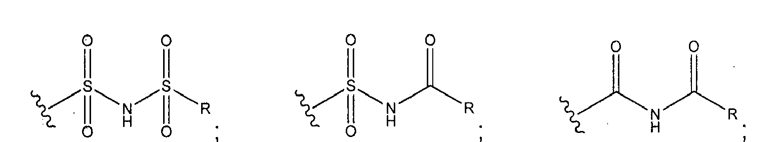 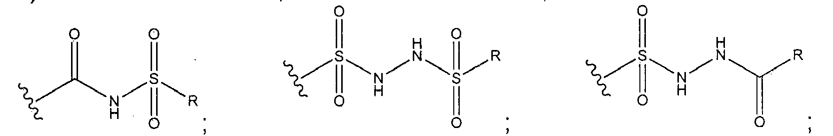 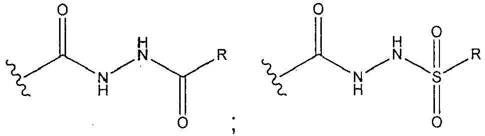 et R = -(C1-C4-alkyle), -O(C1-C4-alkyle), -NH(C1-C4-alkyle), -NH(C1-C4-alcényle), aryle substitué, O-aryle substitué, NH-aryle substitué, -CF₃ et d'autres groupements (C1-C4-alkyle) fluorés;
f) 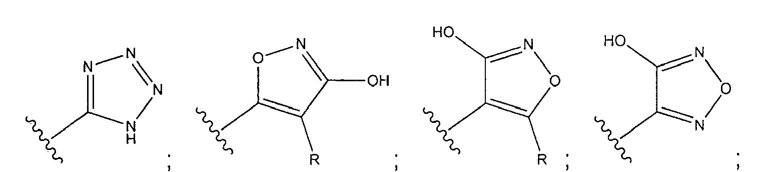 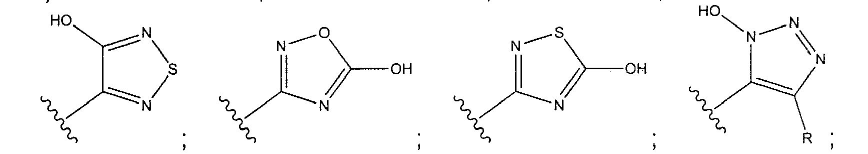 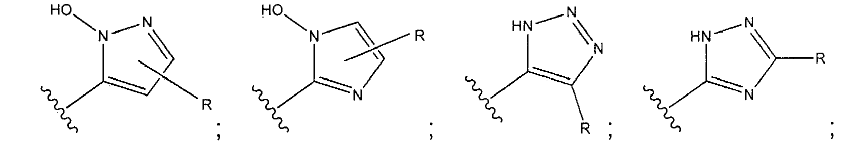 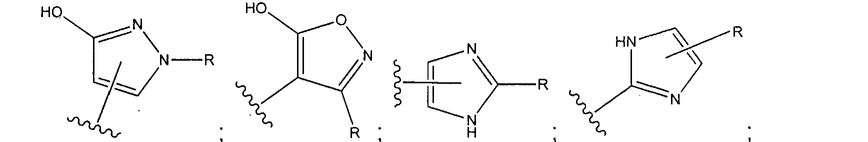 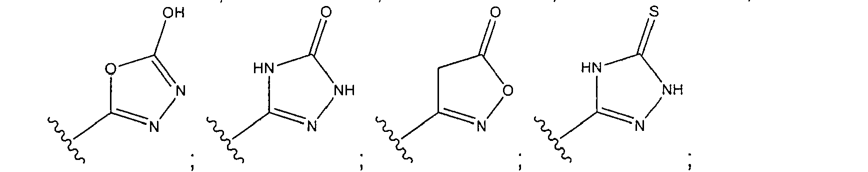 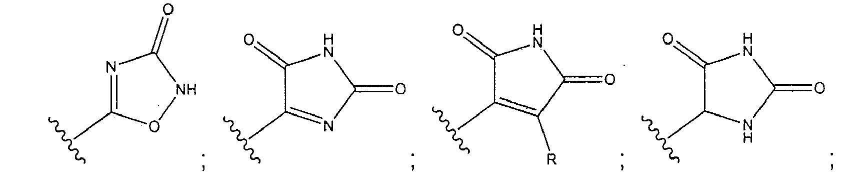 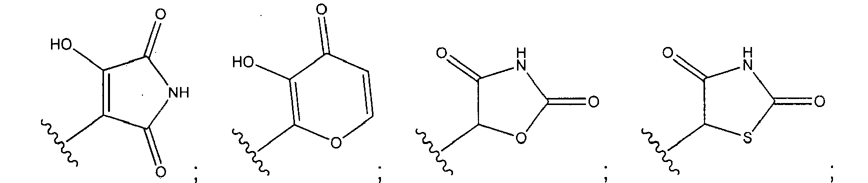 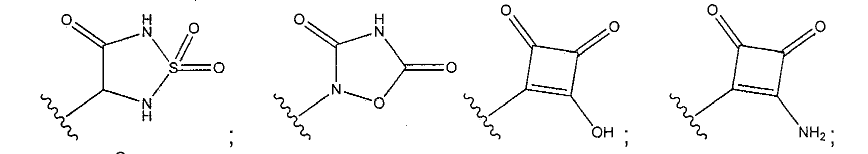 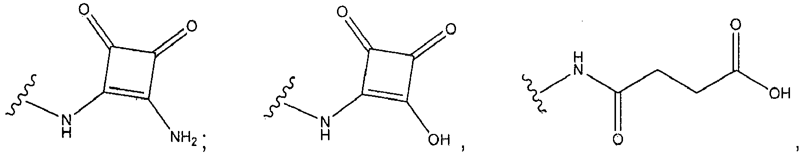 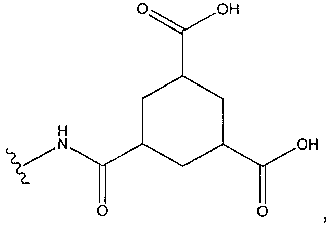 où R = H, -(C1-C4-alkyle), -CF₃ et d'autres groupements (C1-C4-alkyle) fluorés;
g) -OH et -SH, avec la condition que L représente une liaison covalente simple et que -OH ou -SH peut être substituant d'un seul noyau Q1, Q₂ ou Q3 et est connecté à un atome de carbone qui se trouve dans le voisinage d'un atome d'azote
ou
un groupement P_{s'} qui peut être converti en groupement Pₛ dans des conditions physiologiques, ou
un groupement polaire protonable P_{b}, sélectionné à partir du groupe constitué par:
a) 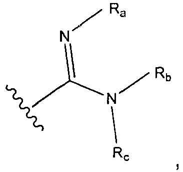 où Rₐ, R_{b} et R_{c} représentent H et -(C1-C4-alkyle), Rₐ et R_{b} peuvent être ensemble -(CH₂CH₂)- ou -(CH₂CH₂CH₂)- et R_{b} et R_{c} ensemble avec l'atome d'azote peuvent être pyrrolidine, pipéridine ou morpholine;
b) 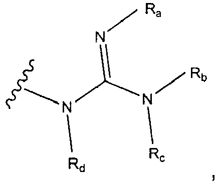 où Rₐ, R_{b}, R_{c} et R_{d} représentent H et -(C1-C4-alkyle), Rₐ et R_{b}, Rₐ et R_{d}, ainsi que R_{b} et R_{d} peuvent être ensemble -(CH₂CH₂)- ou -(CH₂CH₂CH₂)- et R_{b} et R_{c} ensemble avec l'atome d'azote peuvent être pyrrolidine, pipéridine ou morpholine;
c) un groupement amino sélectionné à partir de -NH₂, (C1-C6)alkyl-amine, (C1-C6)dialkyl-amine, pyrrolidine, pipéridine, morpholine ou pipérazine, qui peut être substitué en position 4 par un (C1-C6)alkyle, -(C1-C6) alkanoyle, benzyle, benzoyle ou phényle, où les substituants peuvent optionnellement être substitués par un groupement -COOH
ou
un groupement P_{b}' qui peut être converti en groupement P_{b} dans des conditions physiologiques;
R₃ représente H, (C1-C6)-alkyle, (C1-C6)-alcoxy, -OH, (C1-C6)-alkylthio, (C1-C6)-alkylsulfonyle, -SH, -CF₃, -COOH, -COO((C1-C6)alkyle)), -CONH₂, -CONH((C1-C6)alkyle), -CON((C1-C6)alkyle)₂, nitro, halogène, cyano, amino, (C1-C6)alkyl-amino ou (C1-C6)dialkyl-amino; ou R₃ se trouve sous forme d'une paire d'électrons libre lorsqu'il est lié au un hétéroatome des noyaux Q1, Q₂ ou Q3;
R₄ et R₅ représentent indépendamment l'un de l'autre H, (C1-C6)-alkyle, (C1-C6)-alcoxy, -CF₃, halogène ou lorsque R₄ et R₅ sont connectés en position ortho au noyau, ils peuvent former ensemble un groupement -OCH₂O- ou -OCH₂CH₂O-;
R₇ représente H, -OH, -OCOOR₁₂, -OCOR₁₃, -OCONR₁₄R₁₅, -O-(tétrahydropyran-2-yl), -O-(tétrahydrofuran-2-yl), -O-CHR₁₆-OR₁₇ ou -SiR₁₈R₁₉R₂₀;
R₁₀ et R₁₁ représentent indépendamment l'un de l'autre H, (C1-C6)-alkyle, -CF₃ ou halogène; ou R₁₀ et R₁₁ forment ensemble un groupement -CH₂CH₂-, -CH₂CH₂CH₂-, -CH₂CH₂CH₂CH₂- ou -CH₂CH₂CH₂CH₂CH₂-;
R₁₂ représente (C1-C6)-alkyle, benzyle ou phényle, où éventuellement le groupement alkyle peut être substitué par fluor ou chlore, et éventuellement benzyle et phényle peut être substitué par un ou plusieurs substituants du groupe: (C1-C6)-alkyle, (C1-C6)-alcoxy, -CF₃, cyano, nitro ou halogène;
R₁₃ représente H ou R₁₂;
R₁₄ et R₁₅ représentent indépendamment l'un de l'autre H, (C1-C6)-alkyle, benzyle ou phényle, où éventuellement benzyle et phényle peuvent être substitués par un pu plusieurs substituants du groupe (C1-C6)-alkyle, (C1-C6)-alcoxy, -CF₃, cyano, nitro ou halogène;
R₁₆ représente H ou (C1-C6)-alkyle;
R₁₇ représente (C1-C6)-alkyle, benzyle ou phényle;
R₁₈, R₁₉ et R₂₀ représentent indépendamment l'un de l'autre (C1-C6)-alkyle, benzyle ou phényle;
ou un sel pharmaceutiquement acceptable, une forme tautomérique, énantiomérique ou autre forme stéréoisomérique de celui-ci.

2. Composé selon la revendication 1, dans lequel R₀ représente -CH₃.

3. Composé selon la revendication 1 ou 2, dans lequel R₇ est sélectionné à partir du groupe constitué par : -OH, -OCOCH₃, -OCOOCH₃, -OCH₂OCH₃, -Si(CH₃)₃ et -Si(CH₃)₂C(CH₃)₃.

4. Composé selon l'une quelconque des revendications 1 - 3, dans lequel R₁₂ est sélectionné à partir du groupe constitué par: -COOH, -OH, -SH, -CONH₂, -CONHNH₂, -SO₃H, -SO₂NH₂, -COOCH₃, -COOCH₂CH₃, -COOCH₂CH₂CH₃, -COOCH₂CH₂CH₂CH₃, -COOCH₂CH(CH₂CH₃)₂, -COOCH(CH₃)₂, -COOC(CH₃)₃, -COOCH₂CH₂N(CH₃)₂, -COOCH₂CH₂(morpholin-4-yl) et des groupements 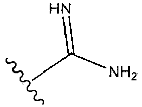 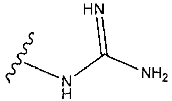 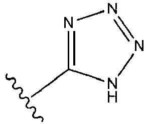 et 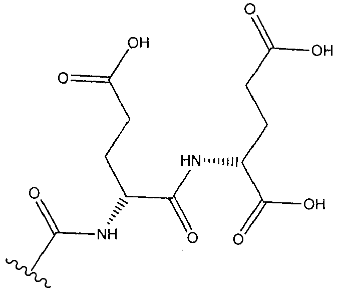 à condition que lorsque R₁₂ représente -OH ou -SH, L représente une liaison covalente simple et que -OH ou -SH est lié à un seul cycle Q₁, Q₂ ou Q₃ à un atome de carbone se trouvant dans le voisinage d'un atome d'azote.

5. Composé selon l'une quelconque des revendications 1 - 4, dans lequel L est sélectionné à partir du groupe constitué par: une liaison, -CH₂-, -CH₂CH₂-, -CH=CH-, -CONH- et -OCH₂-.

6. Composé selon l'une quelconque des revendications 1 - 5, dans lequel X représente N-R₁ et/ou le cycle Q₁ est benzène, pyridine ou pyrimidine, ou les cycles Q₂ et Q₃ sont benzène.

7. Composé selon l'une quelconque des revendications 1 - 6, dans lequel Y représente O, S, -CH₂- ou -CH₂CH₂- et/ou où R₃ est sélectionné à partir du groupe constitué par H, -COOH, -CH₃, -OCH₃, F, Cl, Br et CN.

8. Composé selon l'une quelconque des revendications 1 - 7, dans lequel R₄ et R₅ sont indépendamment l'un de l'autre H ou F et/ou où R₁₀ et R₁₁ sont indépendamment l'un de l'autre H ou CH₃ et/ou où le groupement R₈ est un groupement de formule IV.

9. Composé selon la revendication 1, où le composé de formule I est le suivant:

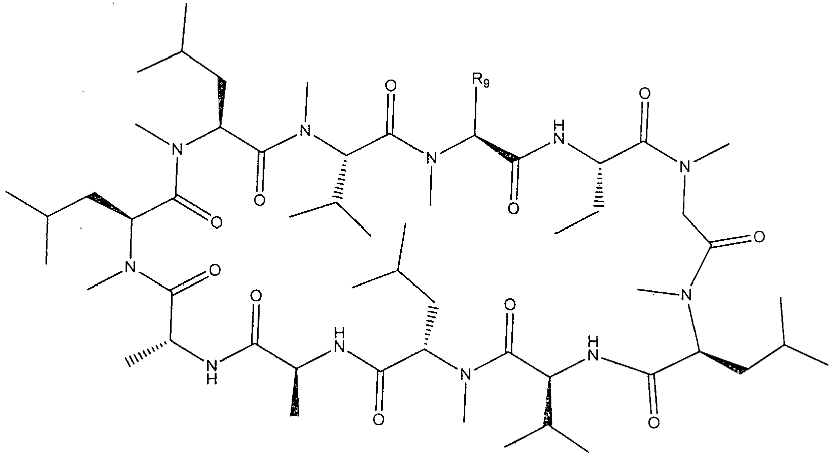

10. Composé selon la revendication 1, où le composé de formule I est le suivant:

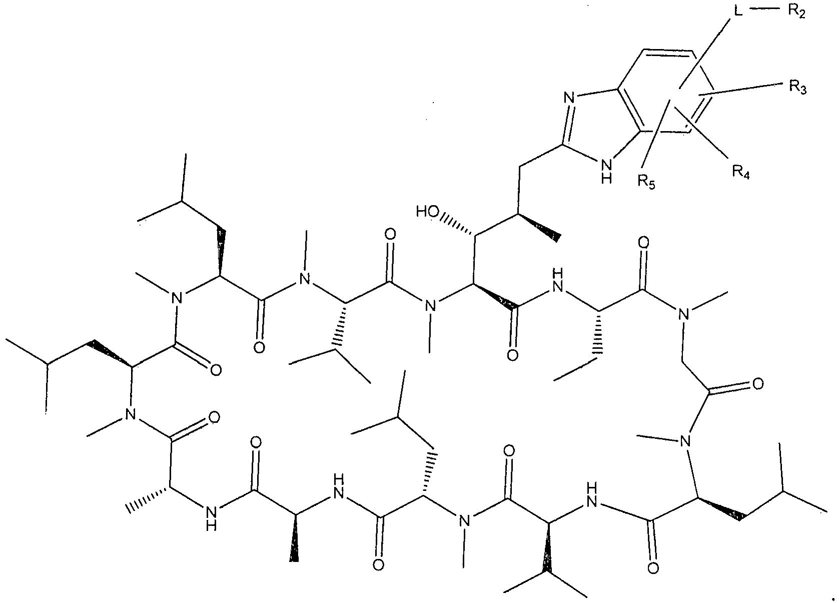

11. Composé selon la revendication 1, où le composé de formule I est le suivant:

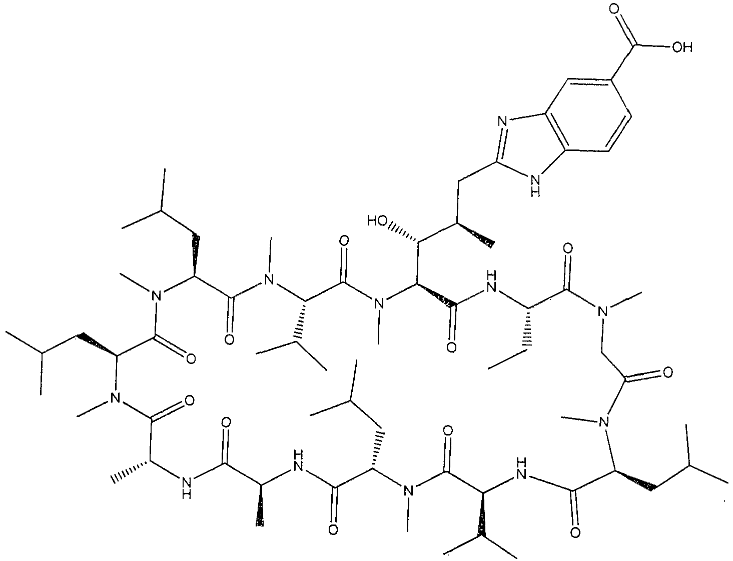

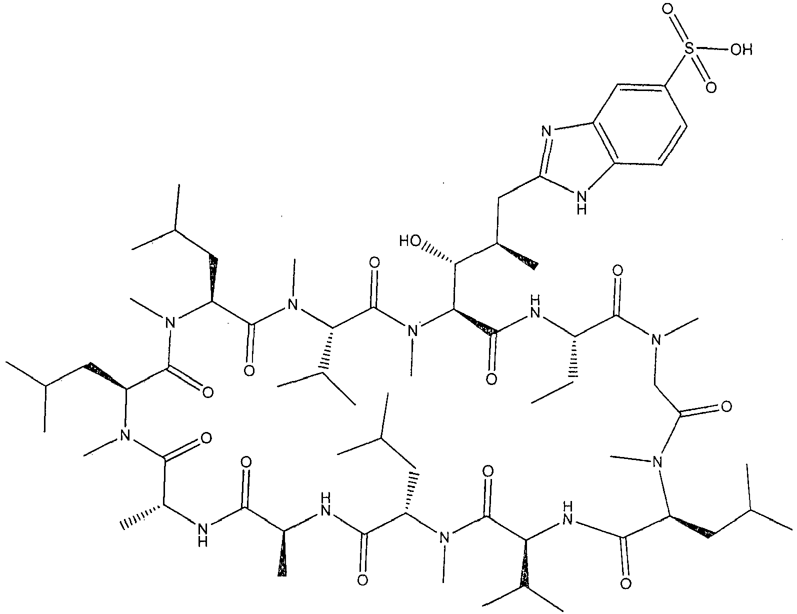

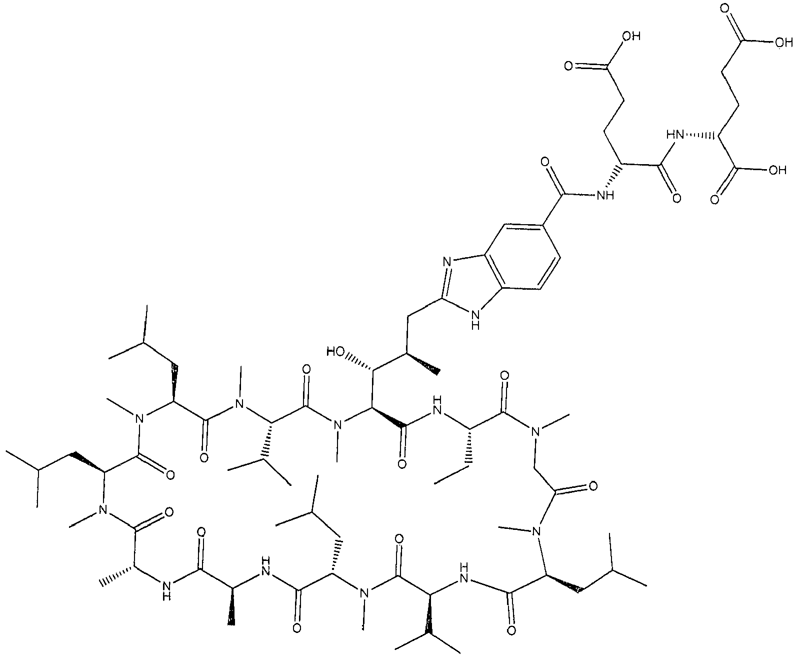

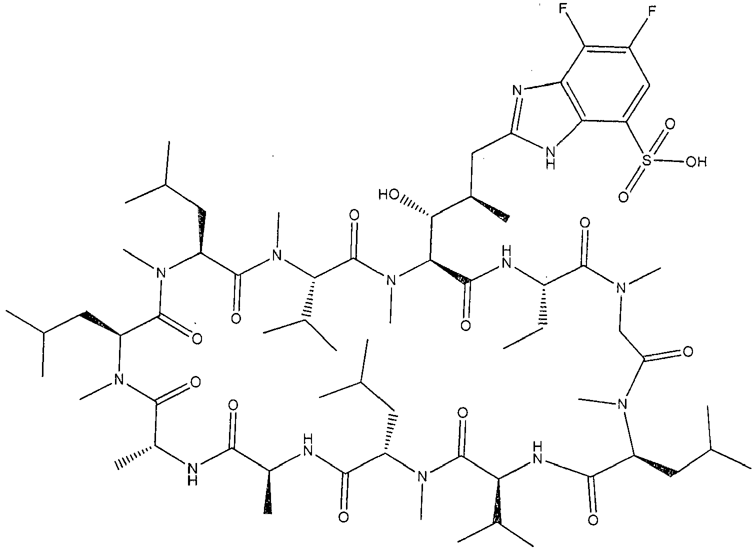

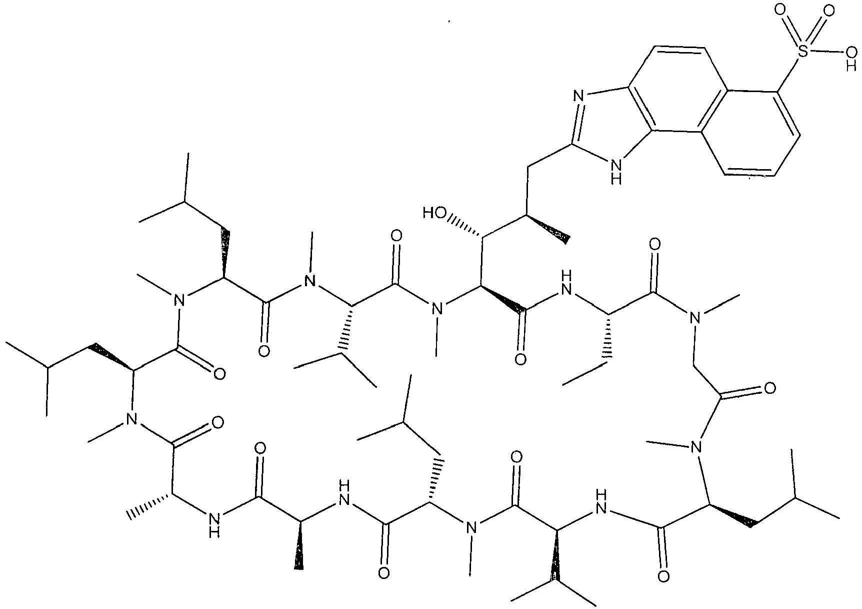

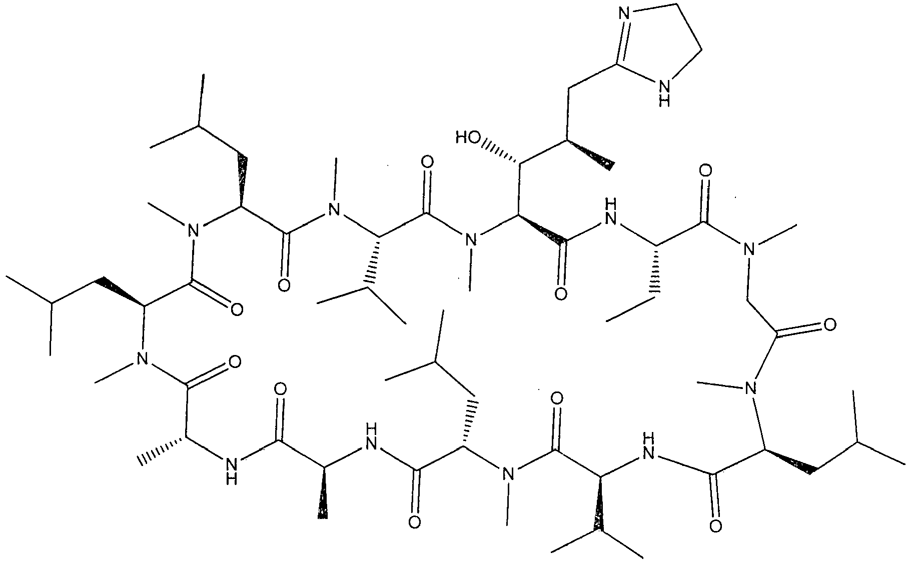

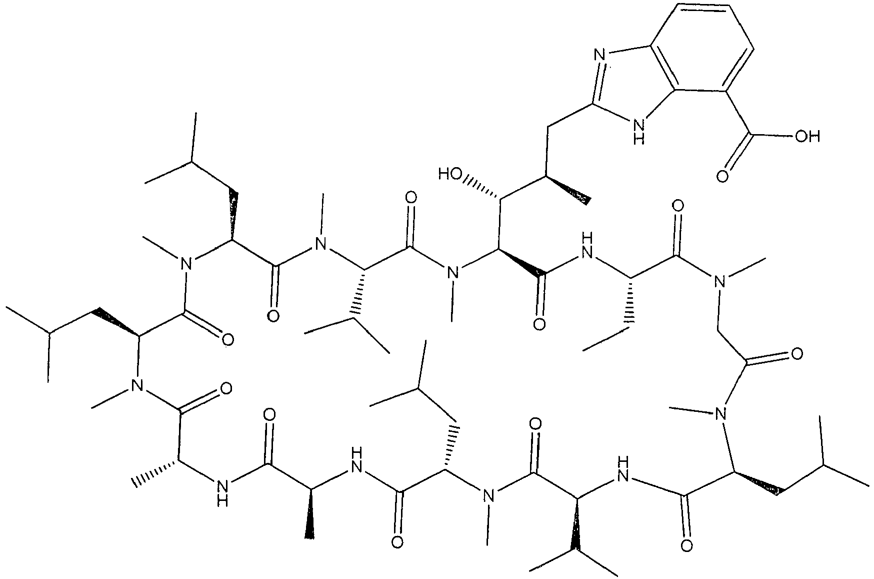

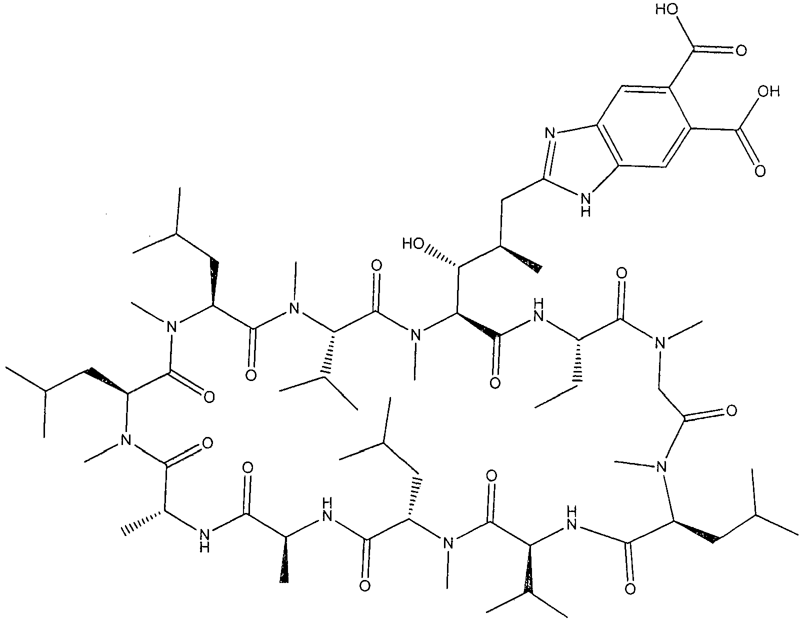

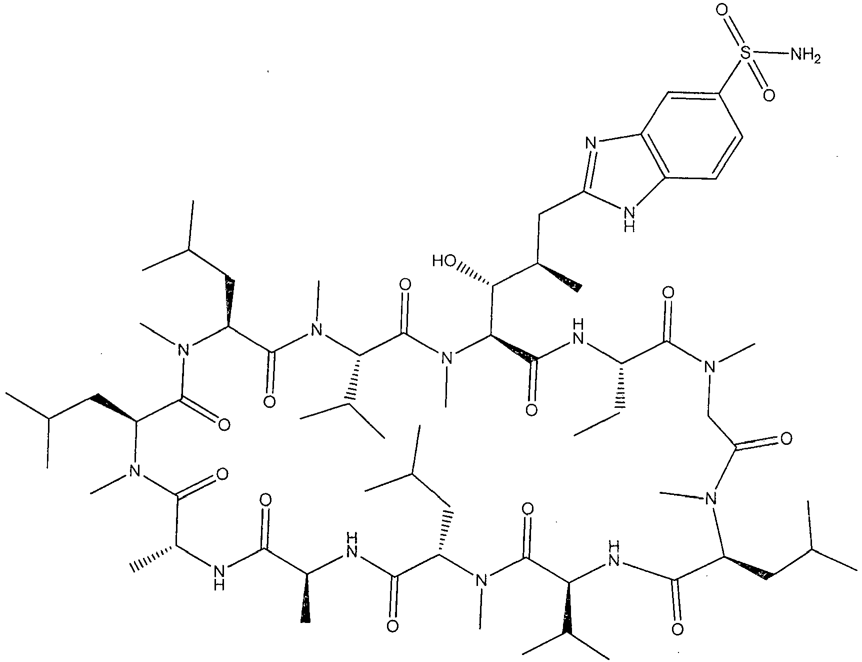

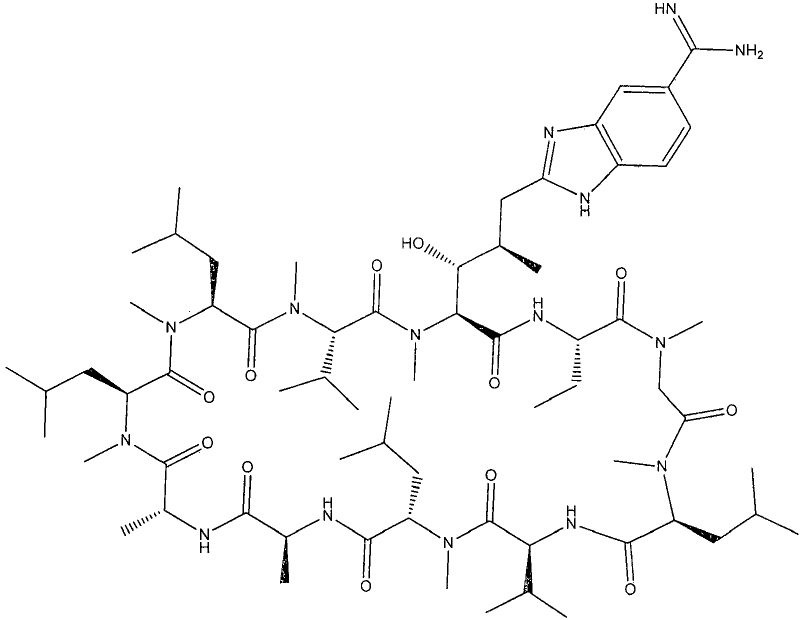

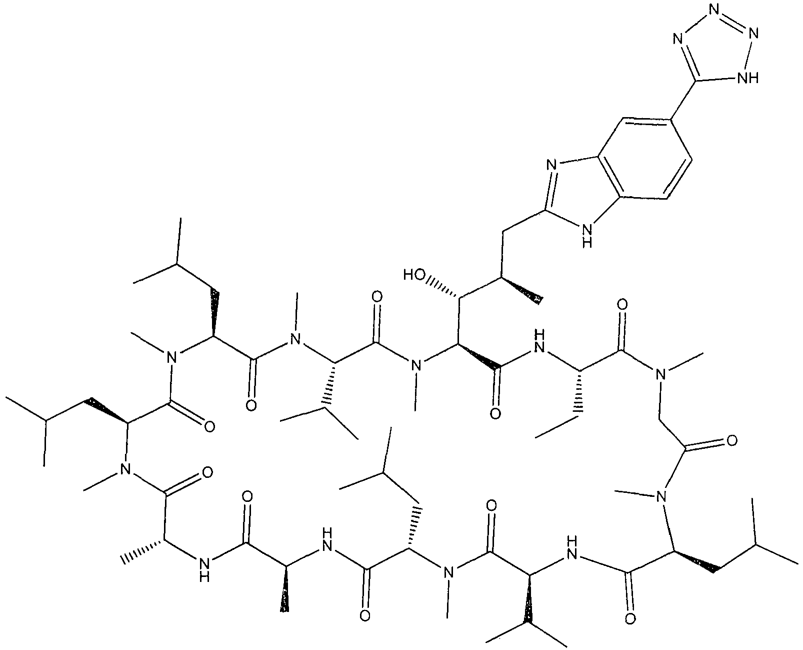

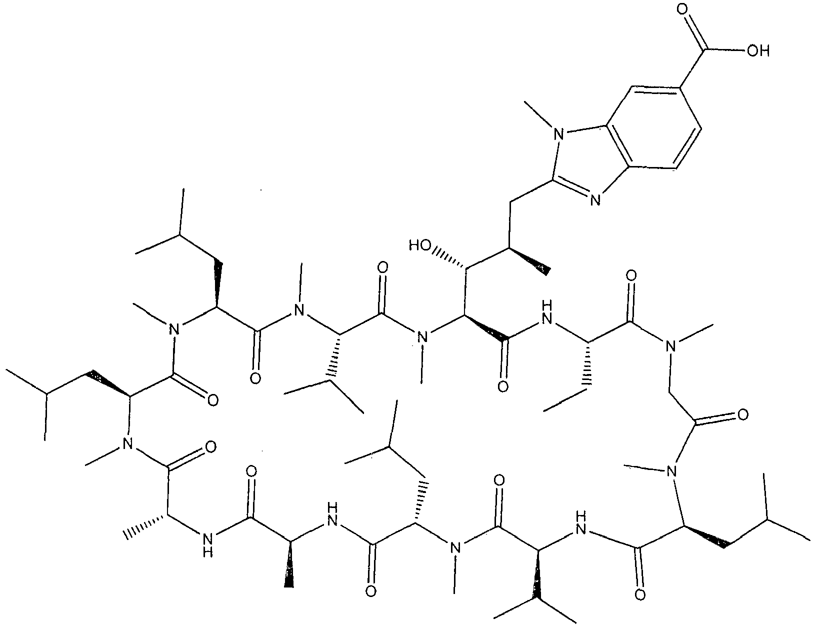

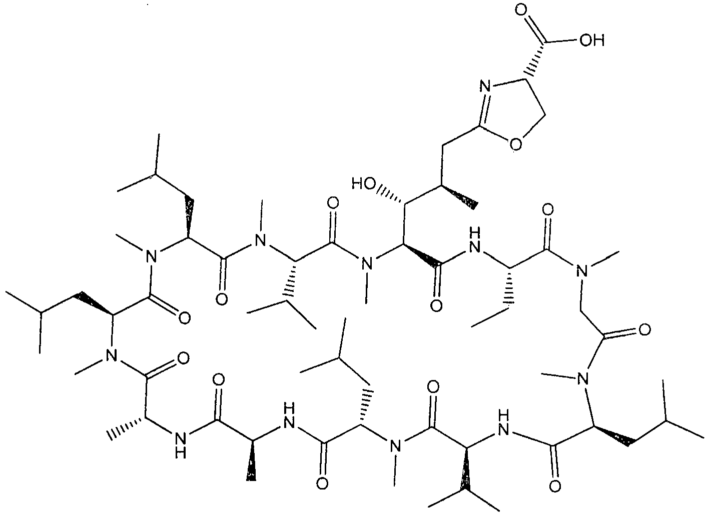

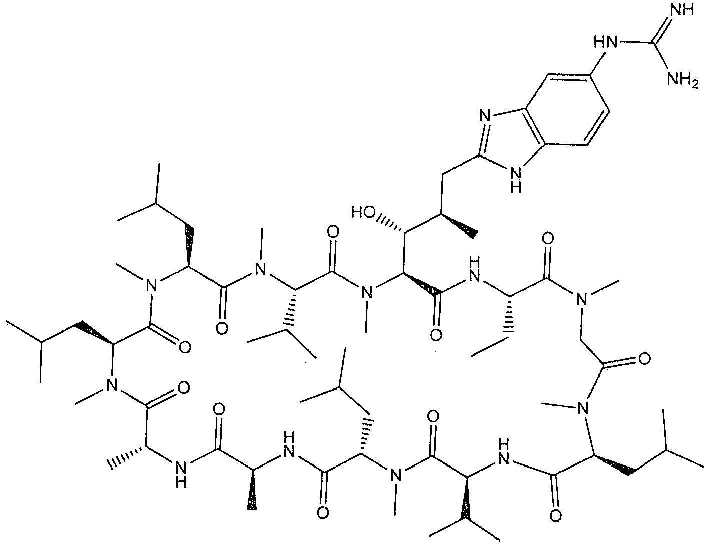

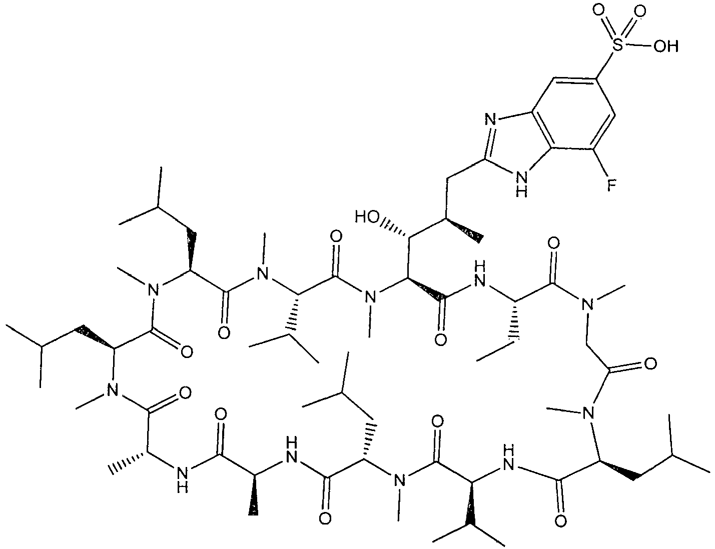

12. Composé couplé à un indicateur comprenant un composé de formule I selon l'une quelconque des revendications 1 à 11, dans lequel un atome terminal est absent et à cette position un indicateur est lié.

13. Composé selon l'une quelconque des revendications 1 à 12 destiné à être utilisé en médicine.

14. Composé selon l'une quelconque des revendications 1 à 12 destiné à être utilisé dans le traitement et/ou le diagnostic
a) des infections virales,
b) des maladies inflammatoires aigues et chroniques,
c) du cancer,
d) des maladies musculaires dégénératives,
e) des maladies neuro-dégénératives, et
f) des lésions associées à l'insuffisance de l'homéostasie calcique.

15. Utilisation d'un dérivé de cyclosporine de formule suivante 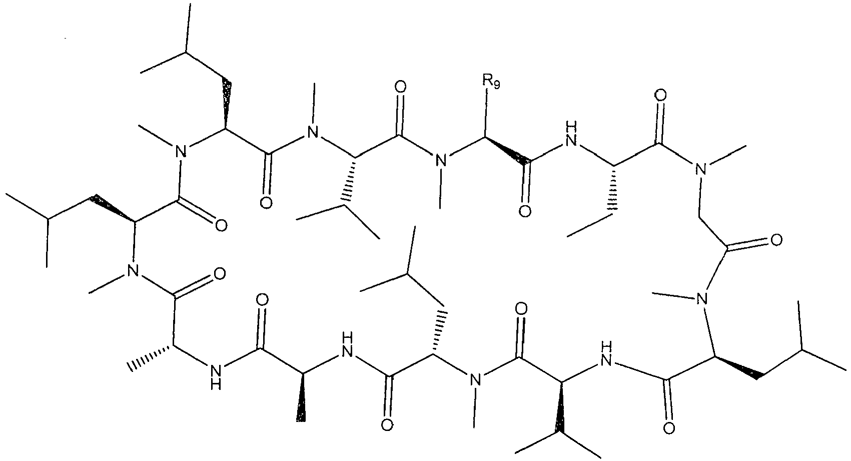 pour la préparation d'un composé selon l'une quelconque des revendications 1 à 12, dans lequel R₉ représente un résidu de la formule suivante: 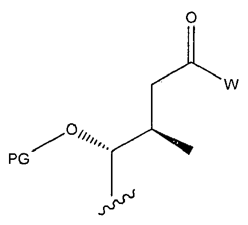 dans laquelle PG représente un groupe protecteur de la fonction alcool, et W est H ou OH, et le résidu respectif est lié à travers la liaison, à la fin de laquelle une ligne ondulée est dessinée.
